(19) Europäisches Patentamt
European Patent Office
Office européen des brevets

(11) EP 0 443 983 B1

(12) **EUROPÄISCHE PATENTSCHRIFT**

(45) Veröffentlichungstag und Bekanntmachung des Hinweises auf die Patenterteilung:
**28.02.1996 Patentblatt 1996/09**

(51) Int. Cl.$^6$: **C07D 257/04**, C07C 233/47, C07C 231/00, C07D 233/64, A61K 31/41, A61K 31/195

(21) Anmeldenummer: **91810098.3**

(22) Anmeldetag: **12.02.1991**

(54) **Acylverbindungen**
Acyl compounds
Composés acylés

(84) Benannte Vertragsstaaten:
**AT BE CH DE DK ES FR GB GR IT LI LU NL SE**

(30) Priorität: **19.02.1990 CH 518/90**
**05.07.1990 CH 2234/90**

(43) Veröffentlichungstag der Anmeldung:
**28.08.1991 Patentblatt 1991/35**

(73) Patentinhaber: **CIBA-GEIGY AG**
**CH-4002 Basel (CH)**

(72) Erfinder:
- **Bühlmayer, Peter, Dr.**
  **CH-4144 Arlesheim (CH)**
- **Ostermayer, Franz, Dr.**
  **CH-4125 Riehen (CH)**
- **Schmidlin, Tibur, Dr.**
  **CH-4056 Basel (CH)**

(56) Entgegenhaltungen:
**EP-A- 0 148 752** **EP-A- 0 253 310**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.9.9/3.4

## Beschreibung

Die Erfindung betrifft Verbindungen der Formel

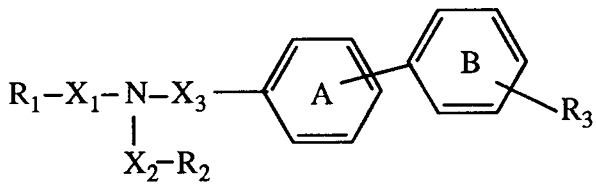

(I),

worin $R_1$ gegebenenfalls durch Halogen oder Hydroxy substituiertes Niederalkyl, Niederalkenyl oder Niederalkinyl oder $C_3$-$C_7$-Cycloalkyl- oder $C_3$-$C_7$-Cycloalkenyl oder Phenylniederalkyl, Phenylniederalkenyl oder Phenylniederalkinyl bedeutet; $X_1$ für CO, $SO_2$ oder -O-C(=O)- steht, wobei das Kohlenstoffatom der Carbonylgruppe an das in der Formel I eingezeichnete Stickstoffatom gebunden ist; $X_2$ gegebenenfalls durch Hydroxy, Carboxy, Amino, Guanidino, $C_3$-$C_7$-Cycloalkyl, $C_3$-$C_7$-Cycloalkenyl, Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen, $C_2$-$C_{10}$-Alkyliden oder $C_3$-$C_7$-Cycloalkylen bedeutet, wobei ein Kohlenstoffatom von $C_1$-$C_{10}$-Alkylen bzw. $C_2$-$C_{10}$-Alkyliden zusätzlich durch $C_2$-$C_6$-Alkylen überbrückt sein kann, und wobei $C_3$-$C_7$-Cycloalkyl oder $C_3$-$C_7$-Cycloalkenyl gegebenenfalls ein- oder mehrfach substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Formyl, Diniederalkoxymethyl oder Oxyniederalkylenoxymethylen; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, 1H-Tetrazol-5-yl, Pyridyl, Hydroxy, Niederalkoxy, Niederalkenyloxy, Phenylniederalkoxy, Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, oder $PO_nH_2$ bedeutet, wobei n für 2 oder 3 steht; $X_3$ $C_1$-$C_{10}$-Alkylen oder $C_2$-$C_{10}$-Alkyliden bedeutet; $R_3$ Carboxy, 5-Tetrazolyl, $SO_3H$, $PO_2H_2$, $PO_3H_2$ oder Halogenniederalkylsulfamoyl ist; und wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, durch Formyl, Diniederalkoxymethyl oder Oxyniederalkylenoxymethylen; wobei mit "nieder" bezeichnete Reste und Gruppen bis
und mit 7 Kohlenstoffatome enthalten; in freier Form oder in Salzform, ein Verfahren zur Herstellung dieser Verbindungen,

die Verwendung dieser Verbindungen und pharmazeutische Präparate, enthaltend eine solche Verbindung I in freier Form oder in Form eines pharmazeutisch verwendbaren Salzes.

Die Verbindungen I können als, insbesondere pharmazeutisch verwendbare, Salze vorliegen. Weisen die Verbindungen I z. B. mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie gegebenenfalls, z.B. durch Halogen, substituierten $C_1$-$C_4$-Alkan- oder Arylsulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Ferner können die Verbindungen I mit mindestens einer aciden Gruppe (beispielsweise COOH oder 5-Tetrazolyl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z. B. Ethyl-, tert.-Butyl-, Diethyl-, Diisopropyl-, Triethyl-, Tributyl- oder Dimethylpropyl-amin, oder einem Mono-, Di- oder Trihydroxyniederalkylamin, z.B. Mono-, Di- oder Triethanolamin. Weiterhin können entsprechende innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden.

Überbrückendes $C_2$-$C_6$-Alkylen ist insbesondere $C_4$-$C_5$-Alkylen.

Ein 5- oder 6-gliedriger und monocyclischer aromatischer Rest, der bis zu vier gleiche oder verschiedene Heteroatome, wie Stickstoff-, Sauerstoff- bzw. Schwefelatome, vorzugsweise ein, zwei, drei oder vier Stickstoffatome, ein Sauerstoff- oder ein Schwefelatom, aufweist, ist z.B. ein 5-gliedriger monoaza-, diaza-, triaza-, tetraaza-, monooxa- oder monothia-cyclischer Arylrest, wie Pyrrolyl, Pyrazolyl, Imidazolyl, Triazolyl, Tetrazolyl, Furyl und Thienyl, während als entsprechender 6-gliedriger Rest insbesondere Pyridyl in Frage kommt. Entsprechende aromatische Reste sind gegebenenfalls ein- oder mehrfach, z.B. zwei- oder dreifach, substituiert, beispielsweise durch gleiche oder verschiedene Reste, z.B. ausgewählt aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O-unterbrochen sind, sowie gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, durch Formyl, Diniederalkoxymethyl oder Oxyniederalkylenoxymethylen.

Als Beispiele für entsprechend verestertes Carboxy seien Niederalkoxy-, Phenylniederalkoxy-, Niederalkenyloxy- und Niederalkoxyniederalkoxy-carbonyl genannt.

Als Beispiele für entsprechend substituierte Aminogruppen in substituiertem Carbamoyl seien Niederalkyl-, Niederalkenyl-, Niederalkinyl-, Phenylniederalkyl-, Phenylniederalkenyl-, Phenylniederalkinyl-, Diniederalkyl-, N-Niederalkyl-N-phenylniederalkyl- und Diphenylniederalkylamino sowie Chinol-1-yl, Isochinol-2-yl, Niederalkylen- und Niederalkylenoxyniederalkylen-amino genannt.

Ein entsprechender aliphatischer Kohlenwasserstoffrest, der durch -O- unterbrochen ist, bedeutet insbesondere Niederalkoxyniederalkyl, -niederalkenyl oder -niederalkinyl, Niederalkenyloxyniederalkyl, -niederalkenyl oder -niederalkinyl.

Vor- und nachstehend sind ungesättigte aliphatische, cycloaliphatische und araliphatische Substituenten in erster Linie nicht über das C-Atom, von dem eine Mehrfachbindung ausgeht, mit einem aromatischen Rest verknüpft.

(Hetero-)Aromatische Reste sind insbesondere, sofern nicht abweichend definiert, jeweils unsubstituiert oder ein- oder mehrfach, z.B. zwei- oder dreifach, insbesondere z.B. durch einen Substituenten ausgewählt aus Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind.

Die Ringe A und B stellen in erster Linie ein 4-Biphenylyl-, ferner 2- oder 3- Biphenylylringsystem dar, wobei der Rest $R_3$ vorzugsweise in ortho-Position des Ringes B lokalisiert ist Entsprechend sind die Ringe A und B gegebenenfalls ein- oder mehrfach, z.B. zwei- oder dreifach, substituiert, beispielsweise durch gleiche oder verschiedene Reste z.B. ausgewählt aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind.

Die vor- und nachstehend verwendeten Allgemeindefinitionen haben, sofern nicht abweichend definiert, folgende Bedeutungen:
Der Ausdruck "Nieder" bedeutet, dass entsprechende Gruppen und Verbindungen jeweils insbesondere bis und mit 7, vorzugsweise bis und mit 4, Kohlenstoffatome enthalten.

Halogen ist insbesondere Halogen mit Atomnummer bis und mit 35, wie Fluor, Chlor oder Brom, und umfasst ferner Iod.

Alkanoyl ist beispielsweise Niederalkanoyl und bedeutet insbesondere $C_2$-$C_7$-Alkanoyl, wie Acetyl, Propionyl, Butyryl, Isobutyryl oder Pivaloyl. Bevorzugt ist $C_2$-$C_5$-Alkanoyl.

Halogenalkylsulfamoyl bedeutet insbesondere Halogen-$C_1$-$C_7$-alkansulfamoyl und ist z.B. Trifluormethan-, Difluormethan-,1,1,2-Trifluorethan- oder Heptafluorpropansulfamoyl. Bevorzugt ist Halogen-$C_1$-$C_4$-alkansulfamoyl.

Niederalkyl bedeutet insbesondere $C_1$-$C_7$-Alkyl, z.B. Methyl, Ethyl, n-Propyl, Isopropyl, n-Butyl, Isobutyl, sek.-Butyl, tert.-Butyl, und umfasst ferner entsprechende Pentyl-, Hexyl- und Heptylreste. Bevorzugt ist $C_1$-$C_4$-Alkyl.

Niederalkenyl bedeutet insbesondere $C_3$-$C_7$-Alkenyl und ist z.B. 2-Propenyl oder 1-, 2-oder 3-Butenyl. Bevorzugt ist $C_3$-$C_5$-Alkenyl.

Niederalkinyl ist insbesondere $C_3$-$C_7$-Alkinyl und bedeutet vorzugsweise Propargyl.

Halogenniederalkyl bedeutet insbesondere Halogen-$C_1$-$C_4$-alkyl, wie Trifluormethyl, 1,1,2-Trifluor-2-chlor-ethyl oder Chlormethyl.

Halogenniederalkenyl bedeutet insbesondere Halogen-$C_3$-$C_5$-alkenyl, wie 3-Chlorallyl.

Halogenniederalkinyl ist insbesondere Halogen-$C_3$-$C_5$-alkinyl, wie 3-Chlorpropargyl.

Hydroxyniederalkyl bedeutet insbesondere Hydroxy-$C_1$-$C_4$-alkyl, wie Hydroxymethyl, 2-Hydroxyethyl oder 3-Hydroxypropyl.

Hydroxyniederalkenyl bedeutet insbesondere Hydroxy-$C_3$-$C_5$-alkenyl, wie 3-Hydroxyallyl.

Hydroxyniederalkinyl bedeutet insbesondere Hydroxy-$C_3$-$C_5$-alkinyl, wie 3-Hydroxypropargyl.

Cycloalkyl ist insbesondere $C_3$-$C_7$-Cycloalkyl und bedeutet z.B. Cyclopropyl, Cyclobutyl, Cyclopentyl, Cyclohexyl und Cycloheptyl. Bevorzugt ist Cyclopentyl und Cyclohexyl.

Cycloalkenyl ist insbesondere $C_3$-$C_7$-Cycloalkenyl und bedeutet vorzugsweise Cyclopent-2-, -3-enyl, Cyclohex-2- und -3-en-yl.

Phenylniederalkyl ist insbesondere Phenyl-$C_1$-$C_4$-alkyl und bedeutet vorzugsweise Benzyl, 1-und 2-Phenethyl, während Phenylniederalkenyl und Phenylniederalkinyl insbesondere Phenyl-$C_3$-$C_5$-alkenyl und -alkinyl bedeuten, insbesondere 3-Phenylallyl und 3-Phenylpropargyl.

Pyrrolyl ist z.B. 2- oder 3-Pyrrolyl. Pyrazolyl ist 3- oder 4-Pyrazolyl. Imidazolyl ist 2- oder 4-Imidazolyl. Triazolyl ist z.B. 1,3,5-1H-Triazol-2-yl oder 1,3,4-Triazol-2-yl. Tetrazolyl ist z.B. 1,2,3,4-Tetrazol-5-yl, Furyl ist 2- oder 3-Furyl und Thienyl ist 2- oder 3-Thienyl, während als Pyridyl 2-, 3- und 4-Pyridyl in Frage kommt.

Alkylen bedeutet insbesondere $C_1$-$C_{10}$-Alkylen oder Niederalkylen, wie $C_1$-$C_7$-Alkylen, und ist geradkettig oder verzweigt und bedeutet insbesondere Methylen, Ethylen, Propylen und Butylen sowie 1,2-Propylen, 2-Methyl-1,3-propylen und 2,2-Dimethyl-1,3-propylen. Bevorzugt ist $C_1$-$C_5$-Alkylen.

Alkyliden bedeutet insbesondere $C_2$-$C_{10}$-Alkyliden, wie Ethyliden, 1,1-oder 2,2-Propyliden, ferner 1,1- oder 2,2-Butyliden oder 1,1-, 2,2- oder 3,3-Pentyliden. Bevorzugt ist $C_2$-$C_5$-Alkyliden.

Cycloalkylen ist insbesondere $C_3$-$C_7$-Cycloalkylen und bedeutet z.B. 1,2-Cyclopropylen, 1,2- oder 1,3-Cyclobutylen, 1,2-, 1,3-Cyclopentylen, 1,2-, 1,3- oder 1,4-Cyclohexylen und 1,2-, 1,3- oder 1,4-Cycloheptylen. Bevorzugt ist 1,3-Cyclopentylen und 1,4-Cyclohexylen.

Niederalkoxy bedeutet insbesondere $C_1$-$C_7$-Alkoxy und ist z.B. Methoxy, Ethoxy, n-Propyloxy, Isopropyloxy, n-Butyloxy, Isobutyloxy, sek.-Butyloxy, tert.-Butyloxy und umfasst ferner entsprechende Pentyloxy-, Hexyloxy- und Heptyloxyreste. Bevorzugt ist $C_1$-$C_4$-Alkoxy.

Niederalkoxyniederalkyl bedeutet insbesondere $C_1$-$C_4$-Alkoxy-$C_1$-$C_4$-alkyl, wie 2-Methoxy-ethyl, 2-Ethoxy-ethyl, 2-n-Propyloxy-ethyl oder Ethoxymethyl.

Niederalkoxyniederalkenyl bzw. -niederalkinyl bedeutet insbesondere $C_1$-$C_5$-Alkoxy-$C_3$-$C_5$-alkenyl bzw. -alkinyl.

Niederalkoxycarbonyl bedeutet insbesondere $C_2$-$C_8$-Alkoxycarbonyl und ist z.B. Methoxy-, Ethoxy-, Propyloxy- oder Pivaloyloxy-carbonyl. Bevorzugt ist $C_2$-$C_5$-Alkoxycarbonyl.

Phenylniederalkoxycarbonyl bedeutet insbesondere Phenyl-$C_1$-$C_4$-alkoxy-carbonyl und ist z.B. Benzyloxy-, 1- oder 2-Phenylethoxy-, 3-Phenylpropyloxy- oder 4-Phenylbutyloxy-carbonyl. Bevorzugt ist Benzyloxycarbonyl.

Niederalkenyloxycarbonyl bedeutet insbesondere $C_3$-$C_5$-Alkenyloxy-carbonyl, vorzugsweise Allyloxycarbonyl, während Niederalkinyloxycarbonyl insbesondere $C_3$-$C_5$-Alkinyloxy-carbonyl, wie Propargyloxycarbonyl, bedeutet.

Niederalkoxyniederalkoxycarbonyl bedeutet insbesondere $C_1$-$C_4$-Alkoxy-$C_1$-$C_4$-alkoxycarbonyl, vorzugsweise Ethoxy-ethoxycarbonyl, Methoxyethoxycarbonyl und Isopropyloxy-ethoxycarbonyl.

Niederalkylenoxyniederalkylen bedeutet insbesondere $C_1$-$C_4$-Alkylenoxy-$C_2$-$C_4$-alkylen, vorzugsweise Ethylenoxyethylen.

Niederalkylamino bedeutet insbesondere $C_1$-$C_7$-Alkylamino und ist z.B. Methyl-, Ethyl-, n-Propyl- und Isopropylamino. Bevorzugt ist $C_1$-$C_4$-Alkylamino.

Niederalkenylamino bedeutet vorzugsweise $C_3$-$C_5$-Alkylamino, wie Allyl-und Methallylamino.

Niederalkinylamino bedeutet vorzugsweise $C_3$-$C_5$-Alkinylamino, wie Propargylamino.

Phenylniederalkylamino bedeutet vorzugsweise Phenyl-$C_1$-$C_4$-alkylamino, insbesondere Benzyl-, 1- und 2-Phenylethylamino.

Phenylniederalkenylamino bedeutet vorzugsweise Phenyl-$C_3$-$C_5$-alkenyl-amino, insbesondere 3-Phenylallylamino und 3-Phenylmethallylamino.

Phenylniederalkenylamino bedeutet vorzugsweise Phenyl-$C_3$-$C_5$-alkinylamino, insbesondere 3-Phenylpropargylamino.

Diniederalkylamino bedeutet insbesondere Di-$C_1$-$C_4$-alkylamino, wie Dimethyl-, Diethyl-, Di-n-propyl-, Methyl-propyl-, Methyl-ethyl-, Methyl-butyl-amino und Dibutylamino.

N-Niederalkyl-N-phenylniederalkylamino bedeutet insbesondere N-$C_1$-$C_4$-Alkyl-N-phenyl-$C_1$-$C_4$-alkylamino, vorzugsweise Methyl-benzyl-amino und Ethyl-benzyl-amino.

Diphenylniederalkylamino bedeutet insbesondere Di-phenyl-$C_1$-$C_4$-alkyl-amino, vorzugsweise Dibenzylamino.

Niederalkylenamino bedeutet insbesondere $C_2$-$C_6$-Alkylenamino, vorzugsweise Pyrrolidin-1-yl oder Piperidin-1-yl.

Niederalkylenoxyniederalkylenamino bedeutet insbesondere $C_2$-$C_3$-Alkylenoxy-$C_2$-$C_3$-alkylenamino, insbesondere Morpholino.

Niederalkanoylamino bedeutet insbesondere $C_1$-$C_5$-Alkanoylamino, wie Formyl-, Acetyl-, Propionyl-, Butyryl- oder Pivaloylamino. Bevorzugt ist $C_2$-$C_5$-Alkanoylamino.

Phenylniederalkanoylamino bedeutet insbesondere Phenyl-$C_2$-$C_5$-alkanoylamino, wie Phenylacetyl- oder Phenylpropionylamino.

Niederalkansulfonylamino bedeutet insbesondere $C_1$-$C_7$-Alkansulfonylamino, wie Methan-, Ethan-, Propan- oder Butansulfonylamino. Bevorzugt ist $C_1$-$C_4$-Alkansulfonylamino.

Niederalkenyloxy bedeutet insbesondere $C_3$-$C_7$-Alkenyloxy und ist z.B. Allyloxy oder But-2-en- oder But-3-enyloxy. Bevorzugt ist $C_3$-$C_5$-Alkenyloxy.

Phenylniederalkoxy bedeutet insbesondere Phenyl-$C_1$-$C_4$-alkoxy, wie Benzyloxy, 1-oder 2-Phenylethoxy, 3-Phenylpropyloxy oder 4-Phenylbutyloxy.

Niederalkenyloxyniederalkyl bedeutet insbesondere $C_3$-$C_5$-Alkenyloxy-$C_1$-$C_4$-alkyl, wie 2-Allyloxyethyl, und Niederalkenyloxyniederalkenyl bzw. -niederalkinyl bedeutet insbesondere $C_3$-$C_5$-Alkenyloxy-$C_3$-$C_5$-alkenyl bzw. -alkinyl.

Ausgedehnte pharmakologische Untersuchungen haben ergeben, dass die Verbindungen I und ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser-Retention. Die Folge der Angiotensin-II-Aktivität manifestiert sich unter anderem in einer Erhöhung des Blutdrucks. Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretion-stimulierenden Effekte zu unterdrücken.

Die Angiotensin-II-antagonisierenden Eigenschaften der Verbindungen der Formel I und ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7,4) unter Einsatz von Peptidaseinhibitoren suspendiert. Die Proben werden 60 Minuten bei 25°C mit $^{125}$I-Angiotensin-II (0,175 nM) und einer variierenden Konzentration an Angiotensin-II oder an Testsubstanz inkubiert. Die Inkubation wird dann durch Zugabe von mit eiskaltem Phosphat gepuffertem Kochsalz beendet und es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die Verbindungen der Formel I und ihre pharmazeurisch verwendbaren Salze werden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammern bei einer anfänglich bestehenden Spannung von 2 g fixiert. Anschliessend werden die Ringe bei 37°C in 20 ml eines Gewebebades getaucht und mit einem Gemisch aus 95 % $O_2$ und 5 % $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die Verbindungen der Formel I und ihre pharmazeutisch verwendbaren Salze werden $IC_{50}$-Werte ab etwa 5 nM bestimmt.

Dass die Verbindungen der Formel I und ihre pharmazeutisch verwendbaren Salze durch Angiotensin-II induzierten Bluthochdruck reduzieren können, kann im Testmodell der normotensiven, narkotisierten Ratte verifiziert werden. Nach Kalibration der Präparationen mit jeweils 0,9 % NaCl (1 ml/kg i.v.), Noradrenalin (1 µg/kg i.v.) bzw. Angiotensin-II (0,3 µg/kg i.v.) werden steigende Dosen (3-6) der Testsubstanz durch Bolusinjektion intravenös injiziert, worauf nach jeder Dosis in 5 Minuten-Intervallen Angiotensin-II bzw. Noradrenalin appliziert wird. Der Blutdruck wird direkt in der Halsschlagader gemessen und mit einem on-line Datenerfassungssystem aufgezeichnet (Buxco). Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen der Formel I und ihre pharmazeutisch verwendbaren Salze ab einer Dosis von etwa 0,3 mg/kg i.v. einen hemmenden Effekt.

Auch im Testmodell der renalen hypertensiven Ratte kann die antihypertensive Aktivität der Verbindungen der Formel I und ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [Helv. Physiol. Acta 24 (1966), 58] vor Verabreichung der Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen der Formel I und ihre pharmazeutisch verwendbaren Salze z.B. als Wirkstoffe in Antihypertensiva verwendet werden, welche z.B. zur Behandlung von Bluthochdruck sowie von Herzinsuffizienz eingesetzt werden. Ein Erfindungsgegenstand ist somit die Verwendung der Verbindungen der Formel I und ihrer pharmazeutisch verwendbaren Salze zur Herstellung von entsprechenden Arzneimitteln und zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Die Erfindung betrifft in erster Linie Verbindungen der Formel I und ihre Salze, worin $R_1$ gegebenenfalls durch Halogen oder Hydroxy substituiertes Niederalkyl, Niederalkenyl oder Niederalkinyl oder $C_3$-$C_7$-Cycloalkyl oder $C_3$-$C_7$-Cycloalkenyl oder Phenylniederalkyl, Phenylniederalkenyl oder Phenylniederalkinyl bedeutet; $X_1$ für CO oder $SO_2$ steht; $X_2$ einen gegebenenfalls durch Hydroxy, $C_3$-$C_7$-Cycloalkyl oder $C_3$-$C_7$-Cycloalkenyl oder Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen oder $C_2$-$C_{10}$-Alkyliden oder $C_3$-$C_7$-Cycloalkylen bedeutet, wobei ein Kohlenstoffatom von $C_1$-$C_{10}$-Alkylen bzw. $C_2$-$C_{10}$-Alkyliden zusätzlich durch $C_2$-$C_6$-Alkylen überbrückt sein kann, und wobei $C_3$-$C_7$-Cycloalkylen gegebenenfalls ein- oder mehrfach substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Formyl, Diniederalkoxymethyl oder Oxyniederalkylenoxymethylen; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Niederalkanoyl-, Phenylniederalkanoyl-, Benzoyl-, Niederalkansulfonyl-, Benzolsulfonyl-amino, Formyl, Diniederalkoxymethyl, Oxynieder-alkylenoxymethylen, Hydroxy, Niederalkoxy, Niederalkenyloxy, Phenylniederalkoxy, Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, oder $PO_nH_2$ bedeutet, wobei n für 2 oder 3 steht; $X_3$ $C_1$-$C_{10}$-Alkylen oder $C_2$-$C_{10}$-Alkyliden bedeutet; $R_3$ Carboxy, 5-Tetrazolyl, $SO_3H$, $PO_2H_2$, $PO_3H_2$ oder Halogenniederalkylsulfamoyl ist; wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, durch Formyl, Diniederalkoxymethyl oder Oxyniederalkylenoxymethylen; in freier Form oder in Salzform.

Die Erfindung betrifft insbesondere Verbindungen der Formel I und ihre Salze, worin $R_1$ gegebenenfalls durch Halogen oder Hydroxy substituiertes Niederalkyl, Niederalkenyl oder Niederalkinyl oder $C_3$-$C_7$-Cycloalkyl oder $C_3$-$C_7$-Cycloalkenyl oder Phenylniederalkyl, Phenylniederalkenyl oder Phenylniederalkinyl bedeutet; $X_1$ für CO oder $SO_2$ steht; $X_2$ gegebenenfalls durch Hydroxy, $C_3$-$C_7$-Cycloalkyl oder $C_3$-$C_7$-Cycloalkenyl oder Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen oder $C_2$-$C_{10}$-Alkyliden bedeutet; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Niederalkenyloxy, Phenylniederalkoxy oder Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, oder $PO_nH_2$ bedeutet, wobei n für 2 oder 3 steht; $X_3$ $-CH_2-$ bedeutet; $R_3$ Carboxy, 5-Tetrazolyl, $SO_3H$, $PO_2H_2$, $PO_3H_2$ oder Halogenniederalkylsulfamoyl ist; und wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_1$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, durch Formyl, Diniederalkoxymethyl oder Oxyniederalkylenoxymethylen; in freier Form oder in Salzform.

Die Erfindung betrifft insbesondere Verbindungen der Formel I und ihre Salze, worin $R_1$ Niederalkyl, Niederalkenyl, Niederalkinyl, Halogenniederalkyl, -niederalkenyl, -niederalkinyl, Hydroxyniederalkyl, -niederalkenyl, -niederalkinyl, Cycloalkyl, Cycloalkenyl, Phenylniederalkyl, Phenylniederalkenyl oder Phenylniederalkinyl bedeutet; $X_1$ für CO oder $SO_2$ steht; $X_2$ Alkylen oder Alkyliden bedeutet, die gegebenenfalls durch Hydroxy, einen Cycloalkyl-, Cycloalkenyl-, einen Phenylrest oder einen 5- oder 6-gliedrigen, monocyclischen heteroaromatischen Rest mit bis zu vier gleichen oder verschiedenen Heteroatomen substituiert sind, wobei die cyclischen Reste ihrerseits gegebenenfalls substituiert sind durch Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Formyl, Diniederalkoxymethyl, Oxyniederalkylenoxymethylen; $R_2$ Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Amino, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Niederalkenyloxy, Phenylniederalkoxy, Phenoxy, $S(O)_m$-R, wobei m für 0, 1 oder 2 und R für Wasserstoff, Niederalkyl, Niederalkenyl oder Niederalkinyl steht, Niederalkanoyl, Sulfamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, oder $PO_nH_2$ bedeutet, wobei n für 2 oder 3 steht; $X_3$ $-CH_2-$ bedeutet; $R_3$ Carboxy, 5-Tetrazolyl, $SO_3H$, $PO_2H_2$, $PO_3H_2$ oder Halogenniederalkylsulfamoyl bedeutet; wobei (hetero-)aromatische Reste einschliesslich der Ringe A und B unabhängig voneinander jeweils gegebenenfalls substituiert sind durch einen oder mehrere Substituenten ausgewählt aus Halogen,

Hydroxy, Niederalkoxy, Niederalkenyloxy, jeweils gegebenenfalls durch Halogen oder Hydroxy substituiertes Niederalkyl, Niederalkenyl, Niederalkinyl, Niederalkoxyniederalkyl, -niederalkenyl, -niederalkinyl, Niederalkenyloxyniederalkyl, -niederalkenyl und -niederalkinyl.

Die Erfindung betrifft insbesondere Verbindungen der Formel I und ihre Salze, worin $X_2$ Alkylen oder Alkyliden bedeutet, die gegebenenfalls durch Hydroxy, einen Cycloalkyl-, Cycloalkenyl-, einen Phenylrest oder einen 5- oder 6-gliedrigen, monocyclischen heteroaromatischen Rest mit bis zu vier gleichen oder verschiedenen Heteroatomen substituiert sind, wobei ein C-Atom von Alkylen bzw. Alkyliden durch $C_2$-$C_6$-Alkylen überbrückt sein kann und wobei die cyclischen Reste ihrerseits gegebenenfalls substituiert sind durch Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Formyl, Diniederalkoxymethyl oder durch Oxyniederalkylenoxymethylen, oder $X_2$ $C_3$-$C_7$-Cycloalkylen bedeutet; $X_3$ Niederalkylen oder Niederalkyliden bedeutet; und die Variablen $X_1$, $R_1$, $R_2$, $R_3$ die unmittelbar vorstehend angegebenen Bedeutungen haben und die (hetero-)aromatischen Ringe einschliesslich der Ringe A und B wie unmittelbar vorstehend angegeben substituiert sein können.

Die Erfindung betrifft insbesondere Verbindungen der Formel I und ihre Salze, worin $R_1$ Niederalkyl, Niederalkenyl, Halogenniederalkyl, -niederalkenyl, Hydroxyniederalkyl, 3-bis 7-gliedriges Cycloalkyl oder Phenylniederalkyl bedeutet; $X_1$ für CO, $SO_2$ oder -O-C(=O)-, wobei das Kohlenstoffatom der Carbonylgruppe an das in der Formel I eingezeichnete Stickstoffatom gebunden ist, steht; $X_2$ $C_1$-$C_{10}$-Alkylen oder $C_1$-$C_7$-Alkyliden, die gegebenenfalls substituiert sind durch Hydroxy, Carboxy, Amino, Guanidino, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder Oxyniederalkylenoxymethylen substituiert sein können; $R_2$ Carboxy, Niederalkoxy-, Phenylniederalkoxy-, Niederalkenyloxy-, Niederalkoxyniederalkoxy-carbonyl, Carbamoyl, in dem die Aminogruppe gegebenenfalls durch Niederalkyl, Phenylniederalkyl mono- oder unabhängig voneinander disubstituiert oder durch Niederalkylen, das gegebenenfalls an zwei benachbarten Kohlenstoffatomen mit einem Benzolring kondensiert ist, oder Niederalkylenoxyniederalkylen disubstituiert ist, Amino, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Phenylniederalkoxy, Phenoxy, $S(O)_m$-R, wobei m für 0, 1 oder 2 und R für Niederalkyl steht, Niederalkanoyl, Sulfamoyl, in dem die Aminogruppe gegebenenfalls durch Niederalkyl, Phenylniederalkyl mono- oder unabhängig voneinander disubstituiert ist, oder $PO_nH_2$ bedeutet, wobei n für 2 oder 3 steht; $X_3$ Methylen ist; $R_3$ Carboxy, 5-Tetrazolyl, $SO_3H$, $PO_2H_2$, $PO_3H_2$ oder Halogenniederalkylsulfamoyl bedeutet; (hetero-)aromatische Reste einschliesslich der Ringe A und B jeweils gegebenenfalls zusätzlich substituiert sind durch einen oder mehrere Substituenten ausgewählt aus Halogen, Hydroxy, Niederalkoxy, jeweils gegebenenfalls durch Halogen oder Hydroxy substituiertes Niederalkyl bzw. Niederalkoxyniederalkyl.

Die Erfindung betrifft insbesondere Verbindungen der Formel I und ihre Salze, worin $R_1$ Niederalkyl, Niederalkenyl, Halogenniederalkyl, -niederalkenyl, Hydroxyniederalkyl, 3- bis 7-gliedriges Cycloalkyl oder Phenylniederalkyl bedeutet; $X_1$ für CO oder $SO_2$ steht; $X_2$ $C_1$-$C_{10}$-Alkylen oder $C_1$-$C_7$-Alkyliden, die gegebenenfalls substituiert sind durch Hydroxy, einen 3- bis 7-gliedrigen Cycloalkyl-, 3-bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder Oxyniederalkylenoxymethylen substituiert sein können; $R_2$ Carboxy, Niederalkoxy-, Phenylniederalkoxy-, Niederalkenyloxy-, Niederalkoxyniederalkoxy-carbonyl, Carbamoyl, in dem die Aminogruppe gegebenenfalls durch Niederalkyl, Phenylniederalkyl mono-oder unabhängig voneinander disubstituiert oder durch Niederalkylen- oder Niederalkylenoxyniederalkylen disubstituiert ist, Amino, in dem die Aminogruppe gegebenenfalls durch Niederalkyl, Phenylniederalkyl mono- oder unabhängig voneinander disubstituiert oder durch Niederalkylen-oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Phenylniederalkoxy, Phenoxy, $S(O)_m$-R, wobei m für 0, 1 oder 2 und R für Niederalkyl steht, Niederalkanoyl, Sulfamoyl, in dem die Aminogruppe gegebenenfalls durch Niederalkyl, Phenylniederalkyl mono- oder unabhängig voneinander disubstituiert ist, oder $PO_nH_2$ bedeutet, wobei n für 2 oder 3 steht; $X_3$ Methylen ist; $R_3$ Carboxy, 5-Tetrazolyl, $SO_3H$, $PO_2H_2$, $PO_3H_2$ oder Halogenniederalkylsulfamoyl bedeutet; (hetero-)aromatische Reste einschliesslich der Ringe A und B jeweils gegebenenfalls zusätzlich substituiert sind durch einen oder mehrere Substituenten ausgewählt aus Halogen, Hydroxy, Niederalkoxy, jeweils gegebenenfalls durch Halogen oder Hydroxy substituiertes Niederalkyl bzw. Niederalkoxyniederalkyl.

Die Erfindung betrifft insbesondere Verbindungen der Formel I und ihre Salze, worin $X_2$ $C_1$-$C_{10}$-Alkylen oder $C_1$-$C_7$-Alkyliden, die gegebenenfalls substituiert sind durch Hydroxy, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder durch Oxyniederalkylenoxymethylen substituiert sein können, wobei ein C-Atom von Alkylen bzw. Alkyliden durch $C_2$-$C_6$-Alkylen überbrückt sein kann, oder $X_2$ $C_3$-$C_7$-Cycloalkylen bedeutet; $X_3$ Niederalkylen oder Niederalkyliden bedeutet und die Variablen $X_1$, $R_1$, $R_2$, $R_3$ die unmittelbar vorstehend angegebenen Bedeutungen haben und die (hetero-)aromatischen Ringe einschliesslich der Ringe A und B wie unmittelbar vorstehend angegeben substituiert sein können.

Die Erfindung betrifft insbesondere Verbindungen der Formel I und ihre Salze, worin die Variablen $R_1$, $X_1$, $R_3$ die jeweils vorstehend angegebenen Bedeutungen haben; $X_2$ gegebenenfalls durch Hydroxy, 3- bis 7-gliedriges Cycloalkyl, Phenyl oder Imidazolyl substituiertes Niederalkylen oder Niederalkyliden bedeutet und $R_2$ Carboxy, Niederalkoxy-, Phenylniederalkoxy-, Niederalkoxyniederalkoxy-carbonyl, Carbamoyl, welches gegebenenfalls durch Niederalkyl, Phenylniederalkyl mono- oder unabhängig voneinander disubstituiert ist, Amino, Niederalkanoyl-, Phenylniederalkanoyl-, Niederalkansulfonylamino, Hydroxy, Niederalkoxy, Phenylniederalkoxy oder Phenoxy bedeutet; $X_3$-$CH_2$- bedeutet; wobei (hetero-)aromatische Reste einschliesslich der Ringe A und B jeweils gegebenenfalls durch einen oder mehrere Substituenten ausgewählt aus Halogen, Trifluormethyl, Hydroxy, Niederalkoxy, Niederalkyl, Hydroxyniederalkyl oder Niederalkoxyniederalkyl substituiert sind.

Die Erfindung betrifft insbesondere Verbindungen der Formel I und ihre Salze, worin $X_2$ gegebenenfalls durch Hydroxy, 3-bis 7-gliedriges Cycloalkyl, 7-gliedriges Cycloalkenyl, Phenyl oder Imidazolyl substituiertes Niederalkylen oder Niederalkyliden bedeutet, wobei ein C-Atom von Niederalkylen bzw. Niederalkyliden durch $C_2$-$C_6$-Alkylen überbrückt sein kann, oder $X_2$ $C_3$-$C_7$-Cycloalkylen bedeutet; und die Variablen $X_1$, $X_3$, $R_1$, $R_2$, $R_3$ die unmittelbar vorstehend angegebenen Bedeutungen haben, die Ringe A und B wie unmittelbar vorstehend angegeben substituiert sein können.

Die Erfindung betrifft insbesondere Verbindungen der Formel

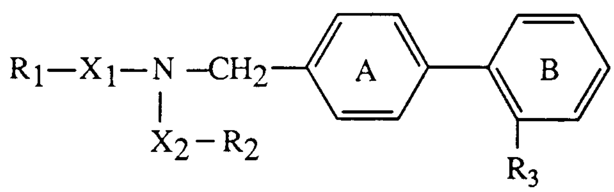

(Ia)

und ihre Salze, worin die Variablen $R_1$, $X_1$, $X_2$, $R_2$ und $R_3$ die jeweils vorstehend angegebenen Bedeutungen haben und die Ringe A und B wie unmittelbar vorstehend angegeben substituiert sein können.

Die Erfindung betrifft insbesondere Verbindungen der Formel Ia und ihre Salze, worin $X_2$ gegebenenfalls durch Hydroxy oder 3-bis 7-gliedriges Cycloalkyl substituiertes Niederalkylen oder Niederalkyliden bedeutet, wobei ein C-Atom von Niederalkylen bzw. Niederalkyliden durch $C_2$-$C_6$-Alkylen, insbesondere $C_4$-$C_5$-Alkylen, überbrückt sein kann, oder worin $X_2$ $C_3$-$C_7$-Cycloalkylen bedeutet, und die Variablen $R_1$, $X_1$, $R_2$ und $R_3$ die jeweils vorstehend angegebenen Bedeutungen haben und die Ringe A und B wie unmittelbar vorstehend angegeben substituiert sein können.

Die Erfindung betrifft insbesondere Verbindungen der Formel Ia und ihre Salze, worin $X_2$ für die Gruppe der Formel

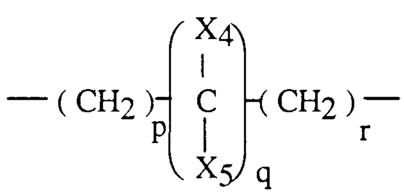

(Ib)

steht, in der p für 0 oder 1, q für 1 und r für 0 oder 1 stehen oder in der p für 1 bis 8 und q sowie r jeweils für 0 stehen; $X_4$ gegebenenfalls durch Hydroxy, 3- bis 7-gliedriges Cycloalkyl, Phenyl oder Imidazolyl substituiertes Niederalkyl oder Phenyl bedeutet und $X_5$ Wasserstoff oder Niederalkyl bedeutet; $R_2$ Carboxy, Niederalkoxycarbonyl, Phenylniederalkoxycarbonyl, Niederalkoxyniederalkoxycarbonyl, Hydroxy, Niederalkoxy, Phenylniederalkoxy, Phenoxy, Amino, Niederalkanoylamino, Phenylniederalkanoylamino oder Niederalkansulfonylamino bedeutet; und die Variablen $R_1$, $X_1$ und $R_3$ die jeweils vorstehend angegebenen Bedeutungen haben; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind.

Die Erfindung betrifft insbesondere Verbindungen der Formel Ia und ihre Salze, worin $X_2$ für die Gruppe der Formel Ib steht, in der p für 0 oder 1, q für 1 und r für 0 oder 1 stehen oder in der p für 1 bis 8 und q sowie r jeweils für 0 stehen; $X_4$ gegebenenfalls durch Hydroxy, 3- bis 7-gliedriges Cycloalkyl, Phenyl oder Imidazolyl substituiertes Niederalkyl oder Phenyl bedeutet und $X_5$ Wasserstoff oder Niederalkyl bedeutet; oder $X_4$ und $X_5$ gemeinsam für $C_2$-$C_6$-Alkylen, insbesondere $C_4$-$C_5$-Alkylen, stehen, oder $X_2$ $C_3$-$C_7$-Cycloalkylen, insbesondere $C_5$-$C_6$-Cycloalkylen, bedeutet; $R_2$ Carboxy, Niederalkoxycarbonyl, Phenylniederalkoxycarbonyl, Niederalkoxyniederalkoxycarbonyl, Hydroxy, Niederalkoxy, Phenylniederalkoxy, Phenoxy, Amino, Niederalkanoylamino, Phenylniederalkanoylamino oder Niederalkansulfonylamino bedeutet; und die Variablen $R_1$, $X_1$ und $R_3$ die jeweils vorstehend angegebenen Bedeutungen haben; wobei (hetero-) aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind.

Die Erfindung betrifft insbesondere Verbindungen der Formel Ia und ihre Salze, worin $R_1$ Niederalkyl, insbesondere $C_3$-$C_5$-Alkyl, oder Niederalkenyl, insbesondere $C_3$-$C_5$-Alkenyl, bedeutet; $X_1$ für CO oder ferner $SO_2$ steht; $X_2$ für die Gruppe der Formel Ib steht, in der p und r für 0 oder 1 und q für 1 stehen; $X_4$ gegebenenfalls durch Hydroxy, 3- bis 7-gliedriges Cycloalkyl, wie Cyclohexyl, durch gegebenenfalls durch Halogen oder Hydroxy substituiertes Phenyl oder Imidazolyl, wie 4-Imidazolyl, substituiertes Niederalkyl, insbesondere $C_1$-$C_4$-Alkyl, oder Phenyl bedeutet; $X_5$ Wasserstoff oder Niederalkyl, wie $C_1$-$C_4$-Alkyl, bedeutet oder $X_4$ und $X_5$ gemeinsam für $C_2$-$C_6$-Alkylen, wie $C_4$-$C_5$-Alkylen, bedeuten, oder $X_2$ $C_3$-$C_7$-Cycloalkylen, wie $C_5$-$C_6$-Cycloalkylen,wie 1,4-Cyclohexylen, bedeutet; $R_2$ Carboxy, Niederalkoxycarbonyl, wie $C_2$-$C_5$-Alkoxycarbonyl, Phenylniederalkoxycarbonyl, wie Phenyl-$C_1$-$C_4$-alkoxycarbonyl, Niederalkoxyniederalkoxycarbonyl, wie $C_1$-$C_4$-Alkoxy-$C_2$-$C_5$-alkoxycarbonyl, Hydroxy oder Niederalkoxy, wie $C_1$-$C_4$-Alkoxy, bedeutet; $R_3$ Carboxy oder 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind.

Die Erfindung betrifft insbesondere Verbindungen der Formel Ia und ihre Salze, worin $R_1$ Niederalkyl, insbesondere $C_3$-$C_5$-Alkyl, oder Niederalkenyl, insbesondere $C_3$-$C_5$-Alkenyl, bedeutet; $X_1$ für CO oder ferner $SO_2$ steht; $X_2$ für die Gruppe der Formel Ib steht, in der p und r für 0 oder 1 und q für 1 stehen; $X_4$ gegebenenfalls durch Hydroxy, 3- bis 7-gliedriges Cycloalkyl, wie Cyclohexyl, durch gegebenenfalls durch Halogen oder Hydroxy substituiertes Phenyl oder Imidazolyl, wie 4-Imidazolyl, substituiertes Niederalkyl, insbesondere $C_1$-$C_4$-Alkyl, oder Phenyl bedeutet; $X_5$ Wasserstoff oder Niederalkyl, wie $C_1$-$C_4$-Alkyl, bedeutet; $R_2$ Carboxy, Niederalkoxycarbonyl,wie $C_2$-$C_5$-Alkoxycarbonyl, Phenylniederalkoxycarbonyl, wie Phenyl-$C_1$-$C_4$-alkoxycarbonyl, Niederalkoxyniederalkoxycarbonyl, wie $C_1$-$C_4$-Alkoxy-$C_2$-$C_5$-alkoxycarbonyl, Hydroxy oder Niederalkoxy, wie $C_1$-$C_4$-Alkoxy, bedeutet; $R_3$ Carboxy oder 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind.

Die Erfindung betrifft insbesondere Verbindungen der Formel Ia und ihre Salze, worin $R_1$ Niederalkyl, insbesondere $C_3$-$C_5$-Alkyl, oder ferner Niederalkenyl, insbesondere $C_3$-$C_5$-Alkenyl, bedeutet; $X_1$ für CO oder ferner $SO_2$ steht; $X_2$ für die Gruppe der Formel Ib steht, in der p für 1-8 und q sowie r für 0 stehen; $R_2$ Hydroxy, Niederalkoxy, wie $C_1$-$C_4$-Alkoxy, Phenylniederalkoxy, wie Phenyl-$C_1$-$C_4$-alkoxy, Phenoxy, Niederalkanoylamino, wie $C_1$-$C_4$-Alkanoylamino, Phenylniederalkanoylamino, wie Phenyl-$C_1$-$C_4$-alkanoylamino, Niederalkansulfonylamino, wie $C_1$-$C_4$-Alkansulfonylamino, bedeutet; $R_3$ Carboxy oder in erster Linie 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind.

Die Erfindung betrifft in erster Linie Verbindungen der Formel Ia und ihre Salze, worin $R_1$ $C_3$-$C_5$-Alkyl oder in zweiter Linie $C_3$-$C_5$-Alkenyl, bedeutet; $X_1$ für CO, ferner $SO_2$ steht; $X_2$ für die Gruppe der Formel Ib steht, in der p und r unabhängig voneinander für 0 oder 1 und q für 1 stehen; $X_4$ $C_1$-$C_4$-Alkyl, wie Methyl, Ethyl, Propyl, Isopropyl, 1- oder 2-Butyl, Hydroxy-$C_1$-$C_4$-alkyl, wie Hydroxymethyl, $C_3$-$C_7$-Cycloalkyl-$C_1$-$C_4$-alkyl, wie Cyclohexylmethyl, Phenyl-$C_1$-$C_4$-alkyl, wie Benzyl, oder Imidazolyl-$C_1$-$C_4$-alkyl, wie Imidazol-4-yl-methyl, bedeutet; $X_5$ Wasserstoff oder $C_1$-$C_4$-Alkyl, wie Methyl, bedeutet; oder $X_4$ und $X_5$ gemeinsam für Tetramethylen, ferner Pentamethylen stehen; $R_2$ Carboxy oder $C_2$-$C_5$-Alkoxycarbonyl, ferner Phenyl-$C_1$-$C_4$-alkoxycarbonyl, wie Benzyloxycarbonyl, bedeutet; $R_3$ Carboxy oder insbesondere 5-Tetrazolyl bedeutet.

Die Erfindung betrifft in erster Linie Verbindungen der Formel Ia und ihre Salze, worin $R_1$ $C_3$-$C_5$-Alkyl oder in zweiter Linie $C_3$-$C_5$-Alkenyl, bedeutet; $X_1$ für CO, ferner $SO_2$ steht; $X_2$ für die Gruppe der Formel Ib steht, in der p und r jeweils für 0 oder 1 und q für 1 stehen; $X_4$ $C_1$-$C_4$-Alkyl, wie Methyl, Ethyl, Propyl, Isopropyl, 1- oder 2-Butyl, Hydroxy-$C_1$-$C_4$-alkyl, wie Hydroxymethyl, $C_3$-$C_7$-Cycloalkyl-$C_1$-$C_4$-alkyl, wie Cyclohexylmethyl, Phenyl-$C_1$-$C_4$-alkyl, wie Benzyl, oder Imidazolyl-$C_1$-$C_4$-alkyl, wie Imidazol-4-yl-methyl, bedeutet; $X_5$ Wasserstoff bedeutet; $R_2$ Carboxy oder $C_2$-$C_5$-Alkoxycarbonyl, ferner Phenyl-$C_1$-$C_4$-alkoxycarbonyl, wie Benzyloxycarbonyl, bedeutet; $R_3$ Carboxy oder 5-Tetrazolyl bedeutet.

Die Erfindung betrifft in erster Linie Verbindungen der Formel Ia und ihre Salze, worin $R_1$ $C_3$-$C_5$-Alkyl, wie Propyl, Butyl oder Pentyl, bedeutet; $X_1$ für CO steht; $X_2$ für die Gruppe der Formel Ib steht, in der q und r für 0 und p für 1 bis 3, insbesondere 2, stehen, oder in der p und q für 1 und r für 0 stehen; $X_4$ $C_1$-$C_4$-Alkyl, wie Methyl, Ethyl, Propyl, Isopropyl, 1- oder 2-Butyl, bedeutet; $X_5$ Wasserstoff oder $C_1$-$C_4$-Alkyl, wie Methyl, bedeutet; $R_2$ Carboxy, $C_2$-$C_5$-Alkoxycarbonyl, wie Methoxy- oder Ethoxycarbonyl, bedeutet; $R_3$ Carboxy oder 5-Tetrazolyl bedeutet.

Die Erfindung betrifft in erster Linie Verbindungen der Formel Ia und ihre Salze, worin $R_1$ $C_3$-$C_5$-Alkyl, wie Propyl, Butyl oder Pentyl, bedeutet; $X_1$ für CO steht; $X_2$ für die Gruppe der Formel Ib steht, in der p für 0 oder 1, r für 0 und q für 1 stehen; $X_4$ $C_1$-$C_4$-Alkyl, wie Methyl, Ethyl, Propyl, Isopropyl, 1- oder 2-Butyl, bedeutet; $X_5$ Wasserstoff oder $C_1$-$C_4$-Alkyl, wie Methyl oder Ethyl, bedeutet oder $X_4$ und $X_5$ gemeinsam für Tetramethylen oder Pentamethylen stehen; $R_2$ Carboxy, $C_2$-$C_5$-Alkoxycarbonyl, wie Methoxy- oder Ethoxycarbonyl, bedeutet; $R_3$ 5-Tetrazolyl bedeutet.

Die Erfindung betrifft in erster Linie Verbindungen der Formel Ia und ihre Salze, worin $R_1$ $C_3$-$C_5$-Alkyl, wie Propyl, Butyl oder Pentyl, bedeutet; $X_1$ für CO steht; $X_2$ für die Gruppe der Formel Ib steht, in der p 0 oder 1 und r für 0 und q für 1 stehen; $X_4$ und $X_5$ gemeinsam für Tetramethylen, ferner Pentamethylen stehen; $R_2$ Carboxy oder $C_2$-$C_5$-Alkoxycarbonyl, wie Methoxy- oder Ethoxycarbonyl, bedeutet; $R_3$ 5-Tetrazolyl bedeutet.

Die Erfindung betrifft in erster Linie Verbindungen der Formel Ia und ihre Salze, worin $R_1$ $C_3$-$C_5$-Alkyl, wie Propyl, Butyl oder Pentyl, bedeutet; $X_1$ für CO steht; $X_2$ für die Gruppe der Formel Ib steht, in der p und r für 0 oder 1 und q für 1 stehen; $X_4$ $C_1$-$C_4$-Alkyl, wie Methyl, Ethyl, Propyl, Isopropyl, 1- oder 2-Butyl, bedeutet; $X_5$ Wasserstoff bedeutet; $R_2$ Carboxy, $C_2$-$C_5$-Alkoxycarbonyl, wie Methoxy- oder Ethoxycarbonyl, bedeutet; $R_3$ 5-Tetrazolyl bedeutet.

Die Erfindung betrifft insbesondere die in den Beispielen aufgeführten neuen Verbindungen sowie die dort beschriebenen Herstellungsweisen.

Gegenstand der Erfindung sind auch Verfahren zur Herstellung der erfindungsgemässen Verbindungen. Die Herstellung von Verbindungen der Formel I und ihrer Salze erfolgt in an sich bekannter Weise und ist z.B. dadurch gekennzeichnet, dass man

a) in einer Verbindung der Formel

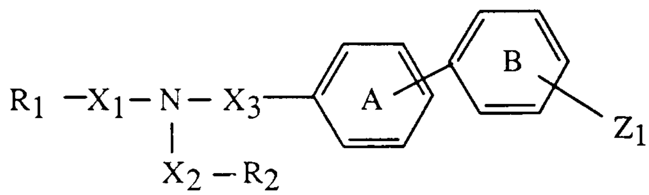 (II)

oder einem Salz davon, worin $Z_1$ einen in $R_3$ überführbaren Rest bedeutet, $Z_1$ in $R_3$ überführt oder

b) eine Verbindung der Formel $R_1$-$X_1$OH (IIIa), ein reaktionsfähiges Derivat davon oder ein Salz davon mit einer Verbindung der Formel

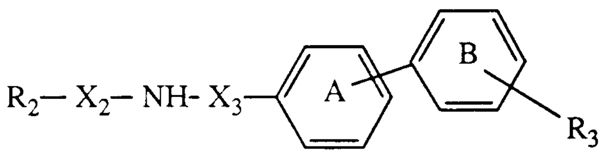 (IIIb)

oder einem Salz davon umsetzt und jeweils, wenn erwünscht, eine verfahrensgemäss oder auf andere Weise erhältliche Verbindung I in freier Form oder in Salzform in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

Salze von Ausgangsmaterialien, die mindestens ein basisches Zentrum aufweisen, beispielsweise der Formel IIIb, sind entsprechende Säureadditionssalze, während Salze von Ausgangsstoffen, die eine acide Gruppe aufweisen, beispielsweise der Formel (IIIa), als Salze mit Basen vorliegen, jeweils wie in Zusammenhang mit entsprechenden Salzen der Formel I vorstehend aufgeführt.

In die Variable $R_3$ überführbare Reste $Z_1$ stellen beispielsweise Cyano, Mercapto, Halogen, die Gruppe $-N_2^+ A^-$, in der $A^-$ ein von einer Säure abgeleitetes Anion bedeutet, Amino sowie von COOH, $SO_3H$, $PO_3H_2$ oder $PO_2H_2$ verschiedene funktionell abgewandelte Formen sowie N-geschütztes 5-Tetrazolyl.

Reaktionsfähige Derivate von Verbindungen der Formel IIIa sind beispielsweise davon abgeleitete aktivierte Ester oder reaktionsfähige Anhydride, ferner reaktionsfähige cyclische Amide.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem

Temperaturbereich von etwa -80°C bis zur Siedetemperatur des Reaktionsmediums, vorzugsweise von etwa -10° bis etwa +200°C, und, falls erforderlich, in einem geschlossenen Gefäss, unter Druck, in einer Inertgasatmosphäre und/oder unter wasserfreien Bedingungen arbeitet.

Verfahrensvariante a):

In 5-Tetrazolyl $R_3$ überführbare Reste $Z_1$ sind beispielsweise Cyano oder geschütztes 5-Tetrazolyl.

Zur Herstellung von Verbindungen der Formel I, worin $R_3$ 5-Tetrazolyl bedeutet, geht man beispielsweise von Ausgangsmaterial der Formel II aus, worin $Z_1$ Cyano bedeutet, und setzt dieses mit einem Azid, wie $HN_3$ oder insbesondere einem Salz, wie Alkalimetallsalz, davon oder mit einem Organozinnazid, wie Tri(nieder)alkyl- oder Triarylzinnazid, um. Bevorzugte Azide sind beispielsweise Natrium- und Kaliumazid sowie Tri-$C_1$-$C_4$-alkyl-, z.B. Triethyl- oder Tributylzinnazid, und Triphenylzinnazid. Bevorzugt wird die Tetrazol-5-yl-Bildung mit solchen Verbindungen der Formel II durchgeführt, worin $R_2$ von Carboxy verschieden ist.

Als Schutzgruppen von geschütztem 5-Tetrazolyl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- und Ethoxymethyl, Niederalkylthiomethyl, wie Methylthiomethyl, Silyl, wie Triniederalkylsilyl,z.B. Dimethyl-tert-butyl- und Triisopropyl-silyl, sowie 2-Cyanoethyl, ferner Niederalkoxyniederalkoxymethyl, wie 2-Methoxyethoxymethyl, Benzyloxymethyl sowie Phenacyl.

Die Abspaltung der Schutzgruppen erfolgt in Anlehnung an bekannte Methoden, beispielsweise wie in J. Green, Protective Groups in Organic Synthesis, Wiley-Interscience (1980) beschrieben. So wird z.B. die Triphenylmethylgruppe üblicherweise durch Hydrolyse, insbesondere in Gegenwart einer Säure, oder Hydrogenolyse in Gegenwart eines Hydrierungskatalysators, 4-Nitrobenzyl z.B. durch Hydrogenolyse in Gegenwart eines Hydrierungskatalysators, Methoxy- oder Ethoxymethyl z.B. durch Behandeln mit einem Triniederalkyl-, wie Triethyl-oder Tributyl-zinn-bromid, Methylthiomethyl z.B. durch Behandeln mit Trifluoressigsäure, Silyreste z.B. durch Behandeln mit Fluoriden, wie Tetraniederalkylammoniumfluoriden, z.B. Tetrabutylammoniumfluorid, oder Alkalimetallfluoriden, z.B. Natriumfluorid, oder 2-Cyanoethyl z.B. durch Hydrolyse, beispielsweise mit Natronlauge, 2-Methoxyethoxymethyl z.B. durch Hydrolyse, z.B. mit Salzsäure, Benzyloxymethyl und Phenacyl z.B. durch Hydrogenolyse in Gegenwart eines Hydrierungskatalysators abgespalten.

Ein in $R_3$= $SO_3H$ überführbärer Rest ist beispielsweise die Mercaptogruppe. Eine solche Gruppe aufweisende Ausgangsverbindungen der Formel II werden beispielsweise durch an sich bekannte Oxidationsverfahren zu solchen Verbindungen der Formel I oxidiert, worin $R_3$ $SO_3H$ ist. Als Oxidationsmittel kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Unter einer in $R_3$ = $PO_3H_2$ überführbaren Gruppe ist beispielsweise eine Gruppe $N_2^+ A^-$ zu verstehen, wobei $A^-$ für ein Anion einer Säure, wie Mineralsäure, steht. Derartige Diazoniumverbindungen werden beispielsweise in an sich bekannter Weise mit einem P(III)-Halogenid, wie $PCl_3$ oder $PBr_3$, umgesetzt und hydrolytisch aufgearbeitet, wobei solche Verbindungen der Formel I erhältlich sind, worin $R_3$ $PO_3H_2$ ist.

Als in Halogenalkylsulphamoyl $R_3$ überführbarer Rest $Z_1$ kommt beispielsweise primäres Amino in Frage.

Zur Herstellung von Verbindungen der Formel I, worin $R_3$ Halogenalkylsulphamoyl bedeutet, setzt man beispielsweise entsprechende Aniline mit einer üblicherweise reaktionsfähig veresterten Halogenalkylsulfonsäure um, wobei gegebenenfalls in Gegenwart einer Base gearbeitet wird. Als bevorzugte reaktionsfähig veresterte Halogensulfonsäure kommt das entsprechende Halogenid, wie Chlorid oder Bromid, in Frage.

Ein in $R_3$ = COOH überführbarer Rest $Z_1$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, Propanol, Isopropanol, n-Butanol, sec- oder tert.-Butanol, während als cycloaliphatischer Alkohol beispielsweise ein 3- bis 8-gliedriges Cycloalkanol, wie Cyclopentanol, -hexanol oder -heptanol, in Frage kommt. Ein aromatischer Alkohol ist beispielsweise ein Phenol oder heterocyclischer Alkohol, welche jeweils gegebenenfalls substituiert sein können, insbesondere Hydroxypyridin, z.B. 2-, 3- oder 4-Hydroxypyridin. Carboxy kann ebenfalls mit einem silyliertem Alkohol verestert sein und bedeutet insbesondere Tri-($C_1$-$C_4$-)-alkylsilyl-($C_1$-$C_4$-)alkoxy-carbonyl, insbesondere Trimethylsilylethoxycarbonyl.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen bzw. 3-Aza-, 3-Niederalkylaza-, 3-Oxo- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- oder N,N-Dipropylcarbamoyl, Pyrrolidino- oder Piperidinocarbonyl, Morpholino-, Piperazino- bzw. 4-Methylpiperazino- sowie Thiomorpholinocarbonyl, Anilinocarbonyl oder durch Niederalkyl, Niederalkoxy und/oder Halogen substituiertes Anilinocarbonyl zu nennen.

Bevorzugtes funktionell abgewandeltes Carboxy ist beispielsweise Niederalkoxycarbonyl, wie Methoxy- oder Ethoxycarbonyl, Tri-($C_1$-$C_4$-)-alkylsilyl-($C_1$-$C_4$-)alkoxy-carbonyl, insbesondere Trimethylsilylethoxycarbonyl, oder Cyano. Verbindungen der Formel I, worin $R_3$ Carboxy ist, können beispielsweise ausgehend von Verbindungen der Formel II, worin $Z_1$ funktionell abgewandeltes Carboxy bedeutet, in an sich bekannter Weise, beispielsweise durch Hydrolyse, insbesondere in Gegenwart einer Base, im Falle von entsprechenden Tri-(C-C-)alkylsilyl-(C-C-)alkoxy-carbonylderivaten z.B. durch Behandeln mit einem Ammoniumfluorid, wie Tetraniederalkylammonium-, z.B. Tetra-n-butyl-ammonium-fluorid, oder im Falle von Benzyloxycarbonylderivaten durch Hydrogenolyse in Gegenwart eines Hydrierungskatalysators, bzw. ausgehend von solchen Verbindungen der Formel II, worin $Z_1$ Hydroxymethyl oder Formyl bedeutet, unter Verwendung üblicher Oxidationsmittel, durch Oxidation hergestellt werden.

Die Oxidation erfolgt beispielsweise in einem inerten Lösungsmittel, wie einer Niederalkancarbonsäure z.B. Essigsäure, einem Keton, z.B. Aceton, einem Ether, z.B. Tetrahydrofuran, einem heterocyclischen Aromaten, z.B. Pyridin, oder Wasser oder einem Gemisch davon, erforderlichenfalls unter Kühlen oder Erwärmen, z.B. von etwa 0° bis etwa 150°C. Als Oxidationsmittel kommen beispielsweise oxidierende Übergangsmetallverbindungen, insbesondere solche mit Elementen der I., VI., oder VIII. Nebengruppe, in Frage. Als Beispiele seien genannt: Silberverbindungen, wie Silbernitrat, -oxid oder -picolinat, Chromverbindungen, wie Chromtrioxid oder Kaliumdichromat, Manganverbindungen, wie Kaliumpermanganat, Tetrabutylammonium- oder Benzyl(triethyl)ammoniumpermanganat. Weitere Oxidationsmittel sind beispielsweise geeignete Verbindungen mit Elementen der 4. Hauptgruppe, wie Bleidioxid, oder Halogen-Sauerstoff-Verbindungen, wie Natriumiodat oder Kaliumperiodat.

So wird beispielsweise Hydroxymethyl und Formyl zu Carboxy $R_3$ oxidiert.

Vorzugsweise eignet sich diese Variante zur Herstellung solcher Verbindungen der Formel I, worin die Variablen Bedeutungen haben, die von ungesättigten Resten verschieden sind.

Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natriumhydroxid, -hydrid, -amid, Natriummethylat, -ethylat, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyldiisopropylamin, N-Methyl-piperidin, Pyridin, Benzyltrimethyl-ammoniumhydroxid, 1,5-Diazabicyclo[4.3.0]non-5-en (DBN) sowie 1,8-Diaza-bicyclo[5.4.0] undec-7-en (DBU) genannt.

Das Ausgangsmaterial der Formel II ist beispielsweise zugänglich, indem man eine Verbindung der Formel $R_2$-$X_2$-$NH_2$ (IIa) mit einer Verbindung der Formel

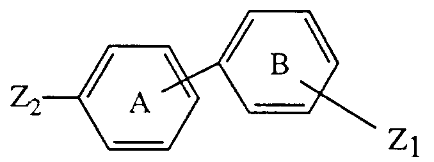 (IIb),

worin $Z_2$ für -$X_3$-$Z_4$ und $Z_4$ für reaktionsfähiges verestertes Hydroxy steht, beispielsweise in Gegenwart einer Base, umsetzt und die so erhältliche Verbindung der Formel

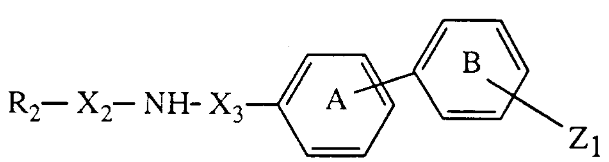 (IIc)

im nächsten Reaktionsschritt mit einer Verbindung der Formel IIIa, z.B. analog Variante b), umsetzt.

Reaktionsfähiges verestertes Hydroxy $Z_4$ ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Brombenzol- oder p-Toluolsulfonyloxy.

Verbindungen der Formel IIb ihrerseits sind beispielsweise aus EP 253,310 bekannt oder können in an sich bekannter Weise hergestellt werden. Verbindungen der Formel (IIa) sind im wesentlichen bekannt oder sind analog an sich bekannter Herstellungsverfahren zugänglich.

Verfahrensvariante b):

Aktivierte Ester von Verbindungen der Formel IIIa sind insbesondere am Verknüpfungskohlenstoffatom des veresterden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der entsprechenden Säure mit einem Niederalkoxyacetylen; Ethoxyacetylen-Methode), oder Ester vom Amidinotyp, wie N,N′-disubstituierte Amidinoester (erhältlich z.B. durch Behandeln der entsprechenden Säure mit einem geeigneten N,N′-disubstituierten Carbodiimid, z.B. N,N′-Dicyclohexylcarbodiimid; Carbodiimid-Methode) oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N′-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester) oder insbesondere Amino- oder Amidoester (erhältlich z.B. durch Behandeln der entsprechenden Säure mit einer N-Hydroxyamino- bzw. N-Hydroxyamido-Verbindung und deren aktivierten Derivaten, z.B. N-Hydroxysuccinimid, N-Hydroxypiperidin, N-Hydroxyphthalimid, N-Hydroxy-5-norbornen- oder norbornan-2,3-dicarbonsäureimid, 1-Hydroxybenzotriazol bzw. Benzotriazol-1-yloxy-phosphoniumsalzen oder Benzotriazol-1-yluroniumsalzen, oder 3-Hydroxy-3,4-dihydro-1,2,3-benzotriazin-4-on, z.B. nach der Anhydrid- oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin, z.B. 1-Ethoxycarbonyl-2-ethoxy-1,2-dihydrochinolin; Methode der gemischten O-Alkylkohlensäureanhydride), Anhydride mit dihalogenierter, insbesondere dichlorierter Phosphorsäure (erhältlich z.B. durch Behandeln der entsprechenden Säure mit Phosphoroxychlorid; Phosphoroxychlorid-methode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diethylaminopropin; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N′-Carbonyldiimidazol; Imidazol-Methode), oder Pyrazolen, z.B. 3,5-Dimethylpyrazol (erhältlich z.B. über das Säurehydrazid durch Behandeln mit Acetylaceton; Pyrazolid-Methode).

Die Kondensation zur Herstellung der Amidbindung kann in an sich bekannter Weise durchgeführt werden, beispielsweise wie in Standardwerken, wie Houben-Weyl, "Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic

Press, London und New York, 1979/1980, oder M. Bodanszky, "Principles of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diethyl-, Dipropyl-, N-Ethyl-N′-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3′-sulfonat und 2-tert-Butyl-5-methylisoxazoliumperchlorat, oder eine geeignete Acylaminoverbindung, z.B. 2-Ethoxy-1-ethoxycarbonyl- 1,2-dihydrochinolin, ferner aktivierte Phosphorsäurederivate, z.B. Diphenylphosphorylazid, Diethylphosphorylcyanid, Phenyl-N-phenylphosphoramidochloridat, Bis-(2-oxo-3-oxazolidinyl)-phosphinsäurechlorid oder 1-Benzotriazolyloxy-tris-(dimethylamino)-phosphonium-hexafluorophosp hat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin.

Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Ether, z.B. Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden.

Das Ausgangsmaterial der Formel IIIb kann man beispielsweise herstellen, indem man eine Verbindung der Formel IIa mit einer Verbindung der Formel

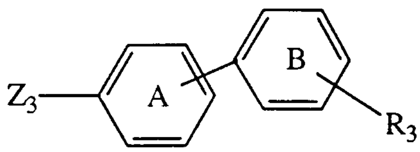 (IIIc),

worin $Z_3$ -$X_3$-$Z_4$ und $Z_4$ reaktionsfähiges verestertes Hydroxy bedeutet, insbesondere in Gegenwart einer der vorstehend aufgeführten Basen, umsetzt. Zur Herstellung von Verbindungen der Formel IIIb, worin $X_3$ -$CH_2$- bedeutet, geht man z.B. von Verbindungen der Formel IIa aus und setzt diese mit Verbindungen der Formel IIIc um, worin $Z_3$ Formyl bedeutet. Die so erhältlichen Schiff′schen Basen werden anschliessend mit Hilfe eines Reduktionsmittels, wie Natriumcyanoborhydrid, reduziert.

Reaktionsfähiges verestertes Hydroxy $Z_4$ ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan-oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Brombenzol- oder p-Toluolsulfonyloxy.

Eine verfahrensgemäss erhältliche erfindungsgemässe Verbindung kann in an sich bekannter Weise in eine andere erfindungsgemässe Verbindung übergeführt werden.

Eine Hydroxy aufweisende erfindungsgemässe Verbindung kann nach an sich bekannten Methoden verethert werden. Die Veretherung kann z.B. mit einem Alkohol, wie gegebenenfalls substituiertem Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide, Methan-, Benzol- oder p-Toluol-sulfonate, in Betracht. Die Veretherung kann z.B. in Gegenwart einer Base, eines Alkalimetallhydrids, -hydroxids, -carbonats oder eines Amins, erfolgen. Umgekehrt können entsprechende Ether, wie Niederalkoxyverbindungen, z.B. mittels starker Säuren, wie Mineralsäuren, z.B. den Halogenwasserstoffsäuren Brom- oder Iodwasserstoffsäure, die vorteilhaft in Form von Pyridiniumhalogeniden vorliegen können, oder mittels Lewissäuren, z.B. Halogeniden von Elementen der 3. Hauptgruppe oder der entsprechenden Nebengruppen, gespalten werden. Diese Umsetzungen können, falls erforderlich, unter Kühlen oder Erwärmen, z.B. einem Temperaturbereich von etwa -20° bis etwa 100°C, in

An- oder Abwesenheit eines Lösungs- oder Verdünnungsmittels, unter Inertgas und/oder unter Druck und gegebenenfalls in einem geschlossenen Gefäss, durchgeführt werden.

Hydroxymethylgruppen aufweisende erfindungsgemässe Verbindungen können beispielsweise ausgehend von entsprechenden Carboxy oder verestertes Carboxy aufweisenden Verbindungen hergestellt werden, wobei entsprechende Verbindungen in an sich bekannter Weise reduziert werden, z.B. durch Reduktion mit einem gegebenenfalls komplexen Hydrid, wie einem Hydrid gebildet aus einem Element der 1. und 3. Hauptgruppe des Periodensystems der Elemente, z.B. Boranat oder Alanat, beispielsweise Lithiumborhydrid, Lithium-, Diisobutylaluminiumhydrid (gegebenenfalls ist ein nachgelagerter Reduktionsschritt unter Verwendung von Alkalimetall-, wie Natriumcyanoborhydrid, erforderlich), ferner Diboran.

Falls ein aromatischer Strukturbestandteil durch (Nieder-)Alkylthio substituiert ist (in $S(O)_m$-R steht m für 0), kann man dieses auf übliche Weise zu entsprechendem (Nieder-)-Alkansulfinyl bzw. - sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig-bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des (Nieder-)Alkylthio zum (Nieder-)Alkansulfonyl. Jedoch setzt man hierbei üblicherweise das Oxidationsmittel im Überschuss ein.

Weist eine der Variablen Amino auf, können entsprechende Verbindungen der Formel I, ihre Tautomeren oder Salze in an sich bekannter Weise N-alkyliert werden; ebenso können Carbamoyl bzw. Carbamoyl aufweisende Reste N-alkyliert werden. Die (Aryl-)-Alkylierung erfolgt z.B. mit einem reaktiven Ester eines (Aryl-)$C_1$-$C_7$-Alkylhalogenids, z.B. -bromid oder -iodid, (Aryl-)$C_1$-$C_7$-Alkylsulfonat, z.B. methansulfonat oder -p-toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft eines Phasentransfer-Katalysators, wie Tetrabutylammoniumbromid oder Benzyltrimethylammoniumchlorid, wobei indes stärker basische Kondensationsmittel, wie Alkalimetallamide, -hydride oder -alkoholate, z.B. Natriumamid, Natriumhydrid oder Natriumethanolat, erforderlich sein können. Ebenso kann Amino in an sich bekannter Weise, z.B analog Variante b), acyliert werden.

In Verbindungen der Formel I, die als Substituenten eine veresterte oder amidierte Carboxygruppe aufweisen, kann man eine solche Gruppe z.B. mittels Hydrolyse, z.B. in Gegenwart eines basischen Mittels, oder eines sauren Mittels, wie einer Mineralsäure, in eine freie Carboxygruppe überführen. Tert-Butyloxycarbonyl beispielsweise kann weiterhin z.B. in an sich bekannter Weise, wie durch Behandeln mit Trihalogen-, wie Trifluoressigsäure, und Benzyloxycarbonyl z.B. durch katalytische Hydrierung in Gegenwart eines Hydrierungskatakysators, z.B. in der nachstehend beschriebenen Weise, in Carboxy überführt werden.

Ferner kann man in Verbindungen der Formel I, die als Substituenten eine Carboxygruppe aufweisen, insbesondere sofern $R_3$ von Carboxy verschieden ist, diese z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z.B. Zinkchlorid, oder eines wasserbindenden Kondensationsmittels, z.B. eines Carbodiimids, wie N,N′-Dicyclohexyl-carbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen der Formel I, worin die Carboxygruppe in freier Form oder in Salz-, wie Ammonium- oder Metall-, z.B. Alkalimetall-, wie Natrium- oder Kaliumsalzform vorliegt, mit einem reaktionsfähigen Ester eines ($C_1$-$C_7$-)Alkylhalogenid, z.B. Methyl- oder Ethyl-bromid oder -iodid, oder einem organischen Sulfonsäureester, wie einem entsprechenden ($C_1$-$C_7$-)Alkylester, z.B. Methansulfonsäure- oder p-Toluolsulfonsäuremethylester oder -ethylester, behandelt.

Verbindungen der Formel I, die als Substituenten eine veresterte Carboxygruppe aufweisen, kann man durch Umesterung, z.B. durch Behandeln mit einem Alkohol, üblicherweise einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-($C_1$-$C_7$-)alkanoats, -($C_1$-$C_7$-)alkanolats oder -cyanids, wie Natriumacetat, -methanolat, -ethylat, -tert-butanolat oder -cyanid, oder eines geeigneten sauren Mittels, gegebenenfalls unter Entfernung des entstehenden Alkohols, z.B. durch Destillation, in andere Esterverbindungen der Formel I umestern. Man kann auch von entsprechenden, sogenannten aktivierten Estern der Formel I ausgehen, die als Substituenten eine aktivierte veresterte Carboxygruppe aufweisen (siehe unten), und diese durch Behandeln mit einem ($C_1$-$C_7$-)Alkanol, in einen anderen Ester umwandeln.

Man kann in Verbindungen der Formel I, die als Substituenten die Carboxylgruppe enthalten, diese auch zuerst in ein reaktionsfähiges Derivat, wie ein Anhydrid, inkl. ein gemischtes Anhydrid, wie ein Säurehalogenid, z.B. -chlorid (z.B. durch Behandeln mit einem Thionylhalogenid, z.B. -chlorid), oder ein Anyhdrid mit einem Ameisensäureester, z.B. -($C_1$-

$C_7$-) alkylester (z.B. durch Behandeln eines Salzes, wie eines Ammonium- oder Alkalimetallsalzes, mit einem Halogen-, wie Chlorameisensäureester, wie ($C_1$-$C_7$-)Alkyl-ester), oder in einen aktivierten Ester, wie Cyanmethyl-, Nitrophenyl-, z.B. 4-Nitrophenyl-, oder Polyhalogenphenyl-, z.B. Pentachlorphenylester (z.B. durch Behandeln mit einer entsprechenden Hydroxyverbindung in Gegenwart eines geeigneten Kondensationsmittels, wie N,N′-Dicyclohexyl-carbodiimid) überführen, und ein solches reaktionsfähiges Derivat dann mit einem Amin umsetzen und so zu Amidverbindungen der Formel I gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit einem Amin in Reaktion bringen. Man kann aber auch andere, nicht-aktivierte Ester, wie ($C_1$-$C_7$-)Alkylester von Verbindungen der Formel I, die als Substituenten z.B. ($C_2$-$C_8$-)Alkoxycarbonyl aufweisen, mit Aminen zur Reaktion bringen.

Weist ein aromatischer Ring als Substituenten ein Wasserstoffatom auf, so kann dieses mit Hilfe eines Halogenierungsmittels in üblicher Weise durch ein Halogenatom ersetzt, z.B. mit Brom, Hypobromsäure, Acylhypobromite oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethylhydantoin, 2,4,4,6-Tetrabrom-2,5-cyclohexandien-1-on, bromiert bzw. mit elementarem Chlor, z.B. in einem halogenierten Kohlenwasserstoff, wie Chloroform, und unter Kühlen, z.B. bis auf etwa -10° bis etwa +100°C, chloriert werden.

Enthält ein aromatischer Ring in den erfindungsgemässen Verbindungen eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. Mineralsäure, wobei die Reaktionstemperatur vorteilhaft unter etwa 5°C gehalten wird.

Die so erhältliche, in Salzform vorliegende Diazoniumgruppe kann man nach analogen Verfahren beispielsweise wie folgt substituieren: durch die Hydroxygruppe analog der Phenolverkochung in Gegenwart von Wasser, durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; durch die Halogenatome Chlor, Brom oder Iod sowie die Cyanogruppe analog der Sandmeyer-Reaktion bei der Umsetzung mit entsprechenden Cu(I)-Salzen, zunächst unter Kühlen, z.B. auf etwa unter 5°C, und anschliessendem Erhitzen, z.B. auf etwa 60° bis etwa 150°C.

Enthalten die Verbindungen der Formel I ungesättigte Reste, wie (Nieder-) Alkenyl oder (Nieder-)Alkinylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle bzw. deren Derivate, z.B. Oxide, geeignet sind, wie Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at und bei Raumtemperatur zwischen etwa -80° bis etwa 200°C, vor allem zwischen Raumtemperatur und etwa 100°C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Weiterhin kann in Verbindungen der Formel I, worin z.B. einer der Reste $R_1$ und/oder $X_2$ Halogen, wie Chlor, aufweist, Halogen durch Umsetzung mit einem gegebenenfalls substituierten Amin, einem Alkohol oder Mercaptan ausgetauscht werden.

Die Erfindung betrifft insbesondere die in den Beispielen beschriebenen Verfahren.

Salze von Verbindungen der Formel I können in an sich bekannter Weise hergestellt werden. So erhält man beispielsweise Säureadditionssalze von Verbindungen der Formel I durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung in freier Form und in Form ihrer Salze sind im Vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen. Beispielsweise weisen Verbindungen der Formel Ia, worin $X_2$ für die Gruppe der Formel Ib, in welcher q für 1 steht und $X_4$ und $X_5$ unterschiedliche Bedeutungen haben, steht, ein asymmetrisches C-Atom auf. In entsprechenden Verbindungen der Formel I, worin $R_2$ beispielsweise gegebenenfalls verestertes oder amidiertes Carboxy oder gegebenenfalls verethertes Hydroxy bedeutet, weist das betreffende asymmetrische C-Atom der Partialstruktur der Formel -$X_2$-$R_2$ vorzugsweise die S-Konfiguration auf.

Erhaltene Racemate und Diastereomerengemische können auf Grund der physikalischchemischen Unterschiede der Bestandteile in bekannter Weise in die reinen Isomeren oder Racemate aufgetrennt werden, beispielsweise durch fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein- oder Apfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen R, $R_1$, $R_2$, $R_3$, $X_1$, $X_2$, $X_3$, $X_4$, $X_5$, m, p, q, und r die für die jeweils bevorzugten Verbindungsgruppen der Formel I angegebenen Bedeutungen haben. Insbesondere sind Verbindungen der Formel IIa, ihre Tautomeren und Salze, worin $Z_1$ Cyano bedeutet, als Ausgangsmaterial bevorzugt.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologische, in erster Linie Angiotensin-II-antagonisierende Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Angiotensin-II-Antagonisten, verwenden.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Celluloseprapäraten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff

vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglyckole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung. Temperaturen werden in Celsiusgraden angegeben. Folgende Laufmittelsysteme für die Chromatographie werden in den nachfolgenden Beispielen verwendet:

Neutrale Systeme

| | |
|---|---|
| N1 Ethylacetat/Hexan | 2:1 |
| N2 Ethylacetat/Hexan | 1:1 |
| N3 Ethylacetat/Hexan | 1:2 |
| N4 Ethylacetat/Hexan | 1:4 |
| N5 Ethylacetat/Hexan | 1:9 |
| N6 $CH_2Cl_2$/Methanol | 95:5 |
| N7 $CH_2Cl_2$/Methanol | 9:1 |
| N8 $CH_2Cl_2$/Methanol | 4:1 |
| N9 $CH_2Cl_2$/Methanol | 2:1 |
| N10 $CH_2Cl_2$/Methanol | 1:1 |

Basische Systeme

| | |
|---|---|
| B1 $CH_2Cl_2$/Methanol/konzentriertes $NH_3$ | 40:10:1 |
| B2 $CH_2Cl_2$/Methanol/konzentriertes $NH_3$ | 50:10:1 |
| B3 $CH_2Cl_2$/Methanol/konzentriertes $NH_3$ | 60:10:1 |
| B4 $CH_2Cl_2$/Methanol/konzentriertes $NH_3$ | 80:10:1 |
| B5 $CH_2Cl_2$/Methanol/konzentriertes $NH_3$ | 100:10:1 |
| B6 Ethylacetat/Ethanol/konzentriertes $NH_3$ | 24:12:4 |
| B7 Toluol/Isopropanol/konzentriertes $NH_3$ | 170:30:2 |

Saure Systeme

| | |
|---|---|
| S1 $CH_2Cl_2$/Methanol/Wasser/Essigsäure | 150:50:10:1 |
| S2 Toluol/Isopropanol/Essigsäure | 170:30:2 |

Mit basischen (d. h. konzentriertes Ammoniak enthaltenden) Laufmittelsystemen chromatographierte Produkte mit sauren funktionellen Gruppen werden nach der Chromatographie in einem organischen Lösungsmittel, z. B. in Diethylether, Essigsäureethylester oder Dichlormethan, aufgenommen. Sodann wird dieses organische Gemisch nacheinander mit (ca. 1 N-) Salzsäure, Wasser und gesättigter Natriumchloridlösung gewaschen und die organische Phase getrocknet und eingedampft. Auf diese Weise erhält man das Produkt mit der freigesetzten sauren funktionellen Gruppe.

Beispiel 1: N-Carboxymethyl-N-pentanoyl-N-[2'-(1H-tetrazol-5-yl)biphenyl-4-ylmethyl]-amin

1,2 g N-(2'-Cyanobiphenyl-4-yl-methyl)-N-methoxycarbonylmethyl-N-pentanoyl-amin, 2,18 g Tributylzinnazid und 40 ml Xylol werden 24 Stunden unter Rückfluss erhitzt. Dann wird das Reaktionsgemisch eingeengt, der Rückstand mit 1 N-Natronlauge versetzt, dieses Gemisch 10 Stunden bei Raumtemperatur gerührt und dann mit Diethylether extrahiert, die wässrige Phase sauer gestellt und anschliessend mit Diethylether extrahiert, diese zweite etherische Phase mit Sole gewaschen, getrocknet und eingeengt und das Rohprodukt mittels Flashchromatographie (100 g Kieselgel; System B1) gereinigt. Amorphes Produkt [$R_f$-Wert: 0,29 ($CH_2Cl_2$/Methanol/konzentriertes Ammoniak = 30:10:1)].

Das Ausgangsmaterial kann beispielsweise wie folgt erhalten werden:

a) 2′-Cyano-4-formyl-biphenyl

250 g 4-Brommethyl-2′-cyano-biphenyl, 150 g Natriumacetat und 2,51 Eisessig werden über Nacht unter Rückfluss erhitzt. Das Gemisch wird anschliessend im Hochvakuum eingeengt und der Rückstand in Ethylacetat aufgenommen. Man extrahiert nacheinander mit Wasser, Natriumhydrogencarbonatlösung und Sole und dampft am Rotationsverdampfer ein. Das Rohprodukt wird in 3,1 l Ethanol gelöst, die Lösung mit 430 ml 2 N-Natronlauge versetzt, das Gemisch über Nacht bei Raumtemperatur gerührt und dann eingeengt und der Rückstand in Ethylacetat aufgenommen. Das Gemisch wird nacheinander mit Wasser und Sodalösung gewaschen und eingeengt. Der Rückstand wird in Hexan suspendiert, die Suspension abgenutscht und der Filterkuchen gewaschen und 20 Stunden bei 60° im Hochvakuum getrocknet. Man erhält so das 2′-Cyano-4-hydroxymethyl-biphenyl in Form eines weissen Pulvers [$^1H$-NMR (DMSO-$d_6$): 4,58 ppm (d, 2 H); 5,3 ppm (t, 1 H); 7,6 bis 8,0 ppm (m, 8 H)].

Eine Lösung von 53 ml Oxalylchlorid in 21 Dichlormethan wird auf - 60° gekühlt. Bei dieser Temperatur wird eine Lösung von 88 ml Dimethylsulfoxid in 150 ml Dichlormethan zugetropft und das Gemisch 2 Minuten nachgerührt. Dann wird bei - 60° eine Lösung von 117 g 2′-Cyano-4-hydroxymethyl-biphenyl in 1 l Dichlormethan zugetropft. Nach beendeter Zugabe (nach ca. 5 Minuten) wird das Gemisch 15 Minuten nachgerührt. Dann werden 390 ml

Triethylamin zugetropft. Man rührt das Gemisch 2 Minuten bei -60° nach, lässt es dann auf Raumtemperatur erwärmen und giesst es auf Wasser. Das Gemisch wird mit Dichlormethan extrahiert und die organische Phase nacheinander mit verdünnter Salzsäure und Sole gewaschen, getrocknet und eingeengt. Der Rückstand wird in Hexan suspendiert, die Suspension abgenutscht, der Filterkuchen gewaschen und das so erhaltene Produkt im Hochvakuum bei 60° getrocknet (Elementaranalyse: 80,7 % C; 4,5 % H; 6,7 % N; 7,7 % O).

b) N-(2′-Cyanobiphenyl-4-ylmethyl)-N-methoxycarbonylmethyl-amin
Ein Gemisch aus 2,0 g 2′-Cyano-4-formyl-biphenyl, 1,22 g 2-Aminoethansäuremethylesterhydrochlorid, 9,6 g Molekularsieb 5A und 26 ml Tetrahydrofuran wird 36 Stunden bei Raumtemperatur gerührt und dann auf 0 bis 5° abgekühlt. Es werden 680 mg Natriumcyanoborhydrid (90 %), gelöst in 4,8 ml Methanol, zugegeben. Das Gemisch wird 24 Stunden bei Raumtemperatur gerührt und dann im Vakuum eingeengt. Das Rohprodukt wird mittels Flashchromatographie (180 g Kieselgel; Essigsäureethylester/Petrolether = 1:1) gereinigt [$^1$H-NMR (DMSO-$d_6$): 3,63 ppm (s, 3 H); 3,79 ppm (s, 2 H); 7,4 bis 8,0 ppm (m, 10 H); 2,6 ppm (1 H)].

c) N-(2′-Cyanobiphenyl-4-ylmethyl)-N-methoxycarbonylmethyl-N-pentanoyl-amin
0,96 g N-(2′-Cyanobiphenyl-4-ymethyl)-N-methoxycarbonylmethyl-amin werden in 9 ml Dichlormethan gelöst. Die Lösung wird mit 1,7 ml Triethylamin und anschliessend bei 0° mit 1,5 ml Pentanoylchlorid versetzt. Man rührt bei Raumtemperatur über Nacht und dampft dann zur Trockne ein: Der Rückstand wird in Diethylether aufgenommen und das etherische Gemisch nacheinander mit Natriumhydrogencarbonatlösung und Sole gewaschen. Flashchromatographie ( 180 g Kieselgel; Essigsäureethylester/Petrolether = 1: 1) liefert das Produkt in Form eines weissen Pulvers [$R_f$-Wert: 0,68 (System N2)].

Beispiel 2: (S)-N-(1-Carboxyethyl)-N-pentanoyl-N-[2′-(1H-tetrazol-5-yl)biphenyl-4-ylmethyl]-amin

Analog Beispiel 1 wird ausgehend von 1,24 g N-Valeryl-N-[(2′-cyanobiphenyl-4-yl)methyl] -(L)-alaninmethylester und 2,73 g Tributylzinnazid wird nach Flashchromatographie (B3) und anschliessendem Umkristallisieren aus Essigester das Produkt als weisses Pulver erhalten. Smp.: 115° (Zers.).

Das Ausgangsmaterial kann beispielsweise wie folgt erhalten werden:

a) N-[(2′-Cyanobiphenyl-4-yl)-methyl]-(L)-alaninmethylester ausgehend von 2,0 g 2′-Cyanobiphenyl-4-carbaldehyd, 1,34 g (L)-Alaninmethylester-Hydrochlorid, 680 mg Natriumcyanoborhydrid und 2,4 g Molekularsieb 5 A und anschliessender Flashchromatographie mit dem System N3. (DC: System N1) $R_f$-Wert: 0,59.

b) N-Valeryl-N-[(2′-cyanobiphenyl-4-yl)-methyl]-(L)-alaninmethylester ausgehend von 1,65 g N-[(2′-Cyanobiphenyl)-methyl]-(L)-alaninmethylester, 2,7 ml Triethylamin und 2,35 ml n-Valeriansäurechlorid und anschliessender Flashchromatographie (N2). (DC:System N2) $R_f$-Wert: 0,62.

Beispiel 3: (S)-N-(1-Methoxycarbonylethyl)-N-pentanoyl-N-[2′-(1H-tetrazol-5-yl)-biphenyl-4-ylmethyl]-amin

0,3 g Säure aus Beispiel 2 werden in 5 ml Methylalkohol gelöst, mit 0,5 ml Salzsäure in Methylalkohol versetzt und während 24 Stunden bei Raumtemperatur gerührt. Das Reaktionsgemisch wird darauf eingeengt, in Methylenchlorid aufgenommen, mit Wasser extrahiert, die organische Phase getrocknet und am Rotationsverdampfer eingeengt. Nach Flashchromatographie (B1) erhält man das Produkt. Smp. des amorphen Materials: 57-59°.

Beispiel 4: N-[1-Carboxy-2-(4-fluorphenyl)-ethyl]-N-pentanoyl-N-[2′-(1H-tetrazol-5-yl)-biphenyl-4-ylmethyl]-amin

Ausgehend von 2,3 g N-Valeryl-N-[(2′-cyanobiphenyl-4-yl)-methyl]-(DL)-p-fluorphenylalaninmethylester und 3,25 g Tributylzinnazid wird nach Flashchromatographie (B1) das Produkt nach Lyophilisation aus tert.-Butylalkohol erhalten. FAB-MS: m/e = 502 $(M+H)^+$.

Das Ausgangsmaterial kann beispielsweise wie folgt erhalten werden:
N-[(2′-Cyanobiphenyl-4-yl)-methyl]-(DL)-p-fluorphenylalaninmethylester ausgehend von 2,33 g 2′-Cyanobiphenyl-4-carbaldehyd, 2,63 g (DL)-p-Fluorphenylalaninmethylester, 790 mg Natriumcyanoborhydrid und 11,0 g Molekularsieb 5 A und anschliessender Flashchromatographie mit System N3. (DC: System N2) $R_f$-Wert: 0,36.

N-Valeryl-N-[(2′-cyanobiphenyl-4-yl)-methyl]-(DL)-p-fluorphenylalaninmethylester ausgehend von 2,1 g N-[(2′-Cyanobiphenyl-4-yl)-methyl]-(DL)-p-fluorphenylalaninmethylester, 1,0 ml Triethylamin und 0,85 ml n-Valeriansäurechlorid und anschliessender Flashchromatographie (N3). (DC: System N2) $R_f$-Wert: 0,64.

Beispiel 5: N-[2-(4-Fluorphenyl)-1-methoxycarbonyl-ethyl]-N-pentanoyl-N-[2′-(1H-tetrazol-5-yl)biphenyl-4-ylmethyl]-amin

Analog Beispiel 3 ausgehend von 1,29 g N-Valeryl-N-[(2′-(1H-tetrazol-5-yl)-biphenyl-4-yl)-methyl]-(DL)-p-fluorphenylalanin gemäss Beispiel 4. FAB-MS: m/e = 516 $(M+H)^+$.

Beispiel 6: N-[2-(4-Fluorphenyl)-1-hydroxymethyl-ethyl]-N-pentanoyl-N-[2′-(1H-tetrazol-5-yl)biphenyl-4-ylmethyl]-amin

0,5 g N-Valeryl-N-[(2′-(1H-tetrazol-5-yl)biphenyl-4-yl)-methyl]-(DL)-p-fluorphenylalanin-methylester aus Beispiel 5 werden in 5 ml Tetrahydrofuran bei -70° mit 1,9 ml Diisobutylaluminiumhydrid versetzt. Nach 20 Minuten gibt man 0,2 ml Methylalkohol zu und lässt auf Raumtemperatur aufwärmen. Das Reaktionsgemisch wird mit Ether und Wasser versetzt, die organische Phase abgetrennt, mit Sole gewaschen, getrocknet und eingeengt. Flashchromatographie (B2) liefert den entsprechenden Aldehyd. Dieser wird bei 0° in 5 ml Ethylalkohol mit 27 mg Natriumborhydrid versetzt und während 3,5 Stunden bei dieser Temperatur gerührt. Nach Abfiltrieren und Einengen wird das Produkt durch Flashchromatographie (N8) und Lyophilisieren aus tert.-Butylalkohol erhalten. FAB-MS: m/e= 488 $(M+H)^+$.

Beispiel 7: N-(2′-Carboxybiphenyl-4-ylmethyl)-N-[1-carboxy-2-(4-fluorphenyl)-ethyl]-N-pentanoyl-amin

N-Valeryl-N-[(2′-carboxybiphenyl-4-yl)-methyl]-(DL)-p-fluorphenylalanin-methylester werden in 10 ml Methylalkohol und 3 ml Wasser mit 0,45 ml 2N NaOH versetzt. Man rührt über Nacht bei Raumtemperatur und neutralisiert anschliessend mit 0,45 ml 2N Salzsäure. Nach Flashchromatographie (B1) und Lyophilisieren aus tert.-Butanol erhält man das amorphe Produkt. FAB-MS: m/e= 478 $(M+H)^+$.

Beispiel 8: N-(2′-Carboxybiphenyl-4-ylmethyl)-N-[2-(4-fluorphenyl)-1-methoxy-carbonyl-ethyl]-N-pentanoyl-amin

840 mg N-Valeryl-N-[(2′-(trimethylsilylethoxycarbonyl)biphenyl-4-yl)-methyl]-(DL)-p-fluorphenylalanin-methylester werden in 10 ml Dimethylformamid mit 15,6 ml einer 0,5 M Lösung von Tetrabutylammoniumfluorid in Tetrahydrofuran versetzt und über Nacht bei Raumtemperatur gerührt. Das Reaktionsgemisch wird eingeengt, in Essigester aufgenommen, mit Wasser und Sole gewaschen, getrocknet und eingeengt. Nach Flashchromatographie (B4) und Lyophilisieren aus tert.-Butanol erhält man das Produkt. FAB-MS: m/e= 492 $(M+H)^+$.

Das Ausgangsmaterial kann beipielsweise wie folgt erhalten werden:
14,2 g 4-Methyl-2′-carboxybiphenyl (EP 253,310) werden in 60 ml Acetonitril und 10,7 ml Pyridin gelöst und 11,4 ml Trimethylsilylethanol zugegeben. Man versetzt mit bei 0° 15,1 g Dicyclohexylcarbodiimid und rührt bei dieser Temperatur während 3 Stunden. Darauf wird das Reaktionsgemisch im Hochvakuum eingedampft, mit Ether versetzt und Dicyclohexylharnstoff abfiltriert. Nach Flashchromatographie (Essigester/Hexan 95:5) erhält man das 4-Methyl-2′-(trimethylsilylethoxycarbonyl)biphenyl als leicht gelbliches Oel. (DC: Essigester/Hexan 95:5) $R_f$-Wert: 0.42.

312 mg 4-Methyl-2′-(trimethylsilylethoxycarbonyl)biphenyl, 178 mg N-Bromsucciniimid, 5 mg Azoisobutyronitril und 15 ml Tetrachlorkohlenstoff werden eine Stunde zum Rückfluss erhitzt. Nach Abkühlen wird das Gemisch eingedampft. Flashchromatographie (Essigester/Hexan 95:5) liefert 4-Brommethyl-2′-(trimethylsilylethoxycarbonyl)biphenyl als leicht gelbliches Oel. $^1$H-NMR ($CFCl_3$): 0 ppm (s, 9 H), 0,7 ppm (t, 2 H), 4,5 ppm (s, 2 H), 7,1-8 ppm Aromaten.

2,8 g 4-Brommethyl-2′-(trimethylsilylethoxycarbonyl)biphenyl und 1,17 g wasserfreies Natriumacetat werden in Eisessig über Nacht bei 65° gerührt und anschliessend 3 Stunden unter Rückfluss gekocht. Das Reaktionsgemisch wird eingedampft, der Rückstand in Essigester aufgenommen, mit Wasser und Natriumhydrogencarbonat gewaschen, die organische Phase getrocknet und eingeengt. Der Rückstand wird in 25 ml Ethanol vorgelegt, 6,3 ml 1N NaOH zugegeben und 30 Minuten bei Raumtemperatur gerührt. Das Gemisch wird im Vakuum eingedampft, mit Essigester versetzt, mit Wasser und Sole gewaschen, getrocknet und eingedampft. Flashchromatographie (N4) liefert 4-Hydroxymethyl-2-(trimethylsilylethoxycarbonyl)biphenyl als farbloses Oel. $^1$H-NMR (DMSO): 0 ppm (s, 9 H), 0,75 ppm (t, 2 H), 4,1 ppm (t, 2 H), 4,73 ppm (d, 2 H), 5,27 ppm (t, 1H), 7,2-7,7 ppm Aromaten.

2′-(Trimethylsilylethoxycarbonyl)biphenyl-4-carbaldehyd wird analog Beispiel 1 a) erhalten ausgehend von 6,5 g 4-Hydroxymethyl-2′-(trimethylsilylethoxycarbonyl)-biphenyl, 1,87 ml Oxalylchlorid, 3,1 ml Dimethylsulfoxid und 13,8 ml Triethylamin und anschliessender Flashchromatographie mit Methylenchlorid. $^1$H-NMR ($CDCl_3$): 0 ppm (s, 9 H), 0,8 ppm (t, 2 H), 4,2 ppm (t, 2 H), 7,2-8,1 ppm Aromaten, 10,1 ppm (s, 1 H).

Analog Beispiel 1 b) erhält man ausgehend von 1,0 g 2′-(Trimethylsilylethoxycarbonyl)-biphenyl-4-carbaldehyd, 3,0 g Molekularsieb 5 A, 0,715 g (D,L)-p-Fluorphenylalaninmethylester-Hydrochlorid und 215 mg Natriumcyanoborhydrid und anschliessender Flashchromatographie (N3) N-[(2′-(Trimethylsilylethoxy-carbonyl)biphenyl-4-yl)-methyl]-(D,L)-p-fluorphenylalanin-methylester. (DC: N3) $R_f$-Wert: 0,64.

Analog Beispiel 1 c) erhält man ausgehend von 0,8 g N-[(2′-(Trimethylsilylethoxycarbonyl)biphenyl-4-yl)-methyl]-(D,L)-p-fluorphenylalanin-methylester, 0,29 ml Triethylamin und 0,25 ml Valerylchlorid nach Flashchromatographie (N3)

N-Valeryl-N-[(2′-(trimethylsilylethoxycarbonyl)biphenyl-4-yl)-methyl] -(D,L)-p-fluorphenylalaninmethylester. (DC: N3) $R_f$-Wert = 0,65.

Beispiel 9: (S)-N-(2′-Carboxybiphenyl-4-ylmethyl)-N-(1-hydroxymethyl-2-phenylethyl)-N-pentanoyl-amin

290 mg N-[3-(p-Fluorphenyl)-1-hydroxy-2-propyl]-N-[2′-(trimethylsilylethoxycarbonyl)-4-yl-methyl]-valeriansäureamid werden in 3 ml Dimethylformamid während 20 Stunden bei Raumtemperatur mit 5,82 ml einer 0,5 molaren Lösung von Tetrabutylammoniumfluorid in Tetrahydrofuran behandelt. Das Gemisch wird im Vakuum eingeengt, in Essigester aufgenommen, mit Wasser und Sole gewaschen und eingeengt. Nach Flashchromatographie (N7) und Lyophilisation erhält man das Produkt als weisses Pulver. FAB-MS: m/e= 446 $(M+H)^+$.

Das Ausgangsmaterial kann beispielsweise wie folgt erhalten werden:
Analog Beispiel 1 b) erhält man ausgehend von 1,5 g 2′-(Trimethylsilylethoxycarbonyl)-biphenyl-4-carbaldehyd, 4,5 g Molekularsieb 5 A, 0,694 g (D,L)-3-Phenyl-2-amino-propan-1-ol und 321 mg Natriumcyanoborhydrid nach Flashchromatographie (B5) N-[(2′-(Trimethylsilylethoxycarbonyl)biphenyl-4-yl)-methyl] -3-(p-fluorphenyl)-2-aminopropan-1-ol. $^1$H-NMR (DMSO): 0 ppm (2 s, 9 H), 0,73 ppm (2 t, 2 H), 2 ppm (b, 1 H), 2,73 ppm (m, 3 H), 3,3 ppm (m, 2 H), 3,83 ppm (s, 2 H), 4,1 ppm (2 t, 2 H), 4,6 ppm (t, 1 H), 7,15-7,8 ppm, m (8 H).

Analog Beispiel 1 c) erhält man ausgehend von 365 mg N-[(2′-(Trimethylsilylethoxycarbonyl)biphenyl-4-yl)-methyl]-3-(phenyl)-2-amino-propan-1-ol, 0,136 ml Triethylamin, 0,112 ml Valerylchlorid und anschliessender Flashchromatographie (N3) N-[3-(Phenyl)-1-hydroxy-2-propyl]-N-[(2′-(trimethylsilylethoxycarbonyl)-4-yl-methyl]-valeriansäureamid. FAB-MS: m/e= 546 $(M+H)^+$.

Beispiel 10: (S)-N-(2′-Carboxybiphenyl-4-ylmethyl)-N-(1-hydroxymethyl-2-imidazol-4-yl-ethyl)-N-pentanoyl-amin

Analog Beispiel 9 erhält man das Produkt ausgehend von 272 mg N-[3-(Imidazol-4-yl)-1-hydroxy-2-propyl]-N-[(2′-(trimethylsilylethoxycarbonyl)biphenyl-4-yl)-methyl]-valeriansäureamid und 5,54 ml Tetrabutylammoniumfluoridlösung nach Flashchromatographie (B1). FAB-MS $(M+H)^+$=436.

Das Ausgangsmaterial kann beispielsweise analog Beispiel 9 wie folgt erhalten werden:
Umsetzung von 1,5 g 2′-(Trimethylsilylethoxycarbonyl)biphenyl-4-carbaldehyd, 0,984 g 3-(Imidazol-4-yl)-2-(S)-amino-propan-1-ol-dihydrochlorid, 321 mg Natriumcyanoborhydrid und 4,5 g Molekularsieb 5 A liefert nach Flashchromatographie (B5) N-[(2′-(Trimethylsilylethoxycarbonyl)biphenyl-4-yl)-methyl]-3-(imidazol-4-yl)-2-aminopropan-1-ol. (DC) $R_f$-Wert (0,36).

Umsetzung von 0,45 g N-[(2′-(Trimethylsilylethoxycarbonyl)biphenyl-4-yl)-methyl]-3-(imidazol-4-yl)-2-(S)-amino-propan-1-ol, 0,152 ml Triethylamin und 0,132 ml Valerylchlorid liefert nach Flashchromatographie (Methylenchlorid-Methanol-conc. Ammoniak: 120-10-1) N-[3-(Imidazol-4-yl)-1-hydroxy-2-propyl]-N-[(2′-(trimethylsilylethoxycarbonyl)biphenyl-4-yl)-methyl]-valeriansäureamid. Bei der Aufarbeitung wird die wässrige Phase leicht basisch gestellt. FAB-MS: m/e= 536 $(M+H)^+$.

Beispiel 11: (R)-N-(1-Carboxyethyl)-N-pentanoyl-N-[2′-(1H-tetrazol-5-yl)biphenyl-4-ylmethyl]-amin

Analog Beispiel 1 wird das Produkt hergestellt ausgehend von 0,84 g N-Valeryl-N-[(2′-cyano-biphenyl-4-yl)-methyl]-(D)-alanin-methylester und 731 mg Tributylzinnazid und anschliessender Flashchromatographie (B1). FAB-MS: m/e = 408 $(M+H)^+$.

Das Ausgangsmaterial kann beispielsweise analog Beispiel 1 b) erhalten werden:
Umsetzung von 2,0 g 2′-Cyanobiphenyl-4-carbaldehyd, 9,6 g Molekularsieb 5 A, 1,34 g (D)-Alaninmethylester-Hydrochlorid und 680 mg Natriumcyanoborhydrid liefert nach Flashchromatographie (N3) N-[(2′-Cyanobiphenyl-4-yl)-methyl]-(D)-alaninmethylester. $^1$H-NMR (DMSO): 1,21 ppm (d, 3 H), 3,63 ppm (s, 3 H), 3,75 ppm (dd, 1 H), 4,56 ppm (d, 2 H), 4,58 ppm (d, 2 H), 5,31 ppm (t, 1 H), 7,4-8 ppm Aromaten.

Umsetzung analog Beispiel 1 c) von 1,25 g N-[(2′-Cyanobiphenyl-4-yl)-methyl]-(D)-alaninmethylester, 2,1 ml Triethylamin und 1,8 ml n-Valeriansäurechlorid liefert nach Flashchromatographie (N3) N-Valeryl-N-[(2′-cyano-biphenyl-4-yl)-methyl]-(D)-alaninmethylester (DC: N2) $R_f$-Wert: 0,61.

Beispiel 12: (1S),(2S)-N-(1-Carboxy-2-methyl-but-1-yl)-N-pentanoyl-N-[2′-(1H-tetrazol-5-yl)biphenyl-4-ylmethyl]-amin

Das Produkt kann ausgehend von 2,0 g N-Valeryl-N-[(2′-cyanobiphenyl-4-yl)-methyl]-(L)-isoleucinmethylester und 3,19 g Tributylzinnazid und anschliessender Flashchromatographie (B1) hergestellt werden. FAB-MS $(M+H)^+$ = 450.

Das Ausgangsmaterial kann beispielsweise analog Beispiel 1 b) erhalten werden:
Die Umsetzung von 2,0 g 2′-Cyanobiphenyl-4-carbaldehyd, 9,6 g Molekularsieb 5 A, 1,76 g (L)-Isoleucinmethylester-Hydrochlorid und 680 mg Natriumcyanoborhydrid liefert nach Flashchromatographie (Essigester-Hexan 1:3) den N-[(2′-Cyanobiphenyl-4-yl)-methyl]-(L)-isoleucinmethylester. $^1$H-NMR (DMSO): 1,21 ppm (d, 3 H), 3,63 ppm (s, 3 H), 3,75 (dd, 1 H), 4,56 ppm (d, 2 H), 4,58 ppm (d, 2 H), 5,31 ppm (t, 1 H), 7,4-8 ppm Aromaten.

Die Umsetzung analog Beispiel 1 c) von 1,80 g N-[(2′-Cyanobiphenyl)-4-yl-methyl]-(L)-isoleucinmethylester, 2,7 ml Triethylamin und 2,35 ml n-Valeriansäurechlorid liefert nach Flashchromatographie (N4) den N-Valeryl-N-[(2′-cyano-biphenyl-4-yl)-methyl]-(L)-isoleucinmethylester. (DC: N3) $R_f$-Wert: 0,43.

Beispiel 13: (1S),(2S)-N-(1-Methoxycarbonyl-2-methyl-but-1-yl)-N-pentanoyl-N-[2′-(1H-tetrazol-5-yl)biphenyl-4-ylmethyl]-amin

Das Produkt kann erhalten werden analog Beispiel 3 ausgehend von 200 mg N-Valeryl-N-[(2′-( 1H-tetrazol-5-yl)biphenyl-4-yl)-methyl]-(L)-isoleucin und anschliessender Flashchromatographie (B1). FAB-MS: m/e= 464 (M+H)$^+$.

Beispiel 14: (S)-N-(1-Carboxybut-1-yl)-N-pentanoyl-N-[2′-(1H-tetrazol-5-yl)biphenyl-4-ylmethyl]-amin

Das Produkt kann analog Beispiel 1 ausgehend von 0,30 g N-Valeryl-N-[(2′-cyano-biphenyl-4-yl)-methyl]-(L)-norvalin-methylester und 490 mg Tributylzinnazid und anschliessender Flashchromatographie (B1) hergestellt werden. FAB-MS (M+H)$^+$ = 436.

Das Ausgangsmaterial kann beispielsweise analog Beispiel 1 b) erhalten werden:

Die Umsetzung von 2,0 g 2′-Cyanobiphenyl-4-carbaldehyd, 9,6 g Molekularsieb 5 A, 1,34 g (L)-Norvalinmethylester-Hydrochlorid und 680 mg Natriumcyanoborhydrid liefert nach Flashchromatographie (N3) den N-[(2′-Cyanobiphenyl-4-yl)-methyl]-(L)-norvalin-methyl-ester. $^1$H-NMR (DMSO): 0,83 ppm (t, 3 H), 1,33 ppm (m, 2 H), 1,55 ppm (m, 2 H), 3,62 ppm (s, 3 H), 3,1 ppm (m, 1 H), 7,3-8 ppm Aromaten.

Die Umsetzung analog Beispiel 1 c) von 1,5 g N-[(2′-Cyanobiphenyl)-4-yl)-methyl]-(L)-norvalinmethylester, 2,35 ml Triethylamin und 2, 15 ml n-Valeriansäurechlorid liefert nach Flashchromatographie (Essigester-Hexan: 1-3) den N-Valeryl-N-[(2′-cyanobiphenyl-4-yl)-methyl]-(L)-norvalinmethylester (DC:B1) $R_f$-Wert: 0,9.

Beispiel 15: (S)-N-(1-Methoxycarbonylbut-1-yl)-N-pentanoyl-N-[2′-(1H-tetrazol-5-yl)-biphenyl-4-ylmethyl]-amin

Das Produkt kann analog Beispiel 3 erhalten werden ausgehend von 200 mg der Verbindung gemäss Beispiel 14 und anschliessender Flashchromatographie (B1). FAB-MS: m/e=464 (M+H)$^+$.

Beispiel 16: (S)-N-(1-Carboxy-2-methyl-prop-1-yl)-N-pentanoyl-N-[2′-(1H-tetrazol-5-yl)-biphenyl-4-ylmethyl]-amin

Das Produkt kann hergestellt werden ausgehend von 1,40 g N-Valeryl-N-[(2′-cyanobiphenyl-4-yl)-methyl]-(L)-valinmethylester und 2,25 g Tributylzinnazid und anschliessender Flashchromatographie (B1). FAB-MS (M+H)$^+$ = 436, Schmelzintervall 105-115° (aus Ethylacetat).

Das Ausgangsmaterial kann beispielsweise analog Beispiel 1 b) erhalten werden:

Umsetzung von 0,5 g 2′-Cyanobiphenyl-4-carbaldehyd, 2,5 g Molekularsieb 5 A, 0,815 g (L)-Valinmethylester-Hydrochlorid und 180 mg Natriumcyanoborhydrid liefert nach Flashchromatographie (N3) den N-[(2′-Cyanobiphenyl-4-yl)-methyl]-(L)-valinmethylester. (DC: N3) $R_f$-Wert: 0,5.

Umsetzung analog Beispiel 1 c) von 1,15 g N-[(2′-Cyanobiphenyl-4-yl)-methyl]-(L)-valinmethylester, 0,625 ml Triethylamin und 0,56 ml n-Valeriansäurechlorid liefert nach Flashchromatographie (N3) den N-Valeryl-N-[(2′-cyanobiphenyl-4-yl)-methyl]-(L)-valinmethylester. (DC: N2) $R_f$-Wert: 0,63.

Beispiel 17: (S)-N-(1-Carboxyethyl)-N-hexanoyl-N-[2′-(1H-tetrazol-5-yl)biphenyl-4-ylmethyl]-amin

Das Produkt kann hergestellt werden ausgehend von 2,4 g N-Caproyl-N-[(2′-cyanobiphenyl-4-yl)-methyl]-(L)-alaninmethylester und 4,05 g Tributylzinnazid und anschliessender Flashchromatographie (B1). FAB-MS: m/e= 422 (M+H)$^+$.

Das Ausgangsmaterial kann beispielsweise analog Beispiel 2 erhalten werden:

Umsetzung von 2,0 g N-[(2′-Cyanobiphenyl)-4-yl-methyl]-(L)-alaninmethylester, 1,23 ml Triethylamin, und 1,22 ml n-Caproylchlorid liefert den N-Caproyl-ageN- [(2′-cyanobiphenyl-4-yl)-methyl]-(L)-alanin-methylester. (DC: N2) $R_f$-Wert: 0,5.

Beispiel 18: (S)-N-Butanoyl-N-(1-carboxyethyl)-N-[2′-(1H-tetrazol-5-yl)biphenyl-4-ylmethyl]-amin

Das Produkt kann hergestellt werden ausgehend von 2,25 g N-Butyryl-N-[(2′-cyanobiphenyl-4-yl)-methyl]-(L)-alaninmethylester und 4,11 g Tributylzinnazid und anschliessender Flashchromatographie (B1). FAB-MS: m/e = 394 (M+H)$^+$.

Das Ausgangsmaterial kann beispielsweise analog Beispiel 2 erhalten werden:
Umsetzung von 2,0 g N-[(2′-Cyanobiphenyl-4-yl)-methyl]-(L)-alaninmethylester, 1,23 ml Triethylamin und 0,92 ml n-Buttersäurechlorid liefert den N-Butyryl-N-[(2′-cyanobiphenyl-4-yl)-methyl]-(L)-alanin-methylester. (DC: N2) $R_f$-Wert: 0,5.

Beispiel 19: (S)-N-(1-Carboxyprop-1-yl)-N-pentanoyl-N-[2′-(1H-tetrazol-5-yl)biphenyl-4-ylmethyl]-amin

Das Produkt kann hergestellt werden ausgehend von 0,68 g N-Valeryl-N-[(2′-cyanobiphenyl-4-yl)-methyl]-(L)-2-aminobuttersäuremethylester und 1,15 g Tributylzinnazid. Kristallisation aus Ether. Smp.: 102- 104°. FAB-MS $(M+H)^+ = 422$.
Das Ausgangsmaterial kann beispielsweise analog Beispiel 1 b) erhalten werden:
Umsetzung von 3,0 g 2′-Cyanobiphenyl-4-carbaldehyd, 14,5 g Molekularsieb 5 A, 2,23 g (L)-2-Aminobuttersäure-Hydrochlorid und 1075 mg Natriumcyanoborhydrid liefert nach Flashchromatographie (N3) den N-[(2′-cyanobiphenyl-4-yl)-methyl]-(L)-2-aminobuttersäuremethylester. $^1$H-NMR (DMSO): 0,88 ppm (t, 3 H), 1,62 ppm (m, 2 H), 2,53 ppm (b, 1 H), 3,15 ppm (m, 1 H), 3,63 ppm (s, 3 H), 3,62 ppm (d, 2 H), 3,81 ppm (d, 1 H).
Umsetzung analog Beispiel 1 c) von 0,54 g N-[(2′-Cyanobiphenyl-4-yl)-methyl]-(L)-2Aminobuttersäuremethylester, 0,33 ml Triethylamin und 0,29 ml N-Valeriansäurechlorid liefert den N-Valeryl-N-[(2′-cyano-biphenyl-4-yl)-methyl]-(L)-2-aminobuttersäuremethylester. (DC: N2) $R_f$-Wert: 0,52.

Beispiel 20: (S)-N-(1-Carboxy-2-cyclohexyl-ethyl)-N-pentanoyl-N-(2′-(1H-tetrazol-5-yl)biphenyl-4-ylmethyl]-amin

Das Produkt kann hergestellt werden ausgehend von 4,0 g N-Valeryl-N-[(2′-cyanobiphenyl-4-yl)-methyl]-(L)-cyclohexylalaninmethylester und 5,8 g Tributylzinnazid und anschliessender Flashchromatographie (B1). FAB-MS $(M+H)^+ = 490$.
Das Ausgangsmaterial kann beispielsweise analog Beispiel 1 b) erhalten werden:
Umsetzung von 9,35 g 2′-Cyanobiphenyl-4-carbaldehyd, 46 g Molekularsieb 5 A, 10,0 g (L)-Cyclohexylalaninmethylester-Hydrochlorid und 3,3 g Natriumcyanoborhydrid liefert nach Flashchromatographie (N3) den N-[(2′-Cyanobiphenyl-4-yl)-methyl]-(L)-cyclohexylalaninmethylester. (DC: N3) $R_f$-Wert: 0,45.
Umsetzung analog Beispiel 1 c) von 9,0 g N-(2′-Cyanobiphenyl-4-ylmethyl)-(L)-cyclohexylalaninmethylester, 4,33 g Triethylamin und 3,75 ml n-Valeriansäurechlorid liefert nach Flashchromatographie (N3) den N-Valeryl-N-[(2′-cyanobiphenyl-4-yl)-methyl](L)-cyclohexylalanin-ester-methylester. (DC: N3) $R_f$-Wert: 0,55.

Beispiel 21: (S)-N-(2-Cyclohexyl-1-methoxycarbonyl-ethyl)-N-pentanoyl-N-[2′-(1-H-tetrazol-5-yl)biphenyl-4-ylmethyl]-amin

Das Produkt kann erhalten werden analog Beispiel 3 ausgehend von 1,02 g der Verbindung aus Beispiel 20. FAB-MS: m/e= 504 $(M+H)^+$.

Beispiel 22: (R)-N-(1-Carboxy-2-methyl-prop-1-yl)-N-pentanoyl-N-[2′-(1H-tetrazol-5-yl)biphenyl-4-ylmethyl]-amin

Das Produkt kann hergestellt werden analog Beispiel 11 ausgehend von 3,8 g N-Valeryl-N-[(2′-cyano-biphenyl-4-yl)-methyl]-(D)-valinmethylester und 6,17 g Tributylzinnazid und anschliessender Flashchromatographie (N8). FAB-MS $(M+H)^+ = 436$.
Das Augangsmaterial kann beispielsweise analog Beispiel 1 b) erhalten werden:
Umsetzung von 4,0 g 2′-Cyanobiphenyl-4-carbaldehyd, 19,3 g Molekularsieb 5 A, 3,8 g (D)-Valinmethylester-Hydrochlorid und 1,43 g Natriumcyanoborhydrid liefert nach Flashchromatographie (N3) den N-[(2′-cyanobiphenyl-4-yl)-methyl]-(D)-Valinmethylester. (DC: N2) $R_f$-Wert: 0,56.
Umsetzung analog Beispiel 1 c) von 3,2 g N-[(2′-Cyanobiphenyl)-4-yl-methyl]-(D)-valinmethylester, 1,82 ml Triethylamin und 1,6 ml N-Valeriansäurechlorid liefert nach Flashchromatographie (N2) den N-Valeryl-N- [(2′-cyano-biphenyl-4-yl)methyl]-(D)-valinmethylester. FAB-MS: m/e= 407 $(M+H)^+$.

Beispiel 23: N-(2-Methoxyethyl)-N-pentanoyl-N-[2′-(1H-tetrazol-5-yl)biphenyl-4-ylmethyl]-amin

Unter Ueberleiten eines schwachen Stickstoffstromes wird eine Lösung von 1,6 g (4,5 mMol) rohem N-[(2′-Cyanobiphenyl-4-yl)-methyl]-N-(2-methoxyethyl)-valeriansäureamid und 1,8 g (5,5 mMol) Tri-n-butylzinnazid in 15 ml o-Xylol während 20-24 Stunden unter Rückfluss erhitzt. Nach dem Abkühlen wird die Lösung mit ca. 30 ml Toluol verdünnt, mit 15 ml 1N wässriger Natronlauge versetzt und während 2 Stunden intensiv gerührt. Die wässrige Phase wird abgetrennt und mit 16 ml 1N wässriger Salzsäure sauer gestellt. Das ausgefällte Produkte wird durch Extraktion mit Aethylacetat isoliert. Man erhält so die rohe Titelverbindung als Oel, das aus wenig Ethylacetat kristallisiert, Smp. 120-122°.
Das Ausgangsmaterial kann beispielsweise wie folgt hergestellt werden:

a) 4-[N-(2-Methoxyethyl)-aminomethyl]-2′-cyanobiphenyl

Eine Lösung von 5,45 g (20 mMol) 4-Brommethyl-2′-cyanobiphenyl in 40 ml 1,4-Dioxan wird mit 7,5 g (100 mMol) 2-Methoxyethylamin versetzt und hierauf 8-10 Stunden unter Rückfluss zum Sieden erhitzt. Nach dem gründlichen Eindampfen im Wasserstralvakuum wird der Eindampfrückstand in 60 ml 2N Salzsäure gelöst und mit 60 ml Ether extrahiert. Die salzsaure Lösung wird abgetrennt und mit conc. Natronlauge alkalisch gestellt. Das ausgefallene Oel wird mit Ether extrahiert, die Etherlösung mit Wasser gewaschen, über Magnesiumsulfat getrocknet und eingedampft. Man erhält so die rohe Titelverbindung als Oel, das in wenig Ether gelöst und mit einer methanolischen Lösung von Salzsäure-Gas versetzt wird. Das so erhaltene kristalline Hydrochlorid wird aus 2-Propanol umkristallisiert und schmilzt bei 174-176°.

b) N-[(2′-Cyanobiphenyl-4-yl)methyl]-N-(2-methoxyethyl)-n-valeri ansäureamid

Zu einem Gemisch von 3,7 g (12,2 mMol) 4-[N-(2-Methoxyethyl)-aminomethyl]2′-cyanobiphenyl-Hydrochlorid und 3,1 g (31 mMol) Triethylamin in 50 ml 1,4-Dioxan werden unter Rühren und Kühlen mit Eiswasser 1,5 g (15 mMol) n-Valerylchlorid getropft. Die Suspension wird 4-6 Stunden bei Raumtemperatur gerührt. Nach dem Eindampfen im Wasserstrahlvakuum wird das Reaktionsgemisch zwischen 20 ml Wasser und 200 ml Ethylacetat verteilt. Die organische Phase wird nacheinander mit je 10 ml 2N Salzsäure, gesättigter $NaHCO_3$-Lösung und Sole gewaschen, über Magnesiumsulfat getrocknet und im Vakuum eingedampft. Die so erhaltene Titelverbindung wird als Oel erhalten ($R_f$-Wert: 0,51 im System B7) und kann roh weiter umgesetzt werden.

Beispiel 24: N-(2-Benzyloxyethyl)-N-pentanoyl-N-[2′-(1H-tetrazol-5-yl)biphenyl-4-ylmethyl]-amin

6,5 g (15,2 mMol) rohes N-(2-Benzyloxyethyl)-N-[(2′-cyanobiphenyl-4-yl)-methyl]n-valeriansäureamid und 6,1 g Tri-n-butylzinnazid werden analog Beispiel 23 umgesetzt und aufgearbeitet. Man erhält so die rohe Titelverbindung, die nach Umkristallisation aus wenig Ethylacetat bei 109-110° schmilzt.

Das Ausgangsmaterial kann beispielsweise auf folgende Weise hergestellt werden:

a) 4-[N-(2-Benzyloxyethyl)-aminomethyl]-2′-cyanobiphenyl

Analog Beispiel 23 a) erhält man aus 2-Benzyloxyethylamin (J. Am. Pharm. Assoc., Sci. Ed. 1952, 41, 257) die Titelverbindung nach flash-chromatographischer Reinigung (Silicagel; Toluol-Methanol 19:1) als gelbliches Oel, das im DC im System B7 einen $R_f$-Wert von 0,48 aufweist.

b) N-(2-Benzyloxyethyl)-N-[(2′-cyanobiphenyl-4-yl)methyl]-n-vale riansäureamid

Analog Beispiel 26 b) erhält man aus 4-[N-(2-Benzyloxyethyl)-aminomethyl]-2′-cyanobiphenyl die Titelverbindung. Sie weist im DC-System B7 einen $R_f$-Wert von 0,71 auf und kann roh weiterverwendet werden.

Beispiel 25: N-(3-Methoxyprop-1-yl)-N-pentanoyl-N-[2′-(1H-tetrazol-5-yl)biphenyl-4-ylmethyl]-amin

Analog Beispiel 23 erhält man aus 2,1 g (5,8 mMol) rohem N-[(2′-Cyanobiphenyl-4-yl)methyl]-N-(3-methoxypropyl)-n-valeriansäureamid und 2,3 g (6,9 mMol) Tri-n-butylzinnazid in 20 ml o-Xylol und flash-chromatographischer Reinigung die Titelverbindung als dickflüssiges Oel mit einem $R_f$-Wert von 0,33 im DC-System B6.

Das Ausgangsmaterial kann beispielsweise auf folgende Weise hergestellt werden:

a) 4-[N-(3-Methoxypropyl)-aminomethyl]-2′-cyanobiphenyl

Analog Beispiel 23 a) erhält man aus 3-Methoxypropylamin die Titelverbindung, die ein Hydrochlorid vom Smp. 183-184° bildet (aus 2-Propanol-Ether).

b) N-[(2′-Cyanobiphenyl-4-yl)-methyl]-N-(3-methoxypropyl)-n-vale riansäureamid

Analog Beispiel 23 b) erhält man aus 25 a) die Titelverbindung. Sie weist im DC-System B7 einen $R_f$-Wert von 0,55 auf und kann roh weiter umgesetzt werden.

Beispiel 26: N-(3-Benzyloxyprop-1-yl)-N-pentanoyl-N-[2′-(1H-tetrazol-5-yl)biphenyl-4-ylmethyl]-amin

5,8 g (13 mMol) der Verbindung 26 b) und 5,3 g (16 mMol) Tri-n-butylzinnazid werden analog Beispiel 23 umgesetzt und aufgearbeitet. Man erhält so die rohe Titelverbindung als Oel, das aus wenig 2-Propanol-Ether zur Kristallisation gebracht wird und dann bei 112-115° schmilzt.

Das Ausgangsmaterial kann beispielsweise auf folgende Weise hergestellt werden:

a) 4-[N-(3-Benzyloxypropyl)-aminomethyl]-2′-cyanobiphenyl

Eine Lösung von 6,0 g (22 mMol) 4-Brommethyl-2′-cyanobiphenyl, 5,8 g (35 mMol) 3-Benzyloxypropylamin und 3,6

g Triethylamin in 50 ml 1,4-Dioxan wird 18 Stunden unter Rückfluss zum Sieden erhitzt. Nach Aufarbeitung analog Beispiel 23 a) erhält man ein Oel, das nach flashchromatographischer Reinigung (Ethanol:Ethylacetat: 1:4) die Titelverbindung ergibt (DC-System B7; Rf-Wert 0,39).

b) N-(3-Benzyloxypropyl)-N-[(2′-cyanobiphenyl-4-yl)methyl]-n-val eriansäureamid
2,0 g (16,7 mMol) n-Valerylchlorid werden unter Kühlung mit einem Wasserbad unter Rühren in eine Lösung von 5,5 g (15,4 mMol) der Verbindung 26 a) und 4,0 g Triethylamin in 40 ml 1,4-Dioxan getropft. Das Reaktionsgemisch wird 5-10 Stunden bei Raumtemperatur gerührt und′wie in Beispiel 23 b) aufgearbeitet. Man erhält so die Titelverbindung als Oel ($R_f$ im System B7: 0,51 ), das für die weitere Umsetzung genügend rein ist.

Beispiel 27: N-(2-Hydroxyethyl)-N-pentanoyl-N-[2′-(1H-tetrazol-5-yl)biphenyl-4-ylmethyl]-amin

Eine Lösung von 2,6 g (5,5 mMol) der in Beispiel 24 beschriebenen Verbindung in 90 ml 1,4-Dioxan wird unter Zusatz von insgesamt 2,0 g Palladium-auf-Kohle-Katalysator (5 %) bei Raumtemperatur solange hydriert, bis in einer DC-Kontrolle (System B6) keine Ausgangsverbindung mehr festzustellen ist (ca. 70 Stunden). Der Katalysator wird abfiltriert, das Filtrat im Vakuum eingedampft und der Rückstand in Ethylacetat gelöst. Durch Waschen der Ethylacetat-Lösung mit Wasser, Trocknen und Eindampfen im Vakuum erhält man einen farblosen Schaum, dessen $^1$H-NMR-Spektrum mit der Struktur der Titelverbindung übereinstimmt und der einen $R_f$-Wert von 0,60 aufweist (DC-System B6).

Beispiel 28: N-(3-Hydroxyprop-1-yl)-N-pentanoyl-N-[2′-(1H-tetrazol-5-yl)biphenyl-4-ylmethyl]-amin

2,7 g (5,5 mMol) der in Beispiel 26 beschriebenen Verbindung werden analog Beispiel 27 hydriert und aufgearbeitet. Man erhält ein gelbliches Oel, das nach flashchromatographischer Reinigung (System S2) die Titelverbindung als farblosen Schaum ergibt, die einen $R_f$-Wert von 0,26 aufweist (System S2).

Beispiel 29: N-(1-Methoxycarbonyl-1-methyl-ethyl)-N-pentanoyl-N-[2′-(1H-tetrazol-5-yl)biphenyl-4-ylmethyl]-amin

Eine Lösung von 9,4 g (24 mMol) rohem 2-Amino-N- [(2′-cyanobiphenyl-4-yl)methyl]-2-methyl-N-valeryl-propansäure-methylester und 9,7 g (29 mMol) Tri-n-butylzinnazid in 120 ml o-Xylol wird 30 Stunden unter Rückfluss zum Sieden erhitzt und dann analog Beispiel 23 aufgearbeitet. Die so als Oel erhaltene, rohe Titelverbindung wird zur Reinigung mit dem System B6 flashchromatographiert. Die so erhaltene Titelverbindung bildet einen Schaum und zeigt einen $R_f$-Wert von 0,39 (System B6).

Das Ausgangsprodukt kann beispielsweise auf folgende Weise erhalten werden:

a) 2-Amino-N-[(2′-cyanobiphenyl-4-yl)methyl]-2-methyl-propansäur e-methylester
Ein Gemisch von 10,9 g (40 mMol) 4-Brommethyl-2′-cyanobiphenyl, 18,4 g (120 mMol) 2-Amino-2-methyl-propansäuremethylester-hydrochlorid (D. Leibfritz et al., Tetrahedron 1982, 38, 2165) und 22 g Kaliumcarbonat in 100 ml Dimethylformamid wird 18-20 Stunden unter Rühren in einem Bad von 80° erwärmt. Die Suspension wird filtriert, das Filtrat im Vakuum eingedampft und der Rückstand zwischen 200 ml Ethylacetat und 50 ml Wasser verteilt. Die organische Phase wird abgetrennt, mit je 30 ml Wasser und Sole gewaschen, getrocknet und eingedampft. Man erhält so die rohe Titelverbindung. Sie bildet ein Hydrochlorid vom Smp. 170-175° (aus 2-Propanol).

b) 2-Amino-N-[(2′-cyanobiphenyl-4-yl)methyl]-2-methyl-N-valerylp ropionsäuremethylester
Eine Lösung von 7,4 g (24 mMol) der Verbindung 29 a) (als Base) und 3,7 g (29 mMol) Ethyldiisopropylamin in 100 ml Methylenchlorid wird unter Rühren tropfenweise mit 3,5 g (29 mMol) Valerylchlorid versetzt. Das Reaktionsgemisch wird 20-25 Stunden bei Raumtemperatur gerührt, bis kein Ausgangsamin mehr im DC festzustellen ist (System B7). Aufarbeitung analog Beispiel 23 b) ergibt die rohe Titelverbindung als gelbliches Oel mit $R_f$ 0,40 (System B7), welches roh weiterverwendet wird.

Beispiel 30: N-(2-Carboxyethyl)-N-pentanoyl-N-[2′-(1H-tetrazol-5-yl)biphenyl-4-ylmethyl]-amin

393 mg N-[(2′-Cyano-biphenyl-4-yl)methyl]-N-valeryl-3-amino-propansäureethylester werden analog Beispiel 1 umgesetzt. Das Rohprodukt wird an Kieselgel 60 (40-63 μm) mit $CH_2Cl_2$-MeOH 95:5 gereinigt, $R_f$ = 0,15 (System N8).

Das Ausgangsmaterial kann beispielsweise folgendermassen hergestellt werden:

a) 3-[(2′-Cyano-biphenyl-4-yl)methylamino]-propansäureethylester
wird analog Beispiel 1 b) aus 4,145 g 2′-Cyanobiphenyl-4-carbaldehyd und 3,135 g 3-Amino-propansäure-ethylester-hydrochlorid erhalten und an Kieselgel 60 (40-63 μm) mit $CH_2Cl_2$-MeOH 95:5 gereinigt, $R_f$ = 0,21 (System N6).

b) N-[(2′-Cyano-biphenyl-4-yl)methyl]-N-valeryl-3-amino-propänsä ure-ethylester
wird analog Beispiel 1 c) aus 1,542 g 3-[(2′-Cyano-biphenyl-4-yl)methylamino]-propansäureethylester erhalten, $R_f$ = 0,66 (System N6).

Beispiel 31: N-(2-Carboxyprop-1-yl)-N-pentanoyl-N-[2′-(1H-tetrazol-5-yl)biphenyl-4-ylmethyl]-amin

785 mg rac-N-[(2′-Cyano-biphenyl-4-yl)methyl]-N-valeryl-3-amino-2-methyl-propansäuremethylester werden analog Beispiel 1 umgesetzt und extraktiv gereinigt, $R_f$ = 0,29 (Sytem N8).
Das Ausgangsmaterial kann beispielsweise folgendermassen hergestellt werden:

a) rac-3-Amino-2-methyl-propansäure-methylester-hydrochlorid
wird aus 10,312 g rac-3-Amino-2-methylpropansäure in 100 ml Methanol durch tropfenweise Zugabe von 7,3 ml Thionylchlorid erhalten, $R_f$ = 0,30 (System N8).

b) rac-3-[(2′-Cyano-biphenyl-4-yl)methylamino]-2-methyl-propansäuremethylester
wird analog Beispiel 1 b) aus 4,145 g 2′-Cyanobiphenyl-4-carbaldehyd und 3,072 g rac-3-Amino-2-methyl-propansäuremethylester-hydrochlorid erhalten und an Kieselgel 60 (40-63 µm) mit $CH_2Cl_2$-MeOH 97:3 gereinigt, $R_f$ = 0,31 (System N6).

c) rac-N-[(2′-Cyano-biphenyl-4-yl)methyl]-N-valeryl-3-amino-2-me thyl-propansäuremethylester
wird analog Beispiel 1 c) aus 1,542 g rac-3-[(2′-Cyano-biphenyl-4-yl)methylamino]-2-methyl-propansäuremethylester erhalten und an Kieselgel 60 (40-63 µm) mit $CH_2Cl_2$-MeOH 98:2 gereinigt, $R_f$ = 0,66 (System N6).

Beispiel 32: N-(1-Carboxy-1-methyl-ethyl)-N-pentanoyl-N-[2′-(1H-tetrazol-5-yl)-biphenyl-4-ylmethyl]-amin

Eine Lösung von 2,6 g (6 mMol) des in Beispiel 29 beschriebenen Esters in 30 ml Methanol wird mit 35 ml wässriger Natronlauge (20 %) versetzt und solange unter Rückfluss und Rühren zum Sieden erhitzt (ca. 35-40 Std.), bis der Ausgangsester im DC (System B6) nicht mehr nachzuweisen ist. Die Lösung wird klar filtriert, das Methanol wird im Vakuum abgedampft und die verbleibende wässrige Lösung mit conc. Salzsäure auf pH 1-2 gebracht. Das ausgefallene Produkt wird mit 200 ml Ethylacetat extrahiert, die organische Phase abgetrennt, mit Sole gewaschen und über $MgSO_4$ getrocknet. Das nach dem Abdampfen des Lösungsmittels isolierte Rohprodukt wird mittels eines Gemisches Methylenchlorid 360 ml, Methanol 40 ml, Wasser 4 ml, Essigsäure 2 ml flashchromatographisch gereinigt. Die einheitlich nur das Produkt enthaltenden Fraktionen werden vereinigt eingedampft und ergeben die Titelverbindung als farblosen Schaum, der im DC (System wie oben erwähnt) einen $R_f$-Wert von 0,33 aufweist.

Beispiel 33: N-(5-Hydroxypent-1-yl)-N-pentanoyl-N-[2′-(1H-tetrazol-5-yl)biphenyl-4-ylmethyl]-amin

Eine Lösung von 6,5 g ( 17 mMol) rohem N-[(2′-Cyanobiphenyl-4-yl)methyl]-N-(5hydroxypentyl)-n-valeriansäureamid und 6,8 g (20,4 mMol) Tri-n-butylzinnazid in 70 ml o-Xylol wird analog Beispiel 23 umgesetzt und aufgearbeitet. Das so erhaltene Rohprodukt wird durch Flash-Chromatographie (System B6) gereinigt. Die das Produkt ($R_f$-Wert 0,20) enthaltenden Fraktionen werden eingedampft. Aus dem so isolierten Ammoniumsalz der Titelverbindung wird das freie Tetrazol mittels 1N Salzsäure freigesetzt und mit Aethylacetat extrahiert. Man erhält so die Titelverbindung als gelblichen, glasartigen Feststoff vom $R_f$-Wert 0,20 (System B6), der aus Ethylacetat kristallin erhalten wird, Smp. 117-118°.
Das Ausgangsmaterial kann beispielsweise auf folgende Weise hergestellt werden:

a) 4-[N-(5-Hydroxypentyl)-aminomethyl]-2′-cyanobiphenyl
Eine Lösung von 6,8 g (25 mMol) 4-Brommethyl-2′-cyanobiphenyl und 12,9 g (125 mMol) 5-Amino-1-pentanol in 50 ml 1,4-Dioxan wird 2-3 Stunden unter Rückfluss zum Sieden erhitzt. Aufarbeitung analog Beispiel 23 a) unter Verwendung von Ethylacetat als Lösungsmittel ergibt die Titelverbindung als Hydrochlorid vom Smp. 189-190° (aus 2-Propanol).

b) N-[(2′-Cyanobiphenyl-4-yl)methyl]-N-(5-hydroxypentyl)-n-valer iansäureamid
Aus 5,1 g (17,3 mMol) der Verbindung 33 a) und 2,3 g (19 mMol) n-Valerylchlorid erhält man unter Verwendung von 9 ml Ethyldiisopropylamin und 50 ml Methylenchlorid analog Beispiel 26b) die Titelverbindung als Oel vom $R_f$ 0,36 (System B7), welches ohne weitere Reinigung weiter umgesetzt wird.

Beispiel 34: N-(1-Carboxyprop-2-yl)-N-pentanoyl-N-[2′-(1H-tetrazol-5-yl)biphenyl-4-ylmethyl]-amin

3,390 g rac-N-[(2′-Cyano-biphenyl-4-yl)methyl]-N-valeryl-3-amino-butansäureethylester werden analog Beispiel 1 umgesetzt und extraktiv gereinigt, $R_f$ = 0,30 (System N8).

Das Ausgangsmaterial kann beispielsweise folgendermassen hergestellt werden:

a) rac-3-[(2′-Cyano-biphenyl-4-yl)methylamino]-butansäure-ethyle ster
wird analog Beispiel 1 b) aus 4,145 g 2′-Cyanobiphenyl-4-carbaldehyd und 4,634 ml rac-3-Amino-butansäure-ethylester erhalten und an Kieselgel 60 (40-63 µm) mit $CH_2Cl_2$-MeOH 98:2 gereinigt, $R_f$ = 0,25 (System N6).

b) rac-N-[(2′-Cyano-biphenyl-4-yl)methyl]-N-valeryl-3-amino-buta nsäure-ethylester
wird analog Beispiel 1 c) aus 7,070 g rac-3-[(2′-Cyano-biphenyl-4-yl)methylamino]butansäure-ethylester erhalten und an Kieselgel 60 (40-63 µm) mit $CH_2Cl_2$-MeOH 99: 1 gereinigt, $R_f$ = 0,36 (System N6).

Beispiel 35: N-(2-Ethoxycarbonyl-3-methyl-but-1-yl)-N-pentanoyl-N-[2′-(1H-tetrazol-5-yl)biphenyl-4-ylmethyl]-amin

2,194 g rac-N- [(2′-Cyano-biphenyl-4-yl)methyl] -N-valeryl-2-(aminomethyl)-3-methylbutansäure-ethylester werden analog Beispiel 1 umgesetzt und an Kieselgel 60 (40-63 µm) mit $CH_2Cl_2$-MeOH gereinigt, $R_f$ = 0,48 (System N8).

Das Ausgangsmaterial kann beispielsweise folgendermassen hergestellt werden:

a) rac-2-[(2′-Cyano-biphenyl-4-yl)methylaminomethyl]-3-methyl-bu tansäure-ethylester
wird analog Beispiel 1 b) aus 4,145 g 2′-Cyanobiphenyl-4-carbaldehyd und 3,180 g rac-2-Aminomethyl-3-methyl-butansäure-ethylester (Miyazaki et al. J. pharm. Soc. Jpn. 77, 415 (1957)) erhalten und an Kieselgel 60 (40-63 µm) mit $CH_2Cl_2$-MeOH 97:3 gereinigt, $R_f$ = 0,48 (System N6).

b) rac-N-[(2′-Cyano-biphenyl-4-yl)methyl]-N-valeryl-2-(aminomethyl)-3-methyl-butansäure-ethylester
wird analog Beispiel 1 c) aus 2,519 g rac-2-[(2′-Cyano-biphenyl-4-yl)methylaminomethyl]-3-methyl-butansäureethylester erhalten und an Kieselgel 60 (40-63 µm) mit $CH_2Cl_2$-MeOH 99: 1 gereinigt, $R_f$ = 0,67 (System N6).

Beispiel 36: N-(2-Carboxy-3-methyl-but-1-yl)-N-pentanoyl-N-[2′-(1H-tetrazol-5-yl)biphenyl-4-ylmethyl]-amin

980 mg rac-N-[(2′-(1H-Tetrazol-5-yl)biphenyl-4-yl)methyl]-N-valeryl-2-(aminomethyl)-3-methyl-bütansäureethylester werden in 3,1 ml 2N NaOH während 72 Stunden auf 100° erhitzt. Neutralisieren mit 3,1 ml 2N HCl und extrahieren mit $CH_2Cl_2$ liefert das Produkt, $R_f$ = 0,30 (System N8).

Beispiel 37: (S)-N-(1-Carboxy-2-methyl-prop-1-yl)-N-pentanoyl-N-[2′-(1H-tetrazol-5-yl)biphenyl-4-ylmethyl]-amin

4,2 g N-Valeryl-N-[(2′-cyanobiphenyl-4-yl)methyl]-(L)-valinbenzylester werden in 40 ml Xylol mit 5,7 g Tri-n-butylzinnazid während 24 Stunden zum Rückfluss erhitzt. Darauf wird zur Trockene eingedampft. Das Rohprodukt wird anschliessend in 40 ml Dioxan aufgenommen, mit 400 mg Palladiumkohle (5%) versetzt und unter Normaldruck bis zur Sättigung hydriert. Es wird vom Katalysator abfiltriert, eingedampft, in Ether aufgenommen und das Produkt mit 18 ml 1N NaOH und 100 ml Wasser extrahiert. Die wässrige Phase wird mit Ether gewaschen und nach Ansäuern mit einem Ueberschuss an 1N Salzsäure mit Essigester extrahiert. Umkristallisieren aus Diisopropylether liefert das reine Produkt vom Smp. 116-117°.

Das Ausgangsmaterial kann beispielsweise folgendermassen hergestellt werden:

a) N-(2′-Cyanobiphenyl-4-yl)methyl]-(L)-valinbenzylester
4,38 g 2′-Cyanobiphenyl-4-carbaldehyd, 8,03 g (L)-Valinbenzylester-Toluolsulfonsäuresalz und 25 g Molekularsieb 5A werden in 80 ml Tetrahydrofuran während 36 Stunden bei Raumtemperatur gerührt und dann auf 0° abgekühlt. Es werden 2, 19 g Natriumcyanoborhydrid (90%), gelöst in 10 ml Methanol, zugegeben, 24 Stunden bei Raumtemperatur gerührt und dann im Vakuum eingeengt. Das Reaktionsgemisch wird darauf filtriert, das Filtrat eingeengt, der Rückstand in Methylenchlorid aufgenommen, dreimal mit Wasser gewaschen, gertrocknet und eingeengt. Der Rückstand wird in Wasser aufgenommen und mit einem Ueberschuss konzentrierter Salzsäure versetzt. Das Produkt wird als Hydrochlorid ausgefällt und abfiltriert. Nach Umkristallisieren aus Essigester/Hexan 1:1 erhält man das reine Produkt vom Smp. 153-155°.

b) N-Valeryl-N-[(2′cyanobiphenyl-4-yl)methyl]-(L)-valinbenzylest er
5,5 g N-[(2′-Cyanobiphenyl-4-yl)methyl]-(L)-valinbenzylester-Hydrochlorid, 4,33 g Diisopropylethylamin und 3 ml Valerylchlorid werden bei Raumtemperatur während 36 Stunden gerührt und anschliessend zur Trockene eingedampft. Der Rückstand wird in Ether aufgenommen, Mit Natriumbicarbonat und Sole gewaschen. Das Rohprodukt wird ohne Reinigung weiterverarbeitet.

Beispiel 38:

In analoger Weise wie vorstehend beschrieben kann man auch die folgenden Verbindungen herstellen:

1. N-(3-Phenoxyprop-1-yl)-N-pentanoyl-N-[2′-(1H-tetrazol-5-yl)biphenyl-4-ylmethyl]amin;
2. N-[2-(4-Hydroxyphenyl)ethyl]-N-pentanoyl-N-[2′-(1H-tetrazol-5-yl)biphenyl-4-ylmethyl]-amin;
3. N-[3-(4-Hydroxyphenyl)prop-1-yl]-N-pentanoyl-N-[2′-(1H-tetrazol-5-yl)biphenyl-4-ylmethyl]-amin;
4. N-(8-Hydroxyokt-1-yl)-N-pentanoyl-N-[2′-(1H-tetrazol-5-yl)biphenyl-4-ylmethyl]amin;
5. N-(2-Methansulfonylaminoethyl)-N-pentanoyl-N-[2′-(1H-tetrazol-5-yl)biphenyl-4-ylmethyl]-amin;
6. N-(3-Acetylaminoprop-1-yl)-N-pentanoyl-N-[2′-(1H-tetrazol-5-yl)biphenyl-4-ylmethyl]-amin;
7. N-(2-Methoxy-2-oxo-1-phenyl-ethyl)-N-pentanoyl-N-[2′-(1H-tetrazol-5-yl)biphenyl-4-ylmethyl]-amin;
8. N-(4-Hydroxybut-2-yl)-N-pentanoyl-N-[2′-(1H-tetrazol-5-yl)biphenyl-4-ylmethyl]amin;
9. N-(2-Hydroxy-1-phenyl-ethyl)-N-pentanoyl-N-[2′-(1H-tetrazol-5-yl)biphenyl-4-ylmethyl]-amin; und
10. N-[3-(4-Hydroxybenzylcarbonylamino)prop-1-yl]-N-pentanoyl-N-[2′-(1H-tetrazol-5-yl)biphenyl-4-ylmethyl]-amin.

Beispiel 39: N-(2-Ethoxycarbonyl-2,2-tetramethylen-ethyl)-N-pentanoyl-N-[2′-(1H-tetrazol-5-yl)biphenyl-4-ylmethyl]-amin

3.75 g N-[(2′-Cyano-biphenyl-4-yl)-methyl]-N-valeryl-1-aminomethyl-c yclopentan-1-carbonsäure-ethylester werden in 200 ml Xylol mit 10.4 g Tri-n-butylzinnazid versetzt und während 41 h zum Rückfluss erhitzt. Darauf wird im Vakuum eingedampft, der Rückstand in 50 ml 2N NaOH-Lösung aufgenommen und 3 mal mit Ether extrahiert. Die wässrige Phase wird sodann mit 30 ml 4N Salzsäure angesäuert und mit Dichlormethan extrahiert. Das Produkt wird durch Eindampfen der zuvor über $Na_2SO_4$ getrockneten organischen Phase als farbloser Schaum erhalten, $R_f$= 0.53 (System N 8). MS (FAB): m/e 490 ($M^+$+H).

Das Ausgangsmaterial kann beispielsweise folgendermassen hergestellt werden:

a) 1-Aminomethyl-cyclopentan-1-carbonsäure-ethylester wird erhalten durch hydrieren von 33 g 1-Cyano-cyclopentan-1-carbonsäure-ethylester (Alfred Bader Chemicals) in 330 ml Ethanol, der ca. 4% Ammoniak enthält, in Gegenwart von 10 g Raney-Nickel bei 45°C und unter Normaldruck. Nach Abfiltrieren vom Katalysator und Entfemen der Lösungsmittel im Vakuum wird das Produkt durch Destillation erhalten, Sdp. 71-74°C bei 0.75 mbar.

b) N-[(2′-Cyano-biphenyl-4-yl)-methyl]-1-aminomethyl-cyclopentan-1-carbonsäure-äthylester wird analog Beispiel 1 b) aus 4.15 g 2′-Cyanobiphenyl-4-carbaldehyd und 4.15 g 1-Aminomethyl-cyclopentan-1-carbonsäure-ethylester erhalten und an Kieselgel 60 (40-63 µm) mit $CH_2Cl_2$/-MeOH (99.5:0.5) gereinigt, $R_f$ = 0.38 (System N 6).

c) N-[(2′-cyano-biphenyl-4-yl)-methyl]-N-valeryl-1-aminomethyl-cyclopentan-1-carbonsäure-äthylester wird analog Beispiel 1 c) aus 4.70 g N-[(2′-Cyano-biphenyl-4-yl)methyl]-1-aminomethyl-cyclopentan-1-carbonsäure-ethylester erhalten und an Kieselgel 60 (40-63 µm) mit $CH_2Cl_2$-MeOH 99.5:0.5 gereinigt, $R_f$ = 0.69 (System N 6).

Beispiel 40: N-(2-Carboxy-2,2-tetramethylen-ethyl)-N-pentanoyl-N-[2′-(1H-tetrazol-5-yl)biphenyl-4-ylmethyl]-amin

0.979 g N-[(2′-(1H-Tetrazol-5-yl)biphenyl-4-yl)methyl]-N-valeryl-1-am inomethyl-cyclopentan-1-carbonsäure-ethylester werden in 10 ml Ethanol gelöst, mit 4 ml 2 N NaOH-Lösung versetzt und während 23 h zum Rückfluss erhitzt. Nach Abkühlen auf Raumtemperatur und Zugabe von 4.5 ml 2N Salzsäure wird eingedampft und das Produkt durch Chromatographie an Kieselgel 60 (40-63 µm) mit $CH_2Cl_2$-MeOH 95:5 isoliert, $R_f$ = 0.35 (System N 8). MS (FAB): m/e 462 ($M^+$+H).

Beispiel 41: N-(3-Ethoxycarbonylcyclohexyl)-N-pentanoyl-N-[2′-(1H-tetrazol-5-yl)biphenyl-4-ylmethyl]-amin und N-(3-Carboxycyclohexyl)-N-pentanoyl-N-(2′-(1H-tetrazol-5-yl)biphenyl-4-ylmethyl]-amin

0.661 g N-[(2′-Cyano-biphenyl-4-yl)methyl]-N-valeryl-3-amin o-cyclohexan-1-carbonsäure-ethylester werden analog Beispiel 1 umgesetzt und extraktiv gereinigt. Das Rohprodukt wird an Kieselgel 60 (40-63 µm) mit $CH_2Cl_2$-MeOH 95:5 gereinigt, $R_f$ = 0.33 (System N 8) für die Säure und $R_f$ = 0.67 (System N 8) für den Ester. MS (FAB): m/e 462 ($M^+$+H), 484 ($M^+$+Na) bzw. m/e 490 ($M^+$ +H), 512 ($M^+$+Na).

Das Ausgangsmaterial kann beispielsweise folgendermassen hergestellt werden:

a) <u>rac-3-[2′-Cyano-biphenyl-4-yl)methylamino]-cyclohex an-1-carbonsäure-ethylester</u> wird aus 2.711 g 4-Brom-methyl-2′-cyano-biphenyl und 2.055 g 3-Amino-cyclohexan-1-carbonsäure-ethylester in Gegenwart von N-Methyl-morpholin bei 10 minütigem Erhitzen auf 160°C erhalten. Das Rohprodukt wird an Kieselgel 60 (40-63 µm) mit $CH_2Cl_2$-MeOH 9: 1 gereinigt, $R_f$ = 0.73 (System N 8).

b) <u>rac-N-[(2′-Cyano-biphenyl-4-yl)methyl]-N-valeryl-3- amino-cyclohexan-1-carbonsäure-ethylester</u> wird analog Beispiel 1 c) aus 0.766 g rac-3-[(2′-Cyano-biphenyl-4-yl)methylamino]-cyclohexan-1-carbonsäure-eth ylester erhalten und an Kieselgel 60 (40-63 µm) mit $CH_2Cl_2$-MeOH 99.5:0.5 gereinigt, $R_f$ = 0.56 (System N 6).

<u>Beispiel 42:</u> <u>cis-N-(4-Carboxycyclohexyl)-N-pentanoyl-N-[2′-(1H-tetrazol-5-yl)biphenyl-4-ylmethyl]-amin</u>

2.700 g cis-N-[(2′-Cyano-biphenyl-4-yl)methyl]-N-valeryl-4-amino-cycl ohexan-1carbonsäure-ethylester werden analog Beispiel 1 umgesetzt und extraktiv gereinigt. $R_f$ = 0.40 (System N 8). MS (FAB): m/e 462 ($M^+$+H).

Das Ausgangsmaterial kann beispielsweise folgendermassen hergestellt werden:

a) <u>cis-4-[(2′-Cyano-biphenyl-4-yl)methylamino]-cyclohexan-1-carb onsäure-ethylester</u> wird analog Beispiel 1 b) aus 4.145 g 2′-Cyanobiphenyl-4-carbaldehyd und 5.137 g 4-Amino-cyclohexan-1-carbonsäure-ethylester erhalten und an Kieselgel 60 (40-63 µm) mit $CH_2Cl_2$-MeOH 99.5:0.5 gereinigt, $R_f$ = 0.18 (System N 6).

b) <u>cis-N-[(2′-Cyano-biphenyl-4-yl)methyl]-N-valeryl-4-amino-cycl ohexan-1-carbonsäure-ethylester</u> wird analog Beispiel 1c aus 2.540 g cis-4-[(2′-Cyano-biphenyl-4-yl)methylamino]-cyclohexan-1-carbonsäure-ethylester erhalten und an Kieselgel 60 (40-63 µm) mit $CH_2Cl_2$-MeOH 98:2 gereinigt, $R_f$ = 0.32 (System N 6).

<u>Beispiel 43:</u> <u>cis-N-(2-Ethoxycarbonylcyclohexyl)-N-pentanoyl-N-[2′-(1H-tetrazol-5-yl)biphenyl-4-ylmethyl]-amin</u>

1.350 g rac-cis-N-[(2′-Cyano-biphenyl-4-yl)methyl]-N-valeryl-2-amino-cyclohexan-1-carbonsäure-ethylester werden analog Beispiel 1 umgesetzt. Das Rohprodukt wird an Kieselgel 60 (40-63 µm) mit $CH_2Cl_2$-MeOH 95:5 gereinigt, $R_f$ = 0.53 (System N 8). MS (FAB): m/e 490 ($M^+$+H).

Das Ausgangsmaterial kann beispielsweise folgendermassen hergestellt werden:

a) <u>rac-cis-2-[(2′-Cyano-biphenyl-4-yl)methylamino]-cyclohexan-1- carbonsäure-ethylester</u> wird analog Beispiel 1 b) aus 4.145 g 2′-Cyanobiphenyl-4-carbaldehyd und 5.137 g rac-cis-2-Amino-cyclohexan-1-carbonsäure-ethylester erhalten und an Kieselgel 60 (40-63 µm) mit $CH_2Cl_2$-MeOH 99:1 gereinigt, $R_f$ = 0.24 (System N 6).

b) <u>rac-cis-N-[(2′-Cyano-biphenyl-4-yl)methyl]-N-valeryl-2-amino-cyclohexan-1-carbonsäure-ethylester</u> wird analog Beispiel lc) aus 2.110 g rac-cis-2-[(2′-Cyano-biphenyl-4-yl)methylamino]-cyclohexan-1-carbonsäure-äthylester erhalten und an Kieselgel 60 (40-63 µm) mit $CH_2Cl_2$-MeOH 98:2 gereinigt, $R_f$ = 0.35 (System N 6).

<u>Beispiel 44:</u> <u>cis-N-(2-Carboxycyclohexyl)-N-pentanoyl-N-[2′-(1H-tetrazol-5-yl)biphenyl-4-ylmethyl]-amin</u>

649 mg rac-cis-N-[(2′-(1H-Tetrazol-5-yl)biphenyl-4-yl)methyl]-N-valeryl-2-aminocyclohexan-1-carbonsäure-ethylester werden zusammen mit 10 ml Ethanol und 2 ml 2 N NaOH während 18 Std. auf 80° erhitzt. Die Mischung wird mit 2 ml 2 N HCl neutralisiert und eingedampft. Das Rohprodukt wird an Kieselgel 60 (40-63 µm) mit $CH_2Cl_2$-MeOH (95:5) gereinigt, $R_f$ = 0.30 (System N 8). MS (FAB): m/e 462 ($M^+$+H), 484 ($M^+$+Na).

<u>Beispiel 45:</u> <u>N-(2-Ethoxycarbonyl-2-ethyl-but-1-yl)-N-pentanoyl-N-[2′-(1H-tetrazol-5-yl)biphenyl-4-ylmethyl]-amin</u>

3.28 g N-[(2′-Cyano-biphenyl-4-yl)methyl]-N-valeryl-2-aminomethyl-2- ethyl-buttersäure-ethylester werden analog Beispiel 1 umgesetzt und extraktiv gereinigt. $R_f$=0.52 (System N 8). MS (FAB): m/e 492 ($M^+$+H), 514 ($M^+$+Na).

Das Ausgangsmaterial kann beispielsweise folgendermassen hergestellt werden:

a) <u>2-Aminomethyl-2-ethyl-buttersäure-ethylester</u> wird erhalten durch hydrieren von 12.83 g 2-Ethyl-2-cyano-buttersäure-ethylester (Pfaltz . Bauer Inc.) in 130 ml Ethanol, der 4% Ammoniak enthält, in Gegenwert von 4 g Raney-Nickel bei 44°C und unter Normaldruck. Nach Abtrennen vom Katalysator wird im Vakuum eingedampft und die dabei zurückbleibende Flüssigkeit im Vakuum destilliert. Sdp. 60-61°C bei 0.70 mbar.

b) <u>N-[(2′-Cyano-biphenyl-4-yl)methyl]-2-aminomethyl-2-ethyl-butt ersäure-ethylester</u> wird aus 2.711 g 4-Brom-methyl-2′-cyano-biphenyl und 4.332 g 2-Aminomethyl-2-ethyl-buttersäure-ethylester analog Beispiel 41 a) erhalten und an Kieselgel 60 (40-63 µm) mit $CH_2Cl_2$-MeOH 97:3 gereinigt, $R_f$ = 0.54 (System N 6).

c) N-[(2′-Cyano-biphenyl-4-yl)methyl]-N-valeryl-2-aminomethyl-2- ethyl-buttersäure-ethylester wird analog Beispiel 1c) aus 3.256 g N-[(2′-Cyano-biphenyl-4-yl) methyl]-2-aminomethyl-2-ethyl-buttersäure-ethylester erhalten und an Kieselgel 60 (40-63 µm) mit $CH_2Cl_2$-MeOH 99: 1 gereinigt, $R_f$ = 0.67 (System N 6).

Beispiel 46: N-(2-Ethoxycarbonyl-2-methyl-prop-1-yl)-N-pentanoyl-N-[2′-(1H-tetrazol-5-yl)biphenyl-4-ylmethyl]-amin

4.21 g N-[(2′-Cyano-biphenyl-4-yl)methyl]-N-valeryl-3-amino-2,2-dime thylpropionsäure-ethylester werden analog Beispiel 1 umgesetzt. Das Rohprodukt wird an Kieselgel 60 (40-63 µm) mit $CH_2Cl_2$-MeOH 9: 1 gereinigt, $R_f$ = 0.60 (System N 8). MS (FAB): m/e 464 ($M^+$+H), 486 ($M^+$+Na).

Das Ausgangsmaterial kann beispielsweise folgendermassen hergestellt werden:

a) N-[(2′-Cyano-biphenyl-4-yl)methyl]-3-amino-2,2-dimethyl-propi onsäure-ethylester wird aus 2.711 g 4-Brom-methyl-2′-cyano-biphenyl und 3.630 g 3-Amino-2,2-dimethylpropionsäure-ethylester analog Beispiel 41a) erhalten und als Rohprodukt weiterverwendet, $R_f$ = 0.54 (System N 6).

b) N-[(2′-Cyano-biphenyl-4-yl)methyl]-N-valeryl-3-amino-2,2-dime thyl-propionsäure-ethylester wird analog Beispiel 1c) aus 3.36 g N-[(2′-Cyano-biphenyl-4-yl)methyl]3-amino-2,2-dimethyl-propionsäure-ethylester erhalten und extraktiv gereinigt, $R_f$ = 0.63 (System N 6).

Beispiel 47: N-{2-[2-(4-Hydroxyphenyl)ethylaminocarbonyl]-2,2-tetramethylen-ethyl}N-pentanoyl-N-(2′-(1H-tetrazol-5-yl)biphenyl-4-ylmethyl]-amin

0.507 g N-[(2′-(1H-Tetrazol-5-yl)biphenyl-4-yl)methyl]-N-valeryl-1-am inomethylcyclopentan-1-carbonsäure wird in 4 ml DMF gelöst und mit 0.210 g Tyramin-hydrochlorid, 0.225 ml Hünig-Base und 0.164 g HOBT versetzt. Das Gemisch wird auf O°C gekühlt und es werden 0.274 g EDCI hinzugefügt. Nach 48 stündigem Rühren bei Raumtemperatur wird im Vakuum eingedampft, der Rückstand in 75 ml Essigester aufgenommen und mit 25 ml 1 N Salzsäure gewaschen. Die organische Phase wird über $Na_2SO_4$ getrocknet und im Vakuum von den Lösungsmittel befreit. Das so erhaltene Rohprodukt wird an Kieselgel 60 (40-63 µm) mit $CH_2Cl_2$-MeOH 95:5 gereinigt, $R_f$ = 0.43 (System N 8). MS (FAB): m/e 581 ($M^+$+H), 603 ($M^+$+Na).

Beispiel 48: (S)-N-{1-[2-(4-Hydroxyphenyl)ethylaminocarbonyl]-2-methyl-prop-1-yl}-N-pentanoyl-N-(2′-(1H-tetrazol-5-yl)bi-phenyl-4-ylmethyl]-amin

0,5 g der Verbindung aus Beispiel 16, 0,21 g Tyramin Hydrochlorid, 0,225 ml N-Aethyldiisopropylamin, 0,164 g 1-Hydroxybenzotriazol und 0,296 g Dicyclohexylcarbodiimid werden während 48 h in 4 ml DMF bei Raumtemperatur gerührt. Nach Abdampfen des Lösungsmittels im Vakuum wird der Rückstand während 1 h in einem Gemisch von 4 ml $CH_2Cl_2$-MeOH-AcOH 94:3:3 verrührt. Nach Eindampfen wird mittels Flashchromatographie aufgetrennt (100 g, System N6). Nach Lyophilisieren aus tert.-Butanol erhält man das Produkt als amorphes Pulver. FAB-MS: m/e = 555 $(M+H)^+$.

Beispiel 49: (S)-N-(1-Carboxy-2,2-dimethyl-prop-1-yl)-N-pentanoyl-N-[2′-(1H-tetrazol-5-yl)biphenyl-4-ylmethyl]-amin

Ausgehend von 240 mg N-Valeryl-N-[(2′-cyanobiphenyl-4-yl)-methyl]-(L)-tert.-leuci nmethylester und 399 mg Tributylzinnazid wird nach Flashchromatographie (B2) das Produkt erhalten. Smp. 122-124°.

Das Ausgangsmaterial kann beispielsweise wie folgt erhalten werden:

a) N-(2′-Cyanobiphenyl-4-yl)-methyl]-(L)-tert.-leucinmethylester ausgehend von 2,5 g 2′-Cyanbiphenyl-4-carbaldehyd, 4,39 g (L)-tert.-Leucinmethylester Hydrochlorid, 895 mg Natriumcyanoborhydrid (85 %) und 12,5 g Molekularsieb 5A und anschliessender Flashchromatographie mit System N3. (DC-System N2) $R_f$-Wert: 0,58.

b) N-Valeryl-N-[(2′-cyanobiphenyl-4-yl)-methyl]-(L)-tert.-leucinmethylester ausgehend von 1,2 g N-(2′-Cyanobiphenyl-4-yl)-methyl]-(L)-tert.-leucinmethylester, 0,65 ml Triethylamin und 0,565 ml n-Valeriansäurechlorid und anschliessender Flashchromatographie (N4). (DC-System N3) $R_f$-Wert: 0,56.

Beispiel 50: (S)-N-(1-Methoxycarbonyl-2-methyl-prop-1-yl)-N-pentanoyl-N-[2′-(1H-tetrazol-5-yl)biphenyl-4-ylmethyl]-amin

0,8 g N-Valeryl-N-[(2′-(1H-tetrazol-5-yl)biphenyl-4-yl)-methyl]-(L )-valinmethylester wird erhalten analog Beispiel 3 ausgehend von 4,4 g N-Valeryl-N-[(2′-(1H-tetrazol-5-yl)biphenyl-4-yl)-methyl]-(L)-valin die in MeOH/HCl verestert werden. Flashchromatographie (Essigester/Hexan 1:3). FAB-MS: m/e = 450 $(M+H)^+$.

Beispiel 51: (S)-N-(1-Hydroxymethyl-2-methyl-prop-1-yl)-N-pentanoyl-N-[2′-(1H-tetrazol-5-yl)biphenyl-4-ylmethyl]-amin

0,8 g N-Valeryl-N-[(2′-(1H-tetrazol-5-yl)biphenyl-4-yl)-methyl]-(L) - valinmethylester werden in 30 ml THF gelöst, bei 5°C mit 83 mg Lithiumborhydrid versetzt und während 24 h bei Raumtemperatur gerührt. Das Reaktionsgemisch wird darauf eingeengt, mit Wasser versetzt, mit Salzsäure auf pH 2 gestellt, wobei eine weisse Fällung eintritt. Es wird mit Essigester extrahiert, mit Wasser und Sole gewaschen, getrocknet und schliesslich mittels Flashchromatographie aufgetrennt ($CH_2Cl_2$-MeOH 5: 1 ). FAB-MS:m/e = 422 $(M+H)^+$.

Beispiel 52: N-(4-Phenoxybut-1-yl)-N-pentanoyl-N-[2′-(1H-tetrazol-5-yl)biphenyl-4-ylmethyl]-amin

3,3 g (7,5 mMol) rohes N-[(2′-Cyanobiphenyl-4-yl)-methyl]-N-(4-phenoxybutyl)-n-valeriansäureamid und 3,0 g (9 mMol) Tri-n-butylzinnazid werden analog Beispiel 23 umgesetzt und aufgearbeitet. Man erhält so die Titelverbindung, die durch Flashchromatographie (Toluol-Methanol 4: 1 ) noch gereinigt wird, als dickflüssiges Oel, $R_f$ 0,50 (System B6).

Das Ausgangsmaterial kann beispielsweise auf folgende Weise hergestellt werden:

a) 4-[N-(4-Phenoxybutyl)-aminomethyl]-2′-cyanobiphenyl.
Analog Beispiel 23a erhält man aus 4-Phenoxybutylamin die Titelverbindung, deren Hydrochlorid bei 103-104° schmilzt (aus Isopropanol-Aethylacetat).

b) N-[(2′-Cyanobiphenyl-4-yl)-methyl]-N-(4-phenoxybutyl)-n-valer iansäureamid.
Analog Beispiel 23b erhält man aus der unter a) beschriebenen Verbindung die Titelverbindung als gelbes Oel vom $R_f$-Wert 0,71 (System B7), das roh weiterverwendet wird.

Beispiel 53: N-(2-Hydroxy-1-phenyl-2-oxo-ethyl)-N-pentanoyl-N-[2′-(1H-tetrazol-5-yl)biphenyl-4-ylmethyl]-amin

Analog Beispiel 1 werden 11,0 g (21 mMol) N-[(2′-Cyanobiphenyl-4-yl)-methyl]-N-valeryl-phenylglycin-benzylester mit 8,5 g (25,5 mMol) Tri-n-butylzinnazid in 60 ml o-Xylol umgesetzt und anschliessend 3 Stunden mit 100 ml 2-n.KOH hydrolysiert. Durch Ansäuern der wässrigen Phase mit 2-n.Salzsäure und Extraktion mit Toluol erhält man die rohe Titelverbindung, die aus wenig Toluol kristallin erhalten wird. Die so erhaltenen Kristalle vom Smp. 145-148 ° enthalten 1/3 Mol-Aequivalent Toluol.

Das Ausgangsmaterial kann beispielsweise wie folgt hergestellt werden:

a) rac. N-[(2′-Cyanobiphenyl-4-yl)-methyl]-phenylglycin-benzylester
24,8 g (60 mMol) rac. Phenylglycin-benzylester-tosylat und 8,2 g (30 mMol) 4-Brommethyl-2′-cyanobiphenyl werden zusammen mit 15,5 g Diisopropylethylamin (Hünigbase) in 60 ml DMF 2 Stunden unter Rühren bei 80° gehalten. Das Reaktionsgemisch wird dann auf Eiswasser gegossen und mit Aethylacetat extrahiert. Das Aethylacetat wird abgetrennt und mit 2-n.Salzsäure verrührt. Das ölig ausfallende Hydrochlorid der Titelverbindung wird abgetrennt, mit Sodalösung in die Base übergeführt und roh weiterverwendet ($R_f$ 0,65 in System B7).

b) N-[(2′-Cyanobiphenyl-4-yl)-methyl]-N-valeryl-phenylglycin-ben zylester
9,4 g (21,7 mMol) der rohen, unter a) beschriebenen Verbindung wird zusammen mit 5,7 g (44 mMol) Hünigbase in 45 ml Methylenchlorid gelöst und mit 3,14 g (26 mMol) Valeriansäurechlorid versetzt. Die Lösung wird 30-40 Stunden stehen gelassen. Aufarbeitung analog Beispiel Ic ergibt die rohe Titelverbindung als dickflüssiges Oel mit Rf-Wert 0,73 (System B7), welches roh weiterverwendet wird.

Beispiel 54: (S)-N-(1-Carboxy-2-methyl-prop-1-yl)-N-pentanoyl-N-[2′-(1H-tetrazol-5-yl)-biphenyl-4-ylmethyl]-amin

Eine Lösung von 21,1 g (40 mMol) N-[(2′-(1H-Tetrazol-5-yl)biphenyl-4-yl)methyl]-N-valeryl-(L)-valinbenzylester in 210 ml Methanol wird unter Zusatz von 4 g Pd/C (10 %) bis zur Aufnahme der berechneten Menge Wasserstoff bei Raumtemperatur hydriert (24 Stunden). Durch Filtration und Eindampfen der Lösung erhält man die rohe Säure. Sie wird zwischen 80 ml 2-n.Kalilauge und 50 ml Aether verteilt. Die wässrige Phase wird abgetrennt, sauer gestellt und die Titelverbindung durch Extraktion mit Ethylacetat isoliert. Sie wird aus Ethylacetat kristallin erhalten und zeigt einen Schmelzintervall von 105-115 ° und eine optische Drehung $[\alpha]_D^{20}$ -69,95°±0,05° (c = 1 % in Methanol).

Das Ausgangsmaterial kann beispielsweise wie folgt hergestellt werden:

a) N-[(2′-Cyanobiphenyl-4-yl)-methyl] -(L)-valinbenzylester
Eine Lösung von 13,6 g (50 mMol) 4-Brommethyl-2′-cyanobiphenyl, 22,8 g (60 mMol) (L)-ValOBz-Tosylat und 34 ml Hünigbase in 100 ml DMF wird 1 Stunde bei 80° gerührt. Das Reaktionsgemisch wird dann abgekühlt, auf 300 ml Eiswasser gegossen und mit 150 ml Ethylacetat extrahiert. Durch Waschen des Extraktes mit wässriger Kaliumbicarbonatlösung, Trocknen und Eindampfen erhält man die rohe Titelverbindung als Oel, das ein Hydrochlorid vom Smp. 172-173° bildet.

b) N-[(2′-Cyanobiphenyl-4-yl)-methyl]-N-valeryl-(L)-valinbenzyle ster
6,2 g ( 15,5 mMol) N-[(2′-Cyanobiphenyl-4-yl)-methyl] -(L)-valinbenzylester und 8,0 ml Hünigbase, gelöst in 50 ml Methylenchlorid werden unter Rühren mit 2,3 ml Valeriansäurechlorid versetzt und analog Beispiel 29b weiterbearbeitet. Man erhält so die Titelverbindung als gelbes Oel, das roh weiterverarbeitet wird ($R_f$-Wert 0,51, Toluol-Methanol 19: 1)

c) (S)-N-(1-Benzyloxycarbonyl-2-methyl-prop-1-yl)-N-pentanoyl-N-[2′-(1H-tetrazol-5-yl)biphenyl-4-ylmethyl]-amin
6,6 g (13,6 mMol) roher N-[(2′-Cyanobiphenyl-4-yl)-methyl]-N-valeryl-(L)-valinbenzylester und 6,0 g (18 mMol) Tributylzinnazid werden in 75 ml o-Xylol 48 Stunden unter Rühren zum Sieden erhitzt. Nach 24 Stunden erfolgt ein Zusatz von 2,0 g Tributylzinnazid. Aufarbeitung analog Beispiel 23 unter Verwendung von 110 ml 1-n.Kalilauge während 20 Minuten ergibt die Titelverbindung als gelbliches Oel, das einen $R_f$-Wert von 0,40 (System S2) und eine optische Drehung $[\alpha]_D^{20}$ - 36,6° (c = 1 % in Methanol) aufweist.

Beispiel 55: (S)-N-(1-Benzyloxycarbonyl-2-methyl-prop-1-yl)-N-pentanoyl-N-[2′-(1H-tetrazol-5-yl)biphenyl-4-ylmethyl]-amin

Eine Lösung von 91 g (ca. 100 mMol) rohem N-[(2′-(1-Triphenylmethyl-tetrazol-5-yl)biphenyl-4-yl)methyl]-N-valeryl-(L)-valinbenzylester in 300 ml Dioxan wird bei 60° mit 300 ml 1-n.Salzsäure versetzt und 2 Stunden bei 60° gehalten. Das Dioxan wird hierauf im Vakuum abgedampft und die wässrige Phase mit 2-n.Kalilauge alkalisch gestellt. Neutrale Teile werden mit Aether extrahiert. Die Wasserphase ergibt durch Ansäuern und Extraktion mit Ethylacetat die rohe Titelverbindung als Oel ($R_f$ 0,40 im System S2).
Das Ausgangsmaterial kann beispielsweise wie folgt hergestellt werden:

a) N-[(2′-(1-Triphenylmethyl-tetrazol-5-yl)biphenyl-4-yl)methyl] - (L)-valinbenzylester
Analog Beispiel 57a erhält man aus 4-Brommethyl-2′-(1-triphenylmethyl-tetrazol-5-yl)biphenyl die Titelverbindung ($R_f$ 0,78 im System B6), die roh weiterverwendet wird.

b) N-[(2′-(1-Triphenylmethyl-tetrazol-5-yl)biphenyl-4-yl)methyl]-N-valeryl-(L)-valinbenzylester
Die unter a) erwähnte Verbindung wird mit 2,5 Aequivalenten Valeriansäurechlorid und 5 Aequivalenten Hünigbase in Methylenchlorid analog Beispiel 29b umgesetzt und aufgearbeitet. Die so erhaltene Titelverbindung wird roh weiterumgesetzt.

Beispiel 56: N-Butanoyl-N-(1-carboxy-1-methyl-ethyl)-N-[2′-(1H-tetrazol-5-yl)biphenyl-4-ylmethyl]-amin

Eine Lösung von 2,1 g (4,2 mMol) 2-Amino-N-butyryl-2-methyl-N-[(2′-(1H-tetrazol-5-yl) biphenyl-4-yl)methyl]-propansäurebenzylester in 20 ml Methanol wird unter Zusatz von 0,2 g Pd/C (10 %) bei 5 bar Anfangsdruck hydriert, bis der Ausgangsbenzylester im DC (System B6, S2) nicht mehr nachzuweisen ist. Durch Filtrarion, Abdampfen des Lösungsmittels und Umkristallisation des Rückstandes aus $CH_3CN$ erhält man die Titelverbindung vom Smp. 187-189°.
Das Ausgangsmaterial kann beispielsweise wie folgt hergestellt werden:

a) 2-Amino-N-(2′-cyanobiphenyl-4-ylmethyl)-2-methyl-propansäure-benzylester
Analog Beispiel 29a erhält man unter Verwendung von 2-Amino-2-methyl-propansäurebenzylester-tosylat die Titelverbindung, die ein Hydrochlorid vom Smp. 200-202° (Ethylacetat-4-n.HCl in absolutem Ethanol) bildet.

b) 2-Amino-N-butyryl-N-(2′-cyanobiphenyl-4-ylmethyl)-2-methylpropansäure-benzylester
Eine Lösung von 6,3 g (15 mMol) des Hydrochlorids der unter a) beschriebenen Verbindung und 10,2 ml (60 mMol) Hünigbase in 60 ml Methylenchlorid wird mit 1,8 g (16 mMol) Buttersäurechlorid versetzt und über Nacht gerührt. Durch weitere Zusätze von Säurechlorid und Hünigbase wird die Reaktion vervollständigt. Aufarbeitung analog Beispiel 23b ergibt die Titelverbindung, die roh weiterumgesetzt wird.

c) 2-Amino-N-butyryl-2-methyl-N-[(2′-(1H-tetrazol-5-yl)biphenyl 4-yl)-methyl]-propansäure-benzylester
Aus der unter b) beschriebenen Verbindung (6 g, roh) und 5,2 g Tributylzinnazid in 50 ml o-Xylol erhält man analog Beispiel 23 die Titelverbindung vom Smp. 203-204° (aus Ethylacetat).

Beispiel 57: N-(4-Hydroxybut-1-yl)-N-pentanoyl-N-[2′-(1H-tetrazol-5-yl)biphenyl-4-ylmethyl]-amin

Analog Beispiel 33 erhält man aus N-[(2′-Cyanobiphenyl-4-yl)methyl]-N-(4-hydroxybutyl)-n-valeriansäureamid die Titelverbindung vom Smp. 110-111° (aus Ethylacetat).

Das Ausgangsmaterial kann beispielsweise wie folgt hergestellt werden:

a) 4-[N-(4-Hydroxybutyl)-aminomethyl]-2′-cyanobiphenyl
Analog Beispiel 33a) erhält man unter Verwendung von 4-Aminobutanol die Titelverbindung als Oel ($R_f$ 0,18 in System B7), das roh weiterverwendet wird.

b) N-[(2′-Cyanobiphenyl-4-yl)-methyl]-N-(4-hydroxybutyl)-n-valeriansäureamid
Analog Beispiel 33b) erhält man aus der unter a) beschriebenen Verbindung die Titelverbindung als Oel ($R_f$ 0,37) das roh weiter umgesetzt wird.

Beispiel 58: (S)-N-(1-Benzyloxycarbonyl-2-methyl-prop-1-yl)-N-[3-brom-2′-(1H-tetrazol-5-yl)-biphenyl-4-ylmethyl]-N-pentanoyl-amin

Eine Lösung von 4,5 g (8 mMol) N-[(3-Brom-2′-cyanobiphenyl-4-yl)methyl]-N-valeryl(L)-valinbenzylester und 3,4 g (10,4 mMol) Tributylzinnazid in 50 ml Xylol wird 20 Stunden unter Rückfluss zum Sieden erhitzt. Aufarbeitung analog Beispiel 54 und "flash"-Chromatographische Reinigung (Toluol-Methanol 4: 1 ) ergibt die Titelverbindung als farblosen Schaum ($R_f$-Wert 0,57, System S2).

Das Ausgangsmaterial kann beispielsweise wie folgt hergestellt werden:

a) 3′-Brom-4′-methyl- 1,1′-biphenyl-2-carbonitril
Eine Suspension von 21,0 g (0,157 Mol) wasserfreiem Aluminiumchlorid in 800 ml Tetrachloräthan wird mit 25,0 g (0,129 Mol) 4′-Methyl-1,1′-biphenyl-2-carbonitril versetzt und unter Rühren auf 60° Innentemperatur gebracht. Sobald das Aluminiumchlorid in Lösung gegangen ist, wird bei 60° Innentemperatur eine Lösung von 20,7 g (0,129 Mol) Brom in 100 ml Tetrachloräthan zugetropft. Das Reaktionsgemisch wird 24 Stunden bei 60° gerührt. Nach Zusatz von weiteren 6,2 g Aluminiumchlorid und Erwärmen auf 60-70° lässt sich in DC (Toluol) kein Ausgangsmaterial mehr feststellen. Das Reaktionsgemisch wird hierauf unter Eiskühlung mit 20 ml conc. Salzsäure zersetzt, die organische Phase abgetrennt und im Vakuum eingedampft. Der dunkle Rückstand wird in Aethylacetat gelöst, mit Wasser und Natriumcarbonat-Lösung gewaschen, getrocknet ($MgSO_4$) und eingedampft. Das Rohprodukt wird flash-chromatographisch gereinigt, wodurch 22,0 g (62 % d. Th.) der Titelverbindung erhalten werden, Smp. 104-106° (aus Cyclohexan).

b) 3′-Brom-4′-brommethyl-1,1′-biphenyl-2-carbonitril
In eine Lösung von 8,9 g (0,033 Mol) 3′-Brom-4′-methyl-1,1′-biphenyl-2-carbonitril in 900 ml Tetrachloräthan werden nach Zugabe von 0,1 g Benzoylperoxid unter UV-Bestrahlung bei 100-110° 5,6 g (0,035 Mol) Brom, gelöst in 20 ml Tetrachloräthan, getropft. Nach 30 Minuten wird das Reaktionsgemisch abgekühlt und im Vakuum eingedampft. Der kristalline Rückstand wird aus Aethylacetat umkristallisiert und ergibt 4,1 g der Titelverbindung vom Smp. 152-153°.

c) N-[(3-Brom-2′-cyanobiphenyl-4-yl)methyl]-(L)-valin-benzyleste r
Eine Lösung von 4,63 g (12,2 mMol) (L)-Valinbenzylester-tosylat und 4,8 ml Hünig-Base in 20 ml DMF wird mit einer Lösung von 3,3 g (9,4 mMol) der unter b) beschriebenen Verbindung versetzt und 4 Stunden bei 100° gerührt. Aufarbeitung analog Beispiel 54a und "flash"-chromatographische Reinigung (n-Hexan-Ethylacetat 4: 1 ) führen zur Titelverbindung als rotbraunem Oel mit $R_f$ 0,21 (n-Hexan-Ethylacetat 4: 1).

d) N-[(3-Brom-2′-cyanobiphenyl-4-yl)methyl]-N-valeryl-(L)-valin benzylester
Aus der unter c) erwähnten Verbindung erhält man analog Beispiel 54b die Titelverbindung als gelbes Oel mit $R_f$ 0,17 (n-Hexan-Ethylacetat).

Beispiel 59: (S)-N-[3-Brom-2′-(1H-tetrazol-5-yl)-biphenyl-4-ylmethyl]-N-(1-carboxy-2-methyl-prop-1-yl)-N-pentanoyl-amin

Eine Lösung von 2,4 g (4 mmol) N-[(3-Brom-2′-(1H-tetrazol-5-yl)biphenyl-4-yl)methyl]-N-valeryl-(L)-valinbenzylester in 90 ml Dioxan wird unter Zusatz von 1,2 g Pd/C (10 %) bei 5 bar und Zimmertemperatur bis zur Aufnahme der berechneten Menge Wasserstoff hydriert. Nach dem Eindampfen der filtrierten Lösung wird der Eindampfrückstand in 2-n.Natronlauge gelöst, mit Aether extrahiert und die Wasserphase mit 2-n.Salzsäure sauer gestellt. Durch Extraktion mit

Ethylacetat, Trocknen und Eindampfen erhält man die Titelverbindung als farblosen Schaum ($R_f$ 0,40, System S2), FAB-MS: m/e = 514 $(M+H)^+$.

Beispiel 60: N-(2-Acetylaminoethyl)-N-pentanoyl-N-[2′-(1H-tetrazol-5-yl)biphenyl-4-ylmethyl]-amin

9,9 g (22 mMol) rohes N-(2-Acetylamino-ethyl)-N-[2′-cyanobiphenyl-4-yl)methyl]-n-valeriansäureamid und 12,3 g (37 mMol) Tributylzinnazid werden in 100 ml Xylolgemisch 30 Stunden unter Rückfluss erhitzt. Der sich abscheidende Niederschlag wird nach dem Abkühlen durch Dekantieren isoliert und anschliessend durch Verrühren zwischen 100 ml Ether und 100 ml 1-n.Kalilauge in Lösung gebracht (3-4 Stunden). Aus der wässrigen, alkalischen Phase wird die Titelverbindung durch Ansäuern mit 2-n.HCl und Extraktion mit viel Ethylacetat isoliert und durch "flash"-Chromatographie (System S2) gereinigt. Man erhält so die Titelverbindung als Feststoff mit einem Schmelzintervall von 74-80°.

Das Ausgangsmaterial kann beispielsweise wie folgt hergestellt werden:

a) N-[2-2′-Cyanobiphenyl-4-yl)methylamino)ethyl]-acetamid
Aus 9,2 g (90 mMol) 2-Aminoethylacetamid und 8,1 g (30 mMol) 4-Brommethyl-2′-cyanobiphenyl in 100 ml Dioxan erhält man analog Beispiel 23a die Titelverbindung als Oel, das roh weiterverwendet wird.

b) N-[(2-Acetylamino-ethyl)-N-[(2′-cyanobiphenyl-4-yl)methyl]-n-valeriansäureamid
Eine Lösung von 4,2 g (8,8 mMol) der unter a) erwähnten Verbindung und 5,0 ml Hünig-Base in 40 ml Methylenchlorid wird mit 2,4 g (20 mMol) Valeriansäurechlorid versetzt und 24 Stunden unter Rückfluss zum Sieden erhitzt. Aufarbeitung analog Beispiel 23b und "flash"-chromatographische Reinigung (n-Hexan-Ethylacetat 4:1)ergeben die Titelverbindung als gelbes Oel mit $R_f$ 0,17 (n-Hexan-Ethylacetat 4:1).

Beispiel 61: N-[2-(n-Butoxycarbonyl)-2,2-tetramethylen-ethyl]-N-pentanoyl-N-[2′-(1H-tetrazol-5-yl)biphenyl-4-ylmethyl]-amin

0,490 g N-[(2′-(1H-Tetrazol-5-yl)biphenyl-4-yl)methyl]-N-valeryl-1-aminomethyl-cyclopentan-1-carbonsäure wird in 20 ml 1-Butanol gelöst, mit Molekularsieb 4Å sowie 0,5 ml 4N Salzsäure versetzt und 48 Stunden zum Rückfluss erhitzt. Das Reaktionsgemisch wird im Vakuum eingedampft und an Kieselgel 60 (40-63 µm) mit $CH_2Cl_2$-MeOH 95:5 gereinigt, $R_f$ = 0,73 (System N8). MS(FAB): $^m/_e$ 518 $(M^++H)$, 540 $(M^++Na)$.

Beispiel 62: N-(2-Ethoxycarbonyl-2,2-pentamethylen-ethyl)-N-pentanoyl-N-[2′-(1H-tetrazol-5-yl)biphenyl-4-ylmethyl]-amin

8,70 g N-[(2′-Cyano-biphenyl-4-yl)methyl]-N-valeryl-1-aminomethyl-cyclohexan-1carbonsäureethylester werden analog Beispiel 1 umgesetzt. Das Rohprodukt wird an Kieselgel 60 (40-63 µm) mit $CH_2Cl_2$-MeOH 95:5 gereinigt, $R_f$ = 0,66 (System N8). MS (FAB): $^m/_e$ 504 $(M^++H)$, 526 $(M^++Na)$, 542 $(M^++K)$.

Das Ausgangsmaterial kann beispielsweise folgendermassen hergestellt werden:

a) 1-Aminomethyl-cyclohexan-1-carbonsäureethylester wird erhalten durch hydrieren von 72,08g 1-Cyano-cyclohexan-1-carbonsäureethylester (T. Kurihara et al. Tet. Lett. 1976, 2455) in 600 ml Aethanol, der ca. 4 % Ammoniak enthält, in Gegenwart von 20 g Raney-Nickel bei 45°C und unter Normaldruck. Nach Entfernen des Katalysators und Lösungsmittels wird das Produkt durch Destillation erhalten, Siedepunkt 72-75°C bei 0,3 mbar.

b) N-[(2′-Cyano-biphenyl-4-yl)methyl]-1-aminomethyl-cyclohexan-1-carbonsäureethylester wird analog Beispiel 41a) aus 5,422 g 4-Brommethyl-2′-cyano-biphenyl und 9,264 g 1-Aminomethyl-cyclohexan-1-carbonsäureethylester erhalten und an Kieselgel 60 (40-63 µm) mit $CH_2Cl_2$-MeOH 97,5:2,5 gereinigt, $R_f$ = 0,67 (System N6).

c) N-[(2′-Cyano-biphenyl-4-yl)methyl]-N-valeryl-1-aminomethyl-1 - carbonsäureethylester wird analog Beispiel 1c) aus 7,12 g N-[(2′-Cyano-biphenyl-4-yl)methyl]-1-aminomethylcyclohexan-1-carbonsäureethylester erhalten und extraktiv gereinigt, $R_f$ = 0,68 (System N6).

Beispiel 63: N-(2-Benzylaminocarbonyl-2,2-tetramethylen-ethyl)-N-pentanoyl-N-[2′-(1H-tetrazol-5-yl)biphenyl-4-ylmethyl]-amin

0,507 g N-[(2′-(1H-Tetrazol-5-yl)biphenyl-4-yl)methyl]-N-valeryl-1-aminomethyl-cyclopentan-1-carbonsäure wird analog Beispiel 48 mit 0,214 g Benzylamin umgesetzt und das Rohprodukt wird an Kieselgel 60 (40-63 µm) mit $CH_2Cl_2$-MeOH 95:5 gereinigt, $R_f$ = 0,49 (System N8). MS (FAB): $^m/_e$ 551 $(M^++H)$, 573 $(M^++Na)$.

Beispiel 64: N-(2-Carboxy-2-ethyl-but-1-yl)-N-pentanoyl-N-[2′-(1H-tetrazol-5-yl)biphenyl-4-ylmethyl]-amin

1,146 g N-[(2′-(1H-Tetrazol-5-yl)biphenyl-4-yl)methyl]-N-valeryl-2-aminomethyl-2-ethyl-buttersäure-ethylester werden in 10 ml Ethanol gelöst, mit 4,66 ml 2N NaOH-Lösung versetzt und 20 Stunden zum Rückfluss erhitzt. Nach Abkühlen auf Raumtemperatur und Zugabe von 4,66 ml 2N Salzsäure wird eingedampft. Das Produkt wird durch Chromatographie an Kieselgel 60 (40-63 µm) mit $CH_2Cl_2$-MeOH 80:20 isoliert, $R_f$ = 0,38 (System N8). MS (FAB):$^m/_e$ 486 ($M^+$+Na), 502 ($M^+$+K).

Beispiel 65: (S)-N-(1-Carboxy-2-methyl-prop-1-yl)-N-ethoxycarbonyl-N-[2′-(1H-tetrazol-5-yl)biphenyl-4-ylmethyl]-amin

0.34 g N-Carboethoxy-N-[(2′-(1H-tetrazol-5-yl)biphenyl-4-yl)-methyl] - (L)-valin-benzylester und 0.17 g Palladiumkohle (10%) werden in 10 ml Tetrahydrofuran unter Normaldruck 20 Stunden bis zur Sättigung hydriert. Es wird vom Katalysator abfiltriert und das Rohprodukt wird mittels Flashchromatographie (25 g Kieselgel, Fliessmittel B1) gereinigt. Amorphes Produkt FAB-MS:m/e = 424 (M+$H^+$).

Das Ausgangsmaterial kann beispielsweise wie folgt erhalten werden:

a) N-Carboethoxy-N-[(2′-Cyanobiphenyl-4-yl)methyl]-(L)-valin-ben zylester
10.0 g N-[(2′-Cyanobiphenyl-4-yl)methyl]-(L)-valin-benzylester werden in 150 ml Chloroform gelöst und bei 0° mit 8.2 ml Diisopropylethylamin versetzt. Man gibt 2.4 ml Chlorameisensäureethylester zu und erhitzt während 3 Stunden zum Rückfluss. Das Reaktionsgemisch wird mit 0.1 M-Salzsäure und Sole gewaschen, getrocknet und eingeengt. Amorphes Produkt. DC (System N3) $R_f$-Wert: 0.45.

b) N-Carboethoxy-N-[(2′-(1H-tetrazol-5-yl)biphenyl-4-yl)methy l]-(L)-valin-benzylester
10.0 g N-Carboethoxy-N-[(2′-Cyanobiphenyl-4-yl)methyl]-(L)-valin-ben zylester und 9.2 g Tributylzinnazid werden in 150 ml Xylol 18 Stunden zum Rückfluss erhitzt. Das Reaktionsgemisch wird eingeengt und der Rückstand während 15 Minuten in 5M etherischer Salzsäure verrührt. Man engt wieder ein, löst den Rückstand in Ether und extrahiert mit kalter 4M Kalilauge. Die Wasserphase wird sauer gestellt und mit Essigester extrahiert. Diese Essigesterphase wird mit Sole gewaschen, über Magnesiumsulfat getrocknet und eingeengt. Das Rohprodukt wird mittels Flashchromatographie (250 g Kieselgel, Fliessmittel N6) gereinigt. Amorphes Produkt, DC (System N6) $R_f$-Wert: 0.22.

Beispiel 66: (S)-N-(1-Carboxy-2-methyl-prop-1-yl)-N-propyloxycarbonyl-N-[2′-(1H-tetrazol-5-yl)biphenyl-4-ylmethyl]-amin

Analog Beispiel 1 ausgehend von 0.14 g N-Carbopropoxy-N-[(2′-(1H-tetrazol-5-yl)biphenyl-4-yl)-methyl]-(L)-valin und 0.07 g Palladiumkohle wird nach Flashchromatographie (B1) das amorphe Produkt erhalten. FAB-MS: m/e = 438 (M+$H^+$).

Das Ausgangsmaterial kann beispielsweise wie folgt erhalten werden:

a) N-Carbopropoxy-N-[(2′-Cyanobiphenyl-4-yl)methyl]-(L)-valin -benzylester ausgehend von 1.0 g N-[(2′-Cyanobiphenyl-4-yl)methyl]-(L)-valin-benzylester 0.8 ml Diisopropylethylamin und 0.34 ml Chlorameisensäurepropylester und anschliessender Flashchromatographie mit System N3. Amorphes Produkt. DC (System N2) $R_f$-Wert: 0.38.

b) N-Carbopropoxy-N-[(2′-(1H-tetrazol-5-yl)biphenyl-4-yl)methy l]-(L)-valin-benzylester ausgehend von 1.04 g N-Carbopropoxy-N-[(2′-Cyanobiphenyl-4-yl)methyl]-(L)-valin-benzylester und 1.1 g Tributylzinnazid und anschliessender Flashchromatographie mit dem System N6. Amorphes Produkt, DC (System N6) $R_f$-Wert: 0.21.

Beispiel 67: (S)-N-Butyloxycarbonyl-N-(1-Carboxy-2-methyl-prop-1-yl)-N-[2′-(1H-tetrazol-5-yl)biphenyl-4-ylmethyl]-amin

Analog Beispiel 1 ausgehend von 0.40 g N-Carbobutoxy-N-[(2′-(1H-tetrazol-5-yl)biphenyl-4-yl)-methyl]-(L)-valin und 0.20 g Palladiumkohle wird nach Flashchromatographie (B1) das amorphe Produkt erhalten. FAB-MS: m/e = 452 (M+$H^+$).

Das Ausgangsmaterial kann beispielsweise wie folgt erhalten werden:

a) N-Carbobutoxy-N-[(2′-Cyanobiphenyl-4-yl)methyl]-(L)-valin -benzylester ausgehend von 1.0 g N-[(2′-Cyanobiphenyl-4-yl)methyl]-(L)-valin-benzylester 0.8 ml Diisopropylethylamin und 0.34 ml Chlorameisensäurebutylester und anschliessender Flashchromatographie mit System N3. Amorphes Produkt. DC (System N2) $R_f$-Wert: 0.41.

b) N-Carbobutoxy-N-[(2′-(1H-tetrazol-5-yl)biphenyl-4-yl)methyl]- (L)-valin-benzylester ausgehend von 1.05 g N-Carbobutoxy-N-[(2′-Cyanobiphenyl-4-yl)methyl]-(L)-valin-benzylester und 1.05 g Tributylzinnazid und anschliessender Flashchromatographie mit dem System N6. Amorphes Produkt, DC (System N6) $R_f$-Wert: 0.17.

Beispiel 68: (S)-N-(1-Carboxy-2-methyl-prop-1-yl)-N-methoxycarbonyl-N-[(2′-(1H-tetrazol-5-yl)biphenyl-4-ylmethyl]-amin

Analog Beispiel 1 ausgehend von 2.40 g N-Carbomethoxy-N-[(2′-(1H-tetrazol-5-yl) biphenyl-4-yl)-methyl]-(L)-valin und 0.50 g Palladiumkohle wird nach Flashchromatographie (B1) das amorphe Produkt erhalten. FAB-MS: m/e = 410 $(M+H^{+})$.

Das Ausgangsmaterial kann beispielsweise wie folgt erhalten werden:

a) N-Carbomethoxy-N-[(2′-Cyanobiphenyl-4-yl)methyl]-(L)-valin -benzylester ausgehend von 4.0 g N-[(2′-Cyanobiphenyl-4-yl)methyl]-(L)-valin-benzylester 3.3 ml Diisopropylethylamin und 0.78 ml Chlorameisensäuremethylester und anschliessender Flashchromatographie mit System N3. Amorphes Produkt. DC (System N3) $R_f$-Wert: 0.34.

b) N-Carbomethoxy-N-[(2′-(1H-tetrazol-5-yl)biphenyl-4-yl)methyl] -(L)-valin-benzylester ausgehend von 3.21 g N-Carbomethoxy-N-[(2′-Cyanobiphenyl-4-yl) methyl]-(L)-valin-benzylester und 3.50 g Tributylzinnazid und anschliessender Flashchromatographie mit dem System N6. Amorphes Produkt, DC (System N6) $R_f$-Wert: 0.26.

Beispiel 69:

In analoger Weise wie in Beispiel 47 beschrieben kann man auch das N-(2-Diethylaminocarbonyl-2,2-tetramethylen-ethyl)-N-pentanoyl-N-[2′-(1H-tetrazol-5-yl)biphenyl-4-ylmethyl]-amin [$R_f$-Wert: 0,47 (System N8)] herstellen.

Beispiel 70:

In analoger Weise wie in Beispiel 47 beschrieben kann man auch das N-(2-Methyl-2-morpholin-4-ylcarbonyl-propyl)-N-pentanoyl-N-[2′-( 1H-tetrazol-5-yl)biphenyl-4-ylmethyl]-amin [$R_f$-Wert: 0,61 (System N8)] herstellen.

Beispiel 71:

In analoger Weise wie in Beispiel 64 beschrieben kann man auch das N-(2-Carboxy-2-methyl-propyl)-N-pentanoyl-N-[2′-( 1H-tetrazol-5-yl)biphenyl-4-ylmethyl]-amin [$R_f$-Wert: 0,39 (System N8)] herstellen.

Beispiel 72:

In analoger Weise wie in Beispiel 40 beschrieben kann man auch das N-(2-Carboxy-2,2-pentamethylen-ethyl)-N-pentanoyl-N-[2′-(1H-tetrazol-5-yl)biphenyl-4-ylmethyl] -amin [$R_f$-Wert: 0,33 (System N8)] herstellen.

Beispiel 73:

Eine Lösung von 1,5 g (2,8 mmol) N-(1-Benzyloxycarbonylcyclopentyl)-N-pentanoyl-N-[2′-(1H-tetrazol-5-yl)biphenyl-4-ylmethyl]-amin in 20 ml Dioxan wird unter Zusatz von 0,3 g Pd/C (10%) in analoger Weise wie in Beispiel 56 beschrieben hydriert. Nach Reinigung durch Flash-Chromatographie (Kieselgel; System S2) erhält man das N-(1-Carboxycyclopentyl)-N-pentanoyl-N-[2′-(1H-tetrazol-5-yl)biphenyl-4-ylmethyl]-amin in Form eines Schaumes [$R_f$-Wert: 0,29 (System S2)].

Das Ausgangsmaterial kann z. B. wie folgt hergestellt werden:

a) Ein Gemisch aus 2,72 g (10 mmol) 4-Brommethyl-2′-cyano-biphenyl, 2,63 g (12 mmol) 1-Aminocyclopentancarbonsäurebenzylester, 3,4 ml (20 mmol) Hünigbase und 10 ml N,N-Dimethylformamid wird unter Rühren 2 Stunden auf 130 bis 140° (Badtemperatur) erhitzt. Nach dem Abkühlen wird das Reaktionsgemisch auf 50 ml Eiswasser gegossen. Durch Extraktion mit Ethylacetat erhält man das rohe N-(1-Benzyloxycarbonylcyclopentyl)-N-(2′-cyano-biphenyl-4-ylmethyl)-amin, das ein zwischen 180 und 182° (Ethanol/Diethylether) schmelzendes Hydrochlorid bildet.

b) Eine Lösung von 2,9 g (6,5 mmol) N-(1-Benzyloxycarbonylcyclopentyl)-N-(2′-cyanobiphenyl-4-ylmethyl)-amin-hydrochlorid und 4,4 ml (26 mmol) Hünigbase in 50 ml Ethylacetat wird mit 1,1 g (9 mmol) Pentanoylchlorid versetzt und das Gemisch 15 Stunden bei 25 bis 30° gerührt. Nach Zusatz von weiteren 0,5 g Pentanoylchlorid wird weitere

8 Stunden gerührt. Das Reaktionsgemisch wird dann mit 10 ml wässriger Ammoniaklösung (5%) versetzt und 0,5 Stunden gerührt. Die Ethylacetatphase wird abgetrennt, nacheinander mit 2 N-Salzsäure, Wasser und Natriumhydrogencarbonatlösung gewaschen, getrocknet und eingedampft. Man erhält so das N-(1-Benzyloxycarbonylcyclopentyl)-N-(2′-cyanobiphenyl-4-ylmethyl)-N-pentanoyl-amin in Form eines braunen Oels [$R_f$-Wert: 0,53 (System B7)], das in roher Form weiterumgesetzt wird.

c) Ein Gemisch aus 3,2 g (6,5 mmol) N-( 1-Benzyloxycarbonylcyclopentyl)-N-(2′-cyanobiphenyl-4-ylmethyl)-N-pentanoyl-amin, 3,3 g (9,8 mmol) Tributylzinnazid und 35 ml o-Xylol wird 24 Stunden unter Rückfluss erhitzt. Aufarbeitung des Gemisches in analoger Weise wie in Beispiel 23 beschrieben ergibt das N-( 1-Benzyloxycarbonylcyclopentyl)-N-pentanoyl-N-[2′-( 1H-tetrazol-5-yl)biphenyl-4-ylmethyl]-amin in Form eines gelben Oels [$R_f$-Wert: 0,37 (System S2)], das in roher Form weiterumgesetzt werden kann.

Beispiel 74:

Eine Lösung von 2,4 g (4,3 mmol) N-(1-Benzyloxycarbonylcyclohexyl)-N-pentanoyl-N-[2′-(1H-tetrazol-5-yl)biphenyl-4-ylmethyl]-amin in 40 ml Dioxan wird unter Zusatz von 0,5 g Pd/C (10%) in analoger Weise wie in Beispiel 73 beschrieben hydriert und aufgearbeitet. Man erhält so das N-(1-Carboxycyclohexyl)-N-pentanoyl-N-[2′-(1H-tetrazol-5-yl)biphenyl-4-ylmethyl]-amin in Form von farblosen Kristallen (aus Ethylacetat), die zwischen 134 und 136° schmelzen.

Das Ausgangsmaterial kann z. B. wie folgt hergestellt werden:

a) Das N-(1-Benzyloxycarbonylcyclohexyl)-N-(2′-cyanobiphenyl-4-ylmethyl)-amin, das ein zwischen 164 und 166° (Isopropanol) schmelzendes Hydrochlorid bildet, erhält man in analoger Weise wie in Beispiel 73a) beschrieben.

b) Eine Lösung von 2,9 g (6,8 mmol) N-(1-Benzyloxycarbonylcyclohexyl)-N-(2′-cyanobiphenyl-4-ylmethyl)-amin und 4,4 ml (26 mmol) Hünigbase in 50 ml Ethylacetat wird mit 1,1 g (9 mmol) Pentanoylchlorid versetzt und das Gemisch 24 Stunden unter Rückfluss erhitzt. Nach dem Abkühlen wird das Reaktionsgemisch mit 20 ml wässriger Ammoniaklösung (2 N) versetzt und 1 Stunde gerührt. Die organische Phase wird abgetrennt, nacheinander mit 2 N-Salzsäure, gesättigter Natriumhydrogencarbonatlösung und Sole gewaschen, getrocknet und eingedampft. Man erhält so das N-(1-Benzyloxycarbonylcyclohexyl)-N-(2′-cyanobiphenyl-4-ylmethyl)-N-pentanoyl-amin in Form eines braunen Oels [$R_f$-Wert: 0,44 (Toluol/Methanol = 19:1)], das in roher Form weiterumgesetzt wird.

c) Ein Gemisch aus 3,3 g (6,5 mmol) N-(1-Benzyloxycarbonylcyclohexyl)-N-(2′-cyanobiphenyl-4-ylmethyl)-N-pentanoyl-amin, 4,1 g ( 12,3 mmol) Tribütylzinnazid und 30 ml o-Xylol wird 44 Stunden unter Rückfluss erhitzt. Aufarbeitung des Gemisches in analoger Weise wie in Beispiel 23 beschrieben ergibt das N-(1-Benzyloxycarbonylcyclohexyl)-N-pentanoyl-N-[2′-(1H-tetrazol-5-yl)biphenyl-4-ylmethyl]-amin in Form von hellbraunen Kristallen, die zwischen 189 und 190° (aus Ethylacetat/Diethylether) schmelzen.

Beispiel 75:

In analoger Weise wie in Beispiel 74 beschrieben kann man auch das N-(1-Carboxy-1-ethyl-prop-1-yl)-N-pentanoyl-N-[2′-(1H-tetrazol-5-yl)biphenyl-4-ylmethyl]-amin [heller Schaum; $R_f$-Wert: 0,35 (System S2)] herstellen.

Beispiel 76:

170 mg (S)-N-(1-Benzyloxycarbonyl-5-benzyloxycarbonylamino-pent-1-yl)N-pentanoyl-N-[2′-(1H-tetrazol-5-yl)biphenyl-4-ylmethyl]-amin werden in 5 ml Methanol gelöst. Die Lösung wird mit 170 mg Palladium/Kohle (10%) versetzt und das Gemisch unter Normaldruck und bei Raumtemperatur bis zur Sättigung hydriert. Das Gemisch wird über Hyflo filtriert und das Filtrat eingedampft, wodurch das reine (S)-N-(5-Amino-1-carboxy-pent-1-yl)-N-pentanoyl-N-[2′-(1H-tetrazol-5-yl)biphenyl-4-ylmethyl]-amin in Form eines weissen Schaumes erhalten wird [MS (FAB): m/z = 465, $(M + H)^+$].

Das Ausgangsmaterial kann z. B. wie folgt hergestellt werden:

a) 5,0 g (S)-2-Amino-6-benzyloxycarbonylamino-hexansäwebenzylester werden in 250 ml N,N-Dimethylformamid gelöst. Die Lösung wird mit 4,33 ml N,N-Diisopropyl-N-ethyl-amin versetzt und das Gemisch auf 80° erwärmt, 30 Minuten gerührt, mit 4,44 g 4-Brommethyl-2′-(1-triphenylmethyl-1H-tetrazol-5-yl)-biphenyl versetzt, 16 Stunden bei 80° gerührt und dann eingedampft. Der Rückstand wird mit Wasser und Ethylacetat aufgearbeitet. Die organische Phase wird getrocknet und mittels Flashchromatographie gereinigt (200 g Kieselgel; System N4). Das (S)-N-(1-Benzyloxycarbonyl-5-benzyloxycarbonylamino-pent-1-yl)-N-[2′-( 1-triphenylmethyl-1H-tetrazol-5-yl)biphenyl-4-ylmethyl]-amin wird in Form eines braunen Oels erhalten [$R_f$-Wert: 0,18 (System N3)].

b) 1,1 g (S)-N-(1-Benzyloxycarbonyl-5-benzyloxycarbonylamino-pent-1-yl)-N-[2′-(1-triphenylmethyl-1H-tetrazol-5-yl)biphenyl-4-ylmethyl]-amin werden in 20 ml $CH_2Cl_2$ gelöst. Die Lösung wird auf 0° gekühlt und mit 0,408 ml N,N-Diisopropyl-N-ethyl-amin und anschliessend mit 0,29 ml Pentanoylchlorid versetzt. Man rührt das Gemisch 15 Minuten in einem Eisbad und dann 16 Stunden bei Raumtemperatur. Das Gemisch wird dann mit $CH_2Cl_2$ verdünnt, nacheinander mit 1 N-Natronlauge, 1 N-Salzsäure, Wasser und Sole gewaschen und über $MgSO_4$ getrocknet. Nach Reinigung mittels Flashchromatographie (200 g Kieselgel; System N3) erhält man das (S)-N-(1-Benzyloxycarbonyl-5-benzyloxycarbonylamino-pent-1-yl)-N-pentanoyl-N-[2′-(1-triphenylmethyl-1H-tetrazol-5-yl)biphenyl-4-ylmethyl]-amin in Form eines bräunlichen Oels [$R_f$-Wert: 0,34 (System N2)].

c) 1,07 g (S)-N-(1-Benzyloxycarbonyl-5-benzyloxycarbonylamino-pent-1-yl)-N-pentanoyl-N-[2′-(1-triphenylmethyl-1H-tetrazol-5-yl)biphenyl-4-ylmethyl]-amin werden in 15 ml Dioxan gelöst. Diese Lösung wird mit 1,5 ml einer Lösung von Chlorwasserstoff in Dioxan (7 N) versetzt und das Gemisch 4,5 Stunden bei 40° gerührt, eingedampft und mittels Flashchromatographie gereinigt (200 g Kieselgel; System N6). Man erhält so das (S)-N-(1 -Benzyloxycarbonyl-5-benzyloxycarbonylamino-pent-1-yl)-N-pentanoyl-N-[2′-(1H-tetrazol-5-yl)biphenyl-4-ylmethyl]-amin [$R_f$-Wert: 0,42 (System N7)].

Beispiel 77:

Ein Gemisch aus 3,64 g N-Butansulfonyl-N-(2′-cyanobiphenyl-4-ylmethyl)-N-(2-ethoxycarbonyl-2,2-pentamethylen-ethyl)-amin, 5,0 g Tributylzinnazid und 20 ml o-Xylol wird 15 Stunden unter Rückfluss erhitzt. Nach dem Abkühlen wird das Gemisch eingedampft. Der Rückstand wird mit 20 ml methanolischer Salzsäure (3 N) versetzt und das Gemisch 1 Stunde gerührt und dann eingedampft. Der Rückstand wird in Diethylether aufgenommen. Die Etherlösung wird mit 1 N-Natronlauge extrahiert. Die wässrige Phase wird mit konzentrierter Salzsäure auf pH 3 angesäuert und mit $CH_2Cl_2$ extrahiert. Die vereinigten organischen Phasen werden über $MgSO_4$ getrocknet und eingeengt. Der Rückstand wird durch Flashchromatographie gereinigt (220 g Kieselgel; $CH_2Cl_2$/Aceton = 9:1). Kristallisation aus Pentan liefert das N-Butansulfonyl-N-(2-ethoxycarbonyl-2,2-pentamethylen-ethyl)-N-[2′-(1H-tetrazol-5-yl)biphenyl-4-ylmethyl]-amin [Smp.: 121° (Zersetzung)].

Das Ausgangsmaterial kann z. B. wie folgt hergestellt werden:

a) 3,0 g 1-Aminomethyl-1-ethoxycarbonyl-cyclohexan werden in 25 ml $CHCl_3$ gelöst. Die Lösung wird bei Raumtemperatur mit 0,7 ml Butansulfonylchlorid versetzt. Das Gemisch wird 5 Stunden unter Rückfluss erhitzt und nach dem Abkühlen eingedampft. Der Rückstand wird in Diethylether aufgenommen. Die etherische Phase wird nacheinander mit 1 N-Salzsäure und Wasser extrahiert, über $MgSO_4$ getrocknet und eingedampft. Der gelbe harzige Rückstand, das rohe N-Butansulfonyl-N-(2-ethoxycarbonyl-2,2-pentamethylen-ethyl)-amin [$R_f$-Wert: 0,64 (System N2)], kann ohne weitere Reinigung weiterumgesetzt werden.

b) 3,75 g N-Butansulfonyl-N-(2-ethoxycarbonyl-2,2-pentamethylen-ethyl)-amin werden in 30 ml Tetrahydrofuran gelöst. Die Lösung wird mit einem Eisbad gekühlt und mit 309 mg Natriumhydrid-Dispersion (80% in Oel) versetzt. Nach dem Erwärmen auf Raumtemperatur werden 3,5 g 4-Brommethyl-2′-cyano-biphenyl zugegeben. Das Gemisch wird 30 Stunden bei Raumtemperatur und dann 4 Stunden bei 60° gerührt und nach dem Abkühlen eingeengt. Der Rückstand wird in Diethylether aufgenommen. Die etherische Phase wird nacheinander mit 1 N-Salzsäure und Wasser extrahiert, getrocknet und eingeengt. Flashchromatographie des Rückstands (300 g Kieselgel; Hexan/tert.-Butylmethylether = 4:1 ) liefert das reine N-Butansulfonyl-N-(2′-cyanobiphenyl-4-ylmethyl)-N-(2-ethoxycarbonyl-2,2-pentamethylen-ethyl)-amin in Form eines gelben Harzes [$R_f$-Wert: 0,46 (Hexan/tert.-Butylmethylether = 1:1)].

Beispiel 78:

1,8 g N-Butansulfonyl-N-(2-ethoxycarbonyl-2,2-pentamethylen-ethyl)-N-[2′-(1H-tetrazol-5-yl)biphenyl-4-ylmethyl]-amin werden in 50 ml Methanol/Wasser (1:1) aufgenommen. Das Gemisch wird mit 5,0 g Natriumhydroxid versetzt, 20 Stunden unter Rückfluss erhitzt, auf Raumtemperatur abgekühlt, mit Wasser verdünnt und mit Ethylacetat extrahiert. Die wässrige Phase wird mit konzentrierter Salzsäure auf pH 3 angesäuert, mit NaCl gesättigt und mit $CH_2Cl_2$ extrahiert. Die vereinigten organischen Phasen werden getrocknet und eingedampft. Umkristallisation aus Diethylether/Hexan liefert das reine N-Butansulfonyl-N-(2-carboxy-2,2-pentamethylen-ethyl)-N-[2′-(1H-tetrazol-5-yl)biphenyl-4-ylmethyl]-amin [Smp.: 123° (Zersetzung)].

Beispiel 79:

In analoger Weise wie in Beispiel 77 beschrieben kann man auch das N-Butansulfonyl-N-(2-ethoxycarbonyl-2-methyl-prop-1 -yl)-N-[2′-(1H-tetrazol-5-yl)biphenyl-4-ylmethyl]-amin (Smp.: 104°) herstellen.

Beispiel 80:

In analoger Weise wie in Beispiel 78 beschrieben kann man auch das N-Butansulfonyl-N-(2-carboxy-2-methyl-prop-1-yl)-N-[2′-( 1H-tetrazol-5-yl)biphenyl-4-ylmethyl]-amin (Smp.: 137°) herstellen.

Beispiel 81:

In analoger Weise wie in Beispiel 77 beschrieben kann man auch das (S)-N-Butansulfonyl-N-( 1-tert.-butoxycarbonylethyl)-N-[2′-(1H-tetrazol-5-yl)biphenyl-4-ylmethyl]-amin herstellen, ausgehend von (S)-2-Aminopropansäure-tert.-butylester.

Beispiel 82:

750 mg (S)-N-Butansulfonyl-N-(1-tert.-butoxycarbonylethyl)-N-[2′-(1H-tetrazol-5-yl)biphenyl-4-ylmethyl]-amin werden 24 Stunden bei 0° mit salzsaurem Eisessig (1,9 N) behandelt. Eindampfen des Gemisches und Flashchromatographie des Rückstands ( 100 g Kieselgel; $CH_2Cl_2$/Ethylacetat/Toluol/Ameisensäure = 40:40:20:4) liefert das (S)-N-Butansulfonyl-N-(1-carboxyethyl)-N-[2′-(1H-tetrazol-5-yl)biphenyl-4-ylmethyl]-amin in Form eines weissen amorphen Pulvers [Smp.: 90° (Zersetzung bei 127°)].

Beispiel 83:

In analoger Weise wie in den Beispielen 77 und 37 beschrieben kann man auch das (S)-N-Butansulfonyl-N-(1-carboxy-2-methyl-prop-1-yl)-N-[2′-(1H-tetrazol-5-yl)biphenyl-4-ylmethyl]-amin [Smp.: 103° (Zersetzung)] herstellen, ausgehend von (S)-2-Amino-3-methyl-butansäurebenzylester-p-toluolsulfonat.

Beispiel 84:

In analoger Weise wie in Beispiel 48 beschrieben kann man auch das (S)-N-(1-Aminocarbonyl-2-methyl-prop-1-yl)-N-pentanoyl-N-[2′-(1H-tetrazol-5-yl)biphenyl-4-ylmethyl]-amin (Smp.: 177 bis 178°) herstellen.

Beispiel 85:

In analoger Weise wie in Beispiel 48 beschrieben kann man auch das (S)-N-(2-Methyl-1-methylaminocarbonyl-prop-1-yl)-N-pentanoyl-N-[2′-(1H-tetrazol-5-yl)biphenyl-4-ylmethyl]-amin (Smp.: 183 bis 184°) herstellen.

Beispiel 86:

In analoger Weise wie in Beispiel 48 beschrieben kann man auch das (S)-N-(1-Dimethylaminocarbonyl-2-methyl-prop-1-yl)-N-pentanoyl-N-[2′-(1H-tetrazol1-5-yl)biphenyl-4-ylmethyl] -amin (Smp.: 179 bis 180°) herstellen.

Beispiel 87:

In analoger Weise wie in Beispiel 48 beschrieben kann man auch das (S)-N-(2-Methyl-1-morpholin-4-ylcarbonyl-prop-1-yl)-N-pentanoyl-N-[2′-( 1H-tetrazol-5-yl)biphenyl-4-ylmethyl]-amin [Smp.: 130° (Zersetzung)] herstellen.

Beispiel 88:

In analoger Weise wie in Beispiel 8 beschrieben kann man auch das (S)-N-(2′-Carboxybiphenyl-4-ylmethyl)-N-(1-carboxy-2-methyl-prop-1-yl)-N-pentanoylamin (Smp.: 66 bis 68°) herstellen.

Beispiel 89:

In analoger Weise wie in Beispiel 16 beschrieben kann man auch das (S)-N-(1,2-Dicarboxyethyl)-N-pentanoyl-N-[2′-( 1H-tetrazol-5-yl)biphenyl-4-ylmethyl]-amin (Smp.: 303 bis 305°) herstellen.

Beispiel 90:

In analoger Weise wie in Beispiel 16 beschrieben kann man auch das (S)-N-(1-Carboxy-2-methyl-prop-1-yl)-N-(5-oxopent-1-en-5-yl)-N-[2′-( 1H-tetrazol-5-yl)biphenyl-4-ylmethyl]-amin (Smp.: 108 bis 109°) herstellen.

Beispiel 91:

In analoger Weise wie vorstehend beschrieben kann man auch die folgenden Verbindungen herstellen:

1. (S)-N-(1-Carboxy-3-phenyl-prop-1-yl)-N-pentanoyl-N-[2′-(1H-tetrazol-5-yl)biphenyl-4-ylmethyl]-amin (Smp.: 124 bis 125°);
2. (S)-N-(2-Cyclohexyl-1-hydroxymethyl-ethyl)-N-pentanoyl-N-[2′-(1H-tetrazol-5-yl)biphenyl-4-ylmethyl]-amin (Smp.: 86 bis 87°);
3. (R)-N-(1-Methoxycarbonyl-2-methyl-prop-1-yl)-N-pentanoyl-N-[2′-(1H-tetrazol-5-yl)biphenyl-4-ylmethyl]-amin (Smp.: 77 bis 78°);
4. (S)-N-(2-Hydroxy-1-methoxycarbonyl-ethyl)-N-pentanoyl-N-[2′-(1H-tetrazol-5-yl)biphenyl-4-ylmethyl]-amin;
5. N-Pentanoyl-N-( 1H-tetrazol-5-ylmethyl)-N-[2′-(1H-tetrazol-5-yl)biphenyl-4-ylmethyl]-amin;
6. N-Pentanoyl-N-pyrid-3-ylmethyl-N-[2′-(1H-tetrazol-5-yl)biphenyl-4-ylmethyl]-amin;
7. (S)-N-(1-Carboxy-4-guanidino-but-1-yl)-N-pentanoyl-N-[2′-(1H-tetrazol-5-yl)biphenyl-4-ylmethyl] -amin-hydrochlorid [$R_f$-Wert: 0,34 ($CH_2Cl_2$/$CH_3OH$/konzentriertes Ammoniak = 20:10:1 )];
8. N-(2-Hydroxy-1-methoxycarbonyl-prop-1-yl)-N-pentanoyl-N-[2′-(1H-tetrazol-5-yl)biphenyl-4-ylmethyl]-amin;
9. N-(1-Benzyloxycarbonyl-1-methyl-ethyl)-N-butanoyl-N-[2′-(1H-tetrazol-5-yl)biphenyl-4-ylmethyl]-amin (Smp.: 203 bis 204°);
10. (S)-N-(1-Carboxy-3-methyl-but-1-yl)-N-pentanoyl-N-[2′-(1H-tetrazol-5-yl)biphenyl-4-ylmethyl]-amin (Smp.: >300°);
11. N-(1-Carboxy-2-hydroxy-ethyl)-N-pentanoyl-N-[2′-(1H-tetrazol-5-yl)biphenyl-4-ylmethyl]-amin;
12. (S)-N-(1-Carboxy-2-hydroxy-ethyl)-N-pentanoyl-N-[2′-(1H-tetrazol-5-yl)biphenyl-4-ylmethyl]-amin;
13. (S )-N-[2-Methyl-1-(2-phenylethylaminocarbonyl)-prop-1-yl]-N-pentanoyl-N-[2′-(1H-tetrazol-5-yl)biphenyl-4-ylmethyl]-amin (Smp.: 109 bis 111 °);
14. (S)-N-(2-Benzyloxy-1-hydroxymethyl-ethyl)-N-pentanoyl-N-[2′-(1H-tetrazol-5-yl)biphenyl-4-ylmethyl] -amin;
15. (S)-N-(1-Carboxy-2-methyl-prop-1-yl)-N-pentanoyl-N-[2′-( 1H-tetrazol-5-yl)biphenyl-3-ylmethyl]-amin (Smp.: 78 bis 79°);
16. (S)-N-(1-Carboxy-2-methyl-prop-1-yl)-N-pentanoyl-N-[3′-(1H-tetrazol-5-yl)biphenyl-4-ylmethyl]-amin (Smp.: 97 bis 98°);
17. (S)-N-[2-Methyl-1-(1,2,3,4-tetrahydrochinol-1-ylcarbonyl)-prop-1 - yl] -N-pentanoylN-[2′-(1H-tetrazol-5-yl)biphenyl-4-ylmethyl]-amin (Smp.: 100 bis 110°);
18. (S)-N-(2-Methyl-1-piperidin-1-ylcarbonyl-prop-1-yl)-N-pentanoyl-N-[2′-(1H-tetrazol-5-yl)biphenyl-4-ylmethyl]-amin (Smp.: 100°);
19. (S)-N-[2-Methyl-1-(1,2,3,4-tetrahydroisochinol-2-ylcarbonyl)-prop-1-yl]-N-pentanoyl-N-[2′-(1H-tetrazol-5-yl)biphenyl-4-ylmethyl]-amin (Smp.: 122°):
20. N-(2-Hydroxymethyl-2-methyl-prop-1-yl)-N-pentanoyl-N-[2′-( 1H-tetrazol-5-yl)biphenyl-4-ylmethyl] -amin [$R_f$-Wert: 0,45 ($CH_2Cl_2$/$CH_3OH$ = 4:1 )];
21. N-Ethoxycarbonyl-N-(2-ethoxycarbonyl-2-methyl-prop-1-yl)-N-[2′-(1H-tetrazol-5-yl)biphenyl-4-ylmethyl] -amin [$R_f$-Wert: 0,64 ($CH_2Cl_2$/$CH_3OH$ = 4:1 )]; und
22. N-(2-Carboxy-2-methyl-prop-1-yl)-N-ethoxycarbonyl-N-[2′-(1H-tetrazol-5-yl)biphenyl-4-ylmethyl]-amin [$R_f$-Wert: 0,32 ($CH_2Cl_2$/$CH_3OH$ = 4:1 )].

Beispiel 92:

Tabletten, enthaltend je 50 mg Wirkstoff, z.B. (S)-N-(1-Carboxy-2-methylprop-1-yl)-N-pentanoyl-N-[2′-( 1H-tetrazol-5-yl)biphenyl-4-ylmethyl]-amin, können wie folgt hergestellt werden:

Zusammensetzung (für 10000 Tabletten):

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 93:

Lacktabletten, enthaltend je 100 mg Wirkstoff, z.B. (S)-N-(1-Carboxy-2-methyl-prop-1-yl)-N-pentanoyl-N-[2′-(1H-tetrazol-5-yl)biphenyl-4-ylmethyl]-amin, können wie folgt hergestellt werden:

Zusammensetzung (für 1000 Tabletten):

| | |
|---|---|
| Wirkstoff | 100,00 g |
| Lactose | 100,00 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Dichlormethan lackiert (Endgewicht der Lacktablette: 283 mg).

Beispiel 94:

In analoger Weise wie in den Beispielen 92 und 93 beschrieben können auch Tabletten und Lacktabletten, enthaltend eine andere Verbindung der Formel I oder ein pharmazeutisch verwendbares Salz einer Verbindung der Formel I, z.B. gemäss einem der Beispiele 1 bis 91, hergestellt werden.

**Patentansprüche**

**Patentansprüche für folgende Vertragsstaaten : AT, BE, CH, DE, DK, FR, GB, IT, LI, LU, NL, SE**

**1.** Eine Verbindung der Formel

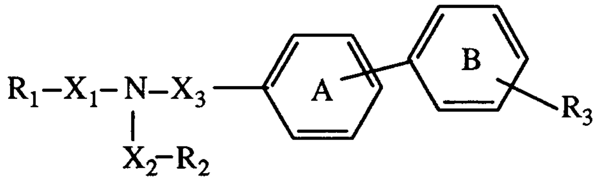

(I),

worin $R_1$ gegebenenfalls durch Halogen oder Hydroxy substituiertes Niederalkyl, Niederalkenyl oder Niederalkinyl oder $C_3$-$C_7$-Cycloalkyl- oder $C_3$-$C_7$-Cycloalkenyl oder Phenylniederalkyl, Phenylniederalkenyl oder Phenylniederalkinyl bedeutet; $X_1$ für CO, $SO_2$ oder -O-C(=O)- steht, wobei das Kohlenstoffatom der Carbonylgruppe an das in der Formel I eingezeichnete Stickstoffatom gebunden ist; $X_2$ gegebenenfalls durch Hydroxy, Carboxy, Amino, Guanidino, $C_3$-$C_7$-Cycloalkyl, $C_3$-$C_7$-Cycloalkenyl, Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen, $C_2$-$C_{10}$-Alkyliden oder $C_3$-$C_7$-Cycloalkylen bedeutet, wobei ein Kohlenstoffatom von $C_1$-$C_{10}$-Alkylen bzw. $C_2$-$C_{10}$-Alkyliden zusätzlich durch $C_2$-$C_6$-Alkylen überbrückt sein kann, und wobei $C_3$-$C_7$-Cycloalkyl oder $C_3$-$C_7$-Cycloalkenyl gegebenenfalls ein- oder mehrfach substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Formyl, Diniederalkoxymethyl oder Oxyniederalkylenoxymethylen; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, 1H-Tetrazol-5-yl, Pyridyl, Hydroxy, Niederalkoxy, Niederalkenyloxy, Phenylniederalkoxy, Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, oder $PO_nH_2$ bedeutet, wobei n für 2 oder 3 steht; $X_3$ $C_1$-$C_{10}$-Alkylen oder $C_2$-$C_{10}$-Alkyliden bedeutet; $R_3$ Carboxy, 5-Tetrazolyl, $SO_3H$, $PO_2H_2$, $PO_3H_2$ oder Halogenniederalkylsulfamoyl ist; und wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind

durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, durch Formyl, Diniederalkoxymethyl oder Oxyniederalkylenoxymethylen; wobei mit "nieder" bezeichnete Reste und Gruppen bis und mit 7 Kohlenstoffatome enthalten; in freier Form oder in Salzform.

2. Eine Verbindung gemäss Anspruch 1 der Formel I, worin $R_1$ gegebenenfalls durch Halogen oder Hydroxy substituiertes Niederalkyl, Niederalkenyl oder Niederalkinyl oder $C_3$-$C_7$-Cycloalkyl oder $C_3$-$C_7$-Cycloalkenyl oder Phenylniederalkyl, Phenylniederalkenyl oder Phenylniederalkinyl bedeutet; $X_1$ für CO oder $SO_2$ steht; $X_2$ einen gegebenenfalls durch Hydroxy, $C_3$-$C_7$-Cycloalkyl oder $C_3$-$C_7$-Cycloalkenyl oder Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen oder $C_2$-$C_{10}$-Alkyliden oder $C_3$-$C_7$-Cycloalkylen bedeutet, wobei ein Kohlenstoffatom von $C_1$-$C_{10}$-Alkylen bzw. $C_2$-$C_{10}$-Alkyliden zusätzlich durch $C_2$-$C_6$-Alkylen überbrückt sein kann, und wobei $C_3$-$C_7$-Cycloalkylen gegebenenfalls ein- oder mehrfach substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Formyl, Diniederalkoxymethyl oder Oxyniederalkylenoxymethylen; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Niederalkenyloxy, Phenylniederalkoxy, Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O-unterbrochen sind, oder $PO_nH_2$ bedeutet, wobei n für 2 oder 3 steht; $X_3$ $C_1$-$C_{10}$-Alkylen oder $C_2$-$C_{10}$-Alkyliden bedeutet; $R_3$ Carboxy, 5-Tetrazolyl, $SO_3H$, $PO_2H_2$, $PO_3H_2$ oder Halogenniederalkylsulfamoyl ist; wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, durch Formyl, Diniederalkoxymethyl oder Oxyniederalkylenoxymethylen; in freier Form oder in Salzform.

3. Eine Verbindung gemäss Anspruch 1 der Formel I, worin $R_1$ gegebenenfalls durch Halogen oder Hydroxy substituiertes Niederalkyl, Niederalkenyl oder Niederalkinyl oder $C_3$-$C_7$-Cycloalkyl oder $C_3$-$C_7$-Cycloalkenyl oder Phenylniederalkyl, Phenylniederalkenyl oder Phenylniederalkinyl bedeutet; $X_1$ für CO oder $SO_2$ steht; $X_2$ gegebenenfalls durch Hydroxy, $C_3$-$C_7$-Cycloalkyl oder $C_3$-$C_7$-Cycloalkenyl oder Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen oder $C_2$-$C_{10}$-Alkyliden bedeutet; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Niederalkenyloxy, Phenylniederalkoxy oder Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, oder $PO_nH_2$ bedeutet, wobei n für 2 oder 3 steht; $X_3$ -$CH_2$- bedeutet; $R_3$ Carboxy, 5-Tetrazolyl, $SO_3H$, $PO_2H_2$, $PO_3H_2$ oder Halogenniederalkylsulfamoyl ist; und wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_1$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, durch Formyl, Diniederalkoxymethyl oder Oxyniederalkylenoxymethylen; in freier Form oder in Salzform.

4. Eine Verbindung gemäss Anspruch 1 der Formel I, worin $R_1$ Niederalkyl, Niederalkenyl, Niederalkinyl, Halogenniederalkyl, -niederalkenyl, -niederalkinyl, Hydroxyniederalkyl, -niederalkenyl, -niederalkinyl, $C_3$-$C_7$-Cycloalkyl, $C_3$-$C_7$-Cycloalkenyl, Phenylniederalkyl, Phenylniederalkenyl oder Phenylniederalkinyl bedeutet; $X_1$ für CO oder $SO_2$ steht; $X_2$ $C_1$-$C_{10}$-Alkylen oder $C_2$-$C_{10}$-Alkyliden bedeutet, die gegebenenfalls durch Hydroxy, einen $C_3$-$C_7$-Cycloalkyl-, $C_3$-$C_7$-Cycloalkenyl-, einen Phenylrest oder einen 5- oder 6-gliedrigen, monocyclischen heteroaromatischen Rest mit bis zu vier gleichen oder verschiedenen Heteroatomen substituiert sind, wobei die cyclischen Reste ihrerseits gegebenenfalls substituiert sind durch Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Formyl, Diniederalkoxymethyl, Oxyniederalkylenoxymethylen; $R_2$ Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Amino, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Niederalkenyloxy, Phenylniederalkoxy, Phenoxy, $S(O)_m$-R, wobei m für 0, 1 oder 2 und R für Wasserstoff, Niederalkyl, Niederalkenyl oder Niederalkinyl steht, Niederalkanoyl, Sulfamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, oder $PO_nH_2$ bedeutet, wobei n für 2 oder 3 steht; $X_3$ -$CH_2$- bedeutet; und $R_3$ Carboxy, 5-Tetrazolyl, $SO_3H$, $PO_2H_2$, $PO_3H_2$ oder Halogenniederalkylsulfamoyl bedeutet; wobei (hetero-)aromatische Reste einschliesslich der Ringe A und B unabhängig voneinander jeweils gegebenenfalls substituiert sind durch einen oder mehrere Substituenten ausgewählt aus Halogen, Hydroxy, Niederalkoxy, Niederalkenyloxy, jeweils gegebenenfalls durch Halogen oder Hydroxy substituiertes Niederalkyl, Niederalkenyl, Niederalkinyl, Niederalkoxyniederalkyl, -niederalkenyl, -niederalkinyl, Niederalkenyloxyniederalkyl, -niederalkenyl und -niederalkinyl, in freier Form oder in Salzform.

5. Eine Verbindung gemäss Anspruch 1 der Formel I, worin $X_2$ $C_1$-$C_{10}$-Alkylen oder $C_2$-$C_{10}$-Alkyliden bedeutet, die gegebenenfalls durch Hydroxy, einen $C_3$-$C_7$-Cycloalkyl-, $C_3$-$C_7$-Cycloalkenyl-, einen Phenylrest oder einen 5- oder 6-gliedrigen, monocyclischen heteroaromatischen Rest mit bis zu vier gleichen oder verschiedenen Heteroatomen substituiert sind, wobei ein C-Atom von $C_1$-$C_{10}$-Alkylen bzw. $C_2$-$C_{10}$-Alkyliden durch $C_2$-$C_6$-Alkylen überbrückt sein kann und wobei die cyclischen Reste ihrerseits gegebenenfalls substituiert sind durch Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Formyl, Diniederalkoxymethyl oder durch Oxyniederalkylenoxymethylen, oder $X_2$ $C_3$-$C_7$-Cycloalkylen bedeutet; $X_3$ Niederalkylen oder Niederalkyliden bedeutet; die Variablen $X_1$, $R_1$, $R_2$, und $R_3$ die in Anspruch 4 angegebenen Bedeutungen haben; und die (hetero)-aromatischen Ringe einschliesslich der Ringe A und B wie in Anspruch 4 angegeben substituiert sein können, in freier Form oder in Salzform.

6. Eine Verbindung gemäss Anspruch 1 der Formel I, worin $R_1$ Niederalkyl, Niederalkenyl, Halogenniederalkyl, -niederalkenyl, Hydroxyniederalkyl, 3- bis 7-gliedriges Cycloalkyl oder Phenylniederalkyl bedeutet; $X_1$ für CO, $SO_2$ oder -O-C(=O)-, wobei das Kohlenstoffatom der Carbonylgruppe an das in der Formel I eingezeichnete Stickstoffatom gebunden ist, steht; $X_2$ $C_1$-$C_{10}$-Alkylen oder $C_1$-$C_7$-Alkyliden, die gegebenenfalls substituiert sind durch Hydroxy, Carboxy, Amino, Guanidino, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder Oxyniederalkylenoxymethylen substituiert sein können; $R_2$ Carboxy, Niederalkoxy-, Phenylniederalkoxy-, Niederalkenyloxy-, Niederalkoxyniederalkoxy-carbonyl, Carbamoyl, in dem die Aminogruppe gegebenenfalls durch Niederalkyl, Phenylniederalkyl mono- oder unabhängig voneinander disubstituiert oder durch Niederalkylen, das gegebenenfalls an zwei benachbarten Kohlenstoffatomen mit einem Benzolring kondensiert ist, oder Niederalkylenoxyniederalkylen disubstituiert ist, Amino, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Phenylniederalkoxy, Phenoxy, $S(O)_m$-R, wobei m für 0, 1 oder 2 und R für Niederalkyl steht, Niederalkanoyl, Sulfamoyl, in dem die Aminogruppe gegebenenfalls durch Niederalkyl, Phenylniederalkyl mono- oder unabhängig voneinander disubstituiert ist, oder $PO_nH_2$ bedeutet, wobei n für 2 oder 3 steht; $X_3$ Methylen ist; $R_3$ Carboxy, 5-Tetrazolyl, $SO_3H$, $PO_2H_2$, $PO_3H_2$ oder Halogenniederalkylsulfamoyl bedeutet; und (hetero-)aromatische Reste einschliesslich der Ringe A und B jeweils gegebenenfalls zusätzlich substituiert sind durch einen oder mehrere Substituenten ausgewählt aus Halogen, Hydroxy, Niederalkoxy, jeweils gegebenenfalls durch Halogen oder Hydroxy substituiertes Niederalkyl bzw. Niederalkoxyniederalkyl, in freier Form oder in Salzform.

7. Eine Verbindung gemäss Anspruch 1 der Formel I, worin $R_1$ Niederalkyl, Niederalkenyl, Halogenniederalkyl, -niederalkenyl, Hydroxyniederalkyl, 3- bis 7-gliedriges Cycloalkyl oder Phenylniederalkyl bedeutet; $X_1$ für CO oder $SO_2$ steht; $X_2$ $C_1$-$C_{10}$-Alkylen oder $C_1$-$C_7$-Alkyliden, die gegebenenfalls substituiert sind durch Hydroxy, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder Oxyniederalkylenoxymethylen substituiert sein können; $R_2$ Carboxy, Niederalkoxy-, Phenylniederalkoxy-, Niederalkenyloxy-, Niederalkoxyniederalkoxy-carbonyl, Carbamoyl, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Amino, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Phenylniederalkoxy, Phenoxy, $S(O)_m$-R, wobei m für 0, 1 oder 2 und R für Niederalkyl steht, Niederalkanoyl, Sulfamoyl, in dem die Aminogruppe gegebenenfalls durch Niederalkyl, Phenylniederalkyl mono- oder unabhängig voneinander disubstituiert ist, oder $PO_nH_2$ bedeutet, wobei n für 2 oder 3 steht; $X_3$ Methylen ist; $R_3$ Carboxy, 5-Tetrazolyl, $SO_3H$, $PO_2H_2$, $PO_3H_2$ oder Halogenniederalkylsulfamoyl bedeutet; und (hetero-)aromatische Reste einschliesslich der Ringe A und B jeweils gegebenenfalls zusätzlich substituiert sind durch einen oder mehrere Substituenten ausgewählt aus Halogen, Hydroxy, Niederalkoxy, jeweils gegebenenfalls durch Halogen oder Hydroxy substituiertes Niederalkyl bzw. Niederalkoxyniederalkyl, in freier Form oder in Salzform.

8. Eine Verbindung gemäss Anspruch 1 der Formel I, worin $X_2$ $C_1$-$C_{10}$-Alkylen oder $C_1$-$C_7$-Alkyliden, die gegebenenfalls substituiert sind durch Hydroxy, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder durch Oxyniederalkylenoxymethylen substituiert sein können, wobei ein C-Atom von $C_1$-$C_{10}$-Alkylen bzw. $C_1$-$C_7$-Alkyliden durch $C_2$-$C_6$-Alkylen überbrückt sein kann, oder $X_2$ $C_3$-$C_7$-Cycloalkylen bedeutet; $X_3$ Niederalkylen oder Niederalkyliden bedeutet; die Variablen $X_1$, $R_1$, $R_2$, $R_3$ die in Anspruch 7 angegebenen Bedeutungen haben; und die (hetero-)aromatischen Ringe einschliesslich der Ringe A und B wie in Anspruch 7 angegeben substituiert sein können, in freier Form oder in Salzform.

9. Eine Verbindung gemäss Anspruch 1 der Formel I, worin die Variablen $R_1$, $X_1$, $R_3$ die jeweils in einem der Ansprüche 1-7 angegebenen Bedeutungen haben; $X_2$ gegebenenfalls durch Hydroxy, 3- bis 7-gliedriges Cycloalkyl, Phenyl oder Imidazolyl substituiertes Niederalkylen oder Niederalkyliden bedeutet und $R_2$ Carboxy, Niederalkoxy-, Phenylniederalkoxy-, Niederalkoxyniederalkoxy-carbonyl, Carbamoyl, welches gegebenenfalls durch Niederalkyl, Phenylniederalkyl mono- oder unabhängig voneinander disubstituiert ist, Amino, Niederalkanoyl-, Phenylniederalkanoyl-, Niederalkansulfonylamino, Hydroxy, Niederalkoxy, Phenylniederalkoxy oder Phenoxy bedeutet; $X_3$ $-CH_2-$ bedeutet; wobei (hetero-)aromatische Reste einschliesslich der Ringe A und B jeweils gegebenenfalls durch einen oder mehrere Substituenten ausgewählt aus Halogen, Trifluormethyl, Hydroxy, Niederalkoxy, Niederalkyl, Hydroxyniederalkyl oder Niederalkoxyniederalkyl substituiert sind, in freier Form oder in Salzform.

10. Eine Verbindung gemäss Anspruch 1 der Formel I, worin $X_2$ gegebenenfalls durch Hydroxy, 3- bis 7-gliedriges Cycloalkyl, 7-gliedriges Cycloalkenyl, Phenyl oder Imidazolyl substituiertes Niederalkylen oder Niederalkyliden bedeutet, wobei ein C-Atom von Niederalkylen bzw. Niederalkyliden durch $C_2$-$C_6$-Alkylen überbrückt sein kann, oder $X_2$ $C_3$-$C_7$-Cycloalkylen bedeutet; die Variablen $X_1$, $X_3$, $R_1$, $R_2$ und $R_3$ die in Anspruch 7 bis 9 angegebenen Bedeutungen haben; und die Ringe A und B wie in Anspruch 9 angegeben substituiert sein können, in freier Form oder in Salzform.

11. Eine Verbindung gemäss Anspruch 1 der Formel

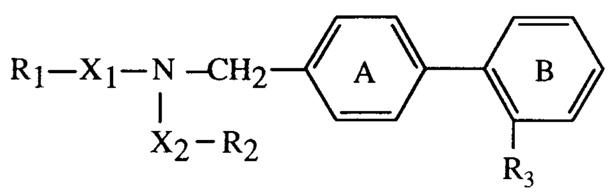

(Ia),

worin die Variablen $R_1$, $X_1$, $X_2$, $R_2$ und $R_3$ die jeweils in einem der Ansprüche 1-10 angegebenen Bedeutungen haben und die Ringe A und B wie in Anspruch 10 angegeben substituiert sein können, in freier Form oder in Salzform.

12. Eine Verbindung gemäss Anspruch 1 der Formel Ia, worin $X_2$ gegebenenfalls durch Hydroxy oder 3- bis 7-gliedriges Cycloalkyl substituiertes Niederalkylen oder Niederalkyliden bedeutet, wobei ein C-Atom von Niederalkylen bzw. Niederalkyliden durch $C_2$-$C_6$-Alkylen, überbrückt sein kann, oder worin $X_2$ $C_3$-$C_7$-Cycloalkylen bedeutet; die Variablen $R_1$, $X_1$, $R_2$ und $R_3$ die jeweils in einem der Ansprüche 1-10 angegebenen Bedeutungen haben; und die Ringe A und B wie in Anspruch 10 angegeben substituiert sein können, in freier Form oder in Salzform.

13. Eine Verbindung gemäss Anspruch 1 der Formel Ia, worin $X_2$ für die Gruppe der Formel

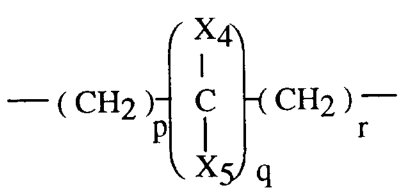

(Ib)

steht, in der p für 0 oder 1, q für 1 und r für 0 oder 1 stehen oder in der p für 1 bis 8 und q sowie r jeweils für 0 stehen; $X_4$ gegebenenfalls durch Hydroxy, 3- bis 7-gliedriges Cycloalkyl, Phenyl oder Imidazolyl substituiertes Niederalkyl oder Phenyl bedeutet; und $X_5$ Wasserstoff oder Niederalkyl bedeutet; $R_2$ Carboxy, Niederalkoxycarbonyl, Phenylniederalkoxycarbonyl, Niederalkoxyniederalkoxycarbonyl, Hydroxy, Niederalkoxy, Phenylniederalkoxy, Phenoxy, Amino, Niederalkanoylamino, Phenylniederalkanoylamino oder Niederalkansulfonylamino bedeutet; und die Variablen $R_1$, $X_1$ und $R_3$ die jeweils in einem der Ansprüche 1-7 angegebenen Bedeutungen haben; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**14.** Eine Verbindung gemäss Anspruch 1 der Formel Ia, worin $X_2$ für die Gruppe der Formel Ib steht, in der p für 0 oder 1, q für 1 und r für 0 oder 1 stehen oder in der p für 1 bis 8 und q sowie r jeweils für 0 stehen; $X_4$ gegebenenfalls durch Hydroxy, 3- bis 7-gliedriges Cycloalkyl, Phenyl oder Imidazolyl substituiertes Niederalkyl oder Phenyl bedeutet; und $X_5$ Wasserstoff oder Niederalkyl bedeutet; oder $X_4$ und $X_5$ gemeinsam für $C_2$-$C_6$-Alkylen, wie $C_4$-$C_5$-Alkylen, stehen; oder $X_2$ $C_3$-$C_7$-Cycloalkylen, wie $C_5$-$C_6$-Cycloalkylen, bedeutet; $R_2$ Carboxy, Niederalkoxycarbonyl, Phenylniederalkoxycarbonyl, Niederalkoxyniederalkoxycarbonyl, Hydroxy, Niederalkoxy, Phenylniederalkoxy, Phenoxy, Amino, Niederalkanoylamino, Phenylniederalkanoylamino oder Niederalkansulfonylamino bedeutet; und die Variablen $R_1$, $X_1$ und $R_3$ die jeweils in einem der Ansprüche 1-7 angegebenen Bedeutungen haben; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**15.** Eine Verbindung gemäss Anspruch 1 der Formel Ia, worin $R_1$ Niederalkyl, wie $C_3$-$C_5$-Alkyl, oder Niederalkenyl, wie $C_3$-$C_5$-Alkenyl, bedeutet; $X_1$ für CO oder ferner $SO_2$ steht; $X_2$ für die Gruppe der Formel Ib steht, in der p und r für 0 oder 1 und q für 1 stehen; $X_4$ gegebenenfalls durch Hydroxy, 3- bis 7-gliedriges Cycloalkyl, wie Cyclohexyl, durch gegebenenfalls durch Halogen oder Hydroxy substituiertes Phenyl oder Imidazolyl, wie 4-Imidazolyl, substituiertes Niederalkyl, wie $C_1$-$C_4$-Alkyl, oder Phenyl bedeutet; und $X_5$ Wasserstoff oder Niederalkyl, wie $C_1$-$C_4$-Alkyl, bedeutet; oder $X_4$ und $X_5$ gemeinsam $C_2$-$C_6$-Alkylen, wie $C_4$-$C_5$-Alkylen, bedeuten; oder $X_2$ $C_3$-$C_7$-Cycloalkylen, wie $C_5$-$C_6$-Cycloalkylen, bedeutet; $R_2$ Carboxy, Niederalkoxycarbonyl, wie $C_2$-$C_5$-Alkoxycarbonyl, Phenylniederalkoxycarbonyl, wie Phenyl-$C_1$-$C_4$-alkoxycarbonyl, Niederalkoxyniederalkoxycarbonyl, wie $C_1$-$C_4$-Alkoxy-$C_2$-$C_5$-alkoxycarbonyl, Hydroxy oder Niederalkoxy, wie $C_1$-$C_4$-Alkoxy, bedeutet; und $R_3$ Carboxy oder 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**16.** Eine Verbindung gemäss Anspruch 1 der Formel Ia, worin $R_1$ Niederalkyl, wie $C_3$-$C_5$-Alkyl, oder Niederalkenyl, wie $C_3$-$C_5$-Alkenyl, bedeutet; $X_1$ für CO oder ferner $SO_2$ steht; $X_2$ für die Gruppe der Formel Ib steht, in der p und r für 0 oder 1 und q für 1 stehen; $X_4$ gegebenenfalls durch Hydroxy, 3- bis 7-gliedriges Cycloalkyl, durch gegebenenfalls durch Halogen oder Hydroxy substituiertes Phenyl oder Imidazolyl, wie 4-Imidazolyl, substituiertes Niederalkyl, wie $C_1$-$C_4$-Alkyl, oder Phenyl bedeutet; und $X_5$ Wasserstoff oder Niederalkyl, wie $C_1$-$C_4$-Alkyl, bedeutet; $R_2$ Carboxy, Niederalkoxycarbonyl, wie $C_2$-$C_5$-Alkoxycarbonyl, Phenylniederalkoxycarbonyl, wie Phenyl-$C_1$-$C_4$-alkoxycarbonyl, Niederalkoxyniederalkoxycarbonyl, wie $C_1$-$C_4$-Alkoxy-$C_2$-$C_5$-alkoxycarbonyl, Hydroxy oder Niederalkoxy, wie $C_1$-$C_4$-Alkoxy, bedeutet; und $R_3$ Carboxy oder 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**17.** Eine Verbindung gemäss Anspruch 1 der Formel Ia, worin $R_1$ Niederalkyl, wie $C_3$-$C_5$-Alkyl, oder ferner Niederalkenyl, wie $C_3$-$C_5$-Alkenyl, bedeutet; $X_1$ für CO oder ferner $SO_2$ steht; $X_2$ für die Gruppe der Formel Ib steht, in der p für eine ganze Zahl von 1 bis 8 und q sowie r für 0 stehen; $R_2$ Hydroxy, Niederalkoxy, wie $C_1$-$C_4$-Alkoxy, Phenylniederalkoxy, wie Phenyl-$C_1$-$C_4$-alkoxy, Phenoxy, Niederalkanoylamino, wie $C_1$-$C_4$-Alkanoylamino, Phenylniederalkanoylamino, wie Phenyl-$C_1$-$C_4$-alkanoylamino, Niederalkansulfonylamino, wie $C_1$-$C_4$-Alkansulfonylamino, bedeutet; und $R_3$ Carboxy oder in erster Linie 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**18.** Eine Verbindung gemäss Anspruch 1 der Formel Ia, worin $R_1$ $C_3$-$C_5$-Alkyl oder in zweiter Linie $C_3$-$C_5$-Alkenyl, bedeutet; $X_1$ für CO, ferner $SO_2$ steht; $X_2$ für die Gruppe der Formel Ib steht, in der p und r unabhängig voneinander für 0 oder 1 und q für 1 stehen; $X_4$ $C_1$-$C_4$-Alkyl, Hydroxy-$C_1$-$C_4$-alkyl, $C_3$-$C_7$-Cycloalkyl-$C_1$-$C_4$-alkyl, Phenyl-$C_1$-$C_4$-alkyl oder Imidazolyl-$C_1$-$C_4$-alkyl bedeutet; und $X_5$ Wasserstoff oder $C_1$-$C_4$-Alkyl bedeutet; oder $X_4$ und $X_5$ gemeinsam für Tetramethylen, ferner Pentamethylen stehen; $R_2$ Carboxy oder $C_2$-$C_5$-Alkoxycarbonyl, ferner Phenyl-$C_1$-$C_4$-alkoxycarbonyl bedeutet; und $R_3$ Carboxy oder 5-Tetrazolyl bedeutet, in freier Form oder in Salzform.

**19.** Eine Verbindung gemäss Anspruch 1 der Formel Ia, worin $R_1$ $C_3$-$C_5$-Alkyl oder in zweiter Linie $C_3$-$C_5$-Alkenyl bedeutet; $X_1$ für CO, ferner $SO_2$ steht; $X_2$ für die Gruppe der Formel Ib steht, in der p und r jeweils für 0 oder 1 und q für

1 stehen; $X_4$ $C_1$-$C_4$-Alkyl, Hydroxy-$C_1$-$C_4$-alkyl, $C_3$-$C_7$-Cycloalkyl-$C_1$-$C_4$-alkyl, Phenyl-$C_1$-$C_4$-alkyl oder Imidazolyl-$C_1$-$C_4$-alkyl bedeutet; und $X_5$ Wasserstoff bedeutet; $R_2$ Carboxy oder $C_2$-$C_5$-Alkoxycarbonyl, ferner Phenyl-$C_1$-$C_4$-alkoxycarbonyl bedeutet; und $R_3$ Carboxy oder 5-Tetrazolyl bedeutet, in freier Form oder in Salzform.

**20.** Eine Verbindung gemäss Anspruch 1 der Formel Ia, worin $R_1$ $C_3$-$C_5$-Alkyl bedeutet; $X_1$ für CO steht; $X_2$ für die Gruppe der Formel Ib steht, in der q und r für 0 und p für 1 bis 3 stehen oder in der p und q für 1 und r für 0 stehen; $X_4$ $C_1$-$C_4$-Alkyl bedeutet; $X_5$ Wasserstoff oder $C_1$-$C_4$-Alkyl bedeutet; $R_2$ Carboxy oder $C_2$-$C_5$-Alkoxycarbonyl bedeutet; und $R_3$ Carboxy oder 5-Tetrazolyl bedeutet, in freier Form oder in Salzform.

**21.** Eine Verbindung gemäss einem der Ansprüche 1-20, worin $R_3$ 5-Tetrazolyl bedeutet, in freier Form oder in Salzform.

**22.** Eine Verbindung gemäss Anspruch 1 der Formel Ia, worin $R_1$ $C_3$-$C_5$-Alkyl bedeutet; $X_1$ für CO steht; $X_2$ für die Gruppe der Formel Ib steht, in der p für 0 oder 1, r für 0 and q für 1 stehen; $X_4$ $C_1$-$C_4$-Alkyl bedeutet; und $X_5$ Wasserstoff oder $C_1$-$C_4$-Alkyl bedeutet; oder $X_4$ und $X_5$ gemeinsam für Tetramethylen oder Pentamethylen stehen; $R_2$ Carboxy, oder $C_2$-$C_5$-Alkoxycarbonyl bedeutet; und $R_3$ 5-Tetrazolyl bedeutet, in freier Form oder in Salzform.

**23.** Eine Verbindung gemäss Anspruch 1 der Formel Ia, worin $R_1$ $C_3$-$C_5$-Alkyl bedeutet; $X_1$ für CO steht; $X_2$ für die Gruppe der Formel Ib steht, in der p für 0 oder 1, r für 0 und q für 1 stehen; $X_4$ und $X_5$ gemeinsam für Tetramethylen, ferner Pentamethylen stehen; $R_2$ Carboxy oder $C_2$-$C_5$-Alkoxycarbonyl bedeutet; und $R_3$ 5-Tetrazolyl bedeutet, in freier Form oder in Salzform.

**24.** Eine Verbindung gemäss Anspruch 1 der Formel Ia, worin $R_1$ $C_3$-$C_5$-Alkyl bedeutet; $X_1$ für CO steht; $X_2$ für die Gruppe der Formel Ib steht, in der p und r für 0 oder 1 und q für 1 stehen; $X_4$ $C_1$-$C_4$-Alkyl bedeutet; und $X_5$ Wasserstoff bedeutet; $R_2$ Carboxy oder $C_2$-$C_5$-Alkoxycarbonyl bedeutet; und $R_3$ 5-Tetrazolyl bedeutet, in freier Form oder in Salzform.

**25.** Eine Verbindung der Formel Ia gemäss einem der Ansprüche 13-24, worin $X_2$ für die Gruppe der Formel Ib steht, q 1 bedeutet und $X_4$ und $X_5$ unterschiedliche Bedeutungen haben, in freier Form oder in Salzform, in welcher das betreffende, die Variablen $X_4$ und $X_5$ aufweisende, asymmetrische C-Atom die S-Konfiguration hat.

**26.** (S)-N-(1-Carboxy-2-methyl-prop-1-yl)-N-pentanoyl-N-[2'-(1H-tetrazol-5-yl)biphenyl-4-ylmethyl]-amin, in freier Form oder in Salzform gemäss Anspruch 1.

**27.** N-(2-Carboxy-2,2-tetramethylen-ethyl)-N-pentanoyl-N-[2'-(1H-tetrazol-5-yl)biphenyl-4-ylmethyl]-amin, in freier Form oder in Salzform gemäss Anspruch 1.

**28.** N-(2-Carboxy-2-ethyl-but-1-yl)-N-pentanoyl-N-[2'-( 1H-tetrazol-5-yl)biphenyl-4-ylmethyl]-amin, in freier Form oder in Salzform gemäss Anspruch 1.

**29.** (S)-N-(1-Carboxy-2-methyl-prop-1-yl)-N-ethoxycarbonyl-N-[2'-(1H-tetrazol-5-yl)biphenyl-4-ylmethyl]-amin, in freier Form oder in Salzform gemäss Anspruch 1.

**30.** N-(1-carboxycyclohexyl)-N-pentanoyl-N-[2'-(1H-tetrazol-5-yl)biphenyl-4-ylmethyl]-amin, in freier Form oder in Salzform gemäss Anspruch 1.

**31.** Eine Verbindung gemäss Anspruch 1 ausgewählt aus der Gruppe bestehend aus:
(S)-N-(1-Carboxyethyl)-N-pentanoyl-N-[2'-(1H-tetrazol-5-yl)biphenyl-4-ylmethyl]-amin,
N-(2-Hydroxyethyl)-N-pentanoyl-N-[2'-(1H-tetrazol-5-yl)biphenyl-4-ylmethyl]-amin,
N-(2-Ethoxycarbonyl-2,2-tetramethylen-ethyl)-N-pentanoyl-N-[2'-(1H-tetrazol-5-yl)biphenyl-4-ylmethyl]-amin,
N-(2-Ethoxycarbonyl-2-ethyl-but-1-yl)-N-pentanoyl-N-[2'-( 1H-tetrazol-5-yl)biphenyl-4-ylmethyl]-amin,
N-(2-Ethoxycarbonyl-2-methyl-prop-1-yl)-N-pentanoyl-N-[2'-(1H-tetrazol-5-yl)biphenyl-4-ylmethyl]-amin,
(S)-N-(1-Hydroxymethyl-2-methyl-prop-1-yl)-N-pentanoyl-N-[2'-(1H-tetrazol-5-yl)biphenyl-4-ylmethyl]-amin,
N-(2-Ethoxycarbonyl-2,2-pentamethylen-ethyl)-N-pentanoyl-N-[2'-(1H-tetrazol-5-yl)biphenyl-4-ylmethyl]-amin,
(S)-N-(1-Carboxy-2-methyl-prop-1-yl)-N-propyloxycarbonyl-N-[2'-(1H-tetrazol-5-yl)biphenyl-4-ylmethyl]-amin,
N-(2-carboxy-2-methyl-propyl)-N-pentanoyl-N-[2'-(1H-tetrazol-5-yl)biphenyl-4-ylmethyl]-amin,
N-(2-carboxy-2,2-pentamethylen-ethyl)-N-pentanoyl-N-[2'-(1H-tetrazol-5-yl)biphenyl-4-ylmethyl]-amin,
(S)-N-(1-aminocarbonyl-2-methyl-prop-1-yl)-N-pentanoyl-N-[2'-(1H-tetrazol-5-yl)biphenyl-4-ylmethyl]-amin und
(S)-N-(1-carboxy-2-methyl-prop-1-yl)-N-(5-oxopent-1-en-5-yl)-N-[2'-(1H-tetrazol-5-yl)biphenyl-4-ylmethyl]-amin,
jeweils in freier Form oder in Salzform.

32. Eine Verbindung gemäss Anspruch 1 ausgewählt aus der Gruppe bestehend aus:
N-Carboxymethyl-N-pentanoyl-N-[2'-(1H-tetrazol-5-yl)biphenyl-4-ylmethyl]-amin,
(S)-N-(1-Methoxycarbonylethyl)-N-pentanoyl-N-[2'-(1H-tetrazol-5-yl)biphenyl-4-ylmethyl]-amin,
N-[1-Carboxy-2-(4-fluorphenyl)-ethyl]-N-pentanoyl-N-[2'-(1H-tetrazol-5-yl)biphenyl-4-ylmethyl]-amin,
N-[2-(4-Fluorphenyl)-1-methoxycarbonyl-ethyl]-N-pentanoyl-N-[2'-(1H-tetrazol-5-yl)biphenyl-4-ylmethyl]-amin,
N-[2-(4-Fluorphenyl)-1-hydroxymethyl-ethyl]-N-pentanoyl-N-[2'-(1H-tetrazol-5-yl)biphenyl-4-ylmethyl]-amin,
N-(2'-Carboxybiphenyl-4-ylmethyl)-N-[1-carboxy-2-(4-fluorphenyl)-ethyl]-N-pentanoyl-amin,
N-(2'-Carboxybiphenyl-4-ylmethyl)-N-[2-(4-fluorphenyl)-1-methoxycarbonyl-ethyl]-N-pentanoyl-amin,
(S)-N-(2'-Carboxybiphenyl-4-ylmethyl)-N-(1-hydroxymethyl-2-phenyl-ethyl)-N-pentanoyl-amin,
(S)-N-(2'-Carboxybiphenyl-4-ylmethyl)-N-(1-hydroxymethyl-2-imidazol-4-yl-ethyl)-N-pentanoyl-amin,
(R)-N-(1-Carboxyethyl)-N-pentanoyl-N-[2'-(1H-tetrazol-5-yl)biphenyl-4-ylmethyl]-amin,
(1S),(2S)-N-(1-Carboxy-2-methyl-but-1-yl)-N-pentanoyl-N-[2'-(1H-tetrazol-5-yl)biphenyl-4-ylmethyl]-amin,
(1S),(2S)-N-(1-Methoxycarbonyl-2-methyl-but-1-yl)-N-pentanoyl-N-[2'-(1H-tetrazol-5-yl)biphenyl-4-ylmethyl]-amin,
(S)-N-(1-Carboxybut-1-yl)-N-pentanoyl-N-[2'-(1H-tetrazol-5-yl)biphenyl-4-ylmethyl]-amin,
(S)-N-(1-Methoxycarbonylbut-1-yl)-N-pentanoyl-N-[2'-(1H-tetrazol-5-yl)biphenyl-4-ylmethyl]-amin,
(S)-N-(1-Carboxyethyl)-N-hexanoyl-N-[2'-(1H-tetrazol-5-yl)biphenyl-4-ylmethyl]-amin,
(S)-N-Butanoyl-N-(1-carboxyethyl)-N-[2'-(1H-tetrazol-5-yl)biphenyl-4-ylmethyl]-amin,
(S)-N-(1-Carboxyprop-1-yl)-N-pentanoyl-N-[2'-(1H-tetrazol-5-yl)biphenyl-4-ylmethyl]-amin,
(S)-N-(1-Carboxy-2-cyclohexyl-ethyl)-N-pentanoyl-N-[2'-(1H-tetrazol-5-yl)biphenyl-4-ylmethyl]-amin,
(S)-N-(2-Cyclohexyl-1-methoxycarbonyl-ethyl)-N-pentanoyl-N-[2'-(1H-tetrazol-5-yl)biphenyl-4-ylmethyl]-amin,
(R)-N-(1-Carboxy-2-methyl-prop-1-yl)-N-pentanoyl-N-[2'-(1H-tetrazol-5-yl)biphenyl-4-ylmethyl]-amin,
N-(2-Methoxyethyl)-N-pentanoyl-N-[2'-(1H-tetrazol-5-yl)biphenyl-4-ylmethyl]-amin,
N-(2-Benzyloxyethyl)-N-pentanoyl-N-[2'-(1H-tetrazol-5-yl)biphenyl-4-ylmethyl]-amin,
N-(3-Methoxyprop-1-yl)-N-pentanoyl-N-[2'-(1H-tetrazol-5-yl)biphenyl-4-ylmethyl]-amin,
N-(3-Benzyloxyprop-1-yl)-N-pentanoyl-N-[2'-(1H-tetrazol-5-yl)biphenyl-4-ylmethyl]-amin,
N-(3-Hydroxyprop-1-yl)-N-pentanoyl-N-[2'-(1H-tetrazol-5-yl)biphenyl-4-ylmethyl]-amin,
N-(1-Methoxycarbonyl-1-methyl-ethyl)-N-pentanoyl-N-[2'-(1H-tetrazol-5-yl)biphenyl-4-ylmethyl]-amin,
N-(2-Carboxyethyl)-N-pentanoyl-N-[2'-(1H-tetrazol-5-yl)biphenyl-4-ylmethyl]-amin,
N-(2-Carboxyprop-1-yl)-N-pentanoyl-N-[2'-(1H-tetrazol-5-yl)biphenyl-4-ylmethyl]-amin,
N-(1-Carboxy-1-methyl-ethyl)-N-pentanoyl-N-[2'-(1H-tetrazol-5-yl)biphenyl-4-ylmethyl]-amin,
N-(5-Hydroxypent-1-yl)-N-pentanoyl-N-[2'-(1H-tetrazol-5-yl)biphenyl-4-ylmethyl]-amin,
N-(1-Carboxyprop-2-yl)-N-pentanoyl-N-[2'-(1H-tetrazol-5-yl)biphenyl-4-ylmethyl]-amin,
N-(2-Ethoxycarbonyl-3-methyl-but-1-yl)-N-pentanoyl-N-[2'-(1H-tetrazol-5-yl)biphenyl-4-ylmethyl]-amin,
N-(2-Carboxy-3-methyl-but-1-yl)-N-pentanoyl-N-[2'-(1H-tetrazol-5-yl)biphenyl-4-ylmethyl]-amin,
N-(3-Phenoxyprop-1-yl)-N-pentanoyl-N-[2'-(1H-tetrazol-5-yl)biphenyl-4-ylmethyl]-amin,
N-[2-(4-Hydroxyphenyl)ethyl]-N-pentanoyl-N-[2'-(1H-tetrazol-5-yl)biphenyl-4-ylmethyl]-amin,
N-[3-(4-Hydroxyphenyl)prop-1-yl]-N-pentanoyl-N-[2'-(1H-tetrazol-5-yl)biphenyl-4-ylmethyl]-amin,
N-(8-Hydroxyoct-1-yl)-N-pentanoyl-N-[2'-(1H-tetrazol-5-yl)biphenyl-4-ylmethyl]-amin,
N-(2-Methansulfonylaminoethyl)-N-pentanoyl-N-[2'-(1H-tetrazol-5-yl)biphenyl-4-ylmethyl]-amin,
N-(3-Acetylaminoprop-1-yl)-N-pentanoyl-N-[2'-(1H-tetrazol-5-yl)biphenyl-4-ylmethyl]-amin,
N-(2-Methoxy-2-oxo-1-phenyl-ethyl)-N-pentanoyl-N-[2'-(1H-tetrazol-5-yl)biphenyl-4-ylmethyl]-amin,
N-(4-Hydroxybut-2-yl)-N-pentanoyl-N-[2'-(1H-tetrazol-5-yl)biphenyl-4-ylmethyl]-amin,
N-(2-Hydroxy-1-phenyl-ethyl)-N-pentanoyl-N-[2'-(1H-tetrazol-5-yl)biphenyl-4-ylmethyl]-amin,
N-[3-(4-Hydroxybenzylcarbonylamino)prop-1-yl]-N-pentanoyl-N-[2'-(1H-tetrazol-5-yl)biphenyl-4-ylmethyl]-amin,
N-(3-Ethoxycarbonylcyclohexyl)-N-pentanoyl-N-[2'-(1H-tetrazol-5-yl)biphenyl-4-ylmethyl]-amin,
N-(3-Carboxycyclohexyl)-N-pentanoyl-N-[2'-(1H-tetrazol-5-yl)biphenyl-4-ylmethyl]-amin,
cis-N-(4-Carboxycyclohexyl)-N-pentanoyl-N-[2'-(1H-tetrazol-5-yl)biphenyl-4-ylmethyl]-amin,
cis-N-(2-Ethoxycarbonylcyclohexyl)-N-pentanoyl-N-[2'-(1H-tetrazol-5-yl)biphenyl-4-ylmethyl]-amin,
cis-N-(2-Carboxycyclohexyl)-N-pentanoyl-N-[2'-(1H-tetrazol-5-yl)biphenyl-4-ylmethyl]-amin,
N-{2-[2-(4-Hydroxyphenyl)ethylaminocarbonyl]-2,2-tetramethylen-ethyl}-N-pentanoyl-N-[2'-(1H-tetrazol-5-yl)biphenyl-4-ylmethyl]-amin,
(S)-N-{1-[2-(4-Hydroxyphenyl)ethylaminocarbonyl]-2-methyl-prop-1-yl}-N-pentanoyl-N-[2'-(1H-tetrazol-5-yl)biphenyl-4-ylmethyl]-amin,
(S)-N-(1-Carboxy-2,2-dimethyl-prop-1-yl)-N-pentanoyl-N-[2'-(1H-tetrazol-5-yl)biphenyl-4-ylmethyl]-amin,
(S)-N-(1-Methoxycarbonyl-2-methyl-prop-1-yl)-N-pentanoyl-N-[2'-(1H-tetrazol-5-yl)biphenyl-4-ylmethyl]-amin,
N-(4-Phenoxybut-1-yl)-N-pentanoyl-N-[2'-(1H-tetrazol-5-yl)biphenyl-4-ylmethyl]-amin,
N-(2-Hydroxy-1-phenyl-2-oxo-ethyl)-N-pentanoyl-N-[2'-(1H-tetrazol-5-yl)biphenyl-4-ylmethyl]-amin,
(S)-N-(1-Benzyloxycarbonyl-2-methyl-prop-1-yl)-N-pentanoyl-N-[2'-(1H-tetrazol-5-yl)biphenyl-4-ylmethyl]-amin,
N-Butanoyl-N-(1-carboxy-1-methyl-ethyl)-N-[2'-(1H-tetrazol-5-yl)biphenyl-4-ylmethyl]-amin,

N-(4-Hydroxybut-1-yl)-N-pentanoyl-N-[2'-(1H-tetrazol-5-yl)biphenyl-4-ylmethyl]-amin,
(S)-N-(1-Benzyloxycarbonyl-2-methyl-prop-1-yl)-N-[3-bromo-2'-(1H-tetrazol-5-yl)biphenyl-4-ylmethyl]-N-pentanoyl-amin,
(S)-N-[3-Brom-2'-(1H-tetrazol-5-yl)-biphenyl-4-ylmethyl]-N-(1-carboxy-2-methyl-prop-1-yl)-N-pentanoyl-amin,
N-(2-Acetylaminoethyl)-N-pentanoyl-N-[2'-(1H-tetrazol-5-yl)biphenyl-4-ylmethyl]-amin,
N-[2-(n-Butoxycarbonyl)-2,2-tetramethylen-ethyl]-N-pentanoyl-N-[2'-(1H-tetrazol-5-yl)biphenyl-4-ylmethyl]-amin,
N-(2-Benzylaminocarbonyl-2,2-tetramethylen-ethyl)-N-pentanoyl-N-[2'-(1H-tetrazol-5-yl)biphenyl-4-ylmethyl]-amin,
(S)-N-Butyloxycarbonyl-N-(1-Carboxy-2-methyl-prop-1-yl)-N-[2'-(1H-tetrazol-5-yl)biphenyl-4-ylmethyl]-amin,
(S)-N-(1-Carboxy-2-methyl-prop-1-yl)-N-methoxycarbonyl-N-[2'-(1H-tetrazol-5-yl)biphenyl-4-ylmethyl]-amin,
N-(2-Diethylaminocarbonyl-2,2-tetramethylen-ethyl)-N-pentanoyl-N-[2'-(1H-tetrazol-5-yl)biphenyl-4-ylmethyl]-amin,
N-(2-Methyl-2-morpholin-4-ylcarbonyl-propyl)-N-pentanoyl-N-[2'-(1H-tetrazol-5-yl)biphenyl-4-ylmethyl]-amin,
N-(1-Carboxycyclopentyl)-N-pentanoyl-N-[2'-(1H-tetrazol-5-yl)biphenyl-4-ylmethyl]-amin,
N-(1-Carboxy-1-ethyl-prop-1-yl)-N-pentanoyl-N-[2'-(1H-tetrazol-5-yl)biphenyl-4-ylmethyl]-amin,
(S)-N-(5-Amino-1-carboxy-pent-1-yl)-N-pentanoyl-N-[2'-(1H-tetrazol-5-yl)biphenyl-4-ylmethyl]-amin,
N-Butansulfonyl-N-(2-ethoxycarbonyl-2,2-pentamethylen-ethyl)-N-[2'-(1H-tetrazol-5-yl)biphenyl-4-ylmethyl]-amin,
N-Butansulfonyl-N-(2-carboxy-2,2-pentamethylen-ethyl)-N-[2'-(1H-tetrazol-5-yl)biphenyl-4-ylmethyl]-amin,
N-Butansulfonyl-N-(2-ethoxycarbonyl-2-methyl-prop-1-yl)-N-[2'-(1H-tetrazol-5-yl)biphenyl-4-ylmethyl]-amin,
N-Butansulfonyl-N-(2-carboxy-2-methyl-prop-1-yl)-N-[2'-(1H-tetrazol-5-yl)biphenyl-4-ylmethyl]-amin,
(S)-N-Butansulfonyl-N-(1-tert.-butoxycarbonylethyl)-N-[2'-(1H-tetrazol-5-yl)biphenyl-4-ylmethyl]-amin,
(S)-N-Butansulfonyl-N-(1-carboxyethyl)-N-[2'-(1H-tetrazol-5-yl)biphenyl-4-ylmethyl]-amin,
(S)-N-Butansulfonyl-N-(1-carboxy-2-methyl-prop-1-yl)-N-[2'-(1H-tetrazol-5-yl)biphenyl-4-ylmethyl]-amin,
(S)-N-(2-Methyl-1-methylaminocarbonyl-prop-1-yl)-N-pentanoyl-N-[2'-(1H-tetrazol-5-yl)biphenyl-4-ylmethyl]-amin,
(S)-N-(1-Dimethylaminocarbonyl-2-methyl-prop-1-yl)-N-pentanoyl-N-[2'-(1H-tetrazol-5-yl)biphenyl-4-ylmethyl]-amin,
(S)-N-(2-Methyl-1-morpholin-4-ylcarbonyl-prop-1-yl)-N-pentanoyl-N-[2'-(1H-tetrazol-5-yl)biphenyl-4-ylmethyl]-amin,
(S)-N-(2'-Carboxybiphenyl-4-ylmethyl)-N-(1-carboxy-2-methyl-prop-1-yl)-N-pentanoyl-amin,
(S)-N-(1,2-Dicarboxyethyl)-N-pentanoyl-N-[2'-(1H-tetrazol-5-yl)biphenyl-4-ylmethyl]-amin,
(S)-N-(1-Carboxy-3-phenyl-prop-1-yl)-N-pentanoyl-N-[2'-(1H-tetrazol-5-yl)biphenyl-4-ylmethyl]-amin,
(S)-N-(2-Cyclohexyl-1-hydroxymethyl-ethyl)-N-pentanoyl-N-[2'-(1H-tetrazol-5-yl)biphenyl-4-ylmethyl]-amin,
(R)-N-(1-Methoxycarbonyl-2-methyl-prop-1-yl)-N-pentanoyl-N-[2'-(1H-tetrazol-5-yl)biphenyl-4-ylmethyl]-amin,
(S)-N-(2-Hydroxy-1-methoxycarbonyl-ethyl)-N-pentanoyl-N-[2'-(1H-tetrazol-5-yl)biphenyl-4-ylmethyl]-amin,
N-Pentanoyl-N-(1H-tetrazol-5-ylmethyl)-N-[2'-(1H-tetrazol-5-yl)biphenyl-4-ylmethyl]-amin,
N-Pentanoyl-N-pyrid-3-ylmethyl-N-[2'-(1H-tetrazol-5-yl)biphenyl-4-ylmethyl]-amin,
(S)-N-(1-Carboxy-4-guanidino-but-1-yl)-N-pentanoyl-N-[2'-(1H-tetrazol-5-yl)biphenyl-4-ylmethyl]-amin,
N-(2-Hydroxy-1-methoxycarbonyl-prop-1-yl)-N-pentanoyl-N-[2'-(1H-tetrazol-5-yl)biphenyl-4-ylmethyl]-amin,
N-(1-Benzyloxycarbonyl-1-methyl-ethyl)-N-butanoyl-N-[2'-(1H-tetrazol-5-yl)biphenyl-4-ylmethyl]-amin,
(S)-N-(1-Carboxy-3-methyl-but-1-yl)-N-pentanoyl-N-[2'-(1H-tetrazol-5-yl)biphenyl-4-ylmethyl]-amin,
N-(1-Carboxy-2-hydroxy-ethyl)-N-pentanoyl-N-[2'-(1H-tetrazol-5-yl)biphenyl-4-ylmethyl]-amin,
(S)-N-(1-Carboxy-2-hydroxy-ethyl)-N-pentanoyl-N-[2'-(1H-tetrazol-5-yl)biphenyl-4-ylmethyl]-amin,
(S)-N-[2-Methyl-1-(2-phenylethylaminocarbonyl)-prop-1-yl]-N-pentanoyl-N-[2'-(1H-tetrazol-5-yl)biphenyl-4-ylmethyl]-amin,
(S)-N-(2-Benzyloxy-1-hydroxymethyl-ethyl)-N-pentanoyl-N-[2'-(1H-tetrazol-5-yl)biphenyl-4-ylmethyl]-amin,
(S)-N-(1-Carboxy-2-methyl-prop-1-yl)-N-pentanoyl-N-[2'-(1H-tetrazol-5-yl)biphenyl-3-ylmethyl]-amin,
(S)-N-(1-Carboxy-2-methyl-prop-1-yl)-N-pentanoyl-N-[3'-(1H-tetrazol-5-yl)biphenyl-4-ylmethyl]-amin,
(S)-N-[2-Methyl-1-(1,2,3,4-tetrahydrochinol-1-ylcarbonyl)-prop-1-yl]-N-pentanoyl-N-[2'-(1H-tetrazol-5-yl)biphenyl-4-ylmethyl]-amin,
(S)-N-(2-Methyl-1-piperidin-1-ylcarbonyl-prop-1-yl)-N-pentanoyl-N-[2'-(1H-tetrazol-5-yl)biphenyl-4-ylmethyl]-amin,
(S)-N-[2-Methyl-1-(1,2,3,4-tetrahydroisochinol-2-ylcarbonyl)-prop-1-yl]-N-pentanoyl-N-[2'-(1H-tetrazol-5-yl)biphenyl-4-ylmethyl]-amin,
N-(2-Hydroxymethyl-2-methyl-prop-1-yl)-N-pentanoyl-N-[2'-(1H-tetrazol-5-yl)biphenyl-4-ylmethyl]-amin,
N-Ethoxycarbonyl-N-(2-ethoxycarbonyl-2-methyl-prop-1-yl)-N-[2'-(1H-tetrazol-5-yl)biphenyl-4-ylmethyl]-amin und
N-(2-Carboxy-2-methyl-prop-1-yl)-N-ethoxycarbonyl-N-[2'-(1H-tetrazol-5-yl)biphenyl-4-ylmethyl]-amin, jeweils in freier Form oder in Salzform.

**33.** Eine Verbindung gemäss einem der Ansprüche 1 bis 32, in freier Form oder in Form eines pharmazeutisch verwendbaren Salzes, zur Anwendung in einem Verfahren zur therapeutischen Behandlung des menschlichen oder tierischen Körpers.

**34.** Eine Verbindung gemäss einem der Ansprüche 1 bis 33, in freier Form oder in Form eines pharmazeutisch verwendbaren Salzes, zur Anwendung als Antihypertensivum.

**35.** Ein pharmazeutisches Präparat, als Wirkstoff enthaltend eine Verbindung gemäss einem der Ansprüche 1 bis 34, in freier Form oder in Form eines pharmazeutisch verwendbaren Salzes, gegebenenfalls neben üblichen pharmazeutischen Hilfsstoffen.

**36.** Ein antihypertensiv wirksames pharmazeutisches Präparat gemäss Anspruch 35, dadurch gekennzeichnet, dass man einen antihypertensiv wirksamen Wirkstoff wählt.

**37.** Verfahren zur Herstellung einer Verbindung der Formel

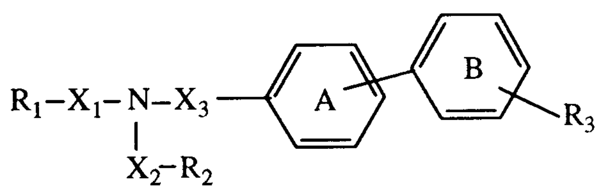

(I) oder

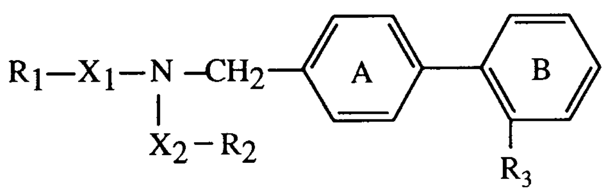

(Ia),

worin $R_1$, $R_2$, $R_3$, $X_1$, $X_2$ und $X_3$ sowie die Substituenten der Ringe A und B die jeweils in einem der Ansprüche 1-25 angegebenen Bedeutungen haben; in freier Form oder in Salzform, dadurch gekennzeichnet, dass man

a) in einer Verbindung der Formel

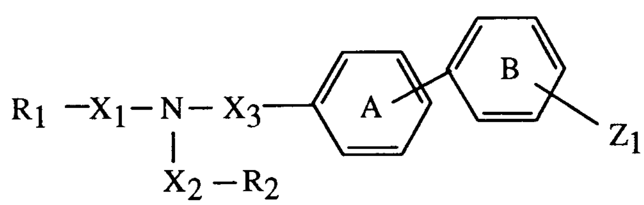

(II)

oder einem Salz davon, worin $Z_1$ einen in $R_3$ überführbaren Rest bedeutet, $Z_1$ in $R_3$ überführt oder
b) eine Verbindung der Formel $R_1$-$X_1$OH (IIIa), ein reaktionsfähiges Derivat davon oder ein Salz davon mit einer Verbindung der Formel

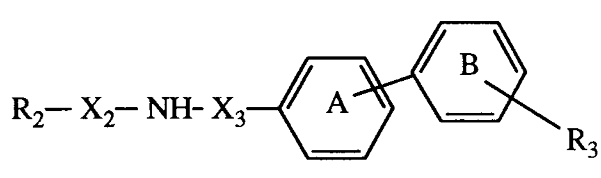

(IIIb)

oder einem Salz davon umsetzt und jeweils, wenn erwünscht, eine verfahrensgemäss oder auf andere Weise erhältliche Verbindung I in freier Form oder in Salzform in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

**38.** Verfahren zur Herstellung eines pharmazeutischen Präparats gemäss Anspruch 35 oder 36, dadurch gekennzeichnet, dass man den Wirkstoff, gegebenenfalls unter Beimischung von üblichen pharmazeutischen Hilfsstoffen, zu einem pharmazeutischen Präparat verarbeitet.

**39.** Verfahren gemäss Anspruch 38 zur Herstellung eines antihypertensiv wirksamen pharmazeutischen Präparats gemäss Anspruch 34, dadurch gekennzeichnet, dass man einen antihypertensiv wirksamen Wirkstoff wählt.

**40.** Verwendung einer Verbindung gemäss einem der Ansprüche 1 bis 34, in freier Form oder in Form eines pharmazeutisch verwendbaren Salzes, zur Herstellung eines pharmazeutischen Präparats.

**41.** Verwendung einer Verbindung gemäss einem der Ansprüche 1 bis 34, in freier Form oder in Form eines pharmazeutisch verwendbaren Salzes, zur Herstellung eines pharmazeutischen Präparats auf nicht-chemischem Wege.

**42.** Verwendung einer Verbindung gemäss einem der Ansprüche 1-34, in freier Form oder in Form eines pharmazeutisch verwendbaren Salzes, zur Herstellung eines Antihypertensivums.

**43.** Verwendung einer Verbindung gemäss einem der Ansprüche 1-34, in freier Form oder in Form eines pharmazeutisch verwendbaren Salzes, zur Herstellung eines pharmazeutischen Präparats zur therapeutischen oder prophylaktischen Behandlung von Herzinsuffizienz.

**44.** Verwendung einer Verbindung gemäss einem der Ansprüche 1-34, in freier Form oder in Form eines pharmazeutisch verwendbaren Salzes, zur Herstellung eines pharmazeutischen Präparats zur therapeutischen oder prophylaktischen Behandlung von Erkrankungen, die durch Angiotensin-II-Aktivität verursacht werden.

**Patentansprüche für folgende Vertragsstaaten : ES, GR**

**1.** Verfahren zur Herstellung einer Verbindung der Formel

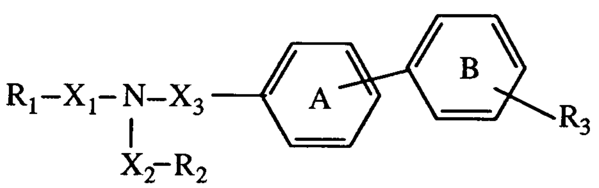

(I),

worin $R_1$ gegebenenfalls durch Halogen oder Hydroxy substituiertes Niederalkyl, Niederalkenyl oder Niederalkinyl oder $C_3$-$C_7$-Cycloalkyl- oder $C_3$-$C_7$-Cycloalkenyl oder Phenylniederalkyl, Phenylniederalkenyl oder Phenylniederalkinyl bedeutet; $X_1$ für CO, $SO_2$ oder -O-C(=O)- steht, wobei das Kohlenstoffatom der Carbonylgruppe an das in der Formel I eingezeichnete Stickstoffatom gebunden ist; $X_2$ gegebenenfalls durch Hydroxy, Carboxy, Amino, Guanidino, $C_3$-$C_7$-Cycloalkyl, $C_3$-$C_7$-Cycloalkenyl, Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen, $C_2$-$C_{10}$-Alkyliden oder $C_3$-$C_7$-Cycloalkylen bedeutet, wobei ein Kohlenstoffatom von $C_1$-$C_{10}$-Alkylen bzw. $C_2$-$C_{10}$-Alkyliden zusätzlich durch $C_2$-$C_6$-Alkylen überbrückt sein kann, und wobei $C_3$-$C_7$-Cycloalkyl oder $C_3$-$C_7$-Cycloalkenyl gegebenenfalls ein- oder mehrfach substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Formyl, Diniederalkoxymethyl oder Oxyniederalkylenoxymethylen; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen

oder $C_2$-$C_{10}$-Alkyliden gegebenenfalls durch -O- unterbrochen oder an zwei benachbarten C-Atomen mit einem Benzolring kondensiert sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, 1H-Tetrazol-5-yl, Pyridyl, Hydroxy, Niederalkoxy, Niederalkenyloxy, Phenylniederalkoxy, Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, oder $PO_nH_2$ bedeutet, wobei n für 2 oder 3 steht; $X_3$ $C_1$-$C_{10}$-Alkylen oder $C_2$-$C_{10}$-Alkyliden bedeutet; $R_3$ Carboxy, 5-Tetrazolyl, $SO_3H$, $PO_2H_2$, $PO_3H_2$ oder Halogenniederalkylsulfamoyl ist; und wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen oder an zwei benachbarten C-Atomen mit einem Benzolring kondensiert sind, durch Formyl, Diniederalkoxymethyl oder Oxyniederalkylenoxymethylen; wobei mit "nieder" bezeichnete Reste und Gruppen bis und mit 7 Kohlenstoffatome enthalten; in freier Form oder in Salzform; dadurch gekennzeichnet, dass man

a) in einer Verbindung der Formel

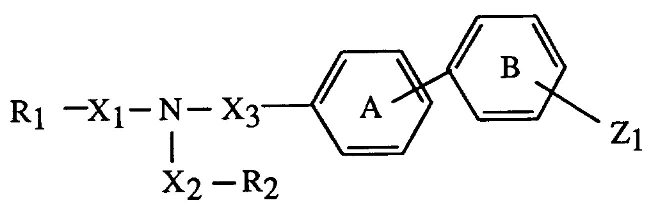 (II)

oder einem Salz davon, worin $Z_1$ einen in $R_3$ überführbaren Rest bedeutet, $Z_1$ in $R_3$ überführt oder

b) eine Verbindung der Formel $R_1$-$X_1$OH (IIIa), ein reaktionsfähiges Derivat davon oder ein Salz davon mit einer Verbindung der Formel

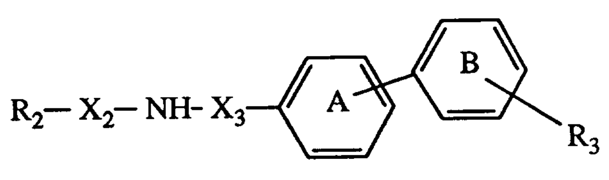 (IIIb)

oder einem Salz davon umsetzt und jeweils, wenn erwünscht, eine verfahrensgemäss oder auf andere Weise erhältliche Verbindung I in freier Form oder in Salzform in eine andere Verbindung I überführt, ein verfahrensgemäss erhaltliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

2. Verfahren gemäss Anspruch 1 zur Herstellung einer Verbindung der Formel I, worin $R_2$ von Carboxy verschieden ist und $R_3$ 5-Tetrazolyl bedeutet, dadurch gekennzeichnet, dass man

(i) von einer Verbindung der Formel (II) ausgeht, worin $Z_1$ Cyano bedeutet, und diese mit $HN_3$ oder einem Alkalimetallsalz davon, mit einem Triniederalkylzinnazid oder Triphenylzinnazid umsetzt; oder

(ii) von einer Verbindung der Formel (II) ausgeht, worin $Z_1$ durch Triphenylmethyl, gegebenenfalls durch Nitro substituiertes Benzyl, Niederalkoxymethyl, Niederalkylthiomethyl, Triniederalkylsilyl, 2-Cyanoethyl, Niederalkoxyniederalkoxymethyl, Benzyloxymethyl oder Phenacyl geschütztes 5-Tetrazolyl bedeutet, und die Schutzgruppe abspaltet und,

wenn erwünscht, eine verfahrensgemäss erhältliche Verbindung der Formel I, worin $R_2$ von Carboxy verschieden ist und $R_3$ 5-Tetrazolyl bedeutet, in eine Verbindung der Formel I überführt, worin $R_2$ Carboxy ist.

3. Verfahren gemäss Anspruch 1 oder 2 zur Herstellung einer Verbindung der Formel I, worin worin $R_1$ gegebenenfalls durch Halogen oder Hydroxy substituiertes Niederalkyl, Niederalkenyl oder Niederalkinyl oder $C_3$-$C_7$-Cycloalkyl oder $C_3$-$C_7$-Cycloalkenyl oder Phenylniederalkyl, Phenylniederalkenyl oder Phenylniederalkinyl bedeutet; $X_1$ für CO oder $SO_2$ steht; $X_2$ einen gegebenenfalls durch Hydroxy, $C_3$-$C_7$-Cycloalkyl oder $C_3$-$C_7$-Cycloalkenyl oder Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen oder $C_2$-$C_{10}$-Alkyliden oder $C_3$-$C_7$-Cycloalkylen bedeutet, wobei ein Kohlenstoffatom von $C_1$-$C_{10}$-Alkylen bzw. $C_2$-$C_{10}$-Alkyliden zusätzlich durch $C_2$-$C_6$-Alkylen überbrückt sein kann, und wobei $C_3$-$C_7$-Cycloalkylen gegebenenfalls ein- oder mehrfach substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Formyl, Diniederalkoxymethyl oder Oxyniederalkylenoxymethylen; $R_2$ Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Niederalkanoyl-, Phenylniederalkanoyl-, Benzoyl-, Niederalkansulfonyl-, Benzolsulfonyl-amino, Formyl, Diniederalkoxymethyl, Oxynieder-alkylenoxymethylen, Hydroxy, Niederalkoxy, Niederalkenyloxy, Phenylniederalkoxy, Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, oder $PO_nH_2$ bedeutet, wobei n für 2 oder 3 steht; $X_3$ $C_1$-$C_{10}$-Alkylen oder $C_2$-$C_{10}$-Alkyliden bedeutet; $R_3$ Carboxy, 5-Tetrazolyl, $SO_3H$, $PO_2H_2$, $PO_3H_2$ oder Halogenniederalkylsulfamoyl ist; wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, durch Formyl, Diniederalkoxymethyl oder Oxyniederalkylenoxymethylen; in freier Form oder in Salzform.

4. Verfahren gemäss Anspruch 1 oder 2 zur Herstellung einer Verbindung der Formel I, worin gegebenenfalls durch Halogen oder Hydroxy substituiertes Niederalkyl, Niederalkenyl oder Niederalkinyl oder $C_3$-$C_7$-Cycloalkyl oder $C_3$-$C_7$-Cycloalkenyl oder Phenylniederalkyl, Phenylniederalkenyl oder Phenylniederalkinyl bedeutet; $X_1$ für CO oder $SO_2$ steht; $X_2$ gegebenenfalls durch Hydroxy, $C_3$-$C_7$-Cycloalkyl oder $C_3$-$C_7$-Cycloalkenyl oder Phenyl oder einen entsprechenden 5- oder 6-gliedrigen und monocyclischen aromatischen Rest, der bis zu vier gleiche oder verschiedene Heteroatome aufweist, substituiertes $C_1$-$C_{10}$-Alkylen oder $C_2$-$C_{10}$-Alkyliden bedeutet; $R_2$ Carboxy, Carboxy,

welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Amino, Amino, welches durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, Niederalkanoyl-, Phenylniederalkanoyl-, Benzoyl-, Niederalkansulfonyl-, Benzolsulfonylamino, Formyl, Diniederalkoxymethyl, Oxyniederalkylenoxymethylen, Hydroxy, Niederalkoxy, Niederalkenyloxy, Phenylniederalkoxy oder Phenoxy, $S(O)_m$-R, wobei m für 0, 1 oder 2 steht und R Wasserstoff, Niederalkyl, Niederalkenyl oder Niederalkinyl bedeutet, Niederalkanoyl, Sulfamoyl, Sulfamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_2$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, oder $PO_nH_2$ bedeutet, wobei n für 2 oder 3 steht; $X_3$ -$CH_2$- bedeutet; $R_3$ Carboxy, 5-Tetrazolyl, $SO_3H$, $PO_2H_2$, $PO_3H_2$ oder Halogenniederalkylsulfamoyl ist; und wobei (hetero-)aromatische Reste einschliesslich der Ringe A und B unabhängig voneinander gegebenenfalls substituiert sind durch Substituenten ausgewählt aus der Gruppe bestehend aus: Halogen, Hydroxy, Niederalkoxy, Niederalkenyloxy, Phenylniederalkoxy, Phenoxy, $S(O)_m$-R und gegebenenfalls durch Halogen oder Hydroxy substituiertes Niederalkyl, Niederalkenyl oder Niederalkinyl, wobei Niederalkyl, Niederalkenyl oder Niederalkinyl gegebenenfalls durch -O- unterbrochen sind, sowie, im Falle von (hetero-)aromatischen Resten, gegebenenfalls zusätzlich substituiert sind durch Carboxy, Carboxy, welches durch einen Alkohol verestert ist, der sich von Niederalkyl, Phenylniederalkyl, Niederalkenyl, Niederalkinyl, Niederalkoxyniederalkyl, Niederalkoxyniederalkenyl oder Niederalkoxyniederalkinyl ableitet, durch Carbamoyl, Carbamoyl, in welchem die Aminogruppe durch Niederalkyl, Niederalkenyl, Niederalkinyl, Phenylniederalkyl, Phenylniederalkenyl oder Phenylniederalkinyl mono- oder unabhängig voneinander disubstituiert oder durch $C_1$-$C_{10}$-Alkylen oder $C_1$-$C_{10}$-Alkyliden disubstituiert ist, wobei $C_1$-$C_{10}$-Alkylen oder $C_2$-$C_{10}$-Alkyliden gegebenenfalls durch -O- unterbrochen sind, durch Formyl, Diniederalkoxymethyl oder Oxyniederalkylenoxymethylen; in freier Form oder in Salzform.

**5.** Verfahren gemäss Anspruch 1 oder 2 zur Herstellung einer Verbindung der Formel I, worin $R_1$ $R_1$ Niederalkyl, Niederalkenyl, Niederalkinyl, Halogenniederalkyl, -niederalkenyl, -niederalkinyl, Hydroxyniederalkyl, -niederalkenyl, -niederalkinyl, $C_3$-$C_7$-Cycloalkyl, $C_3$-$C_7$-Cycloalkenyl, Phenylniederalkyl, Phenylniederalkenyl oder Phenylniederalkinyl bedeutet; $X_1$ für CO oder $SO_2$ steht; $X_2$ $C_1$-$C_{10}$-Alkylen oder $C_2$-$C_{10}$-Alkyliden bedeutet, die gegebenenfalls durch Hydroxy, einen $C_3$-$C_7$-Cycloalkyl-, $C_3$-$C_7$-Cycloalkenyl-, einen Phenylrest oder einen 5- oder 6-gliedrigen, monocyclischen heteroaromatischen Rest mit bis zu vier gleichen oder verschiedenen Heteroatomen substituiert sind, wobei die cyclischen Reste ihrerseits gegebenenfalls substituiert sind durch Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Formyl, Diniederalkoxymethyl, Oxyniederalkylenoxymethylen; $R_2$ Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Amino, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Niederalkenyloxy, Phenylniederalkoxy, Phenoxy, $S(O)_m$-R, wobei m für 0, 1 oder 2 und R für Wasserstoff, Niederalkyl, Niederalkenyl oder Niederalkinyl steht, Niederalkanoyl, Sulfamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, oder $PO_nH_2$ bedeutet, wobei n für 2 oder 3 steht; $X_3$ -$CH_2$- bedeutet; und $R_3$ Carboxy, 5-Tetrazolyl, $SO_3H$, $PO_2H_2$, $PO_3H_2$ oder Halogenniederalkylsulfamoyl bedeutet; wobei (hetero-)aromatische Reste einschliesslich der Ringe A und B unabhängig voneinander jeweils gegebenenfalls substituiert sind durch einen oder mehrere Substituenten ausgewählt aus Halogen, Hydroxy, Niederalkoxy, Niederalkenyloxy, jeweils gegebenenfalls durch Halogen oder Hydroxy substituiertes Niederalkyl, Niederalkenyl,

Niederalkinyl, Niederalkoxyniederalkyl, -niederalkenyl, -niederalkinyl, Niederalkenyloxyniederalkyl, -niederalkenyl und -niederalkinyl, in freier Form oder in Salzform.

6. Verfahren gemäss Anspruch 1 oder 2 zur Herstellung einer Verbindung der Formel I, worin $X_2$ $C_1$-$C_{10}$-Alkylen oder $C_2$-$C_{10}$-Alkyliden bedeutet, die gegebenenfalls durch Hydroxy, einen $C_3$-$C_7$-Cycloalkyl-, $C_3$-$C_7$-Cycloalkenyl-, einen Phenylrest oder einen 5- oder 6-gliedrigen, monocyclischen heteroaromatischen Rest mit bis zu vier gleichen oder verschiedenen Heteroatomen substituiert sind, wobei ein C-Atom von $C_1$-$C_{10}$-Alkylen bzw. $C_2$-$C_{10}$-Alkyliden durch $C_2$-$C_6$-Alkylen überbrückt sein kann und wobei die cyclischen Reste ihrerseits gegebenenfalls substituiert sind durch Carboxy, welches gegebenenfalls verestert ist mit einem Alkohol, der sich von Niederalkyl, Phenylniederalkyl, Niederalkenyl, Niederalkinyl, Niederalkoxyniederalkyl, -niederalkenyl oder -niederalkinyl ableitet, Carbamoyl, in dem die Aminogruppe gegebenenfalls durch Niederalkyl, Niederalkenyl, Niederalkinyl, Phenylniederalkyl, Phenylniederalkenyl, Phenylniederalkinyl mono- oder unabhängig voneinander disubstituiert oder durch Niederalkylen- oder Niederalkylenoxyniederalkylen disubstituiert ist, Formyl, Diniederalkoxymethyl oder durch Oxyniederalkylenoxymethylen, oder $X_2$ $C_3$-$C_7$-Cycloalkylen bedeutet; $X_3$ Niederalkylen oder Niederalkyliden bedeutet; die Variablen $X_1$, $R_1$, $R_2$, und $R_3$ die in Anspruch 5 angegebenen Bedeutungen haben; und die (hetero-)aromatischen Ringe einschliesslich der Ringe A und B wie in Anspruch 5 angegeben substituiert sein können, in freier Form oder in Salzform.

7. Verfahren gemäss Anspruch 1 oder 2 zur Herstellung einer Verbindung der Formel I, worin $R_1$ Niederalkyl, Niederalkenyl, Halogenniederalkyl, -niederalkenyl, Hydroxyniederalkyl, 3- bis 7-gliedriges Cycloalkyl oder Phenylniederalkyl bedeutet; $X_1$ für CO, $SO_2$ oder
-O-C(=O)-, wobei das Kohlenstoffatom der Carbonylgruppe an das in der Formel I eingezeichnete Stickstoffatom gebunden ist, steht; $X_2$ $C_1$-$C_{10}$-Alkylen oder $C_1$-$C_7$-Alkyliden, die gegebenenfalls substituiert sind durch Hydroxy, Carboxy, Amino, Guanidino, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder Oxyniederalkylenoxymethylen substituiert sein können; $R_2$ Carboxy, Niederalkoxy-, Phenylniederalkoxy-, Niederalkenyloxy-, Niederalkoxyniederalkoxy-carbonyl, Carbamoyl, in dem die Aminogruppe gegebenenfalls durch Niederalkyl, Phenylniederalkyl mono- oder unabhängig voneinander disubstituiert oder durch Niederalkylen, das gegebenenfalls an zwei benachbarten Kohlenstoffatomen mit einem Benzolring kondensiert ist, oder Niederalkylenoxyniederalkylen disubstituiert ist, Amino, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Phenylniederalkoxy, Phenoxy, $S(O)_m$-R, wobei m für 0, 1 oder 2 und R für Niederalkyl steht, Niederalkanoyl, Sulfamoyl, in dem die Aminogruppe gegebenenfalls durch Niederalkyl, Phenylniederalkyl mono- oder unabhängig voneinander disubstituiert ist, oder $PO_nH_2$ bedeutet, wobei n für 2 oder 3 steht; $X_3$ Methylen ist; $R_3$ Carboxy, 5-Tetrazolyl, $SO_3H$, $PO_2H_2$, $PO_3H_2$ oder Halogenniederalkylsulfamoyl bedeutet; und (hetero-)aromatische Reste einschliesslich der Ringe A und B jeweils gegebenenfalls zusätzlich substituiert sind durch einen oder mehrere Substituenten ausgewählt aus Halogen, Hydroxy, Niederalkoxy, jeweils gegebenenfalls durch Halogen oder Hydroxy substituiertes Niederalkyl bzw. Niederalkoxyniederalkyl, in freier Form oder in Salzform.

8. Verfahren gemäss Anspruch 1 oder 2 zur Herstellung einer Verbindung der Formel I, worin $R_1$ Niederalkyl, Niederalkenyl, Halogenniederalkyl, -niederalkenyl, Hydroxyniederalkyl, 3- bis 7-gliedriges Cycloalkyl oder Phenylniederalkyl bedeutet; $X_1$ für CO oder $SO_2$ steht; $X_2$ $C_1$-$C_{10}$-Alkylen oder $C_1$-$C_7$-Alkyliden, die gegebenenfalls substituiert sind durch Hydroxy, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder Oxyniederalkylenoxymethylen substituiert sein können; $R_2$ Carboxy, Niederalkoxy-, Phenylniederalkoxy-, Niederalkenyloxy-, Niederalkoxyniederalkoxy-carbonyl, Carbamoyl, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Amino, in dem die Aminogruppe gegebenenfalls durch Niederalkyl, Phenylniederalkyl mono- oder unabhängig voneinander disubstituiert oder durch Niederalkylen- oder Niederalkylenoxyniederalkylen disubstituiert ist, Niederalkanoyl-, Phenylniederalkanoyl-, Benzoyl-, Niederalkansulfonyl-, Benzolsulfonyl-amino, Formyl, Diniederalkoxymethyl, Oxyniederalkylenoxymethylen, Hydroxy, Niederalkoxy, Phenylniederalkoxy, Phenoxy, $S(O)_m$-R, wobei m für 0, 1 oder 2 und R für Niederalkyl steht, Niederalkanoyl, Sulfamoyl, in dem die Aminogruppe gegebenenfalls durch Niederalkyl, Phenylniederalkyl mono-

oder unabhängig voneinander disubstituiert ist, oder $PO_nH_2$ bedeutet, wobei n für 2 oder 3 steht; $X_3$ Methylen ist; $R_3$ Carboxy, 5-Tetrazolyl, $SO_3H$, $PO_2H_2$, $PO_3H_2$ oder Halogenniederalkylsulfamoyl bedeutet; und (hetero-)aromatische Reste einschliesslich der Ringe A und B jeweils gegebenenfalls zusätzlich substituiert sind durch einen oder mehrere Substituenten ausgewählt aus Halogen, Hydroxy, Niederalkoxy, jeweils gegebenenfalls durch Halogen oder Hydroxy substituiertes Niederalkyl bzw. Niederalkoxyniederalkyl, in freier Form oder in Salzform.

**9.** Verfahren gemäss Anspruch 1 oder 2 zur Herstellung einer Verbindung der Formel I, worin $X_2$ $C_1$-$C_{10}$-Alkylen oder $C_1$-$C_7$-Alkyliden, die gegebenenfalls substituiert sind durch Hydroxy, einen 3- bis 7-gliedrigen Cycloalkyl-, 3- bis 7-gliedrigen Cycloalkenyl-, Phenyl-, Pyrrolyl-, Pyrazolyl-, Imidazolyl-, Triazolyl-, Tetrazolyl-, Furyl-, Thienyl- oder Pyridylrest, welche ihrerseits gegebenenfalls zusätzlich durch Carboxy, Niederalkoxycarbonyl, Phenylniederalkoxycarbonyl, Carbamoyl, in dem die Aminogruppe gegebenenfalls durch Niederalkyl oder Phenylniederalkyl mono- oder unabhängig voneinander disubstituiert ist, Formyl, Diniederalkoxymethyl oder durch Oxyniederalkylenoxymethylen substituiert sein können, wobei ein C-Atom von $C_1$-$C_{10}$-Alkylen bzw. $C_1$-$C_7$-Alkyliden durch $C_2$-$C_6$-Alkylen überbrückt sein kann, oder $X_2$ $C_3$-$C_7$-Cycloalkylen bedeutet; $X_3$ Niederalkylen oder Niederalkyliden bedeutet; die Variablen $X_1$, $R_1$, $R_2$, $R_3$ die in Anspruch 8 angegebenen Bedeutungen haben; und die (hetero-)aromatischen Ringe einschliesslich der Ringe A und B wie in Anspruch 8 angegeben substituiert sein können, in freier Form oder in Salzform.

**10.** Verfahren gemäss Anspruch 1 oder 2 zur Herstellung einer Verbindung der Formel I, worin die Variablen $R_1$, $X_1$, $R_3$ die jeweils in einem der Ansprüche 1-8 angegebenen Bedeutungen haben; $X_2$ gegebenenfalls durch Hydroxy, 3- bis 7-gliedriges Cycloalkyl, Phenyl oder Imidazolyl substituiertes Niederalkylen oder Niederalkyliden bedeutet und $R_2$ Carboxy, Niederalkoxy-, Phenylniederalkoxy-, Niederalkoxyniederalkoxy-carbonyl, Carbamoyl, welches gegebenenfalls durch Niederalkyl, Phenylniederalkyl mono- oder unabhängig voneinander disubstituiert ist, Amino, Niederalkanoyl-, Phenylniederalkanoyl-, Niederalkansulfonylamino, Hydroxy, Niederalkoxy, Phenylniederalkoxy oder Phenoxy bedeutet; $X_3$ -$CH_2$- bedeutet; wobei (hetero-)aromatische Reste einschliesslich der Ringe A und B jeweils gegebenenfalls durch einen oder mehrere Substituenten ausgewählt aus Halogen, Trifluormethyl, Hydroxy, Niederalkoxy, Niederalkyl, Hydroxyniederalkyl oder Niederalkoxyniederalkyl substituiert sind, in freier Form oder in Salzform.

**11.** Verfahren gemäss Anspruch 1 oder 2 zur Herstellung einer Verbindung der Formel I, worin $X_2$ gegebenenfalls durch Hydroxy, 3- bis 7-gliedriges Cycloalkyl, 7-gliedriges Cycloalkenyl, Phenyl oder Imidazolyl substituiertes Niederalkylen oder Niederalkyliden bedeutet, wobei ein C-Atom von Niederalkylen bzw. Niederalkyliden durch $C_2$-$C_6$-Alkylen überbrückt sein kann, oder $X_2$ $C_3$-$C_7$-Cycloalkylen bedeutet; die Variablen $X_1$, $X_3$, $R_1$, $R_2$ und $R_3$ die in Anspruch 8 bis 10 angegebenen Bedeutungen haben; und die Ringe A und B wie in Anspruch 10 angegeben substituiert sein können, in freier Form oder in Salzform.

**12.** Verfahren gemäss Anspruch 1 oder 2 zur Herstellung einer Verbindung der Formel

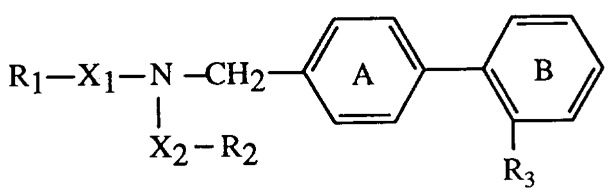

(Ia),

worin die Variablen $R_1$, $X_1$, $X_2$, $R_2$ und $R_3$ die jeweils in einem der Ansprüche 1 oder 3-11 angegebenen Bedeutungen haben und die Ringe A und B wie in Anspruch 11 angegeben substituiert sein können, in freier Form oder in Salzform.

**13.** Verfahren gemäss Anspruch 1 oder 2 zur Herstellung einer Verbindung der Formel Ia, worin $X_2$ gegebenenfalls durch Hydroxy oder 3- bis 7-gliedriges Cycloalkyl substituiertes Niederalkylen oder Niederalkyliden bedeutet, wobei ein C-Atom von Niederalkylen bzw. Niederalkyliden durch $C_2$-$C_6$-Alkylen, überbrückt sein kann, oder worin $X_2$ $C_3$-$C_7$-Cycloalkylen bedeutet; die Variablen $R_1$, $X_1$, $R_2$ und $R_3$ die jeweils in einem der Ansprüche 1 oder 3-11 angegebenen Bedeutungen haben; und die Ringe A und B wie in Anspruch 11 angegeben substituiert sein können, in freier Form oder in Salzform.

**14.** Verfahren gemäss Anspruch 1 oder 2 zur Herstellung einer Verbindung der Formel Ia, worin $X_2$ für die Gruppe der Formel

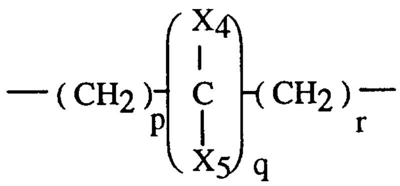

(Ib)

steht, in der p für 0 oder 1, q für 1 und r für 0 oder 1 stehen oder in der p für 1 bis 8 und q sowie r jeweils für 0 stehen; $X_4$ gegebenenfalls durch Hydroxy, 3- bis 7-gliedriges Cycloalkyl, Phenyl oder Imidazolyl substituiertes Niederalkyl oder Phenyl bedeutet; und $X_5$ Wasserstoff oder Niederalkyl bedeutet; $R_2$ Carboxy, Niederalkoxycarbonyl, Phenylniederalkoxycarbonyl, Niederalkoxyniederalkoxycarbonyl, Hydroxy, Niederalkoxy, Phenylniederalkoxy, Phenoxy, Amino, Niederalkanoylamino, Phenylniederalkanoylamino oder Niederalkansulfonylamino bedeutet; und die Variablen $R_1$, $X_1$ und $R_3$ die jeweils in einem der Ansprüche 1 oder 3-8 angegebenen Bedeutungen haben; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**15.** Verfahren gemäss Anspruch 1 oder 2 zur Herstellung einer Verbindung der Formel Ia, worin $X_2$ für die Gruppe der Formel Ib steht, in der p für 0 oder 1, q für 1 und r für 0 oder 1 stehen oder in der p für 1 bis 8 und q sowie r jeweils für 0 stehen; $X_4$ gegebenenfalls durch Hydroxy, 3- bis 7-gliedriges Cycloalkyl, Phenyl oder Imidazolyl substituiertes Niederalkyl oder Phenyl bedeutet; und $X_5$ Wasserstoff oder Niederalkyl bedeutet; oder $X_4$ und $X_5$ gemeinsam für $C_2$-$C_6$-Alkylen, wie $C_4$-$C_5$-Alkylen, stehen; oder $X_2$ $C_3$-$C_7$-Cycloalkylen, wie $C_5$-$C_6$-Cycloalkylen, bedeutet; $R_2$ Carboxy, Niederalkoxycarbonyl, Phenylniederalkoxycarbonyl, Niederalkoxyniederalkoxycarbonyl, Hydroxy, Niederalkoxy, Phenylniederalkoxy, Phenoxy, Amino, Niederalkanoylamino, Phenylniederalkanoylamino oder Niederalkansulfonylamino bedeutet; und die Variablen $R_1$, $X_1$ und $R_3$ die jeweils in einem der Ansprüche 1 oder 3-8 angegebenen Bedeutungen haben; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**16.** Verfahren gemäss Anspruch 1 oder 2 zur Herstellung einer Verbindung der Formel Ia, worin $R_1$ Niederalkyl, wie $C_3$-$C_5$-Alkyl, oder Niederalkenyl, wie $C_3$-$C_5$-Alkenyl, bedeutet; $X_1$ für CO oder ferner $SO_2$ steht; $X_2$ für die Gruppe der Formel Ib steht, in der p und r für 0 oder 1 und q für 1 stehen; $X_4$ gegebenenfalls durch Hydroxy, 3- bis 7-gliedriges Cycloalkyl, wie Cyclohexyl, durch gegebenenfalls durch Halogen oder Hydroxy substituiertes Phenyl oder Imidazolyl, wie 4-Imidazolyl, substituiertes Niederalkyl, wie $C_1$-$C_4$-Alkyl, oder Phenyl bedeutet; und $X_5$ Wasserstoff oder Niederalkyl, wie $C_1$-$C_4$-Alkyl, bedeutet; oder $X_4$ und $X_5$ gemeinsam $C_2$-$C_6$-Alkylen, wie $C_4$-$C_5$-Alkylen, bedeuten; oder $X_2$ $C_3$-$C_7$-Cycloalkylen, wie $C_5$-$C_6$-Cycloalkylen, bedeutet; $R_2$ Carboxy, Niederalkoxycarbonyl, wie $C_2$-$C_5$-Alkoxycarbonyl, Phenylniederalkoxycarbonyl, wie Phenyl-$C_1$-$C_4$-alkoxycarbonyl, Niederalkoxyniederalkoxycarbonyl, wie $C_1$-$C_4$-Alkoxy-$C_2$-$C_5$-alkoxycarbonyl, Hydroxy oder Niederalkoxy, wie $C_1$-$C_4$-Alkoxy, bedeutet; und $R_3$ Carboxy oder 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**17.** Verfahren gemäss Anspruch 1 oder 2 zur Herstellung einer Verbindung der Formel Ia, worin $R_1$ Niederalkyl, wie $C_3$-$C_5$-Alkyl, oder Niederalkenyl, wie $C_3$-$C_5$-Alkenyl, bedeutet; $X_1$ für CO oder ferner $SO_2$ steht; $X_2$ für die Gruppe der Formel Ib steht, in der p und r für 0 oder 1 und q für 1 stehen; $X_4$ gegebenenfalls durch Hydroxy, 3- bis 7-gliedriges Cycloalkyl, durch gegebenenfalls durch Halogen oder Hydroxy substituiertes Phenyl oder Imidazolyl, wie 4-Imidazolyl, substituiertes Niederalkyl, wie $C_1$-$C_4$-Alkyl, oder Phenyl bedeutet; und $X_5$ Wasserstoff oder Niederalkyl, wie $C_1$-$C_4$-Alkyl, bedeutet; $R_2$ Carboxy, Niederalkoxycarbonyl, wie $C_2$-$C_5$-Alkoxycarbonyl, Phenylniederalkoxycarbonyl, wie Phenyl-$C_1$-$C_4$-alkoxycarbonyl, Niederalkoxyniederalkoxycarbonyl, wie $C_1$-$C_4$-Alkoxy-$C_2$-$C_5$-alkoxycarbonyl, Hydroxy oder Niederalkoxy, wie $C_1$-$C_4$-Alkoxy, bedeutet; und $R_3$ Carboxy oder 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**18.** Verfahren gemäss Anspruch 1 oder 2 zur Herstellung einer Verbindung der Formel Ia, worin $R_1$ Niederalkyl, wie $C_3$-$C_5$-Alkyl, oder ferner Niederalkenyl, wie $C_3$-$C_5$-Alkenyl, bedeutet; $X_1$ für CO oder ferner $SO_2$ steht; $X_2$ für die Gruppe der Formel Ib steht, in der p für eine ganze Zahl von 1 bis 8 und q sowie r für 0 stehen; $R_2$ Hydroxy, Nie-

deralkoxy, wie $C_1$-$C_4$-Alkoxy, Phenylniederalkoxy, wie Phenyl-$C_1$-$C_4$-alkoxy, Phenoxy, Niederalkanoylamino, wie $C_1$-$C_4$-Alkanoylamino, Phenylniederalkanoylamino, wie Phenyl-$C_1$-$C_4$-alkanoylamino, Niederalkansulfonylamino, wie $C_1$-$C_4$-Alkansulfonylamino, bedeutet; und $R_3$ Carboxy oder in erster Linie 5-Tetrazolyl bedeutet; wobei (hetero-)aromatische Reste einschliesslich der Ringe A und B jeweils gegebenenfalls durch Halogen, Trifluormethyl, Hydroxy, Niederalkoxy, Niederalkyl oder Hydroxyniederalkyl substituiert sind, in freier Form oder in Salzform.

**19.** Verfahren gemäss Anspruch 1 oder 2 zur Herstellung einer Verbindung der Formel Ia, worin $R_1$ $C_3$-$C_5$-Alkyl oder in zweiter Linie $C_3$-$C_5$-Alkenyl, bedeutet; $X_1$ für CO, ferner $SO_2$ steht; $X_2$ für die Gruppe der Formel Ib steht, in der p und r unabhängig voneinander für 0 oder 1 und q für 1 stehen; $X_4$ $C_1$-$C_4$-Alkyl, Hydroxy-$C_1$-$C_4$-alkyl, $C_3$-$C_7$-Cycloalkyl-$C_1$-$C_4$-alkyl, Phenyl-$C_1$-$C_4$-alkyl oder Imidazolyl-$C_1$-$C_4$-alkyl bedeutet; und $X_5$ Wasserstoff oder $C_1$-$C_4$-Alkyl bedeutet; oder $X_4$ und $X_5$ gemeinsam für Tetramethylen, ferner Pentamethylen stehen; $R_2$ Carboxy oder $C_2$-$C_5$-Alkoxycarbonyl, ferner Phenyl-$C_1$-$C_4$-alkoxycarbonyl bedeutet; und $R_3$ Carboxy oder 5-Tetrazolyl bedeutet, in freier Form oder in Salzform.

**20.** Verfahren gemäss Anspruch 1 oder 2 zur Herstellung einer Verbindung der Formel Ia, worin $R_1$ $C_3$-$C_5$-Alkyl oder in zweiter Linie $C_3$-$C_5$-Alkenyl bedeutet; $X_1$ für CO, ferner $SO_2$ steht; $X_2$ für die Gruppe der Formel Ib steht, in der p und r jeweils für 0 oder 1 und q für 1 stehen; $X_4$ $C_1$-$C_4$-Alkyl, Hydroxy-$C_1$-$C_4$-alkyl, $C_3$-$C_7$-Cycloalkyl-$C_1$-$C_4$-alkyl, Phenyl-$C_1$-$C_4$-alkyl oder Imidazolyl-$C_1$-$C_4$-alkyl bedeutet; und $X_5$ Wasserstoff bedeutet; $R_2$ Carboxy oder $C_2$-$C_5$-Alkoxycarbonyl, ferner Phenyl-$C_1$-$C_4$-alkoxycarbonyl bedeutet; und $R_3$ Carboxy oder 5-Tetrazolyl bedeutet, in freier Form oder in Salzform.

**21.** Verfahren gemäss Anspruch 1 oder 2 zur Herstellung einer Verbindung der Formel Ia, worin $R_1$ $C_3$-$C_5$-Alkyl bedeutet; $X_1$ für CO steht; $X_2$ für die Gruppe der Formel Ib steht, in der q und r für 0 und p für 1 bis 3 stehen oder in der p und q für 1 und r für 0 stehen; $X_4$ $C_1$-$C_4$-Alkyl bedeutet; $X_5$ Wasserstoff oder $C_1$-$C_4$-Alkyl bedeutet; $R_2$ Carboxy oder $C_2$-$C_5$-Alkoxycarbonyl bedeutet; und $R_3$ Carboxy oder 5-Tetrazolyl bedeutet, in freier Form oder in Salzform.

**22.** Verfahren gemäss Anspruch 1 oder 2 zur Herstellung einer Verbindung der Formel Ia, worin $R_3$ 5-Tetrazolyl bedeutet, in freier Form oder in Salzform.

**23.** Verfahren gemäss Anspruch 1 oder 2 zur Herstellung einer Verbindung der Formel Ia, worin $R_1$ $C_3$-$C_5$-Alkyl bedeutet; $X_1$ für CO steht; $X_2$ für die Gruppe der Formel Ib steht, in der p für 0 oder 1, r für 0 und q für 1 stehen; $X_4$ $C_1$-$C_4$-Alkyl bedeutet; und $X_5$ Wasserstoff oder $C_1$-$C_4$-Alkyl bedeutet; oder $X_4$ und $X_5$ gemeinsam für Tetramethylen oder Pentamethylen stehen; $R_2$ Carboxy, oder $C_2$-$C_5$-Alkoxycarbonyl bedeutet; und $R_3$ 5-Tetrazolyl bedeutet, in freier Form oder in Salzform.

**24.** Verfahren gemäss Anspruch 1 oder 2 zur Herstellung einer Verbindung der Formel Ia, worin $_1$ $C_3$-$C_5$-Alkyl bedeutet; $X_1$ für CO steht; $X_2$ für die Gruppe der Formel Ib steht, in der p für 0 oder 1, r für 0 und q für 1 stehen; $X_4$ und $X_5$ gemeinsam für Tetramethylen, ferner Pentamethylen stehen; $R_2$ Carboxy oder $C_2$-$C_5$-Alkoxycarbonyl bedeutet; und $R_3$ 5-Tetrazolyl bedeutet, in freier Form oder in Salzform.

**25.** Verfahren gemäss Anspruch 1 oder 2 zur Herstellung einer Verbindung der Formel Ia, worin $R_1$ $C_3$-$C_5$-Alkyl bedeutet; $X_1$ für CO steht; $X_2$ für die Gruppe der Formel Ib steht, in der p und r für 0 oder 1 und q für 1 stehen; $X_4$ $C_1$-$C_4$-Alkyl bedeutet; und $X_5$ Wasserstoff bedeutet; $R_2$ Carboxy oder $C_2$-$C_5$-Alkoxycarbonyl bedeutet; und $R_3$ 5-Tetrazolyl bedeutet, in freier Form oder in Salzform.

**26.** Verfahren gemäss Anspruch 1 oder 2 zur Herstellung einer Verbindung der Formel Ia gemäss einem der Ansprüche 14-25, worin $X_2$ für die Gruppe der Formel Ib steht, q 1 bedeutet und $X_4$ und $X_5$ unterschiedliche Bedeutungen haben, in freier Form oder in Salzform, in welcher das betreffende, die Variablen $X_4$ und $X_5$ aufweisende, asymmetrische C-Atom die S-Konfiguration hat.

**27.** Verfahren gemäss Anspruch 1 oder 2 zur Herstellung von (S)-N-(1-Carboxy-2-methyl-prop-1-yl)-N-pentanoyl-N-[2'-(1H-tetrazol-5-yl)biphenyl-4-ylmethyl]-amin, in freier Form oder in Salzform gemäss Anspruch 1.

**28.** Verfahren gemäss Anspruch 1 oder 2 zur Herstellung von N-(2-Carboxy-2,2-tetramethylen-ethyl)-N-pentanoyl-N-[2'-(1H-tetrazol-5-yl)biphenyl-4-ylmethyl]-amin, in freier Form oder in Salzform gemäss Anspruch 1.

**29.** Verfahren gemäss Anspruch 1 oder 2 zur Herstellung von N-(2-Carboxy-2-ethyl-but-1-yl)-N-pentanoyl-N-[2'-(1H-tetrazol-5-yl)biphenyl-4-ylmethyl]-amin, in freier Form oder in Salzform gemäss Anspruch 1.

**30.** Verfahren gemäss Anspruch 1 oder 2 zur Herstellung von S)-N-(1-Carboxy-2-methyl-prop-1-yl)-N-ethoxycarbonyl-N-[2'-(1H-tetrazol-5-yl)biphenyl-4-ylmethyl]-amin, in freier Form oder in Salzform gemäss Anspruch 1.

**31.** Verfahren gemäss Anspruch 1 oder 2 zur Herstellung von N-(1-carboxycyclohexyl)-N-pentanoyl-N-[2'-(1H-tetrazol-5-yl)biphenyl-4-ylmethyl]-amin, in freier Form oder in Salzform gemäss Anspruch 1.

**32.** Verfahren gemäss Anspruch 1 oder 2 zur Herstellung einer Verbindung gemäss Anspruch 1 ausgewählt aus der Gruppe bestehend aus:
(S)-N-(1-Carboxyethyl)-N-pentanoyl-N-[2'-(1H-tetrazol-5-yl)biphenyl-4-ylmethyl]-amin,
N-(2-Hydroxyethyl)-N-pentanoyl-N-[2'-(1H-tetrazol-5-yl)biphenyl-4-ylmethyl]-amin,
N-(2-Ethoxycarbonyl-2,2-tetramethylen-ethyl)-N-pentanoyl-N-[2'-(1H-tetrazol-5-yl)biphenyl-4-ylmethyl]-amin,
N-(2-Ethoxycarbonyl-2-ethyl-but-1-yl)-N-pentanoyl-N-[2'-(1H-tetrazol-5-yl)biphenyl-4-ylmethyl]-amin,
N-(2-Ethoxycarbonyl-2-methyl-prop-1-yl)-N-pentanoyl-N-[2'-(1H-tetrazol-5-yl) biphenyl-4-ylmethyl]-amin,
(S)-N-(1-Hydroxymethyl-2-methyl-prop-1-yl)-N-pentanoyl-N-[2'-(1H-tetrazol-5-yl)biphenyl-4-ylmethyl]-amin,
N-(2-Ethoxycarbonyl-2,2-pentamethylen-ethyl)-N-pentanoyl-N-[2'-(1H-tetrazol-5-yl)biphenyl-4-ylmethyl]-amin,
(S)-N-(1-Carboxy-2-methyl-prop-1-yl)-N-propyloxycarbonyl-N-[2'-(1H-tetrazol-5-yl)biphenyl-4-ylmethyl]-amin,
N-(2-carboxy-2-methyl-propyl)-N-pentanoyl-N-[2'-(1H-tetrazol-5-yl)biphenyl-4-ylmethyl]-amin,
N-(2-carboxy-2,2-pentamethylen-ethyl)-N-pentanoyl-N-[2'-(1H-tetrazol-5-yl)biphenyl-4-ylmethyl]-amin,
(S)-N-(1-aminocarbonyl-2-methyl-prop-1-yl)-N-pentanoyl-N-[2'-(1H-tetrazol-5-yl)biphenyl-4-ylmethyl]-amin und
(S)-N-(1-carboxy-2-methyl-prop-1-yl)-N-(5-oxopent-1-en-5-yl)-N-[2'-(1H-tetrazol-5-yl)biphenyl-4-ylmethyl]-amin,
jeweils in freier Form oder in Salzform.

**33.** Verfahren gemäss Anspruch 1 oder 2 zur Herstellung einer Verbindung gemäss Anspruch 1 ausgewählt aus der Gruppe bestehend aus:
N-Carboxymethyl-N-pentanoyl-N-[2'-(1H-tetrazol-5-yl)biphenyl-4-ylmethyl]-amin,
(S)-N-(1-Methoxycarbonylethyl)-N-pentanoyl-N-[2'-(1H-tetrazol-5-yl)biphenyl-4-ylmethyl]-amin,
N-[1-Carboxy-2-(4-fluorphenyl)-ethyl]-N-pentanoyl-N-[2'-(1H-tetrazol-5-yl)biphenyl-4-ylmethyl]-amin,
N-[2-(4-Fluorphenyl)-1-methoxycarbonyl-ethyl]-N-pentanoyl-N-[2'-(1H-tetrazol-5-yl)biphenyl-4-ylmethyl]-amin,
N-[2-(4-Fluorphenyl)-1-hydroxymethyl-ethyl]-N-pentanoyl-N-[2'-(1H-tetrazol-5-yl)biphenyl-4-ylmethyl]-amin,
N-(2'-Carboxybiphenyl-4-ylmethyl)-N-[1-carboxy-2-(4-fluorphenyl)-ethyl]-N-pentanoyl-amin,
N-(2'-Carboxybiphenyl-4-ylmethyl)-N-[2-(4-fluorphenyl)-1-methoxycarbonyl-ethyl]-N-pentanoyl-amin,
(S)-N-(2'-Carboxybiphenyl-4-ylmethyl)-N-(1-hydroxymethyl-2-phenyl-ethyl)-N-pentanoyl-amin,
(S)-N-(2'-Carboxybiphenyl-4-ylmethyl)-N-(1-hydroxymethyl-2-imidazol-4-yl-ethyl)-N-pentanoyl-amin,
(R)-N-(1-Carboxyethyl)-N-pentanoyl-N-[2'-(1H-tetrazol-5-yl)biphenyl-4-ylmethyl]-amin,
(1S),(2S)-N-(1-Carboxy-2-methyl-but-1-yl)-N-pentanoyl-N-[2'-(1H-tetrazol-5-yl)biphenyl-4-ylmethyl]-amin,
(1S),(2S)-N-(1-Methoxycarbonyl-2-methyl-but-1-yl)-N-pentanoyl-N-[2'-(1H-tetrazol-5-yl)biphenyl-4-ylmethyl]-amin,
(S)-N-(1-Carboxybut-1-yl)-N-pentanoyl-N-[2'-(1H-tetrazol-5-yl)biphenyl-4-ylmethyl]-amin,
(S)-N-(1-Methoxycarbonylbut-1-yl)-N-pentanoyl-N-[2'-(1H-tetrazol-5-yl)biphenyl-4-ylmethyl]-amin,
(S)-N-(1-Carboxyethyl)-N-hexanoyl-N-[2'-(1H-tetrazol-5-yl)biphenyl-4-ylmethyl]-amin,
(S)-N-Butanoyl-N-(1-carboxyethyl)-N-[2'-(1H-tetrazol-5-yl)biphenyl-4-ylmethyl]-amin,
(S)-N-(1-Carboxyprop-1-yl)-N-pentanoyl-N-[2'-(1H-tetrazol-5-yl)biphenyl-4-ylmethyl]-amin,
(S)-N-(1-Carboxy-2-cyclohexyl-ethyl)-N-pentanoyl-N-[2'-(1H-tetrazol-5-yl)biphenyl-4-ylmethyl]-amin,
(S)-N-(2-Cyclohexyl-1-methoxycarbonyl-ethyl)-N-pentanoyl-N-[2'-(1H-tetrazol-5-yl)biphenyl-4-ylmethyl]-amin,
(R)-N-(1-Carboxy-2-methyl-prop-1-yl)-N-pentanoyl-N-[2'-(1H-tetrazol-5-yl)biphenyl-4-ylmethyl]-amin,
N-(2-Methoxyethyl)-N-pentanoyl-N-[2'-(1H-tetrazol-5-yl)biphenyl-4-ylmethyl]-amin,
N-(2-Benzyloxyethyl)-N-pentanoyl-N-[2'-(1H-tetrazol-5-yl)biphenyl-4-ylmethyl]-amin,
N-(3-Methoxyprop-1-yl)-N-pentanoyl-N-[2'-(1H-tetrazol-5-yl)biphenyl-4-ylmethyl]-amin,
N-(3-Benzyloxyprop-1-yl)-N-pentanoyl-N-[2'-(1H-tetrazol-5-yl)biphenyl-4-ylmethyl]-amin,
N-(3-Hydroxyprop-1-yl)-N-pentanoyl-N-[2'-(1H-tetrazol-5-yl)biphenyl-4-ylmethyl]-amin,
N-(1-Methoxycarbonyl-1-methyl-ethyl)-N-pentanoyl-N-[2'-(1H-tetrazol-5-yl)biphenyl-4-ylmethyl]-amin,
N-(2-Carboxyethyl)-N-pentanoyl-N-[2'-(1H-tetrazol-5-yl)biphenyl-4-ylmethyl]-amin,
N-(2-Carboxyprop-1-yl)-N-pentanoyl-N-[2'-(1H-tetrazol-5-yl)biphenyl-4-ylmethyl]-amin,
N-(1-Carboxy-1-methyl-ethyl)-N-pentanoyl-N-[2'-(1H-tetrazol-5-yl)biphenyl-4-ylmethyl]-amin,
N-(5-Hydroxypent-1-yl)-N-pentanoyl-N-[2'-(1H-tetrazol-5-yl)biphenyl-4-ylmethyl]-amin,
N-(1-Carboxyprop-2-yl)-N-pentanoyl-N-[2'-(1H-tetrazol-5-yl)biphenyl-4-ylmethyl]-amin,
N-(2-Ethoxycarbonyl-3-methyl-but-1-yl)-N-pentanoyl-N-[2'-(1H-tetrazol-5-yl)biphenyl-4-ylmethyl]-amin,
N-(2-Carboxy-3-methyl-but-1-yl)-N-pentanoyl-N-[2'-(1H-tetrazol-5-yl)biphenyl-4-ylmethyl]-amin,
N-(3-Phenoxyprop-1-yl)-N-pentanoyl-N-[2'-(1H-tetrazol-5-yl)biphenyl-4-ylmethyl]-amin,

N-[2-(4-Hydroxyphenyl)ethyl]-N-pentanoyl-N-[2'-(1H-tetrazol-5-yl)biphenyl-4-ylmethyl]-amin,
N-[3-(4-Hydroxyphenyl)prop-1-yl]-N-pentanoyl-N-[2'-(1H-tetrazol-5-yl)biphenyl-4-ylmethyl]-amin,
N-(8-Hydroxyoct-1-yl)-N-pentanoyl-N-[2'-(1H-tetrazol-5-yl)biphenyl-4-ylmethyl]-amin,
N-(2-Methansulfonylaminoethyl)-N-pentanoyl-N-[2'-(1H-tetrazol-5-yl)biphenyl-4-ylmethyl]-amin,
N-(3-Acetylaminoprop-1-yl)-N-pentanoyl-N-[2'-(1H-tetrazol-5-yl)biphenyl-4-ylmethyl]-amin,
N-(2-Methoxy-2-oxo-1-phenyl-ethyl)-N-pentanoyl-N-[2'-(1H-tetrazol-5-yl)biphenyl-4-ylmethyl]-amin,
N-(4-Hydroxybut-2-yl)-N-pentanoyl-N-[2'-(1H-tetrazol-5-yl)biphenyl-4-ylmethyl]-amin,
N-(2-Hydroxy-1-phenyl-ethyl)-N-pentanoyl-N-[2'-(1H-tetrazol-5-yl)biphenyl-4-ylmethyl]-amin,
N-[3-(4-Hydroxybenzylcarbonylamino)prop-1-yl]-N-pentanoyl-N-[2'-(1H-tetrazol-5-yl)biphenyl-4-ylmethyl]-amin,
N-(3-Ethoxycarbonylcyclohexyl)-N-pentanoyl-N-[2'-(1H-tetrazol-5-yl)biphenyl-4-ylmethyl]-amin,
N-(3-Carboxycyclohexyl)-N-pentanoyl-N-[2'-(1H-tetrazol-5-yl)biphenyl-4-ylmethyl]-amin,
cis-N-(4-Carboxycyclohexyl)-N-pentanoyl-N-[2'-(1H-tetrazol-5-yl)biphenyl-4-ylmethyl]-amin,
cis-N-(2-Ethoxycarbonylcyclohexyl)-N-pentanoyl-N-[2'-(1H-tetrazol-5-yl)biphenyl-4-ylmethyl]-amin,
cis-N-(2-Carboxycyclohexyl)-N-pentanoyl-N-[2'-(1H-tetrazol-5-yl)biphenyl-4-ylmethyl]-amin,
N-{2-[2-(4-Hydroxyphenyl)ethylaminocarbonyl]-2,2-tetramethylen-ethyl}-N-pentanoyl-N-[2'-(1H-tetrazol-5-yl)biphenyl-4-ylmethyl]-amin,
(S)-N-{1-[2-(4-Hydroxyphenyl)ethylaminocarbonyl]-2-methyl-prop-1-yl}-N-pentanoyl-N-[2'-(1H-tetrazol-5-yl)biphenyl-4-ylmethyl]-amin,
(S)-N-(1-Carboxy-2,2-dimethyl-prop-1-yl)-N-pentanoyl-N-[2'-(1H-tetrazol-5-yl)biphenyl-4-ylmethyl]-amin,
(S)-N-(1-Methoxycarbonyl-2-methyl-prop-1-yl)-N-pentanoyl-N-[2'-(1H-tetrazol-5-yl)biphenyl-4-ylmethyl]-amin,
N-(4-Phenoxybut-1-yl)-N-pentanoyl-N-[2'-(1H-tetrazol-5-yl)biphenyl-4-ylmethyl]-amin,
N-(2-Hydroxy-1-phenyl-2-oxo-ethyl)-N-pentanoyl-N-[2'-(1H-tetrazol-5-yl)biphenyl-4-ylmethyl]-amin,
(S)-N-(1-Benzyloxycarbonyl-2-methyl-prop-1-yl)-N-pentanoyl-N-[2'-(1H-tetrazol-5-yl)biphenyl-4-ylmethyl]-amin,
N-Butanoyl-N-(1-carboxy-1-methyl-ethyl)-N-[2'-(1H-tetrazol-5-yl)biphenyl-4-ylmethyl]-amin,
N-(4-Hydroxybut-1-yl)-N-pentanoyl-N-[2'-(1H-tetrazol-5-yl)biphenyl-4-ylmethyl]-amin,
(S)-N-(1-Benzyloxycarbonyl-2-methyl-prop-1-yl)-N-[3-bromo-2'-(1H-tetrazol-5-yl)biphenyl-4-ylmethyl]-N-pentanoyl-amin,
(S)-N-[3-Brom-2'-(1H-tetrazol-5-yl)-biphenyl-4-ylmethyl]-N-(1-carboxy-2-methyl-prop-1-yl)-N-pentanoyl-amin,
N-(2-Acetylaminoethyl)-N-pentanoyl-N-[2'-(1H-tetrazol-5-yl)biphenyl-4-ylmethyl]-amin,
N-[2-(n-Butoxycarbonyl)-2,2-tetramethylen-ethyl]-N-pentanoyl-N-[2'-(1H-tetrazol-5-yl)biphenyl-4-ylmethyl]-amin,
N-(2-Benzylaminocarbonyl-2,2-tetramethylen-ethyl)-N-pentanoyl-N-[2'-(1H-tetrazol-5-yl)biphenyl-4-ylmethyl]-amin,
(S)-N-Butyloxycarbonyl-N-(1-Carboxy-2-methyl-prop-1-yl)-N-[2'-(1H-tetrazol-5-yl)biphenyl-4-ylmethyl]-amin,
(S)-N-(1-Carboxy-2-methyl-prop-1-yl)-N-methoxycarbonyl-N-[2'-(1H-tetrazol-5-yl)biphenyl-4-ylmethyl]-amin,
N-(2-Diethylaminocarbonyl-2,2-tetramethylen-ethyl)-N-pentanoyl-N-[2'-(1H-tetrazol-5-yl)biphenyl-4-ylmethyl]-amin,
N-(2-Methyl-2-morpholin-4-ylcarbonyl-propyl)-N-pentanoyl-N-[2'-(1H-tetrazol-5-yl)biphenyl-4-ylmethyl]-amin,
N-(1-Carboxycyclopentyl)-N-pentanoyl-N-[2'-(1H-tetrazol-5-yl)biphenyl-4-ylmethyl]-amin,
N-(1-Carboxy-1-ethyl-prop-1-yl)-N-pentanoyl-N-[2'-(1H-tetrazol-5-yl)biphenyl-4-ylmethyl]-amin,
(S)-N-(5-Amino-1-carboxy-pent-1-yl)-N-pentanoyl-N-[2'-(1H-tetrazol-5-yl)biphenyl-4-ylmethyl]-amin,
N-Butansulfonyl-N-(2-ethoxycarbonyl-2,2-pentamethylen-ethyl)-N-[2'-(1H-tetrazol-5-yl)biphenyl-4-ylmethyl]-amin,
N-Butansulfonyl-N-(2-carboxy-2,2-pentamethylen-ethyl)-N-[2'-(1H-tetrazol-5-yl)biphenyl-4-ylmethyl]-amin,
N-Butansulfonyl-N-(2-ethoxycarbonyl-2-methyl-prop-1-yl)-N-[2'-(1H-tetrazol-5-yl)biphenyl-4-ylmethyl]-amin,
N-Butansulfonyl-N-(2-carboxy-2-methyl-prop-1-yl)-N-[2'-(1H-tetrazol-5-yl)-biphenyl-4-ylmethyl]-amin,
(S)-N-Butansulfonyl-N-(1-tert.-butoxycarbonylethyl)-N-[2'-(1H-tetrazol-5-yl)biphenyl-4-ylmethyl]-amin,
(S)-N-Butansulfonyl-N-(1-carboxyethyl)-N-[2'-(1H-tetrazol-5-yl)biphenyl-4-ylmethyl]-amin,
(S)-N-Butansulfonyl-N-(1-carboxy-2-methyl-prop-1-yl)-N-[2'-(1H-tetrazol-5-yl)biphenyl-4-ylmethyl]-amin,
(S)-N-(2-Methyl-1-methylaminocarbonyl-prop-1-yl)-N-pentanoyl-N-[2'-(1H-tetrazol-5-yl)biphenyl-4-ylmethyl]-amin,
(S)-N-(1-Dimethylaminocarbonyl-2-methyl-prop-1-yl)-N-pentanoyl-N-[2'-(1H-tetrazol-5-yl)biphenyl-4-ylmethyl]-amin,
(S)-N-(2-Methyl-1-morpholin-4-ylcarbonyl-prop-1-yl)-N-pentanoyl-N-[2'-(1H-tetrazol-5-yl)biphenyl-4-ylmethyl]-amin,
(S)-N-(2'-Carboxybiphenyl-4-ylmethyl)-N-(1-carboxy-2-methyl-prop-1-yl)-N-pentanoyl-amin,
(S)-N-(1,2-Dicarboxyethyl)-N-pentanoyl-N-[2'-(1H-tetrazol-5-yl)biphenyl-4-ylmethyl]-amin,
(S)-N-(1-Carboxy-3-phenyl-prop-1-yl)-N-pentanoyl-N-[2'-(1H-tetrazol-5-yl)biphenyl-4-ylmethyl]-amin,
(S)-N-(2-Cyclohexyl-1-hydroxymethyl-ethyl)-N-pentanoyl-N-[2'-(1H-tetrazol-5-yl)biphenyl-4-ylmethyl]-amin,
(R)-N-(1-Methoxycarbonyl-2-methyl-prop-1-yl)-N-pentanoyl-N-[2'-(1H-tetrazol-5-yl)biphenyl-4-ylmethyl]-amin,
(S)-N-(2-Hydroxy-1-methoxycarbonyl-ethyl)-N-pentanoyl-N-[2'-(1H-tetrazol-5-yl)biphenyl-4-ylmethyl]-amin,
N-Pentanoyl-N-(1H-tetrazol-5-ylmethyl)-N-[2'-(1H-tetrazol-5-yl)biphenyl-4-ylmethyl]-amin,

N-Pentanoyl-N-pyrid-3-ylmethyl-N-[2'-(1H-tetrazol-5-yl)biphenyl-4-ylmethyl]-amin,
(S)-N-(1-Carboxy-4-guanidino-but-1-yl)-N-pentanoyl-N-[2'-(1H-tetrazol-5-yl)biphenyl-4-ylmethyl]-amin,
N-(2-Hydroxy-1-methoxycarbonyl-prop-1-yl)-N-pentanoyl-N-[2'-(1H-tetrazol-5-yl)biphenyl-4-ylmethyl]-amin,
N-(1-Benzyloxycarbonyl-1-methyl-ethyl)-N-butanoyl-N-[2'-(1H-tetrazol-5-yl)biphenyl-4-ylmethyl]-amin,
(S)-N-(1-Carboxy-3-methyl-but-1-yl)-N-pentanoyl-N-[2'-(1H-tetrazol-5-yl)biphenyl-4-ylmethyl]-amin,
N-(1-Carboxy-2-hydroxy-ethyl)-N-pentanoyl-N-[2'-(1H-tetrazol-5-yl)biphenyl- 4-ylmethyl]-amin, (S)-N-(1-Carboxy-2-hydroxy-ethyl)-N-pentanoyl-N-[2'-(1H-tetrazol-5-yl)biphenyl-4-ylmethyl]-amin,
(S)-N-[2-Methyl-1-(2-phenylethylaminocarbonyl)-prop-1-yl]-N-pentanoyl-N-[2'-(1H-tetrazol-5-yl)biphenyl-4-ylmethyl]-amin,
(S)-N-(2-Benzyloxy-1-hydroxymethyl-ethyl)-N-pentanoyl-N-[2'-(1H-tetrazol-5-yl)biphenyl-4-ylmethyl]-amin,
(S)-N-(1-Carboxy-2-methyl-prop-1-yl)-N-pentanoyl-N-[2'-(1H-tetrazol-5-yl)biphenyl-3-ylmethyl]-amin,
(S)-N-(1-Carboxy-2-methyl-prop-1-yl)-N-pentanoyl-N-[3'-(1H-tetrazol-5-yl)biphenyl-4-ylmethyl]-amin,
(S)-N-[2-Methyl-1-(1,2,3,4-tetrahydrochinol-1-ylcarbonyl)-prop-1-yl]-N-pentanoyl-N-[2'-(1H-tetrazol-5-yl)biphenyl-4-ylmethyl]-amin,
(S)-N-(2-Methyl-1-piperidin-1-ylcarbonyl-prop-1-yl)-N-pentanoyl-N-[2'-(1H-tetrazol-5-yl)biphenyl-4-ylmethyl]-amin,
(S)-N-[2-Methyl-1-(1,2,3,4-tetrahydroisochinol-2-ylcarbonyl)-prop-1-yl]-N-pentanoyl-N-[2'-(1H-tetrazol-5-yl)biphenyl-4-ylmethyl]-amin,
N-(2-Hydroxymethyl-2-methyl-prop-1-yl)-N-pentanoyl-N-[2'-(1H-tetrazol-5-yl)biphenyl-4-ylmethyl]-amin,
N-Ethoxycarbonyl-N-(2-ethoxycarbonyl-2-methyl-prop-1-yl)-N-[2'-(1H-tetrazol-5-yl)biphenyl-4-ylmethyl]-amin und
N-(2-Carboxy-2-methyl-prop-1-yl)-N-ethoxycarbonyl-N-[2'-(1H-tetrazol-5-yl)biphenyl-4-ylmethyl]-amin, jeweils in freier Form oder in Salzform.

34. Verfahren zur Herstellung eines pharmazeutischen Präparats als Wirkstoff enthaltend eine Verbindung gemäss einem der Ansprüche 1 oder 3 bis 33, in freier Form oder in Form eines pharmazeutisch verwendbaren Salzes, gegebenenfalls neben üblichen pharmazeutischen Hilfsstoffen, dadurch gekennzeichnet, dass man den Wirkstoff, gegebenenfalls unter Beimischung von üblichen pharmazeutischen Hilfsstoffen, zu einem pharmazeutischen Präparat verarbeitet.

35. Verwendung einer Verbindung gemäss einem der Ansprüche 1 oder 3-33, in freier Form oder in Form eines pharmazeutisch verwendbaren Salzes, zur Herstellung eines Antihypertensivums.

36. Verwendung einer Verbindung gemäss einem der Ansprüche 1 oder 3-33, in freier Form oder in Form eines pharmazeutisch verwendbaren Salzes, zur Herstellung eines pharmazeutischen Präparats zur therapeutischen oder prophylaktischen Behandlung von Herzinsuffizienz.

37. Verwendung einer Verbindung gemäss einem der Ansprüche 1 oder 3-33, in freier Form oder in Form eines pharmazeutisch verwendbaren Salzes, zur Herstellung eines pharmazeutischen Präparats zur therapeutischen oder prophylaktischen Behandlung von Erkrankungen, die durch Angiotensin-II-Aktivität verursacht werden.

## Claims

**Claims for the following Contracting States : AT, BE, CH, DE, DK, FR, GB, IT, LI, LU, NL, SE**

1. A compound of the formula

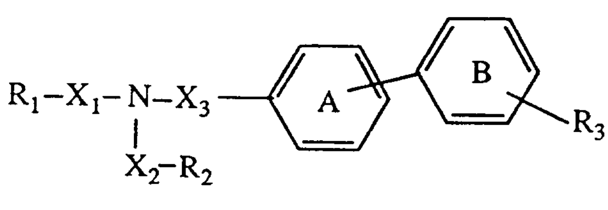

(I),

in which $R_1$ is lower alkyl, lower alkenyl or lower alkynyl or $C_3$-$C_7$cycloalkyl or $C_3$-$C_7$cycloalkenyl or phenyl-lower alkyl, phenyl-lower alkenyl or phenyl-lower alkynyl, each of which is unsubstituted or substituted by halogen or hydroxyl; $X_1$ is CO, $SO_2$ or -O-C(=O)-, the carbon atom of the carbonyl group being bonded to the nitrogen atom drawn in in the formula 1; $X_2$ is $C_1$-$C_{10}$alkylene, $C_2$-$C_{10}$alkylidene or $C_3$-$C_7$cycloalkylene which is unsubstituted or substituted by hydroxyl, carboxyl, amino, guanidino, $C_3$-$C_7$cycloalkyl, $C_3$-$C_7$cycloalkenyl, phenyl or a corresponding

5- or 6-membered and monocyclic aromatic radical which has up to four identical or different heteroatoms, it being possible for a carbon atom of $C_1$-$C_{10}$alkylene or $C_2$-$C_{10}$alkylidene additionally to be bridged by $C_2$-$C_6$alkylene, and $C_3$-$C_7$cycloalkyl or $C_3$-$C_7$cycloalkenyl being unsubstituted, monosubstituted or polysubstituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, formyl, di-lower alkoxymethyl or oxy-lower alkylenoxymethylene; $R_2$ is carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, amino, amino which is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or fused at two adjacent C atoms to a benzene ring, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, 1H-tetrazol-5-yl, pyridyl, hydroxyl, lower alkoxy, lower alkenyloxy, phenyl-lower alkoxy phenoxy, $S(O)_m$-R, m being 0, 1 or 2 and R being hydrogen, lower alkyl, lower alkenyl or lower alkynyl, lower alkanoyl, sulfamoyl, sulfamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, or is $PO_nH_2$, n being 2 or 3; $X_3$ is $C_1$-$C_{10}$alkylene or $C_2$-$C_{10}$alkylidene; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B independently of one another being unsubstituted or substituted by substituents selected from the group consisting of: halogen hydroxyl lower alkoxy, lower alkenyloxy, phenyl-lower alkoxy, phenoxy, $S(O)_m$-R and lower alkyl, lower alkenyl or lower alkynyl, each of which is unsubstituted or substituted by halogen or hydroxyl, lower alkyl, lower alkenyl or lower alkynyl being uninterrupted or interrupted by -O-, and, in the case of (hetero)aromatic radicals, additionally being unsubstituted or substituted by carboxyl, carboxyl which is esterified by an alcohol which is derived from lower alkyl, phenyl-lower alkyl, lower alkenyl lower alkynyl, lower alkoxy-lower alkyl, lower alkoxy-lower alkenyl or lower alkoxy-lower alkynyl, by carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, by formyl, di-lower alkoxymethyl or oxy-lower alkylenoxymethylene; radicals and groups designated by "lower" containing up to and including 7 carbon atoms; in free form or in salt form.

2. A compound according to claim 1 of the formula I, in which $R_1$ is lower alkyl, lower alkenyl or lower alkynyl or $C_3$-$C_7$cycloalkyl or $C_3$-$C_7$cycloalkenyl or phenyl-lower alkyl, phenyl-lower alkenyl or phenyl-lower alkynyl, each of which is unsubstituted or substituted by halogen or hydroxyl; $X_1$ is CO or $SO_2$; $X_2$ is $C_1$-$C_{10}$alkylene or $C_2$-$C_{10}$alkylidene or $C_3$-$C_7$cycloalkylene which is unsubstituted or substituted by hydroxyl, $C_3$-$C_7$cycloalkyl or $C_3$-$C_7$cycloalkenyl or phenyl or a corresponding 5- or 6-membered and monocyclic aromatic radical which has up to 4 identical or different heteroatoms, it being possible for a carbon atom of $C_1$-$C_{10}$alkylene or $C_2$-$C_{10}$alkylidene additionally to be bridged by $C_2$-$C_6$alkylene, and $C_3$-$C_7$cycloalkylene being unsubstituted, monosubstituted or polysubstituted by carboxyl, carboxyl which is esterified by an alcohol which is derived from lower alkyl, phenyl-lower alkyl, lower alkenyl, lower alkynyl, lower alkoxy-lower alkyl lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, formyl, di-lower alkoxymethyl or oxy-lower alkylenoxymethylene; $R_2$ is carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, amino, amino which is monosubstituted or independently of one another disubstituted by lower alkyl, lower alkenyl, lower alkynyl, phenyl-lower alkyl, phenyl-lower alkenyl or

phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, lower alkanoyl- phenyl-lower alkanoyl-, benzoyl- lower alkanesulfonyl- or benzenesulfonyl-amino formyl, di-lower alkoxymethyl, oxy-lower alkylenoxymethylene, hydroxyl, lower alkoxy, lower alkenyloxy, phenyl-lower alkoxy, phenoxy, $S(O)_m$-R, m being 0, 1 or 2 and R being hydrogen, lower alkyl, lower alkenyl or lower alkynyl, lower alkanoyl, sulfamoyl, sulfamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, or is $PO_nH_2$, n being 2 or 3; $X_3$ is $C_1$-$C_{10}$alkylene or $C_2$-$C_{10}$alkylidene; $R_3$ is carboxyl, 5-tetrazolyl, $SO_3H$, $PO_2H_2$, $PO_3H_2$ or halo-lower alkylsulfamoyl; (hetero)aromatic radicals including the rings A and B independently of one another being unsubstituted or substituted by substituents selected from the group consisting of: halogen, hydroxyl, lower alkoxy, lower alkenyloxy, phenyl-lower alkoxy, phenoxy, $S(O)_m$-R and lower alkyl, lower alkenyl or lower alkynyl, each of which is unsubstituted or substituted by halogen or hydroxyl, lower alkyl, lower alkenyl or lower alkynyl being uninterrupted or interrupted by -O-, and, in the case of (hetero)aromatic radicals, additionally unsubstituted or substituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, by carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, by formyl, di-lower alkoxymethyl or oxy-lower alkylenoxymethylene; in free form or in salt form.

3. A compound according to claim 1 of the formula I, in which $R_1$ is lower alkyl, lower alkenyl or lower alkynyl or $C_3$-$C_7$cycloalkyl or $C_3$-$C_7$cycloalkenyl or phenyl-lower alkyl, phenyl-lower alkenyl or phenyl-lower alkynyl, each of which is unsubstituted or substituted by halogen or hydroxyl; $X_1$ is CO or $SO_2$; $X_2$ is $C_1$-$C_{10}$alkylene or $C_2$-$C_{10}$alkylidene which is unsubstituted or substituted by hydroxyl, $C_3$-$C_7$cycloalkyl or $C_3$-$C_7$cycloalkenyl or phenyl or a corresponding 5- or 6-membered and monocyclic aromatic radical which has up to 4 identical or different heteroatoms; $R_2$ is carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl phenyl-lower alkenyl or phenyl-lower alkynyl or is disubstituted by $C_1$-$C_{10}$alkylene or $C_2$-$C_{10}$alkylidene $C_1$-$C_{10}$alkylene or $C_2$-$C_{10}$alkylidene being uninterrupted or interrupted by -O-, amino, amino which is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, lower alkanoyl-, phenyl-lower alkanoyl-, benzoyl- lower alkanesulfonyl- or benzenesulfonyl-amino, formyl, di-lower alkoxymethyl, oxy-lower alkylenoxymethylene, hydroxyl, lower alkoxy, lower alkenyloxy, phenyl-lower alkoxy or phenoxy, $S(O)_m$-R, m being 0, 1 or 2 and R being hydrogen, lower alkyl, lower alkenyl or lower alkynyl, lower alkanoyl, sulfamoyl, sulfamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, or is $PO_nH_2$, n being 2 or 3; $X_3$ is $-CH_2-$; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B independently of one another being unsubstituted or substituted by substituents selected from the group consisting of: halogen, hydroxyl, lower alkoxy, lower alkenyloxy, phenyl-lower alkoxy, phenoxy, $S(O)_m$-R and lower alkyl, lower alkenyl or lower alkynyl, each of which is unsubstituted or substituted by halogen or hydroxyl, lower alkyl, lower alkenyl or lower alkynyl being uninterrupted or interrupted by -O-, and, in the case of (hetero)aromatic radicals, additionally unsubstituted or substituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, by carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_1$-$C_{10}$alkylidene, $C_1$-$C_{10}$alkylene or $C_2$-$C_{10}$alkylidene being uninterrupted or interrupted by -O-, by formyl, di-lower alkoxymethyl or oxy-lower alkylenoxymethylene; in free form or in salt form.

4. A compound according to claim 1 of the formula I, in which $R_1$ is lower alkyl, lower alkenyl, lower alkynyl, halo-lower alkyl, -lower alkenyl or -lower alkynyl, hydroxy-lower alkyl, -lower alkenyl or -lower alkynyl, $C_3$-$C_7$cycloalkyl, $C_3$-$C_7$cycloalkenyl, phenyl-lower alkyl, phenyl-lower alkenyl or phenyl-lower alkynyl; $X_1$ is CO or $SO_2$; $X_2$ is $C_1$-$C_{10}$alkylene or $C_2$-$C_{10}$alkylidene, each of which is unsubstituted or substituted by hydroxyl, a $C_3$-$C_7$cycloalkyl, $C_3$-$C_7$cycloalkenyl or a phenyl radical or a 5- or 6-membered, monocyclic heteroaromatic radical having up to four identical or different heteroatoms, the cyclic radicals for their part being unsubstituted or substituted by carboxyl

which can be esterified with an alcohol which is derived from lower alkyl, phenyl-lower alkyl, lower alkenyl, lower alkynyl, lower alkoxy-lower alkyl, -lower alkenyl or -lower alkynyl, carbamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene or lower alkylenoxy-lower alkylene, formyl, di-lower alkoxymethyl or oxy-lower alkylenoxymethylene; $R_2$ is carboxyl which can be esterified with an alcohol which is derived from lower alkyl, phenyl-lower alkyl, lower alkenyl, lower alkynyl, or lower alkoxy-lower alkyl, -lower alkenyl or -lower alkynyl, carbamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower alkylenoxy-lower alkylene, amino in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower-alkylenoxy-lower alkylene, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, lower alkenyloxy, phenyl-lower alkoxy, phenoxy, $S(O)_m$-R, m being 0, 1 or 2 and R being hydrogen, lower alkyl, lower alkenyl or lower alkynyl, lower alkanoyl, sulfamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower alkylenoxy-lower alkylene, or is $PO_nH_2$, n being 2 or 3; $X_3$ is $-CH_2-$; and $R_3$ is carboxyl, 5-tetrazolyl, $SO_3H$, $PO_2H_2$, $PO_3H_2$ or halo-lower alkylsulfamoyl; (hetero)aromatic radicals including the rings A and B independently of one another each being unsubstituted or substituted by one or more substituents selected from halogen, hydroxyl, lower alkoxy, lower alkenyloxy, or lower alkyl, lower alkenyl, lower alkynyl, lower alkoxy-lower alkyl, -lower alkenyl or -lower alkynyl, lower alkenyloxy-lower alkyl, -lower alkenyl and -lower alkynyl, each of which is unsubstituted or substituted by halogen or hydroxyl, in free form or in salt form.

**5.** A compound according to claim 1 of the formula I, in which $X_2$ is $C_1$-$C_{10}$alkylene or $C_2$-$C_{10}$alkylidene, each of which is unsubstituted or substituted by hydroxyl, a $C_3$-$C_7$cycloalkyl, $C_3$-$C_7$cycloalkenyl or a phenyl radical or a 5- or 6-membered, monocyclic heteroaromatic radical having up to four identical or different heteroatoms, it being possible for a C atom of $C_1$-$C_{10}$alkylene or $C_2$-$C_{10}$alkylidene to be bridged by $C_2$-$C_6$alkylene and the cyclic radicals for their part being unsubstituted or substituted by carboxyl which can be esterified with an alcohol which is derived from lower alkyl, phenyl-lower alkyl, lower alkenyl, lower alkynyl, or lower alkoxy-lower alkyl, -lower alkenyl or -lower alkynyl, carbamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower alkylenoxy-lower alkylene, formyl, di-lower alkoxymethyl or by oxy-lower alkylenoxymethylene, or $X_2$ is $C_3$-$C_7$cycloalkylene; $X_3$ is lower alkylene or lower alkylidene; the variables $X_1$, $R_1$, $R_2$ and $R_3$ are as defined in claim 4; and the (hetero)aromatic rings including the rings A and B can be substituted as indicated in claim 4, in free form or in salt form.

**6.** A compound according to claim 1 of the formula I, in which $R_1$ is lower alkyl, lower alkenyl, halo-lower alkyl or -lower alkenyl, hydroxy-lower alkyl, 3- to 7-membered cycloalkyl or phenyl-lower alkyl; $X_1$ is CO, $SO_2$ or -O-C(=O)-, the carbon atom of the carbonyl group being bonded to the nitrogen atom drawn in in the formula I; $X_2$ is $C_1$-$C_{10}$alkylene or $C_1$-$C_7$alkylidene, each of which is unsubstituted or substituted by hydroxyl, carboxyl, amino, guanidino, a 3- to 7-membered cycloalkyl, 3- to 7-membered cycloalkenyl, phenyl, pyrrolyl, pyrazolyl, imidazolyl, triazolyl, tetrazolyl, furyl, thienyl or pyridyl radical can which for its part additionally be unsubstituted or substituted by carboxyl, lower alkoxycarbonyl, phenyl-lower alkoxycarbonyl, carbamoyl in which the amino group is unsubstituted, monosubstituted or independently of one another disubstituted by lower alkyl or phenyl-lower alkyl, formyl, di-lower alkoxymethyl or oxy-lower alkylenoxymethylene; $R_2$ is carboxyl, lower alkoxy-, phenyl-lower alkoxy-, lower alkenyloxy- or lower alkoxy-lower alkoxy-carbonyl, carbamoyl in which the amino group is unsubstituted, monosubstituted or independently of one another disubstituted by lower alkyl or phenyl-lower alkyl or is disubstituted by lower alkylene which can be fused at two adjacent carbon atoms to a benzene ring, or lower alkylenoxy-lower alkylene, amino in which the amino group is unsubstituted, monosubstituted or independently of one another disubstituted by lower alkyl or phenyl-lower alkyl or is disubstituted by lower alkylene- or lower alkylenoxy-lower alkylene, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, phenyl-lower alkoxy, phenoxy, $S(O)_m$-R, m being 0, 1 or 2 and R being lower alkyl, lower alkanoyl, sulfamoyl in which the amino group is unsubstituted, monosubstituted or independently of one another disubstituted by lower alkyl or phenyl-lower alkyl, or is $PO_nH_2$, n being 2 or 3; $X_3$ is methylene; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B are each additionally unsubstituted or substituted by one or more substituents selected from halogen, hydroxyl, lower alkoxy, or lower alkyl or lower alkoxy-lower alkyl, each of which is unsubstituted or substituted by halogen or hydroxyl, in free form or in salt form.

7. A compound according to claim 1 of the formula I, in which $R_1$ is lower alkyl, lower alkenyl, halo-lower alkyl or -lower alkenyl, hydroxy-lower alkyl, 3- to 7-membered cycloalkyl or phenyl-lower alkyl; $X_1$ is CO or $SO_2$; $X_2$ is $C_1$-$C_{10}$alkylene or $C_1$-$C_7$alkylidene, each of which is unsubstituted or substituted by hydroxyl, a 3- to 7-membered cycloalkyl, 3- to 7-membered cycloalkenyl, phenyl, pyrrolyl, pyrazolyl, imidazolyl, triazolyl, tetrazolyl, furyl, thienyl or pyridyl radical which for its part can additionally be unsubstituted or substituted by carboxyl, lower alkoxycarbonyl, phenyl-lower alkoxycarbonyl, carbamoyl in which the amino group is unsubstituted, monosubstituted or independently of one another disubstituted by lower alkyl or phenyl-lower alkyl formyl, di-lower alkoxymethyl or oxy-lower alkylenoxymethylene; $R_2$ is carboxyl, lower alkoxy-, phenyl-lower alkoxy-, lower alkenyloxy- or lower alkoxy-lower alkoxy-carbonyl, carbamoyl in which the amino group is unsubstituted, monosubstituted or independently of one another disubstituted by lower alkyl or phenyl-lower alkyl or is disubstituted by lower alkylene- or lower alkylenoxy-lower alkylene, amino in which the amino group is unsubstituted, monosubstituted or independently of one another disubstituted by lower alkyl or phenyl-lower alkyl or is disubstituted by lower alkylene- or lower alkylenoxy-lower alkylene, lower alkanoyl, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, phenyl-lower alkoxy, phenoxy, $S(O)_m$-R, m being 0, 1 or 2 and R being lower alkyl, lower alkanoyl, sulfamoyl in which the amino group is unsubstituted, monosubstituted or independently of one another disubstituted by lower alkyl or phenyl-lower alkyl, or is $PO_nH_2$, n being 2 or 3; $X_3$ is methylene; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B are in each case additionally unsubstituted or substituted by one or more substituents selected from halogen, hydroxyl, lower alkoxy, or lower alkyl or lower alkoxy-lower alkyl, each of which is unsubstituted or substituted by halogen or hydroxyl, In free form or in salt form.

8. A compound according to claim 1 of the formula I, in which $X_2$ is $C_1$-$C_{10}$alkylene or $C_1$-$C_7$alkylidene, each of which is unsubstituted or substituted by hydroxyl, a 3- to 7-membered cycloalkyl, 3- to 7-membered cycloalkenyl, phenyl, pyrrolyl, pyrazolyl, imidazolyl, triazolyl, tetrazolyl, furyl, thienyl or pyridyl radical which for its part can additionally be substituted by carboxyl, lower alkoxycarbonyl, phenyl-lower alkoxycarbonyl, carbamoyl in which the amino group is unsubstituted, monosubstituted or independently of one another disubstituted by lower alkyl or phenyl-lower alkyl, formyl, di-lower alkoxymethyl or by oxy-lower alkylenoxymethylene, it being possible for a C atom of $C_1$-$C_{10}$alkylene or $C_1$-$C_7$alkylidene to be bridged by $C_2$-$C_6$alkylene, or $X_2$ is $C_3$-$C_7$cycloalkylene; $X_3$ is lower alkylene or lower alkylidene; the variables $X_1$, $R_1$, $R_2$ and $R_3$ are as defined in claim 7; and the (hetero)aromatic rings including the rings A and B can be substituted as indicated in claim 7, in free form or in salt form.

9. A compound according to claim 1 of the formula I, in which the variables $R_1$, $X_1$ and $R_3$ are as defined in each case in any one of claims 1-7; $X_2$ is lower alkylene or lower alkylidene, each of which is unsubstituted or substituted by hydroxyl, 3- to 7-membered cycloalkyl, phenyl or imidazolyl and $R_2$ is carboxyl, lower alkoxy-, phenyl-lower alkoxy- or lower alkoxy-lower alkoxy-carbonyl, carbamoyl which is unsubstituted, monosubstituted or independently of one another disubstituted by lower alkyl or phenyl-lower alkyl, amino, lower alkanoyl-, phenyl-lower alkanoyl- or lower alkanesulfonylamino, hydroxyl, lower alkoxy, phenyl-lower alkoxy or phenoxy; $X_3$ is $-CH_2-$; (hetero)aromatic radicals including the rings A and B each being unsubstituted or substituted by one or more substituents selected from halogen, trifluoromethyl, hydroxyl, lower alkoxy, lower alkyl, hydroxy-lower alkyl or lower alkoxy-lower alkyl, in free form or in salt form.

10. A compound according to claim 1 of the formula I, in which $X_2$ is lower alkylene or lower alkylidene which is unsubstituted or substituted by hydroxyl, 3- to 7-membered cycloalkyl, 7-membered cycloalkenyl, phenyl or imidazolyl, where a C atom of lower alkylene or lower alkylidene can be bridged by $C_2$-$C_6$alkylene, or $X_2$ is $C_3$-$C_7$cycloalkylene; the variables $X_1$, $X_3$, $R_1$, $R_2$ and $R_3$ are as defined in claims 7 to 9; and the rings A and B can be substituted as indicated in claim 9, in free form or in salt form.

11. A compound according to claim 1 of the formula

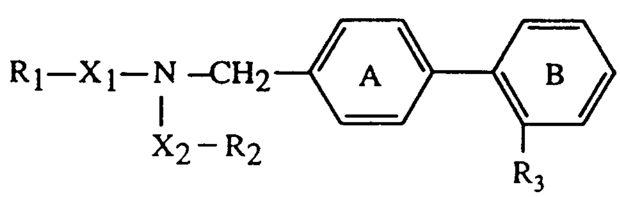

(Ia),

in which the variables $R_1$, $X_1$, $X_2$, $R_2$ and $R_3$ are as defined in each case in any one of claims 1-10 and the rings A and B can be substituted as indicated in claim 10, in free form or in salt form.

**12.** A compound according to claim 1 of the formula Ia, in which $X_2$ is lower alkylene or lower alkylidene, which is unsubstituted or substituted by hydroxyl or 3- to 7-membered cycloalkyl, it being possible for a C atom of lower alkylene or lower alkylidene to be bridged by $C_2$-$C_6$alkylene, or in which $X_2$ is $C_3$-$C_7$cycloalkylene; the variables $R_1$,$X_1$, $R_2$ and $R_3$ are as defined in each case in any one of claims 1-10; and the rings A and B can be substituted as indicated in claim 10, in free form or in salt form.

**13.** A compound according to claim 1 of the formula Ia, in which $X_2$ is the group of the formula

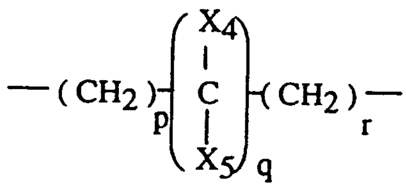

(Ib)

in which p is 0 or 1, q is 1 and r is 0 or 1 or in which p is 1 to 8 and q and r are in each case 0; $X_4$ is lower alkyl or phenyl which is unsubstituted or substituted by hydroxyl, 3- to 7-membered cycloalkyl, phenyl or imidazolyl; and $X_5$ is hydrogen or lower alkyl; $R_2$ is carboxyl, lower alkoxycarbonyl, phenyl-lower alkoxycarbonyl, lower alkoxy-lower alkoxycarbonyl, hydroxyl, lower alkoxy, phenyl-lower alkoxy, phenoxy, amino, lower alkanoylamino, phenyl-lower alkanoylamino or lower alkanesulfonylamino; and the variables $R_1$, $X_1$ and $R_3$ are as defined in each case in any one of claims 1-7; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**14.** A compound according to claim 1 of the formula Ia, in which $X_2$ is the group of the formula Ib in which p is 0 or 1, q is 1 and r is 0 or 1 or in which p is 1 to 8 and q and r are each 0; $X_4$ is lower alkyl or phenyl, which is unsubstituted or substituted by hydroxyl, 3- to 7-membered cycloalkyl, phenyl or imidazolyl; and $X_5$ is hydrogen or lower alkyl; or $X_4$ and $X_5$ together are $C_2$-$C_6$alkylene, such as $C_4$-$C_5$alkylene; or $X_2$ is $C_3$-$C_7$cycloalkylene, such as $C_5$-$C_6$cycloalkylene; $R_2$ is carboxyl, lower alkoxycarbonyl, phenyl-lower alkoxycarbonyl, lower alkoxy-lower alkoxycarbonyl, hydroxyl, lower alkoxy, phenyl-lower alkoxy, phenoxy, amino, lower alkanoylamino, phenyl-lower alkanoylamino or lower alkanesulfonylamino; and the variables $R_1$, $X_1$ and $R_3$ are as defined in each case in any one of claims 1-7; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**15.** A compound according to claim 1 of the formula Ia, in which $R_1$ is lower alkyl, such as $C_3$-$C_5$alkyl, or lower alkenyl, such as $C_3$-$C_5$alkenyl; $X_1$ is CO or else $SO_2$; $X_2$ is the group of the formula Ib in which p and r are 0 or 1 and q is 1; $X_4$ is lower alkyl, such as $C_1$-$C_4$alkyl, or phenyl, which is unsubstituted or substituted by hydroxyl, 3- to 7-membered cycloalkyl, such as cyclohexyl, or by phenyl or imidazolyl which is unsubstituted or substituted by halogen or hydroxyl, such as 4-imidazolyl; and $X_5$ is hydrogen or lower alkyl, such as $C_1$-$C_4$alkyl; or $X_4$ and $X_5$ together are $C_2$-$C_6$alkylene, such as $C_4$-$C_5$alkylene; or $X_2$ is $C_3$-$C_7$cycloalkylene, such as $C_5$-$C_6$cycloalkylene; $R_2$ is carboxyl, lower alkoxycarbonyl, such as $C_2$-$C_5$alkoxycarbonyl, phenyl-lower alkoxycarbonyl such as phenyl-$C_1$-$C_4$alkoxycarbonyl, lower alkoxyl-lower alkoxycarbonyl, such as $C_1$-$C_4$alkoxy-$C_2$-$C_5$alkoxycarbonyl, hydroxyl or lower alkoxy, such as $C_1$-$C_4$alkoxy; and $R_3$ is carboxyl or 5-tetrazolyl; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**16.** A compound according to claim 1 of the formula Ia, in which $R_1$ is lower alkyl, such as $C_3$-$C_5$alkyl, or lower alkenyl, such as $C_3$-$C_5$alkenyl; $X_1$ is CO or else $SO_2$; $X_2$ is the group of the formula Ib in which p and r are 0 or 1 and q is 1; $X_4$ is lower alkyl, such as $C_1$-$C_4$alkyl, or phenyl, which is unsubstituted or substituted by hydroxyl, 3- to 7-membered cycloalkyl, or by phenyl or imidazolyl which is unsubstituted or substituted by halogen or hydroxyl, such as 4-imidazolyl; and $X_5$ is hydrogen or lower alkyl, such as $C_1$-$C_4$alkyl; $R_2$ is carboxyl, lower alkoxycarbonyl, such as $C_2$-$C_5$alkoxycarbonyl, phenyl-lower alkoxycarbonyl, such as phenyl-$C_1$-$C_4$alkoxycarbonyl, lower alkoxy-lower alkoxycarbonyl, such as $C_1$-$C_4$alkoxy-$C_2$-$C_5$alkoxycarbonyl, hydroxyl or lower alkoxy, such as $C_1$-$C_4$alkoxy; and $R_3$ is carboxyl or 5-tetrazolyl; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**17.** A compound according to claim 1 of the formula Ia, in which $R_1$ is lower alkyl, such as $C_3$-$C_5$alkyl, or else lower alkenyl, such as $C_3$-$C_5$alkenyl; $X_1$ is CO or else $SO_2$; $X_2$ is the group of the formula Ib in which p is an integer from

1 to 8 and q and r are 0; $R_2$ is hydroxyl, lower alkoxy, such as $C_1$-$C_4$alkoxy, phenyl-lower alkoxy, such as phenyl-$C_1$-$C_4$alkoxy, phenoxy, lower alkanoylamino, such as $C_1$-$C_4$alkanoylamino, phenyl-lower alkanoylamino, such as phenyl-$C_1$-$C_4$alkanoylamino, or lower alkanesulfonylamino, such as $C_1$-$C_4$alkanesulfonylamino; and $R_3$ is carboxyl or primarily 5-terrazolyl; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**18.** A compound according to claim 1 of the formula Ia, in which $R_1$ is $C_3$-$C_5$alkyl or secondarily $C_3$-$C_5$alkenyl; $X_1$ is CO or else $SO_2$; $X_2$ is the group of the formula Ib in which p and r independently of one another are 0 or 1 and q is 1; $X_4$ is $C_1$-$C_4$alkyl, hydroxy-$C_1$-$C_4$alkyl, $C_3$-$C_7$cycloalkyl-$C_1$-$C_4$alkyl, phenyl-$C_1$-$C_4$alkyl or imidazolyl-$C_1$-$C_4$alkyl; and $X_5$ is hydrogen or $C_1$-$C_4$alkyl; or $X_4$ and $X_5$ together are tetramethylene or else pentamethylene; $R_2$ is carboxyl or $C_2$-$C_5$alkoxycarbonyl or else phenyl-$C_1$-$C_4$alkoxycarbonyl; and $R_3$ is carboxyl or 5-tetrazolyl, in free form or in salt form.

**19.** A compound according to claim 1 of the formula Ia, in which $R_1$ is $C_3$-$C_5$alkyl or secondarily $C_3$-$C_5$alkenyl; $X_1$ is CO or else $SO_2$; $X_2$ is the group of the formula Ib in which p and r are each 0 or 1 and q is 1; $X_4$ is $C_1$-$C_4$alkyl, hydroxy-$C_1$-$C_4$alkyl, $C_3$-$C_7$cycloalkyl-$C_1$-$C_4$alkyl, phenyl-$C_1$-$C_4$alkyl or imidazolyl-$C_1$-$C_4$alkyl; and $X_5$ is hydrogen; $R_2$ is carboxyl or $C_2$-$C_5$alkoxcarbonyl or else phenyl-$C_{1_}C_4$alkoxycarbonyl; and $R_3$ is carboxyl or 5-tetrazolyl, in free form or in salt form.

**20.** A compound according to claim 1 of the formula Ia, in which $R_1$ is $C_3$-$C_5$alkyl; $X_1$ is CO; $X_2$ is the group of the formula Ib in which q and r are 0 and p is 1 to 3 or in which p and q are 1 and r is 0; $X_4$ is $C_1$-$C_4$alkyl; $X_5$ is hydrogen or $C_1$-$C_4$alkyl; $R_2$ is carboxyl or $C_2$-$C_5$alkoxycarbonyl; and $R_3$ is carboxyl or 5-tetrazolyl, in free form or in salt form.

**21.** A compound according to any any one of claims 1-20, in which $R_3$ is 5-tetrazolyl, in free form or in salt form.

**22.** A compound according to claim 1 of the formula Ia, in which $R_1$ is $C_3$-$C_5$alkyl; $X_1$ is CO; $X_2$ is the group of the formula Ib in which p is 0 or 1, r is 0 and q is 1; $X_4$ is $C_1$-$C_4$alkyl; and $X_5$ is hydrogen or $C_1$-$C_4$alkyl; or $X_4$ and $X_5$ together are tetramethylene or pentamethylene; $R_2$ is carboxyl or $C_2$-$C_5$alkoxycarbonyl; and $R_3$ is 5-tetrazolyl, in free form or in salt form.

**23.** A compound according to claim 1 of the formula Ia, in which $R_1$ is $C_3$-$C_5$alkyl; $X_1$ is CO; $X_2$ is the group of the formula Ib in which p is 0 or 1, r is 0 and q is 1; $X_4$ and $X_5$ together are tetramethylene or else pentamethylene; $R_2$ is carboxyl or $C_2$-$C_5$alkoxycarbonyl; and $R_3$ is 5-tetrazolyl, in free form or in salt form.

**24.** A compound according to claim 1 of the formula Ia, in which $R_1$ is $C_3$-$C_5$alkyl; $X_1$ is CO; $X_2$ is the group of the formula Ib in which p and r are 0 or 1 and q is 1; $X_4$ is $C_1$-$C_4$alkyl; and $X_5$ is hydrogen; $R_2$ is carboxyl or $C_2$-$C_5$alkoxycarbonyl; and $R_3$ is 5-tetrazolyl, in free form or in salt form.

**25.** A compound of the formula Ia according to any one of claims 13-24, in which $X_2$ is the group of the formula Ib, q is 1 and $X_4$ and $X_5$ are defined differently, in free form or in salt form, in which the asymmetric C atom in question containing the variables $X_4$ and $X_5$ has the S configuration.

**26.** (S)-N-(1-Carboxy-2-methylprop-1-yl)-N-pentanoyl-N-[2'-(1H-tetrazol-5-yl)-biphenyl-4-ylmethyl]amine, in free form or in salt form according to claim 1.

**27.** N-(2-Carboxy-2,2-tetramethyleneethyl)-N-pentanoyl-N-[2'-(1H-tetrazol-5-yl)biphenyl-4-ylmethyl]amine, in free form or in salt form according to claim 1.

**28.** N-(2-Carboxy-2-ethylbut-1-yl)-N-pentanoyl-N-[2'-(1H-tetrazol-5-yl)biphenyl-4-ylmethyl]amine, in free form or in salt form according to claim 1.

**29.** (S)-N-(1-Carboxy-2-methylprop-1-yl)-N-ethoxycarbonyl-N-[2'-(1H-tetrazol-5-yl)biphenyl-4-ylmethyl]amine, in free form or in salt form according to claim 1.

**30.** N-(1-Carboxycyclohexyl)-N-pentanoyl-N-[2'-(1H-tetrazol-5-yl)biphenyl-4-ylmethyl]-amine, in free form or in salt form according to claim 1.

**31.** A compound according to claim 1 selected from the group consisting of:
(S)-N-(1-carboxyethyl)-N-pentanoyl-N-[2'-(1H-tetrazol-5-yl)biphenyl-4-ylmethyl]amine,

N-(2-hydroxyethyl)-N-pentanoyl-N-(2'-(1H-tetrazol-5-yl)biphenyl-4-ylmethyl]amine, N-(2-ethoxycarbonyl-2,2-tetramethyleneethyl)-N-pentanoyl-N-[2'-(1H-tetrazol-5-yl)biphenyl-4-ylmethyl]amine, N-(2-ethoxycarbonyl-2-ethylbut-1-yl)-N-pentanoyl-N-[2'-(1H-tetrazol-5-yl)biphenyl-4-ylmethyl]amine, N(2-ethoxycarbonyl)-2-methylprop-1-yl)-N-pentanoyl-N-[2'-(1H-tetrazol-5-yl)-biphenyl-4-ylmethyl]amine, (S)-N-(1-hydroxymethyl-2-methylprop-1-yl)-N-pentanoyl-N-[2'-1H-tetrazol-5-yl)biphenyl-4-ylmethyl]amine, N-(2-ethoxycarbonyl-2,2-pentamethyleneethyl)-N-pentanoyl-N-[2'-(1H-tetrazol-5-yl)biphenyl-4-ylmethyl]amine, (S)-N-(1-carboxy-2-methylprop-1-yl)-N-propyloxycarbonyl-N-[2'-(1H-tetrazol-5-yl)biphenyl-4-ylmethyl]amine, N-(2-carboxy-2-methylpropyl)-N-pentanoyl-N-[2'-(1H-tetrazol-5-yl)biphenyl-4-ylmethyl]amine, N-(2-carboxy-2,2-pentamethyleneethyl)-N-pentanoyl-N-[2'-(1H-tetrazol-5-yl)biphenyl-4-ylmethyl]amine, (S)-N-(1-aminocarbonyl-2-methylprop-1-yl)-N-pentanoyl-N-[2'-(1H-tetrazol-5-yl)biphenyl-4-ylmethyl]amine and (S)-N-(1-carboxy-2-methylprop-1-yl)-N-(5-oxopent-1-en-5-yl)-N-[2'-(1H-tetrazol-5-yl)biphenyl-4-ylmethyl]amine, in each case in free form or in salt form.

**32.** A compound according to claim 1 selected from the group consisting of: N-carboxymethyl-N-pentanoyl-N-[2'-(1H-tetrazol-5-yl)biphenyl-4-ylmethyl]amine, (S)-N-(1-methoxycarbonylethyl)-N-pentanoyl-N-[2'-(1H-tetrazol-5-yl)biphenyl-4-ylmethyl]amine, N-[1-carboxy-2-(4-fluorophenyl)ethyl]-N-pentanoyl-N-[2'-(1H-tetrazol-5-yl)biphenyl-4-ylmethyl]amine, N-[2-(4-fluorophenyl)-1-methoxycarbonylethyl]-N-pentanoyl-N-[2'-(1H-tetrazol-5-yl)biphenyl-4-ylmethyl]amine, N-[2-(4-fluorophenyl)-1-hydroxymethylethyl]-N-pentanoyl-N-[2'-(1H-tetrazol-5-yl)biphenyl-4-ylmethyl]amine, N-(2'-carboxybiphenyl-4-ylmethyl)-N-[1-carboxy-2-(4-fluorophenyl)ethyl]-N-pentanoyl-amine, N-(2'-carboxybiphenyl-4-ylmethyl)-N-[2-(4-fluorophenyl)-1-methoxycarbonylethyl]-N-pentanoylamine, (S)-N-(2'-carboxybiphenyl-4-ylmethyl)-N-(1-hydroxymethyl-2-phenylethyl)-N-pentanoylamine, (S)-N-(2'-carboxybiphenyl-4-ylmethyl)-N-(1-hydroxymethyl-2-imidazol-4-ylethyl)-N-pentanoylamine, (R)-N-(1-carboxyethyl)-N-pentanoyl-N-[2'-(1H-tetrazol-5-yl)biphenyl-4-ylmethyl]amine, (1S),(2S)-N-(1-carboxy-2-methylbut-1-yl)-N-pentanoyl-N-[2'-(1H-tetrazol-5-yl)biphenyl-4-ylmethyl]amine, (1S),(2S)-N-(1-methoxycarbonyl-2-methylbut-1-yl)-N-pentanoyl-N-[2'-(1H-tetrazol-5-yl)biphenyl-4-ylmethyl]amine, (S)-N-(1-carboxybut-1-yl)-N-pentanoyl-N-[2'-(1H-tetrazol-5-yl)biphenyl-4-ylmethyl]-amine, (S)-N-(1-methoxycarbonylbut-1-yl)-N-pentanoyl-N-[2'-(1H-tetrazol-5-yl)biphenyl-4-ylmethyl]amine, (S)-N-(1-carboxyethyl)-N-hexanoyl-N-[2'-(1H-tetrazol-5-yl)biphenyl-4-ylmethyl]amine, (S)-N-butanoyl-N-(1-carboxyethyl)-N-[2'-(1H-tetrazol-5-yl)biphenyl-4-ylmethyl]amine, (S)-N-(1-carboxyprop-1-yl)-N-pentanoyl-N-[2'-(1H-tetrazol-5-yl)biphenyl-4-ylmethyl]-amine, (S)-N-(1-carboxy-2-cyclohexylethyl)-N-pentanoyl-N-[2'-(1H-tetrazol-5-yl)biphenyl-4-ylmethyl]amine, (S)-N-(2-cyclohexyl-1-methoxycarbonylethyl)-N-pentanoyl-N-[2'-(1H-tetrazol-5-yl)biphenyl-4-ylmethyl]amine, (R)-N-(1-carboxy-2-methylprop-1-yl)-N-pentanoyl-N-[2'-(1H-tetrazol-5-yl)biphenyl-4-ylmethyl]amine, N-(2-methoxyethyl)-N-pentanoyl-N-[2'-(1H-tetrazol-5-yl)biphenyl-4-ylmethyl]amine, N-(2-benzyloxyethyl)-N-pentanoyl-N-[2'-(1H-tetrazol-5-yl)biphenyl-4-ylmethyl]amine, N-(3-methoxyprop-1-yl)-N-pentanoyl-N-[2'-(1H-tetrazol-5-yl)biphenyl-4-ylmethyl]-amine, N-(3-benzyloxyprop-1-yl)-N-pentanoyl-N-[2'-1H-tetrazol-5-yl)biphenyl-4-ylmethyl]-amine, N-(3-hydroxyprop-1-yl)-N-penanol-N-[2'-(1H-tetrazol-5-yl)biphenyl-4-ylmethyl]-amine, N-(1-methoxycarbonyl-1-methylethyl)-N-pentanoyl-N-[2'-(1H-tetrazol-5-yl)biphenyl-4-ylmethyl]amine, N-(2-carboxyethyl)-N-pentanoyl-N-[2'-(1H-tetrazol-5-yl)biphenyl-4-ylmethyl]amine. N-(2-carboxyprop-1-yl)-N-pentanoyl-N-[2'-(1H-tetrazol-5-yl)biphenyl-4-ylmethyl]-amine, N-(1-carboxy-1-methylethyl)-N-pentanoyl-N-[2'-(1H-tetrazol-5-yl)biphenyl-4-ylmethyl]amine, N-(5-hydroxypent-1-yl)-N-pentanoyl-N-[2'-(1H-tetrazol-5-yl)biphenyl-4-ylmethyl]amine, N-(1-carboxyprop-2-yl)-N-pentanoyl-N-[2'-(1H-tetrazol-5-yl)biphenyl-4-ylmethyl]amine, N-(2-ethoxycarbonyl-3-methylbut-1-yl)-N-pentanoyl-N-[2'-(1H-tetrazol-5-yl)biphenyl-4-ylmethyl]amine, N-(2-carboxy-3-methylbut-1-yl)-N-pentanoyl-N-[2'-(1H-tetrazol-5-yl)biphenyl-4-ylmethyl]amine, N[(3-phenoxyprop-1-yl)-N-pentanoyl-N-[2'-(1H-tetrazol-5-yl)biphenyl-4-ylmethyl]amine, N-[2-(4-hydroxyphenyl)ethyl]-N-pentanoyl-N-[2'-(1H-tetrazol-5-yl)biphenyl-4-ylmethyl]amine, N-[3-(4-hydroxyphenyl)prop-1-yl]-N-pentanoyl-N-[2'-(1H-tetrazol-5-yl)biphenyl-4-ylmethyl]amine, N-(8-hydroxyoct-1-yl)-N-pentanoyl-N-[2'-(1H-tetrazol-5-yl)biphenyl-4-ylmethyl]amine, N-(2-methanesulfonylaminoethyl)-N-pentanoyl-N-[2'-(1H-tetrazol-5-yl)biphenyl-4-ylmethyl]amine, N-(3-acetylaminoprop-1-yl)-N-pentanoyl-N-[2'-(1H-tetrazol-5-yl)biphenyl-4-ylmethyl]-amine, N-(2-methoxy-2-oxo-1-phenylethyl)-N-pentanoyl-N-[2'-(1H-tetrazol-5-yl)biphenyl-4-ylmethyl]amine, N-(4-hydroxybut-2-yl)-N-pentanoyl-N-[2'-(1H-tetrazol-5-yl)biphenyl-4-ylmethyl]amine, N-(2-hydroxy-1-phenylethyl)-N-pentanoyl-N-[2'-(1H-tetrazol-5-yl)biphenyl-4-ylmethyl]amine, N-[3-(4-hydroxybenzylcarbonylamino)prop-1-yl]-N-pentanoyl-N-[2'-(1H-tetrazol-5-yl)biphenyl-4-ylmethyl]amine, N-(3-ethoxycarbonylcyclohexyl)-N-pentanoyl-N-[2'-(1H-tetrazol-5-yl)biphenyl-4-ylmethyl]amine, N-(3-carboxycyclohexyl)-N-pentanoyl-N-[2'-(1H-tetrazol-5-yl)biphenyl-4-ylmethyl]-amine,

cis-N-(4-carboxycyclohexyl)-N-pentanoyl-N-[2'-(1H-tetrazol-5-yl)biphenyl-4-ylmethyl]amine,
cis-N-(2-ethoxycarbonylcyclohexyl)-N-pentanoyl-N-[2'-(1H-tetrazol-5-yl)biphenyl-4-ylmethyl]amine,
cis-N-(2-carboxycyclohexyl)-N-pentanoyl-N-[2'-(1H-tetrazol-5-yl)biphenyl-4-ylmethyl]amine.
N-[2-[2-(4-hydroxyphenyl)ethylaminocarbonyl]-2,2-tetramethyleneethyl)-N-pentanoyl-N-[2'-(1H-tetrazol-5-yl)biphenyl-4-ylmethyl]amine,
(S)-N-{1-[2-(4-hydroxyphenyl)ethylaminocarbonyl]-2-methylprop-1-yl}-N-pentanoyl-N-[2'-(1H-tetrazol-5-yl)biphenyl-4-ylmethyl]amine,
(S)-N-(1-carboxy-2,2-dimethylprop-1-yl)-N-pentanoyl-N-[2'-(1H-tetrazol-5-yl)biphenyl-4-ylmethyl]amine,
(S)-N-(1-methoxycarbonyl-2-methylprop-1-yl)-N-pentanoyl-N-[2'-(1H-tetrazol-5-yl)biphenyl-4-ylmethyl]amine,
N-(4-phenoxybut-1-yl)-N-pentanoyl-N-[2'-(1H-tetrazol-5-yl)biphenyl-4-ylmethyl]amine,
N-(2-hydroxy-1-phenyl-2-oxoethyl)-N-pentanoyl-N-[2'-(1H-tetrazol-5-yl)biphenyl-4-ylmethyl]amine,
(S)-N-(1-benzyloxycarbonyl-2-methylprop-1-yl)-N-pentanoyl-N-[2'-(1H-tetrazol-5-yl)biphenyl-4-ylmethyl]amine,
N-butanoyl-N-(1-carboxy-1-methylethyl)-N-[2'-(1H-tetrazol-5-yl)biphenyl-4-ylmethyl]-amine,
N-(4-hydroxybut-1-yl)-N-pentanoyl-N-[2'-(1H-tetrazol-5-yl)biphenyl-4-ylmethyl]amine,
(S)-N-(1-benzyloxycarbonyl-2-methylprop-1-yl)-N-[3-bromo-2'-(1H-tetrazol-5-yl)biphenyl-4-ylmethyl]-N-pentanoylamine,
(S)-N-[3-bromo-2'-(1H-tetrazol-5-yl)biphenyl-4-ylmethyl]-N-(1-carboxy-2-methyl-prop-1-yl)-N-pentanoylamine,
N-(2-acetylaminoethyl)-N-pentanoyl-N-[2'-(1H-tetrazol-5-yl)biphenyl-4-ylmethyl]amine,
N-[2-(n-butoxycarbonyl)-2,2-tetramethyleneethyl]-N-pentanoyl-N-[2'-(1H-tetrazol-5-yl)biphenyl-4-ylmethyl]amine,
N-(2-benzylaminocarbonyl-2,2-tetramethyleneethyl)-N-pentanoyl-N-[2'-(1H-tetrazol-5-yl)biphenyl-4-ylmethyl]amine,
(S)-N-butyloxycarbonyl-N-(1-carboxy-2-methylprop-1-yl)-N-[2'-(1H-tetrazol-5-yl)biphenyl-4-ylmethyl]amine,
(S)-N-(1-carboxy-2-methylprop-1-yl)-N-methoxycarbonyl-N-[2'-(1H-tetrazol-5-yl)biphenyl-4-ylmethyl]amine,
N-(2-diethylaminocarbonyl-2,2-tetramethyleneethyl)-N-pentanoyl-[2'-(1H-tetrazol-5-yl)biphenyl-4-ylmethyl]amine,
N-(2-methyl-2-morpholin-4-ylcarbonylpropyl)-N-pentanoyl-N-[2'-(1H-tetrazol-5-yl)biphenyl-4-ylmethyl]amine,
N-(1-carboxycyclopentyl)-N-pentanoyl-N-[2'-(1H-tetrazol-5-yl)biphenyl-4-ylmethyl]-amine,
N-(1-carboxy-1-ethylprop-1-yl)-N-pentanoyl-N-[2'-(1H-tetrazol-5-yl)biphenyl-4-ylmethyl]amine,
(S)-N-(5-amino-1-carboxypent-1-yl)-N-pentanoyl-N-[2'-(1H-tetrazol-5-yl)biphenyl-4-ylmethyl]amine,
N-butanesulfonyl-N-(2-ethoxycarbonyl-2,2-pentamethyleneethyl)-N-[2'-(1H-tetrazol-5-yl)biphenyl-4-ylmethyl]amine,
N-butanesulfonyl-N-(2-carboxy-2,2-pentamethyleneethyl)-N-[2'-(1H-tetrazol-5-yl)biphenyl-4-ylmethyl]amine,
N-butanesulfonyl-N-(2-ethoxycarbonyl-2-methylprop-1-yl)-N-[2'-(1H-tetrazol-5-yl)biphenyl-4-ylmethyl]amine,
N-butanesulfonyl-N-(2-carboxy-2-methylprop-1-yl)-N-[2'-(1H-tetrazol-5-yl)biphenyl-4-ylmethyl]amine,
(S)-N-butanesulfonyl-N-(1-tert-butoxycarbonylethyl)-N-[2'-(1H-tetrazol-5-yl)biphenyl-4-ylmethyl]amine,
(S)-N-butanesulfonyl-N-(1-carboxyethyl)-N-[2'-(1H-tetrazol-5-yl)biphenyl-4-ylmethyl]-amine,
(S)-N-butanesulfonyl-N-(1-carboxy-2-methylprop-1-yl)-N-[2'-(1H-tetrazol-5-yl)biphenyl-4-ylmethyl]amine,
(2)-N-(2-methyl-1-methylaminocarbonylprop-1-yl)-N-pentanoyl-N-[2'-(1H-tetrazol-5-yl)biphenyl-4-ylmethyl]amine,
(S)-N-(1-dimethylaminocarbonyl-2-methylprop-1-yl)-N-pentanoyl-N-[2'-(1H-tetrazol-5-yl)biphenyl-4-ylmethyl]amine,
(S)-N-(2-methyl-1-morpholin-4-ylcarbonylprop-1-yl)-N-pentanoyl-N-[2'-(1H-tetrazol-5-yl)biphenyl-4-ylmethyl]amine,
(S)-N-(2'-carboxybiphenyl-4-ylmethyl)-N-(1-carboxy-2-methylprop-1-yl)-N-pentanoyl-amine,
(S)-N-(1,2-dicarboxyethyl)-N-pentanoyl-N-[2'-(1H-tetrazol-5-yl)biphenyl-4-ylmethyl]-amine,
(S)-N-(1-carboxy-3-phenylprop-1-yl)-N-pentanoyl-N-[2'-(1H-tetrazol-5-yl)biphenyl-4-ylmethyl]amine,
(S)-N-(2-cyclohexyl-1-hydroxymethylethyl)-N-pentanoyl-N-[2'-(1H-tetrazol-5-yl)biphenyl-4-ylmethyl]amine,
(R)-N-(1-methoxycarbonyl-2-methylprop-1-yl)-N-pentanoyl-N-[2'-(1H-tetrazol-5-yl)biphenyl-4-ylmethyl]amine.
(S)-N-(2-hydroxy-1-methoxycarbonylethyl)-N-pentanoyl-N-[2'-(1H-tetrazol-5-yl)biphenyl-4-ylmethyl]amine,
N-pentanoyl-N-(1H-tetrazol-5-ylmethyl)-N-[2'-(1H-tetrazol-5-yl)biphenyl-4-ylmethyl]-amine,
N-pentanoyl-N-pyrid-3-ylmethyl-N-[2'-(1H-tetrazol-5-yl)biphenyl-4-ylmethyl]amine,
(S)-N-(1-carboxy-4-guanidinobut-1-yl)-N-pentanoyl-N-[2'-(1H-tetrazol-5-yl)biphenyl-4-ylmethyl]amine,
N-(2-hydroxy-1-methoxycarbonylprop-1-yl)-N-pentanoyl-N-[2'-(1H-tetrazol-5-yl)biphenyl-4-ylmethyl]amine,
N-(1-benzyloxycarbonyl-1-methylethyl)-N-butanoyl-N-[2'-(1H-tetrazol-5-yl)biphenyl-4-ylmethyl]amine,
(S)-N-(1-carboxy-3-methylbut-1-yl)-N-pentanoyl-N-[2'-(1H-tetrazol-5-yl)biphenyl-4-ylmethyl]amine,
N-(1-carboxy-2-hydroxyethyl)-N-pentanoyl-N-[2'-(1H-tetrazol-5-yl)biphenyl-4-yl methyl]amine,
(S)-N-(1-carboxy-2-hydroxyethyl)-N-pentanoyl-N-[2'-(1H-tetrazol-5-yl)biphenyl-4-ylmethyl]amine,
(S)-N-(2-methyl-1-(2-phenylethylaminocarbonyl)prop-1-yl]-N-pentanoyl-N-[2'-(1H-tetrazol-5-yl)biphenyl-4-ylmethyl]amine,
(S)-N-(2-benzyloxy-1-hydroxymethylethyl)-N-pentanoyl-N-[2'-(1H-tetrazol-5-yl)biphenyl-4-ylmethyl]amine,
(S)-N-(1-carboxy-2-methylprop-1-yl)-N-pentanoyl-N-[2'-(1H-tetrazol-5-yl)biphenyl-3-ylmethyl]amine,

(S)-N-(1-carboxy-2-methylprop-1-yl)-N-pentanoyl-N-[3'-(1H-tetrazol-5-yl)biphenyl-4-ylmethyl]amine,
(S)-N-[2-methyl-1-(1,2,3,4-tetrahydroquinol-1-ylcarbonyl)prop-1-yl]-N-pentanoyl-N-[2'-(1H-tetrazol-5-yl)biphenyl-4-ylmethyl]amine,
(S)-N-(2-methyl-1-piperidin-1-ylcarbonylprop-1-yl)-N-pentanoyl-N-[2'-(1H-tetrazol-5-yl)biphenyl-4-ylmethyl]amine,
(S)-N-[2-methyl-1-(1,2,3,4-tetrahydroisoquinol-2-ylcarbonyl)prop-1-yl]-N-pentanoyl-N-[2'-(1H-tetrazol-5-yl)biphenyl-4-ylmethyl]amine,
N-(2-hydroxymethyl-2-methylprop-1-yl)-N-pentanoyl-N-[2'-(1H-tetrazol-5-yl)biphenyl-4-ylmethyl]amine,
N-ethoxycarbonyl-N-(2-ethoxycarbonyl-2-methylprop-1-yl)-N-[2'-(1H-tetrazol-5-yl)biphenyl-4-ylmethyl]amine and
N-(2-carboxy-2-methylprop-1-yl)-N-ethoxycarbonyl-N-[2'-(1H-tetrazol-5-yl)biphenyl-4-ylmethyl]amine, in each case in free form or in salt form.

**33.** A compound according to any one of claims 1 to 32, in free form or in the form of a pharmaceutically utilizable salt, for use in a method for the therapeutic treatment of the human or animal body.

**34.** A compound according to any one of claims 1 to 33, in free form or in the form of a pharmaceutically, utilizable salt, for use as an antihypertensive.

**35.** A pharmaceutical preparation comprising as active compound a compound according to any one of claims 1 to 34, in free form or in the form of a pharmaceutically utilizable salt, if appropriate in addition to customary pharmaceutical excipients.

**36.** An antihypertensive pharmaceutical preparation according to claim 35, wherein an antihypertensive active ingredient is selected.

**37.** A process for the preparation of a compound of the formula

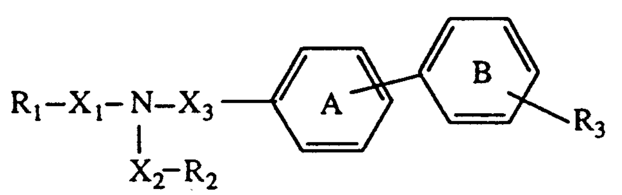

(I) or

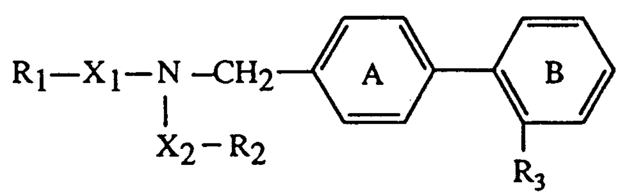

(Ia),

in which $R_1$, $R_2$, $R_3$, $X_1$, $X_2$ and $X_3$ and the substituents of the rings A and B are as defined in each case in any one of claims 1-25; in free form or in salt form, wherein

a) in a compound of the formula

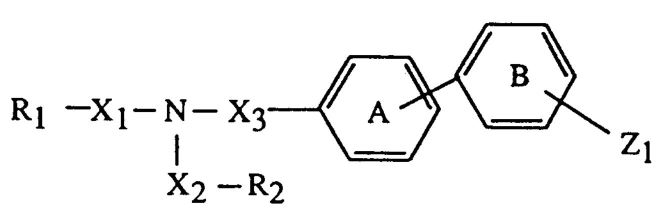

(II)

or a salt thereof in which $Z_1$ is a radical which can be converted into $R_3$, $Z_1$ is converted to $R_3$ or

b) a compound of the formula $R_1$-$X_1$OH (IIIa), a reactive derivative thereof or a salt thereof is reacted with a compound of the formula

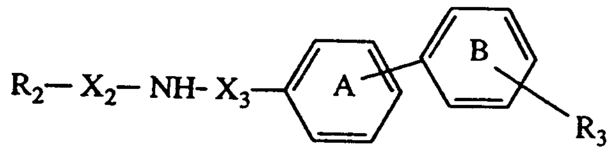

(IIIb)

or a salt thereof and in each case, if desired, a compound I in free form or in salt form obtainable according to the process or in another way is converted into another compound I, a mixture of isomers obtainable according to the process is separated and the desired isomer is isolated and/or a free compound I obtainable according to the process is converted into a salt or a salt of a compound I obtainable according to the process is converted into the free compound I or into another salt.

**38.** A process for the production of a pharmaceutical preparation according to claim 35 or 36, wherein the active ingredient, if appropriate with admixture of customary pharmaceutical excipients, is processed to give a pharmaceutical preparation.

**39.** A process according to claim 38 for the production of an antihypertensive pharmaceutical preparation according to claim 34, wherein an antihypertensive active ingredient is selected.

**40.** The use of a compound according to any one of claims 1 to 34, in free form or in the form of a pharmaceutically utilizable salt, for the production of a pharmaceutical preparation.

**41.** The use of a compound according to any one of claims 1 to 34, in free form or in the form of a pharmaceutically utilizable salt, for the production of a pharmaceutical preparation by a non-chemical route.

**42.** The use of a compound according to any one of claims 1 to 34, in free form or in the form of a pharmaceutically utilizable salt, for the production of an antihypertensive.

**43.** The use of a compound according to any one of claims 1 to 34, in free form or in the form of a pharmaceutically utilizable salt, for the production of a pharmaceutical preparation for the therapeutic or prophylactic treatment of cardiac insufficiency.

**44.** The use of a compound according to any one of claims 1 to 34, in free form or in the form of a pharmaceutically utilizable salt, for the production of a pharmaceutical preparation for the therapeutic or prophylactic treatment of disorders which are caused by angiotensin II activity.

**Claims for the following Contracting States : ES, GR**

**1.** A process for the preparation of a compound of the formula

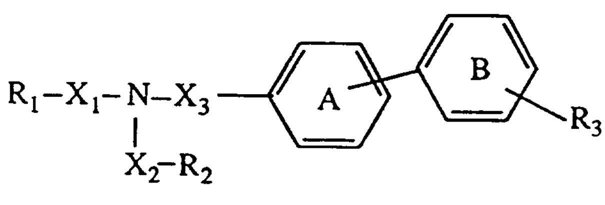

(I),

in which $R_1$ is lower alkyl, lower alkenyl or lower alkynyl or $C_3$-$C_7$cycloalkyl or $C_3$-$C_7$cycloalkenyl or phenyl-lower alkyl, phenyl-lower alkenyl or phenyl-lower alkynyl, each of which is unsubstituted or substituted by halogen or hydroxyl; $X_1$ is CO, $SO_2$ or -O-C(=O)-, the carbon atom of the carbonyl group being bonded to the nitrogen atom drawn in in the formula I; $X_2$ is $C_1$-$C_{10}$alkylene, $C_2$-$C_{10}$alkylidene or $C_3$-$C_7$cycloalkylene which is unsubstituted or substituted by hydroxyl, carboxyl, amino, guanidino, $C_3$-$C_7$cycloalkyl, $C_3$-$C_7$cycloalkenyl, phenyl or a corresponding 5- or 6-membered and monocyclic aromatic radical which has up to four identical or different heteroatoms, it being

possible for a carbon atom of $C_1$-$C_{10}$alkylene or $C_2$-$C_{10}$alkylidene additionally to be bridged by $C_2$-$C_6$alkylene, and $C_3$-$C_7$cycloalkyl or $C_3$-$C_7$cycloalkenyl being unsubstituted, monosubstituted or polysubstituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, formyl, di-lower alkoxymethyl or oxy-lower alkylenoxymethylene; $R_2$ is carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, amino, amino which is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or fused at two adjacent C atoms to a benzene ring, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, 1H-tetrazol-5-yl, pyridyl, hydroxyl, lower alkoxy, lower alkenyloxy, phenyl-lower alkoxy, phenoxy, $S(O)_m$-R, m being 0, 1 or 2 and R being hydrogen, lower alkyl, lower alkenyl or lower alkynyl, lower alkanoyl, sulfamoyl, sulfamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, or is $PO_nH_2$, n being 2 or 3; $X_3$ is $C_1$-$C_{10}$alkylene or $C_2$-$C_{10}$alkylidene; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B independently of one another being unsubstituted or substituted by substituents selected from the group consisting of: halogen, hydroxyl, lower alkoxy, lower alkenyloxy, phenyl-lower alkoxy, phenoxy, $S(O)_m$-R and lower alkyl, lower alkenyl or lower alkynyl, each of which is unsubstituted or substituted by halogen or hydroxyl, lower alkyl, lower alkenyl or lower alkynyl being uninterrupted or interrupted by -O-, and, in the case of (hetero)aromatic radicals, additionally being unsubstituted or substituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, by carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O- or being fused at two adjacent C atoms to a benzene ring, by formyl, di-lower alkoxymethyl or oxy-lower alkylenoxymethylene; radicals and groups designated by "lower" containing up to and including 7 carbon atoms; in free form or in salt form; wherein

a) in a compound of the formula

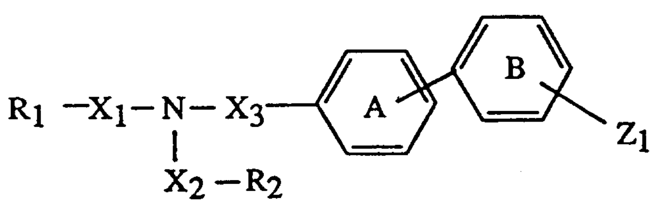

(II)

or a salt thereof in which $Z_1$ is a radical which can be converted into $R_3$, $Z_1$ is converted to $R_3$ or

b) a compound of the formula $R_1$-$X_1$OH (IIIa), a reactive derivative thereof or a salt thereof is reacted with a compound of the formula

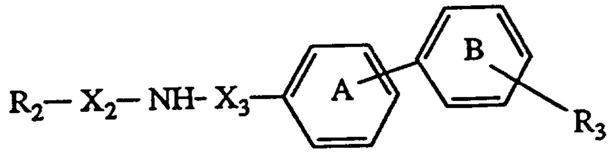

(IIIb)

or a salt thereof and in each case, if desired, a compound I in free form or in salt form obtainable according to the process or in another way is converted into another compound I, a mixture of isomers obtainable according to the process is separated and the desired isomer is isolated and/or a free compound I obtainable according to the process is converted into a salt or a salt of a compound I obtainable according to the process is converted into the free compound I or into another salt.

**2.** A process according to claim 1 for the preparation of a compound of the formula I, in which $R_2$ is other than carboxyl and $R_3$ is 5-tetrazolyl, wherein

(i) the starting compound used is a compound of the formula (II) in which $Z_1$ is cyano, and this is reacted with $HN_3$ or an alkali metal salt thereof, with a tri-lower alkyltin azide or triphenyltin azide; or
(ii) the starting compound used is a compound of the formula (II) in which $Z_1$ is 5-tetrazolyl protected by triphenylmethyl, benzyl which is unsubstituted or substituted by nitro, lower alkoxymethyl, lower alkylthiomethyl, tri-lower alkylsilyl, 2-cyanoethyl, lower alkoxy-lower alkoxymethyl, benzyloxymethyl or phenacyl, and the protective group is removed and,

if desired, a compound of the formula I in which $R_2$ is other than carboxyl and $R_3$ is 5-tetrazolyl obtainable according to the process is converted into a compound of the formula I in which $R_2$ is carboxyl.

**3.** A process according to claim 1 or 2 for the preparation of a compound of the formula I, in which $R_1$ is lower alkyl, lower alkenyl or lower alkynyl or $C_3$-$C_7$cycloalkyl or $C_3$-$C_7$cycloalkenyl or phenyl-lower alkyl, phenyl-lower alkenyl or phenyl-lower alkynyl, each of which is unsubstituted or substituted by halogen or hydroxyl; $X_1$ is CO or $SO_2$; $X_2$ is $C_1$-$C_{10}$alkylene or $C_2$-$C_{10}$alkylidene or $C_3$-$C_7$cycloalkylene which is unsubstituted or substituted by hydroxyl, $C_3$-$C_7$cycloalkyl or $C_3$-$C_7$cycloalkenyl or phenyl or a corresponding 5- or 6-membered and monocyclic aromatic radical which has up to 4 identical or different heteroatoms, it being possible for a carbon atom of $C_1$-$C_{10}$alkylene or $C_2$-$C_{10}$alkylidene additionally to be bridged by $C_2$-$C_6$alkylene, and $C_3$-$C_7$cycloalkylene being unsubstituted, monosubstituted or polysubstituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, formyl, di-lower alkoxymethyl or oxy-lower alkylenoxymethylene; $R_2$ is carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl lower alkenyl lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, amino, amino which is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, lower alkenyloxy, phenyl-lower alkoxy, phenoxy, $S(O)_m$-R, m being 0, 1 or 2 and R being hydrogen, lower alkyl, lower alkenyl or lower alkynyl, lower alkanoyl, sulfamoyl, sulfamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, or is $PO_nH_2$, n being 2 or 3; $X_3$ is $C_1$-$C_{10}$alkylene or $C_2$-$C_{10}$alkylidene; $R_3$ is carboxyl, 5-tetrazolyl, $SO_3H$, $PO_2H_2$, $PO_3H_2$ or halo-lower alkylsulfamoyl; (hetero)aromatic radicals including the rings A and B independently of one another being

unsubstituted or substituted by substituents selected from the group consisting of: halogen, hydroxyl, lower alkoxy, lower alkenyloxy, phenyl-lower alkoxy, phenoxy, $S(O)_m$-R and lower alkyl, lower alkenyl or lower alkynyl, each of which is unsubstituted or substituted by halogen or hydroxyl, lower alkyl, lower alkenyl or lower alkynyl being uninterrupted or interrupted by -O-, and, in the case of (hetero)aromatic radicals, additionally unsubstituted or substituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, by carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, by formyl, di-lower alkoxymethyl or oxy-lower alkylenoxymethylene; in free form or in salt form.

4. A process according to claim 1 or 2 for the preparation of a compound of the formula I, in which $R_1$ is lower alkyl, lower alkenyl or lower alkynyl or $C_3$-$C_7$cycloalkyl or $C_3$-$C_7$cycloalkenyl or phenyl-lower alkyl phenyl-lower alkenyl or phenyl-lower alkynyl, each of which is unsubstituted or substituted by halogen or hydroxyl; $X_1$ is CO or $SO_2$; $X_2$ is $C_1$-$C_{10}$alkylene or $C_2$-$C_{10}$alkylidene which is unsubstituted or substituted by hydroxyl, $C_3$-$C_7$cycloalkyl or $C_3$-$C_7$cycloalkenyl or phenyl or a corresponding 5- or 6-membered and monocyclic aromatic radical which has up to 4 identical or different heteroatoms; $R_2$ is carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, amino, amino which is monosubstituted or independently of one another disubstituted by lower alkyl, lower alkenyl, lower alkynyl, phenyl-lower alkynyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, lower alkenyloxy, phenyl-lower alkoxy or phenoxy, $S(O)_m$-R, m being 0, 1 or 2 and R being hydrogen, lower alkyl, lower alkenyl or lower alkynyl, lower alkanoyl, sulfamoyl, sulfamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_2$-$C_{10}$alkylidene, $C_1$-$C_{10}$alkylene or $C_2$-$C_{10}$alkylidene being uninterrupted or interrupted by -O-, or is $PO_nH_2$, n being 2 or 3; $X_3$ is $-CH_2-$; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B independently of one another being unsubstituted or substituted by substituents selected from the group consisting of: halogen, hydroxyl, lower alkoxy, lower alkenyloxy, phenyl-lower alkoxy, phenoxy, $S(O)_m$-R and lower alkyl, lower alkenyl or lower alkynyl, each of which is unsubstituted or substituted by halogen or hydroxyl, lower alkyl, lower alkenyl or lower alkynyl being uninterrupted or interrupted by -O-, and, in the case of (hetero)aromatic radicals, additionally unsubstituted or substituted by carboxyl, carboxyl which is esterified by an alcohol which is derived from lower alkyl, phenyl-lower alkyl, lower alkenyl, lower alkynyl, lower alkoxy-lower alkyl, lower alkoxy-lower alkenyl or lower alkoxy-lower alkynyl, by carbamoyl, carbamoyl in which the amino group is monosubstituted or independently of one another disubstituted by lower alkyl, lower alkenyl, lower alkynyl, phenyl-lower alkyl, phenyl-lower alkenyl or phenyl-lower alkynyl or is disubstituted by $C_1$-$C_{10}$alkylene or $C_1$-$C_{10}$alkylidene, $C_1$-$C_{10}$alkylene or $C_2$-$C_{10}$alkylidene being uninterrupted or interrupted by -O-, by formyl, di-lower alkoxymethyl or oxy-lower alkylenoxymethylene; in free form or in salt form.

5. A process according to claim 1 or 2 for the preparation of a compound of the formula I, in which $R_1$ is lower alkyl, lower alkenyl, lower alkynyl, halo-lower alkyl, -lower alkenyl or -lower alkynyl, hydroxy-lower alkyl, -lower alkenyl or -lower alkynyl, $C_3$-$C_7$cycloalkyl, $C_3$-$C_7$cycloalkenyl, phenyl-lower alkyl, phenyl-lower alkenyl or phenyl-lower alkynyl: $X_1$ is CO or $SO_2$; $X_2$ is $C_1$-$C_{10}$alkylene or $C_2$-$C_{10}$alkylidene, each of which is unsubstituted or substituted by hydroxyl a $C_3$-$C_7$cycloalkyl, $C_3$-$C_7$cycloalkenyl or a phenyl radical or a 5- or 6-membered, monocyclic heteroaromatic radical having up to four identical or different heteroatoms, the cyclic radicals for their part being unsubstituted or substituted by carboxyl which can be esterified with an alcohol which is derived from lower alkyl, phenyl-lower alkyl, lower alkenyl, lower alkynyl, lower alkoxy-lower alkyl, -lower alkenyl or -lower alkynyl, carbamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene or lower alkylenoxy-lower alkylene, formyl, di-lower alkoxymethyl or oxy-lower alkylenoxymethylene; $R_2$ is carboxyl which can be esterified with an alcohol which is derived from lower alkyl, phenyl-lower alkyl, lower alkenyl, lower alkynyl, or lower alkoxy-lower alkyl, -lower alkenyl or -lower alkynyl, carbamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower alkylenoxy-lower alkylene, amino in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower-alkylenoxy-lower alkylene, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, hydroxyl lower alkoxy, lower alkenyloxy, phenyl-lower alkoxy, phenoxy, $S(O)_m$-R, m being 0, 1 or 2 and R being hydrogen, lower alkyl, lower alkenyl or lower alkynyl, lower alkanoyl, sulfamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower alkylenoxy-lower alkylene, or is $PO_nH_2$, n being 2 or 3; $X_3$ is $-CH_2-$; and $R_3$ is carboxyl, 5-tetrazolyl, $SO_3H$, $PO_2H_2$, $PO_3H_2$ or halo-lower alkylsulfamoyl; (hetero)aromatic radicals including the rings A and B independently of one another each being unsubstituted or substituted by one or more substituents selected from halogen, hydroxyl lower alkoxy, lower alkenyloxy, or lower alkyl, lower alkenyl, lower alkynyl, lower alkoxy-lower alkyl, -lower alkenyl or -lower alkynyl, lower alkenyloxy-lower alkyl, -lower alkenyl and -lower alkynyl, each of which is unsubstituted or substituted by halogen or hydroxyl, in free form or in salt form.

6. A process according to claim 1 or 2 for the preparation of a compound of the formula I, in which $X_2$ is $C_1$-$C_{10}$alkylene or $C_2$-$C_{10}$alkylidene, each of which is unsubstituted or substituted by hydroxyl, a $C_3$-$C_7$cycloalkyl, $C_3$-$C_7$cycloalkenyl or a phenyl radical or a 5-or 6-membered, monocyclic heteroaromatic radical having up to four identical or different heteroatoms, it being possible for a C atom of $C_1$-$C_{10}$alkylene or $C_2$-$C_{10}$alkylidene to be bridged by $C_2$-$C_6$alkylene and the cyclic radicals for their part being unsubstituted or substituted by carboxyl which can be esterified with an alcohol which is derived from lower alkyl, phenyl-lower alkyl, lower alkenyl, lower alkynyl, or lower alkoxy, -lower alkyl-lower alkenyl or -lower alkynyl, carbamoyl in which the amino group is unsubstituted, monosubstituted or independently of one another disubstituted by lower alkyl, lower alkenyl, lower alkynyl, phenyl-lower alkyl, phenyl-lower alkenyl or phenyl-lower alkynyl or is disubstituted by lower alkylene- or lower alkylenoxy-lower alkylene, formyl, di-lower alkoxymethyl or by oxy-lower alkylenoxymethylene, or $X_2$ is $C_3$-$C_7$cycloalkylene; $X_3$ is lower alkylene or lower alkylidene; the variables $X_1$, $R_1$, $R_2$ and $R_3$ are as defined in claim 5; and the (hetero)aromatic rings including the rings A and B can be substituted as indicated in claim 5, in free form or in salt form.

7. A process according to claim 1 or 2 for the preparation of a compound of the formula I, in which $R_1$ is lower alkyl, lower alkenyl, halo-lower alkyl or -lower alkenyl, hydroxy-lower alkyl, 3- to 7-membered cycloalkyl or phenyl-lower alkyl; $X_1$ is CO, $SO_2$ or
-O-C(=O)-, the carbon atom of the carbonyl group being bonded to the nitrogen atom drawn in in the formula I; $X_2$ is $C_1$-$C_{10}$alkylene or $C_1$-$C_7$alkylidene, each of which is unsubstituted or substituted by hydroxyl, carboxyl, amino, guanidino, a 3- to 7-membered cycloalkyl, 3- to 7-membered cycloalkenyl, phenyl, pyrrolyl, pyrazolyl, imidazolyl, triazolyl, tetrazolyl, furyl, thienyl or pyridyl radical which for its part can additionally be unsubstituted or substituted by carboxyl, lower alkoxycarbonyl, phenyl-lower alkoxycarbonyl, carbamoyl in which the amino group is unsubstituted, monosubstituted or independently of one another disubstituted by lower alkyl or phenyl-lower alkyl, formyl, di-lower alkoxymethyl or oxy-lower alkylenoxymethylene; $R_2$ is carboxyl, lower alkoxy-, phenyl-lower alkoxy-, lower alkenyloxy- or lower alkoxy-lower alkoxy-carbonyl, carbamoyl in which the amino group is unsubstituted, monosubstituted or independently of one another disubstituted by lower alkyl or phenyl-lower alkyl or is disubstituted by lower alkylene which can be fused at two adjacent carbon atoms to a benzene ring, or lower alkylenoxy-lower alkylene, amino in which the amino group is unsubstituted, monosubstituted or independently of one another disubstituted by lower alkyl or phenyl-lower alkyl or is disubstituted by lower alkylene- or lower alkylenoxy-lower alkylene, lower alkanoyl-, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, phenyl-lower alkoxy, phenoxy, $S(O)_m$-R, m being 0, 1 or 2 and R being lower alkyl, lower alkanoyl, sulfamoyl in which the amino group is unsubstituted, monosubstituted or independently of one another disubstituted by lower alkyl or phenyl-lower alkyl, or is $PO_nH_2$, n being 2 or 3; $X_3$ is methylene; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B are each additionally unsubstituted or substituted by one or more substituents selected from halogen, hydroxyl, lower alkoxy, or lower alkyl or lower alkoxy-lower alkyl, each of which is unsubstituted or substituted by halogen or hydroxyl, in free form or in salt form.

8. A process according to claim 1 or 2 for the preparation of a compound of the formula I, in which $R_1$ is lower alkyl, lower alkenyl, halo-lower alkyl or -lower alkenyl, hydroxy-lower alkyl, 3- to 7-membered cycloalkyl or phenyl-lower alkyl; $X_1$ is CO or $SO_2$; $X_2$ is $C_1$-$C_{10}$alkylene or $C_1$-$C_7$alkylidene, each of which is unsubstituted or substituted by hydroxyl, a 3- to 7-membered cycloalkyl, 3- to 7-membered cycloalkenyl, phenyl, pyrrolyl, pyrazolyl, imidazolyl, triazolyl, tetrazolyl, furyl, thienyl or pyridyl radical which for its part can additionally be unsubstituted or substituted by carboxyl, lower alkoxycarbonyl, phenyl-lower alkoxycarbonyl, carbamoyl in which the amino group is unsubstituted, monosubstituted or independently of one another disubstituted by lower alkyl or phenyl-lower alkyl, formyl, di-lower

alkoxymethyl or oxy-lower alkylenoxymethylene; $R_2$ is carboxyl, lower alkoxy-, phenyl-lower alkoxy-, lower alkenyloxy- or lower alkoxy-lower alkoxy-carbonyl, carbamoyl in which the amino group is unsubstituted, monosubstituted or independently of one another disubstituted by lower alkyl or phenyl-lower alkyl or is disubstituted by lower alkylene- or lower alkylenoxy-lower alkylene, amino in which the amino group is unsubstituted, monosubstituted or independently of one another disubstituted by lower alkyl or phenyl-lower alkyl or is disubstituted by lower alkylene- or lower alkylenoxy-lower alkylene, lower alkanoyl, phenyl-lower alkanoyl-, benzoyl-, lower alkanesulfonyl- or benzenesulfonyl-amino, formyl, di-lower alkoxymethyl, oxy-lower alkylenoxymethylene, hydroxyl, lower alkoxy, phenyl-lower alkoxy, phenoxy, $S(O)_m$-R, m being 0, 1 or 2 and R being lower alkyl, lower alkanoyl, sulfamoyl in which the amino group is unsubstituted, monosubstituted or independently of one another disubstituted by lower alkyl or phenyl-lower alkyl, or is $PO_nH_2$, n being 2 or 3; $X_3$ is methylene; $R_3$ is carboxyl, 5-tetrazolyl, $SO_3H$, $PO_2H_2$, $PO_3H_2$ or halo-lower alkylsulfamoyl; and (hetero)aromatic radicals including the rings A and B are in each case additionally unsubstituted or substituted by one or more substituents selected from halogen, hydroxyl, lower alkoxy, or lower alkyl or lower alkoxy-lower alkyl, each of which is unsubstituted or substituted by halogen or hydroxyl, in free form or in salt form.

**9.** A process according to claim 1 or 2 for the preparation of a compound of the formula I, in which $X_2$ is $C_1$-$C_{10}$alkylene or $C_1$-$C_7$alkylidene, each of which is unsubstituted or substituted by hydroxyl, a 3- to 7-membered cycloalkyl, 3- to 7-membered cycloalkenyl, phenyl, pyrrolyl, pyrazolyl, imidazolyl, triazolyl, tetrazolyl, furyl, thienyl or pyridyl radical which for its part can additionally be substituted by carboxyl, lower alkoxycarbonyl, phenyl-lower alkoxycarbonyl, carbamoyl in which the amino group is unsubstituted, monosubstituted or independently of one another disubstituted by lower alkyl or phenyl-lower alkyl, formyl, di-lower alkoxymethyl or by oxy-lower alkylenoxymethylene, it being possible for a C atom of $C_1$-$C_{10}$alkylene or $C_1$-$C_7$alkylidene to be bridged by $C_2$-$C_6$alkylene, or $X_2$ is $C_3$-$C_7$cycloalkylene; $X_3$ is lower alkylene or lower alkylidene; the variables $X_1$, $R_1$, $R_2$ and $R_3$ are as defined in claim 8; and the (hetero)aromatic rings including the rings A and B can be substituted as indicated in claim 8, in free form or in salt form.

**10.** A process according to claim 1 or 2 for the preparation of a compound of the formula I, in which the variables $R_1$,$X_1$ and $R_3$ are as defined in each case in any one of claims 1-8; $X_2$ is lower alkylene or lower alkylidene, each of which is unsubstituted or substituted by hydroxyl, 3- to 7-membered cycloalkyl, phenyl or imidazolyl and $R_2$ is carboxyl, lower alkoxy-, phenyl-lower alkoxy- or lower alkoxy-lower alkoxy-carbonyl, carbamoyl which is unsubstituted, monosubstituted or independently of one another disubstituted by lower alkyl or phenyl-lower alkyl, amino, lower alkanoyl-, phenyl-lower alkanoyl- or lower alkanesulfonylamino, hydroxyl, lower alkoxy, phenyl-lower alkoxy or phenoxy; $X_3$ is $-CH_2-$; (hetero)aromatic radicals including the rings A and B each being unsubstituted or substituted by one or more substituents selected from halogen, trifluoromethyl, hydroxyl, lower alkoxy, lower alkyl, hydroxy-lower alkyl or lower alkoxy-lower alkyl, in free form or in salt form.

**11.** A process according to claim 1 or 2 for the preparation of a compound of the formula I, in which $X_2$ is lower alkylene or lower alkylidene which is unsubstituted or substituted by hydroxyl, 3- to 7-membered cycloalkyl, 7-membered cycloalkenyl, phenyl or imidazolyl, where a C atom of lower alkylene or lower alkylidene can be bridged by $C_2$-$C_6$alkylene, or $X_2$ is $C_3$-$C_7$cycloalkylene; the variables $X_1$, $X_3$, $R_1$, $R_2$ and $R_3$ are as defined in claim 8 to 10; and the rings A and B can be substituted as indicated in claim 10, in free form or in salt form.

**12.** A process according to claim 1 or 2 for the preparation of a compound of the formula

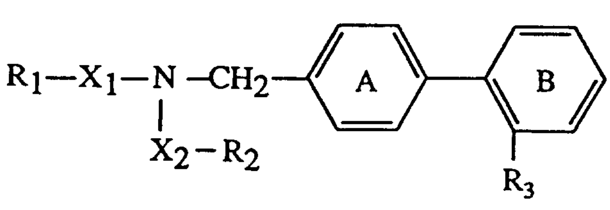

(Ia),

in which the variables $R_1$, $X_1$, $X_2$, $R_2$ and $R_3$ are as defined in each case in any one of claims 1 or 3-11 and the rings A and B can be substituted as indicated in claim 11, in free form or in salt form.

**13.** A process according to claim 1 or 2 for the preparation of a compound of the formula Ia, in which $X_2$ is lower alkylene or lower alkylidene, which is unsubstituted or substituted by hydroxyl or 3- to 7-membered cycloalkyl, it being possible for a C atom of lower alkylene or lower alkylidene to be bridged by $C_2$-$C_6$alkylene, or in which $X_2$ is $C_3$-

$C_7$cycloalkylene; the variables $R_1$,$X_1$, $R_2$ and $R_3$ are as defined in each case in any one of claims 1 or 3-11; and the rings A and B can be substituted as indicated in claim 11, in free form or in salt form.

**14.** A process according to claim 1 or 2 for the preparation of a compound of the formula Ia, in which $X_2$ is the group of the formula

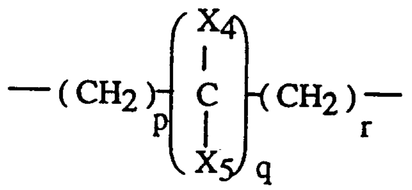

(Ib)

in which p is 0 or 1, q is 1 and r is 0 or 1 or in which p is 1 to 8 and q and r are in each case 0; $X_4$ is lower alkyl or phenyl which is unsubstituted or substituted by hydroxyl, 3- to 7-membered cycloalkyl, phenyl or imidazolyl; and $X_5$ is hydrogen or lower alkyl; $R_2$ is carboxyl, lower alkoxycarbonyl, phenyl-lower alkoxycarbonyl, lower alkoxy-lower alkoxycarbonyl, hydroxyl, lower alkoxy, phenyl-lower alkoxy, phenoxy, amino, lower alkanoylamino, phenyl-lower alkanoylamino or lower alkanesulfonylamino; and the variables $R_1$, $X_1$ and $R_3$ are as defined in each case in any one of claims 1 or 3-8; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**15.** A process according to claim 1 or 2 for the preparation of a compound of the formula Ia, in which $X_2$ is the group of the formula Ib in which p is 0 or 1, q is 1 and r is 0 or 1 or in which p is 1 to 8 and q and r are each 0; $X_4$ is lower alkyl or phenyl, which is unsubstituted or substituted by hydroxyl, 3- to 7-membered cycloalkyl, phenyl or imidazolyl; and $X_5$ is hydrogen or lower alkyl; or $X_4$ and $X_5$ together are $C_2$-$C_6$alkylene, such as $C_4$-$C_5$alkylene; or $X_2$ is $C_3$-$C_7$cycloalkylene such as $C_5$-$C_6$cycloalkylene; $R_2$ is carboxyl, lower alkoxycarbonyl, phenyl-lower alkoxycarbonyl, lower alkoxy-lower alkoxycarbonyl, hydroxyl, lower alkoxy, phenyl-lower alkoxy, phenoxy, amino, lower alkanoylamino, phenyl-lower alkanoylamino or lower alkanesulfonylamino; and the variables $R_1$, $X_1$ and $R_3$ are as defined in each case in any one of claims 1 or 3-8; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**16.** A process according to claim 1 or 2 for the preparation of a compound of the formula Ia, in which $R_1$ is lower alkyl, such as $C_3$-$C_5$alkyl, or lower alkenyl, such as $C_3$-$C_5$alkenyl; $X_1$ is CO or else $SO_2$; $X_2$ is the group of the formula Ib in which p and r are 0 or 1 and q is 1; $X_4$ is lower alkyl, such as $C_1$-$C_4$alkyl, or phenyl, which is unsubstituted or substituted by hydroxyl, 3- to 7-membered cycloalkyl, such as cyclohexyl, or by phenyl or imidazolyl which is unsubstituted or substituted by halogen or hydroxyl, such as 4-imidazolyl; and $X_5$ is hydrogen or lower alkyl, such as $C_1$-$C_4$alkyl; or $X_4$ and $X_5$ together are $C_2$-$C_6$alkylene, such as $C_4$-$C_5$alkylene; or $X_2$ is $C_3$-$C_7$cycloalkylene, such as $C_5$-$C_6$cycloalkylene; $R_2$ is carboxyl, lower alkoxycarbonyl, such as $C_2$-$C_5$alkoxycarbonyl, phenyl-lower alkoxycarbonyl, such as phenyl-$C_1$-$C_4$alkoxycarbonyl, lower alkoxy-lower alkoxycarbonyl, such as $C_1$-$C_4$alkoxy-$C_2$-$C_5$alkoxycarbonyl, hydroxyl or lower alkoxy, such as $C_1$-$C_4$alkoxy; and $R_3$ is carboxyl or 5-tetrazolyl; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**17.** A process according to claim 1 or 2 for the preparation of a compound of the formula Ia, in which $R_1$ is lower alkyl, such as $C_3$-$C_5$alkyl, or lower alkenyl, such as $C_3$-$C_5$alkenyl; $X_1$ is CO or else $SO_2$; $X_2$ is the group of the formula Ib in which p and r are 0 or 1 and q is 1; $X_4$ is lower alkyl, such as $C_1$-$C_4$alkyl, or phenyl, which is unsubstituted or substituted by hydroxyl, 3- to 7-membered cycloalkyl, or by phenyl or imidazolyl which is unsubstituted or substituted by halogen or hydroxyl, such as 4-imidazolyl; and $X_5$ is hydrogen or lower alkyl, such as $C_1$-$C_4$alkyl; $R_2$ is carboxyl, lower alkoxycarbonyl, such as $C_2$-$C_5$alkoxycarbonyl, phenyl-lower alkoxycarbonyl, such as phenyl-$C_1$-$C_4$alkoxycarbonyl, lower alkoxy-lower alkoxycarbonyl, such as $C_1$-$C_4$alkoxy-$C_2$-$C_5$alkoxycarbonyl, hydroxyl or lower alkoxy, such as $C_1$-$C_4$alkoxy; and $R_3$ is carboxyl or 5-tetrazolyl; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**18.** A process according to claim 1 or 2 for the preparation of a compound of the formula Ia, in which $R_1$ is lower alkyl, such as $C_3$-$C_5$alkyl, or else lower alkenyl, such as $C_3$-$C_5$alkenyl; $X_1$ is CO or else $SO_2$; $X_2$ is the group of the formula Ib in which p is an integer from 1 to 8 and q and r are 0; $R_2$ is hydroxyl, lower alkoxy, such as $C_1$-$C_4$alkoxy, phenyl-

lower alkoxy, such as phenyl-$C_1$-$C_4$alkoxy, phenoxy, lower alkanoylamino, such as $C_1$-$C_4$alkanoylamino, phenyl-lower alkanoylamino, such as phenyl-$C_1$-$C_4$alkanoylamino, or lower alkanesulfonylamino, such as $C_1$-$C_4$alkanesulfonylamino; and $R_3$ is carboxyl or primarily 5-tetrazolyl; (hetero)aromatic radicals including the rings A and B each being unsubstituted or substituted by halogen, trifluoromethyl, hydroxyl, lower alkoxy, lower alkyl or hydroxy-lower alkyl, in free form or in salt form.

**19.** A process according to claim 1 or 2 for the preparation of a compound of the formula Ia, in which $R_1$ is $C_3$-$C_5$alkyl or secondarily $C_3$-$C_5$alkenyl; $X_1$ is CO, also $SO_2$; $X_2$ is the group of the formula Ib in which p and r independently of one another are 0 or 1 and q is 1; $X_4$ is $C_1$-$C_4$alkyl, hydroxy-$C_1$-$C_4$alkyl, $C_3$-$C_7$cycloalkyl-$C_1$-$C_4$alkyl, phenyl-$C_1$-$C_4$alkyl or imidazolyl-$C_1$-$C_4$alkyl; and $X_5$ is hydrogen or $C_1$-$C_4$alkyl; or $X_4$ and $X_5$ together are tetramethylene, and also pentamethylene; $R_2$ is carboxyl or $C_2$-$C_5$alkoxycarbonyl, and also phenyl-$C_1$-$C_4$alkoxycarbonyl; and $R_3$ is carboxyl or 5-tetrazolyl, in free form or in salt form.

**20.** A process according to claim 1 or 2 for the preparation of a compound of the formula Ia, in which $R_1$ is $C_3$-$C_5$alkyl or secondarily $C_3$-$C_5$alkenyl; $X_1$ is CO, and also $SO_2$; $X_2$ is the group of the formula Ib in which p and r are each 0 or 1 and q is 1; $X_4$ is $C_1$-$C_4$alkyl, hydroxy-$C_1$-$C_4$alkyl, $C_3$-$C_7$cycloalkyl-$C_1$-$C_4$alkyl, phenyl-$C_1$-$C_4$alkyl or imidazolyl-$C_1$-$C_4$alkyl; and $X_5$ is hydrogen; $R_2$ is carboxyl or $C_2$-$C_5$alkoxycarbonyl, and also phenyl-$C_1$-$C_4$alkoxycarbonyl; and $R_3$ is carboxyl or 5-tetrazolyl, in free form or in salt form.

**21.** A process according to claim 1 or 2 for the preparation of a compound of the formula Ia, in which $R_1$ is $C_3$-$C_5$alkyl; $X_1$ is CO; $X_2$ is the group of the formula Ib in which q and r are 0 and p is 1 to 3 or in which p and q are 1 and r is 0; $X_4$ is $C_1$-$C_4$alkyl; $X_5$ is hydrogen or $C_1$-$C_4$alkyl; $R_2$ is carboxyl or $C_2$-$C_5$alkoxycarbonyl; and $R_3$ is carboxyl or 5-tetrazolyl, in free form or in salt form.

**22.** A process according to claim 1 or 2 for the preparation of a compound of the formula Ia, in which $R_3$ is 5-tetrazolyl, in free form or in salt form.

**23.** A process according to claim 1 or 2 for the preparation of a compound of the formula Ia, in which $R_1$ is $C_3$-$C_5$alkyl; $X_1$ is CO; $X_2$ is the group of the formula Ib in which p is 0 or 1, r is 0 and q is 1; $X_4$ is $C_1$-$C_4$alkyl; and $X_5$ is hydrogen or $C_1$-$C_4$alkyl; or $X_4$ and $X_5$ together are tetramethylene or pentamethylene; $R_2$ is carboxyl or $C_2$-$C_5$alkoxycarbonyl; and $R_3$ is 5-tetrazolyl, in free form or in salt form.

**24.** A process according to claim 1 or 2 for the preparation of a compound of the formula Ia, in which $R_1$ is $C_3$-$C_5$alkyl; $X_1$ is CO; $X_2$ is the group of the formula Ib in which p is 0 or 1, r is 0 and q is 1; $X_4$ and $X_5$ together are tetramethylene, and also pentamethylene; $R_2$ is carboxyl or $C_2$-$C_5$alkoxycarbonyl; and $R_3$ is 5-tetrazolyl, in free form or in salt form.

**25.** A process according to claim 1 or 2 for the preparation of a compound of the formula Ia, in which $R_1$ is $C_3$-$C_5$alkyl; $X_1$ is CO; $X_2$ is the group of the formula Ib in which p and r are 0 or 1 and q is 1; $X_4$ is $C_1$-$C_4$alkyl; and $X_5$ is hydrogen; $R_2$ is carboxyl or $C_2$-$C_5$alkoxycarbonyl; and $R_3$ is 5-tetrazolyl, in free form or in salt form.

**26.** A process according to claim 1 or 2 for the preparation of a compound of the formula Ia according to any one of claims 14-25, in which $X_2$ is the group of the formula Ib, q is 1 and $X_4$ and $X_5$ are defined differently, in free form or in salt form, in which the asymmetric C atom in question containing the variables $X_4$ and $X_5$ has the S configuration.

**27.** A process according to claim 1 or 2 for the preparation of (S)-N-(1-carboxy-2-methylprop-1-yl)-N-pentanoyl-N-[2'-(1H-te trazol-5-yl)biphenyl-4-yl-methyl]amine, in free form or in salt form according to claim 1.

**28.** A process according to claim 1 or 2 for the preparation of N-(2-carboxy-2,2-tetramethyleneethyl)-N-pentanoyl-N-[2'-(1H-tetrazol-5-yl)biphenyl-4-ylmethyl]amine, in free form or in salt form according to claim 1.

**29.** A process according to claim 1 or 2 for the preparation of N-(2-carboxy-2-ethylbut-1-yl)-N-pentanoyl-N-[2'-(1H-tetrazol-5-yl)biphenyl-4-ylmethyl]amine, in free form or in salt form according to claim 1.

**30.** A processs according to claim 1 or 2 for the preparation of (S)-N-(1-carboxy-2-methylprop-1-yl)-N-ethoxycarbonyl-N-[2'-(1H-tetrazol-5-yl)biphenyl-4-ylmethyl]amine, in free form or in salt form according to claim 1.

**31.** A process according to claim 1 or 2 for the preparation of N-(1-carboxycyclohexyl)-N-pentanoyl-N-[2'-(1H-tetrazol-5-yl)biphenyl-4-ylmethyl]-amine, in free form or in salt form according to claim 1.

32. A process according to claim 1 or 2 for the preparation of a compound according to claim 1 selected from the group consisting of:
(S)-N-(1-carboxyethyl)-N-pentanoyl-N-[2'-(1H-tetrazol-5-yl)biphenyl-4-ylmethyl]amine,
N-(2-hydroxyethyl)-N-pentanoyl-N-[2'-(1H-tetrazol-5-yl)biphenyl-4-ylmethyl]amine,
N-(2-ethoxycarbonyl-2,2-tetramethyleneethyl)-N-pentanoyl-N-[2'-(1H-tetrazol-5-yl)biphenyl-4-ylmethyl]amine,
N-(2-ethoxycarbonyl-2-ethylbut-1-yl)-N-pentanoyl-N-[2'-(1H-tetrazol-5-yl)biphenyl-4-ylmethyl]amine,
N(2-ethoxycarbonyl)-2-methylprop-1-yl)-N-pentamoyl-N-[2'-(1H-tetrazol-5-yl)-biphenyl-4-ylmethyl]amine,
(S)-N-(1-hydroxymethyl-2-methylprop-1-yl)-N-pentanoyl-N-[2'-1H-tetrazol-5-yl)biphenyl-4-ylmethyl]amine,
N-(2-ethoxycarbonyl-2,2-pentamethyleneethyl)-N-pentanoyl-N-[2'-(1H-tetrazol-5-yl)biphenyl-4-ylmethyl]amine,
(S)-N-(1-carboxy-2-methylprop-1-yl)-N-propyloxycarbonyl-N-[2'-1H-tetrazol-5-yl)biphenyl-4-ylmethyl]amine,
N-(2-carboxy-2-methylpropyl)-N-pentanoyl-N-[2'-(1H-tetrazol-5-yl)biphenyl-4-ylmethyl]amine,
N-(2-carboxy-2,2-pentamethyleneethyl)-N-pentanoyl-N-[2'-(1H-tetrazol-5-yl)biphenyl-4-ylmethyl]amine,
(S)-N-(1-aminocarbonyl-2-methylprop-1-yl)-N-pentanoyl-N-[2'-(1H-tetrazol-5-yl)biphenyl-4-ylmethyl]amine and
(S)-N-(1-carboxy-2-methylprop-1-yl)-N-(5-oxopent-1-en-5-yl)-N-[2'-(1H-tetrazol-5-yl)biphenyl-4-ylmethyl]amine, in each case in free form or in salt form.

33. A process according to claim 1 or 2 for the preparation of a compound according to claim 1 selected from the group consisting of:
N-carboxymethyl-N-pentanoyl-N-[2'-(1H-tetrazol-5-yl)biphenyl-4-ylmethyl]amine,
(S)-N-(1-methoxycarbonylethyl)-N-pentanoyl-N-[2'-(1H-tetrazol-5-yl)biphenyl-4-ylmethyl]amine,
N-[1-carboxy-2-(4-fluorophenyl)ethyl]-N-pentanoyl-N-[2'-(1H-tetrazol-5-yl)biphenyl-4-ylmethyl]amine,
N-[2-(4-fluorophenyl)-1-methoxycarbonylethyl]-N-pentanoyl-N-[2'-(1H-tetrazol-5-yl)biphenyl-4-ylmethyl]amine,
N-[2-(4-fluorophenyl)-1-hydroxymethylethyl]-N-pentanoyl-N-[2'-(1H-tetrazol-5-yl)biphenyl-4-ylmethyl]amine,
N-(2'-carboxybiphenyl-4-ylmethyl)-N-[1-carboxy-2-(4-fluorop henyl)ethyl]-N-pentanyol-amine,
N-(2'-carboxybiphenyl-4-ylmethyl)-N-[2-(4-fluorophenyl)-1-methoxycarbonyethyl]-N-pentanoylamine,
(S)-N-(2'-carboxybiphenyl-4-ylmethyl)-N-(1-hydroxymethyl-2-phenylethyl)-N-pentanoylamine,
(S)-N-(2'-carboxybiphenyl-4-ylmethyl)-N-(1-hydroxymethyl-2-imidazol-4-ylethyl)N-pentanoylamine,
(R)-N-(1-carboxyethyl)-N-pentanoyl-N-[2'-(1H-tetrazol-5-yl)biphenyl-4-ylmethyl]amine,
(1S),(2S)-N-(1-carboxy-2-methylbut-1-yl)-N-pentanoyl-N-[2'-(1H-tetrazol-5-yl)biphenyl-4-ylmethyl]amine,
(1S),(2S)-N-(1-methoxycarbonyl-2-methylbut-1-yl)-N-pentanoyl-N-[2'-(1H-tetrazol-5-yl)biphenyl-4-ylmethyl]amine,
(S)-N-(1-carboxybut-1-yl)-N-pentanoyl-N-[2'-(1H-tetrazol-5-yl)biphenyl-4-ylmethyl]-amine,
(S)-N-(1-methoxycarbonylbut-1-yl)-N-pentanoyl-N-[2'-(1H-tetrazol-5-yl)biphenyl-4-ylmethyl]amine,
(S)-N-(1-carboxyethyl-N-hexanoyl-N-[2'-(1H-tetrazol-5-yl)biphenyl-4-ylmethyl]amine,
(S)-N-butanoyl-N-(1-carboxyethyl)-N-[2'-(1H-tetrazol-5-yl)biphenyl-4-ylmethyl]amine,
(S)-N-(1-carboxyprop-1-yl)-N-pentanoyl-N-[2'-(1H-tetrazol-5-yl)biphenyl-4-ylmethyl]-amine,
(S)-N-(1-carboxy-2-cyclohexylethyl)-N-pentanoyl-N-[2'-(1H-tetrazol-5-yl)biphenyl-4-ylmethyl]amine,
(S)-N-(2-cyclohexyl-1-methoxycarbonylethyl)-N-pentanoyl-N-[2'-(1H-tetrazol-5-yl)biphenyl-4-ylmethyl]amine,
(R)-N-(1-carboxy-2-methylprop-1-yl)-N-pentanoyl-N-[2'-(1H-tetrazol-5-yl)biphenyl-4-ylmethyl]amine,
N-(2-methoxyethyl-N-pentanoyl-N-[2'-(1H-tetrazol-5-yl)biphenyl-4-ylmethyl]amine,
N-(2-benzyloxyethyl)-N-pentanoyl-N-[2'-(1H-tetrazol-5-yl)biphenyl-4-ylmethyl]amine,
N-(3-methoxyprop-1-yl)-N-pentanoyl-N-[2'-(1H-tetrazol-5-yl)biphenyl-4-ylmethyl]-amine,
N-(3-benzyloxyprop-1-yl)-N-pentanoyl-N-[2'-1H-tetrazol-5-yl)biphenyl-4-ylmethyl]-amine,
N-(3-hydroxyprop-1-yl)-N-pentanoyl-N-[2'-(1H-tetrazol-5-yl)biphenyl-4-ylmethyl]-amine,
N-(1-methoxycarbonyl-1-methylethyl)-N-pentanoyl-N-[2'-(1H-tetrazol-5-yl)biphenyl-4-ylmethyl]amine,
N-(2-carboxyethyl)-N-pentanoyl-N-[2'-(1H-tetrazol-5-yl)biphenyl-4-ylmethyl]amine,
N-(2-carboxyprop-1-yl)-N-pentanoyl-N-[2'-(1H-tetrazol-5-yl)biphenyl-4-ylmethyl]-amine,
N-(1-carboxy-1-methylethyl)-N-pentanoyl-N-[2'-(1H-tetrazol-5-yl)biphenyl-4-ylmethyl]amine,
N-(5-hydroxypent-1-yl)-N-pentanoyl-N-[2'-(1H-tetrazol-5-yl)biphenyl-4-ylmethyl]amine,
N-(1-carboxyprop-2-yl)-N-pentanoyl-N-[2'-(1H-tetrazol-5-yl)biphenyl-4-ylmethyl]amine,
N-(2-ethoxycarbonyl-3-methylbut-1-yl)-N-pentanoyl-N-[2'-(1H-tetrazol-5-yl)biphenyl-4-ylmethyl]amine,
N-(2-carboxy-3-methylbut-1-yl)-N-pentanoyl-N-[2'-(1H-tetrazol-5-yl)biphenyl-4-ylmethyl]amine,
N[(3-phenoxyprop-1-yl)-N-pentanoyl-N-[2'-(1H-tetrazol-5-yl)biphenyl-4-ylmethyl]amine,
N-[2-(4-hydroxyphenyl)ethyl]-N-pentanoyl-N-[2'-(1H-tetrazol-5-yl)biphenyl-4-ylmethyl]amine,
N-[3-(4-hydroxyphenyl)prop-1-yl]-N-pentanoyl-N-[2'-(1H-tetrazol-5-yl)biphenyl-4-ylmethyl]amine,
N-(8-hydroxyoct-1-yl)-N-pentanoyl-N-[2'-(1H-tetrazol-5-yl)biphenyl-4-ylmethyl]amine,
N-(2-methanesulfonylaminoethyl)-N-pentanoyl-N-[2'-(1H-tetrazol-5-yl)biphenyl-4-ylmethyl]amine,
N-(3-acetylaminoprop-1-yl)-N-pentanoyl-N-[2'-(1H-tetrazol-5-yl)biphenyl-4-ylmethyl]-amine,
N-(2-methoxy-2-oxo-1-phenylethyl)-N-pentanoyl-N-[2'-(1H-tetrazol-5-yl)biphenyl-4-ylmethyl]amine,
N-(4-hydroxybut-2-yl)-N-pentanoyl-N-[2'-(1H-tetrazol-5-yl)biphenyl-4-ylmethyl]amine,

N-(2-hydroxyl-1-phenylethyl)-N-pentanoyl-N-[2'-(1H-tetrazol-5-yl)biphenyl-4-ylmethyl]amine,
N-[3-(4-hydroxybenzylcarbonylamino)prop-1-yl]-N-pentanoyl-N-[2'-(1H-tetrazol-5-yl)biphenyl-4-ylmethyl]amine,
N-(3-ethoxycarbonylcyclohexyl)-N-pentanoyl-N-[2'-(1H-tetrazol-5-yl)biphenyl-4-ylmethyl]amine,
N-(3-carboxycyclohexyl)-N-pentanoyl-N-[2'-(1H-tetrazol-5-yl)biphenyl-4-methyl]-amine,
cis-N-(4-carboxycyclohexyl)-N-pentanoyl-N-[2'-(1H-tetrazol-5-yl)biphenyl-4-ylmethyl]amine,
cis-N-(2-ethoxycarbonylcyclohexyl)-N-pentanoyl-N-[2'-(1H-tetrazol-5-yl)biphenyl-4-ylmethyl]amine,
cis-N-(2-carboxycyclohexyl)-N-pentanoyl-N-[2'-(1H-tetrazol-5-yl)biphenyl-4-ylmethyl]amine,
N-{2-[2-(4-hydroxyphenyl)ethylaminocarbonyl]-2,2-tetramethyleneethyl}-N-pentanoyl-N-[2'-(1H-tetrazol-5-yl)biphenyl-4-ylmethyl]amine,
(S)-N-{1-[2-(4-hydroxyphenyl)ethylaminocarbonyl]-2-methylprop-1-yl}-N-pentanoyl-N-[2'-(1H-tetrazol-5-yl)biphenyl-4-ylmethyl]amine,
(S)-N-(1-carboxy-2,2-dimethylprop-1-yl)-N-pentanoyl-N-[2'-(1H-tetrazol-5-yl)biphenyl-4-ylmethyl]amine,
(S)-N-(1-methoxycarbonyl-2-methylprop-1-yl)-N-pentanoyl-N-[2'-(1H-tetrazol-5-yl)biphenyl-4-ylmethyl]amine,
N-(4-phenoxybut-1-yl)-N-pentanoyl-N-[2'-(1H-tetrazol-5-yl)biphenyl-4-ylmethyl]amine,
N-(2-hydroxy-1-phenyl-2-oxoethyl)-N-pentanoyl-N-[2'-(1H-tetrazol-5-yl)biphenyl-4-ylmethyl]amine,
(S)-N-(1-benzyloxycarbonyl-2-methylprop-1-yl)-N-pentanoyl-N-[2'-(1H-tetrazol-5-yl)biphenyl-4-ylmethyl]amine,
N-butanoyl-N-(1-carboxy-1-methylethyl)-N-[2'-(1H-tetrazol-5-yl)biphenyl-4-ylmethyl]-amine,
N-(4-hydroxybut-1-yl)-N-pentanoyl-N-[2'-(1H-tetrazol-5-yl)biphenyl-4-ylmethyl]amine,
(S)-N-(1-benzyloxycarbonyl-2-methylprop-1-yl)-N-[3-bromo-2'-(1H-tetrazol-5-yl)-biphenyl-4-ylmethyl]-N-pentanoylamine,
(S)-N-[3-bromo-2'-(1H-tetrazol-5-yl)biphenyl-4-ylmethyl]-N-(1-carboxy-2-methylprop-1-yl)-N-pentanoylamine,
N-(2-acetylaminoethyl)-N-pentanoyl-N-[2'-(1H-tetrazol-5-yl)biphenyl-4-ylmethyl]amine,
N-[2-(n-butoxycarbonyl)-2,2-tetramethyleneethyl]-N-pentanoyl-N-[2'-(1H-tetrazol-5-yl)biphenyl-4-ylmethyl]amine,
N-(2-benzylaminocarbonyl-2,2-tetramethyleneethyl)-N-pentanoyl-N-[2'-(1H-tetrazol-5-yl)biphenyl-4-ylmethyl]amine,
(S)-N-butyloxycarbonyl-N-(1-carboxy-2-methylprop-1-yl)-N-[2'-(1H-tetrazol-5-yl)biphenyl-4-ylmethyl]amine,
(S)-N-(1-carboxy-2-methylprop-1-yl)-N-methoxycarbonyl-N-[2'-(1H-tetrazol-5-yl)biphenyl-4-ylmethyl]amine,
N-(2-diethylaminocarbonyl-2,2-tetramethyleneethyl)-N-pentanoyl-[2'-(1H-tetrazol-5-yl)biphenyl-4-ylmethyl]amine,
N-(2-methyl-2-morpholin-4-ylcarbonylpropyl)-N-pentanoyl-N-[2'-(1H-tetrazol-5-yl)biphenyl-4-ylmethyl]amine,
N-(1-carboxycyclopentyl)-N-pentanoyl-N-[2'-(1H-tetrazol-5-yl)biphenyl-4-ylmethyl]-amine,
N-(1-carboxy-1-ethylprop-1-yl)-N-pentanoyl-N-[2'-(1H-tetrazol-5-yl)biphenyl-4-ylmethyl]amine,
(S)-N-(5-amino-1-carboxypent-1-yl)-N-pentanoyl-N-[2'-(1H-tetrazol-5-yl)biphenyl-4-ylmethyl]amine,
N-butanesulfonyl-N-(2-ethoxycarbonyl-2,2-pentamethyleneethyl)-N-[2'-(1H-tetrazol-5-yl)biphenyl-4-ylmethyl]amine,
N-butanesulfonyl-N-(2-carboxy-2,2-pentamethyleneethyl)-N-[2'-(1H-tetrazol-5-yl)biphenyl-4-ylmethyl]amine,
N-butanesulfonyl-N-(2-ethoxycarbonyl-2-methylprop-1-yl)-N-[2'-(1H-tetrazol-5-yl)biphenyl-4-ylmethyl]amine,
N-butanesulfonyl-N-(2-carboxy-2-methylprop-1-yl)-N-[2'-(1H-tetrazol-5-yl)biphenyl-4-ylmethyl]amine,
(S)-N-butanesulfonyl-N-(1-tert-butoxycarbonylethyl)-N-[2'-(1H-tetrazol-5-yl)biphenyl-4-ylmethyl]amine,
(S)-N-butanesulfonyl-N-(1-carboxyethyl)-N-[2'-(1H-tetrazol-5-yl)biphenyl-4-ylmethyl]-amine,
(S)-N-butanesulfonyl-N-(1-carboxy-2-methylprop-1-yl)-N-[2'-(1H-tetrazol-5-yl)biphenyl-4-ylmethyl]amine,
(S)-N-(2-methyl-1-methylaminocarbonylprop-1-yl)-N-pentanoyl-N-[2'-(1H-tetrazol-5-yl)biphenyl-4-ylmethyl]amine,
(S)-N-(1-dimethylaminocarbonyl-2-methylprop-1-yl)-N-pentanoyl-N-[2'-(1H-tetrazol-5-yl)biphenyl-4-ylmethyl]amine,
(S)-N-(2-methyl-1-morpholin-4-ylcarbonylprop-1-yl)-N-pentanoyl-N-[2'-(1H-tetrazol-5-yl)biphenyl-4-ylmethyl]amine,
(S)-N-(2'-carboxybiphenyl-4-ylmethyl)-N-(1-carboxy-2-methylprop-1-yl)-N-pentanoyl-amine,
(S)-N-(1,2-dicarboxyethyl)-N-pentanoyl-N-[2'-(1H-tetrazol-5-yl)-biphenyl-4-ylmethyl]-amine,
(S)-N-(1-carboxy-3-phenylprop-1-yl)-N-pentanoyl-N-[2'-(1H-tetrazol-5-yl)biphenyl-4-ylmethyl]amine,
(S)-N-(2-cyclohexyl-1-hydroxymethylethyl)-N-pentanoyl-N-[2'-(1H-tetrazol-5-yl)biphenyl-4-ylmethyl]amine,
(R)-N-(1-methoxycarbonyl-2-methylprop-1-yl)-N-pentanoyl-N-[2'-(1H-tetrazol-5-yl)biphenyl-4-ylmethyl]amine,
(S)-N-(2-hydroxy-1-methoxycarbonylethyl)-N-pentanoyl-N-[2'-(1H-tetrazol-5-yl)biphenyl-4-ylmethyl]amine,
N-pentanoyl-N-(1H-tetrazol-5-ylmethyl)-N-[2'-(1H-tetrazol-5-yl)biphenyl-4-ylmethyl]-amine,
N-pentanoyl-N-pyrid-3-ylmethyl-N-[2'-(1H-tetrazol-5-yl)biphenyl-4-ylmethyl]amine,
(S)-N-(1-carboxy-4-guanidinobut-1-yl)-N-pentanoyl-N-[2'-(1H-tetrazol-5-yl)biphenyl-4-ylmethyl]amine,
N-(2-hydroxy-1-methoxycarbonylprop-1-yl)-N-pentanoyl-N-[2'-(1H-tetrazol-5-yl)biphenyl-4-ylmethyl]amine,
N-(1-benzyloxycarbonyl-1-methylethyl)-N-butanoyl-N-[2'-(1H-tetrazol-5-yl)biphenyl-4-ylmethyl]amine, (S)-N-(1-carboxy-3-methylbut-1-yl)-N-pentanoyl-N-[2'-(1H-tetrazol-5-yl)biphenyl-4-ylmethyl]amine,
N-(1-carboxy-2-hydroxyethyl)-N-pentanoyl-N-[2'-(1H-tetrazol-5-yl)biphenyl-4-yl-methyl]amine,
(S)-N-(1-carboxy-2-hydroxyethyl)-N-pentanoyl-N-[2'-(1H-tetrazol-5-yl)biphenyl-4-ylmethyl]amine,

(S)-N-(2-methyl-1-(2-phenylethylaminocarbonyl)prop-1-yl]-N-pentanoyl-N-[2'-(1H-tetrazol-5-yl)biphenyl-4-ylmethyl]amine,
(S)-N-(2-benzyloxy-1-hydroxymethylethyl)-N-pentanoyl-N-[2'-(1H-tetrazol-5-yl)biphenyl-4-ylmethyl]amine,
(S)-N-(1-carboxy-2-methylprop-1-yl)-N-pentanoyl-N-[2'-(1H-tetrazol-5-yl)biphenyl-3-ylmethyl]amine,
(S)-N-(1-carboxy-2-methylprop-1-yl)-N-pentanoyl-N-[3'-(1H-tetrazol-5-yl)biphenyl-4-ylmethyl]amine,
(S)-N-[2-methyl-1-(1,2,3,4-tetrahydroquinol-1-ylcarbonyl)prop-1-yl]-N-pentanoyl-N-[2'-(1H-tetrazol-5-yl)biphenyl-4-ylmethyl]amine,
(S)-N-(2-methyl-1-piperidin-1-ylcarbonylprop-1-yl)-N-pentanoyl-N-[2'-(1H-tetrazol-5-yl)biphenyl-4-ylmethyl]amine,
(S)-N-[2-methyl-1-(1,2,3,4-tetrahydroisoquinol-2-ylcarbonyl)prop-1-yl]-N-pentanoyl-N-[2'-(1H-tetrazol-5-yl)biphenyl-4-ylmethyl]amine,
N-(2-hydroxymethyl-2-methylprop-1-yl)-N-pentanoyl-N-[2'-(1H-tetrazol-5-yl)-biphenyl-4-ylmethyl]amine,
N-ethoxycarbonyl-N-(2-ethoxycarbonyl-2-methylprop-1-yl)-N-[2'-(1H-tetrazol-5-yl)-biphenyl-4-ylmethyl]amine and
N-(2-carboxy-2-methylprop-1-yl)-N-ethoxycarbonyl-N-[2'-(1H-tetrazol-5-yl)biphenyl-4-ylmethyl]amine, in each case in free form or in salt form.

**34.** A process for the production of a pharmaceutical preparation comprising as active ingredient a compound according to any one of claims 1 or 3 to 33, in free form or in the form of a pharmaceutically utilizable salt, if appropriate in addition to customary pharmaceutical excipients, wherein the active ingredient, if appropriate with admixture of customary pharmaceutical excipients, is processed to give a pharmaceutical preparation.

**35.** The use of a compound according to any one of claims 1 or 3 to 33, in free form or in the form of a pharmaceutically utilizable salt, for the production of an antihypertensive.

**36.** The use of a compound according to any one of claims 1 or 3 to 33, in free form or in the form of a pharmaceutically utilizable salt, for the production of a pharmaceutical preparation for the therapeutic or prophylactic treatment of cardiac insufficiency.

**37.** The use of a compound according to any one of claims 1 or 3 to 33, in free form or in the form of a pharmaceutically utilizable salt, for the production of a pharmaceutical preparation for the therapeutic or prophylactic treatment of disorders which are caused by angiotensin II activity.

**Revendications**

**Revendications pour les Etats contractants suivants : AT, BE, CH, DE, DK, FR, GB, IT, LI, LU, NL, SE**

**1.** Composé de formule

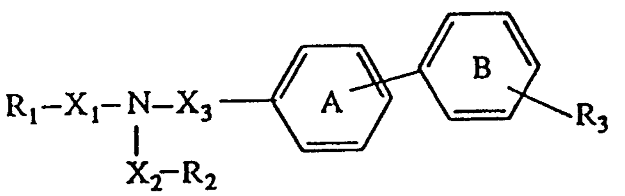

(I),

dans laquelle $R_1$ représente un radical alkyle inférieur, alcényle inférieur ou alcynyle inférieur, éventuellement substitué par un atome d'halogène ou par le groupe hydroxy, ou un radical cycloalkyle en $C_3$-$C_7$ ou cycloalcényle en $C_3$-$C_7$ ou phényl-alkyle inférieur, phényl-alcényle inférieur ou phényl-alcynyle inférieur; $X_1$ représente CO, $SO_2$ ou -O-C(=O)-, l'atome de carbone du groupe carbonyle étant lié à l'atome d'azote indiqué dans la formule I; $X_2$ représente un radical alkylène en $C_1$-$C_{10}$, alkylidène en $C_2$-$C_{10}$ ou cycloalkylène en $C_3$-$C_7$, éventuellement substitué par un groupe hydroxy, carboxy, amino, guanidino, cycloalkyle en $C_3$-$C_7$, cycloalcényle en $C_3$-$C_7$, phényle ou par un groupe aromatique monocyclique et à 5 ou 6 chaînons correspondant, qui comporte jusqu'à 4 hétéroatomes identiques ou différents, un atome de carbone du radical alkylène en $C_1$-$C_{10}$ ou alkylidène en $C_2$-$C_{10}$ pouvant en outre être ponté par un groupe alkylène en $C_2$-$C_6$, et un radical cycloalkyle en $C_3$-$C_7$ ou cycloalcényle en $C_3$-$C_7$ étant éventuellement une ou plusieurs fois substitué par un ou des groupes carboxy, carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, ou par un ou des groupes carbamoyle, carbamoyle dont le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou

phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, ou par un ou des groupes formyle, di-(alcoxy inférieur)-méthyle ou oxy-(alkylène-oxy inférieur)-méthylène; $R_2$ représente le groupe carboxy ou un groupe carboxy qui est estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, le groupe carbamoyle ou un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, le groupe amino ou un groupe amino qui est monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, un groupe alcanoyle inférieur, phényl-(alcanoyl inférieur)-, benzoyl-, (alcane inférieur)-sulfonyl-, benzènesulfonyl-amino, formyle, di-(alcoxy inférieur)-méthyle, oxy-(alkylène-oxy inférieur)-méthylène, 1H-tétrazol-5-yle, pyridyle, hydroxy, alcoxy inférieur, alcényloxy inférieur, phényl-alcoxy inférieur, phénoxy, $S(O)_m$-R, m étant 0, 1 ou 2 et R représentant un atome d'hydrogène ou un radical alkyle inférieur, alcényle inférieur ou alcynyle inférieur, ou représente un groupe alcanoyle inférieur, sulfamoyle, sulfamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, ou représente $PO_nH_2$, n étant 2 ou 3; $X_3$ représente un groupe alkylène en $C_1$-$C_{10}$ ou alkylidène en $C_2$-$C_{10}$; $R_3$ est le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, étant éventuellement substitués, indépendamment les uns des autres, par des substituants choisis parmi des atomes d'halogène et des groupes hydroxy, alcoxy inférieur, alcényloxy inférieur, phényl-alcoxy inférieur, phénoxy, $S(O)_m$-R et des groupes alkyle inférieur, alcényle inférieur ou alcynyle inférieur éventuellement substitués par un atome d'halogène ou par le groupe hydroxy, les radicaux alkyle inférieur, alcényle inférieur ou alcynyle inférieur étant éventuellement interrompus par -O-, ainsi que, dans le cas de radicaux (hétéro)aromatiques, éventuellement substitués en outre par le groupe carboxy ou un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, par le groupe carbamoyle ou un groupe carbamoyle dans lequel le fragment amino est monosubstitué ou disubstitué, indépendanment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$ -$C_{10}$ étant éventuellement interrompus par -O- ou condensés, au niveau de deux atomes de carbone contigus, avec un cycle benzénique, par le groupe formyle ou par un groupe di-(alcoxy inférieur)-méthyle ou oxy(alkylène-oxy inférieur)-méthylène; les radicaux et groupes désignés par "inférieur" contenant jusqu'à 7 atomes de carbone; sous forme libre ou sous forme de sel.

**2.** composé selon la revendication 1, de formule I, dans lequel $R_1$ représente un radical alkyle inférieur, alcényle inférieur ou alcynyle inférieur éventuellement substitué par un atome d'halogène ou par le groupe hydroxy, ou un radical cycloalkyle en $C_3$-$C_7$ ou cycloalcényle en $C_3$-$C_7$ ou phényl-alkyle inférieur, phényl-alcényle inférieur ou phényl-alcynyle inférieur; $X_1$ représente CO ou $SO_2$; $X_2$ représente un radical alkylène en $C_1$-$C_{10}$ ou alkylidène en $C_2$-$C_{10}$ ou cycloalkylène en $C_3$-$C_7$, éventuellement substitué par un groupe hydroxy, cycloalkyle en $C_3$-$C_7$ ou cycloalcényle en $C_3$-$C_7$ ou phényle ou par un radical aromatique correspondant, monocyclique à 5 ou 6 chaînons, qui comporte jusqu'à 4 hétéroatomes identiques ou différents, un atome de carbone du radical alkylène en $C_1$-$C_{10}$ ou alkylidène en $C_2$-$C_{10}$ pouvant en outre être ponté par des groupes alkylène en $C_2$-$C_6$, et les groupes cycloalkylène en $C_3$-$C_7$ étant éventuellement substitués une ou plusieurs fois par un ou plusieurs groupes carboxy, carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, ou par le groupe carbamoyle ou un groupe carbamoyle dans lequel le fragment amino est monosubstitué ou disubstitué, indépendamment l'un de l'autre, par un ou des groupes alkyle inférieur, alcényle inférièur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, le groupe formyle, un groupe di(alcoxy inférieur)-méthyle ou oxy-(alkylène-oxy inférieur)-méthylène; $R_2$

représente le radical carboxy ou un radical carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, ou le radical carbamoyle ou un radical carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, le radical amino ou un radical amino qui est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente un radical (alcanoyl inférieur)-, phényl-(alcanoyl inférieur)-, benzoyl-, (alcane inférieur)-sulfonyl-, benzènesulfonyl-amino, formyle, di-(alcoxy inférieur)-méthyle, oxy-(alkylène-oxy inférieur)-méthylène, hydroxy, alcoxy inférieur, alcényloxy inférieur, phényl-alcoxy inférieur, phénoxy, $S(O)_m$-R, m représentant 0, 1 ou 2 et R représentant un atome d'hydrogène ou un groupe alkyle inférieur, alcényle inférieur ou alcynyle inférieur, un radical alcanoyle inférieur, le radical sulfamoyle ou un radical sulfamoyle dans lequel le fragment amino est monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente $PO_nH_2$, n représentant 2 ou 3; $X_3$ représente un radical alkylène en $C_1$-$C_{10}$ ou alkylidène en $C_2$-$C_{10}$; $R_3$ est le groupe carboxy, 5-tétrazolyle, $SO_3H$, $PO_2H_2$, $PO_3H_2$ ou un groupe halogéno(alkyl inférieur)-sulfamoyle; les radicaux (hétéro)aromatiques, y compris les cycles A et B, étant éventuellement substitués, indépendamment les uns des autres, par des substituants choisis parmi des atomes d'halogène et des groupes hydroxy, alcoxy inférieur, alcényloxy inférieur, phényl-alcoxy inférieur, phénoxy, $S(O)_m$-R et des radicaux alkyle inférieur, alcényle inférieur ou alcynyle inférieur éventuellement substitués par un atome d'halogène ou par le groupe hydroxy, les radicaux alkyle inférieur, alcényle inférieur ou alcynyle inférieur étant éventuellement interrompus par -O-, ainsi que, dans le cas des radicaux (hétéro)aromatiques, éventuellement substitués en outre par le groupe carboxy, un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, par le groupe carbamoyle, par un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou par le groupe formyle, un groupe di-(alcoxy inférieur)-méthyle ou oxy(alkylène-oxy inférieur)-méthylène; sous forme libre ou sous forme de sel.

3. Composé selon la revendication 1, de formule I, dans lequel $R_1$ représente un radical alkyle inférieur, alcényle inférieur ou alcynyle inférieur éventuellement substitué par un atome d'halogène ou par le groupe hydroxy, ou un radical cycloalkyle en $C_3$-$C_7$ ou cycloalcényle en $C_3$-$C_7$ ou phényl-alkyle inférieur, phényl-alcényle inférieur ou phényl-alcynyle inférieur; $X_1$ représente CO ou $SO_2$; $X_2$ représente un radical alkylène en $C_1$-$C_{10}$ ou alkylidène en $C_2$-$C_{10}$ éventuellement substitué par un groupe hydroxy, cycloalkyle en $C_3$-$C_7$ ou cycloalcényle en $C_3$-$C_7$ ou phényle ou par un radical aromatique correspondant, monocyclique et à 5 ou 6 chaînons, qui comporte jusqu'à 4 hétéroatomes identiques ou différents; $R_2$ représente le groupe carboxy ou un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, le groupe carbamoyle ou un groupe carbamoyle dans lequel le fragment amino est mono-substitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente le groupe amino ou un groupe amino qui est monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente un groupe (alcanoyl inférieur)-, phényl-(alcanoyl inférieur)-, benzoyl-, (alcane inférieur)-sulfonyl-, benzènesulfonylamino, formyle, di-(alcoxy inférieur)-méthyle, oxy-(alkylène-oxy inférieur)-méthylène, hydroxy, alcoxy inférieur, alcényloxy inférieur, phényl-alcoxy inférieur ou phénoxy, $S(O)_m$-R, m représentant 0, 1 ou 2 et R représentant un atome d'hydrogène ou un groupe alkyle inférieur, alcényle inférieur ou alcynyle inférieur, un radical alcanoyle inférieur, ou représente le radical sulfamoyle ou un radical sulfamoyle dans lequel le fragment amino est monosubstitué ou disubstitué, indépendamment l'un de l'autre, par un ou des groupes alkyle inférieur, alcényle inférieur, alcynyle

inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O-, ou représente $PO_nH_2$, n étant 2 ou 3; $X_3$ représente -$CH_2$-; $R_3$ est le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, étant éventuellement substitués, indépendamment les uns des autres, par des substituants choisis parmi des atomes d'halogène et des groupes hydroxy, alcoxy inférieur, alcényloxy inférieur, phényl-alcoxy inférieur, phénoxy, $S(O)_m$-R et des radicaux alkyie inférieur, alcényle inférieur ou alcynyle inférieur éventuellement substitués par un atome d'halogène ou par le groupe hydroxy, les radicaux alkyle inférieur, alcényle inférieur ou alcynyle inférieur étant éventuellement interrompus par -O-, et, dans le cas de radicaux (hétéro)aromatiques, étant éventuellement substitués en outre par le groupe carboxy ou par un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, par le groupe carbamoyle ou par un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou par le groupe formyle, un groupe di-(alcoxy inférieur)-méthyle ou oxy-(alkylène-oxy inférieur)-méthylène; sous forme libre ou sous forme de sel.

4. Composé selon la revendication 1, de formule I, dans lequel $R_1$ représente un radical alkyle inférieur, alcényle inférieur, alcynyle inférieur, halogéno-alkyle inférieur, -alcényle inférieur, -alcynyle inférieur, hydroxyalkyle inférieur, -alcényle inférieur, -alcynyle inférieur, cycloalkyle en $C_3$-$C_7$, cycloalcényle en $C_3$-$C_7$, phényl-alkyle inférieur, phényl-alcényle inférieur ou phényl-alcynyle inférieur; $X_1$ représente CO ou $SO_2$; $X_2$ représente un radical alkylène en $C_1$-$C_{10}$ ou alkylidène en $C_2$-$C_{10}$ qui sont éventuellement substitués par le groupe hydroxy ou par un radical cycloalkyle en $C_3$-$C_7$, cycloalcényle en $C_3$-$C_7$, phényle ou par un radical hétéroaromatique monocyclique à 5 ou 6 chaînons, comportant jusqu'à 4 hétéroatomes identiques ou différents, les radicaux cycliques étant pour leur part éventuellement substitués par un groupe carboxy éventuellement estérifié par un alcool dérivant d'un reste alkyle inférieur, phényl-alkyle inférieur, alcényle inférieur, alcynyle inférieur, (alcoxy inférieur)-alkyle inférieur, -alcényle inférieur ou -alcynyle inférieur, ou par un groupe carbamoyle dans lequel le fragment amino est éventuellement monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, le radical formyle, di-(alcoxy inférieur)-méthyle, oxy-(alkylène-oxy inférieur)-méthylène; $R_2$ représente un groupe carboxy éventuellement estérifié par un alcool dérivant d'un reste alkyle inférieur, phényl-alkyle inférieur, alcényle inférieur, alcynyle inférieur, (alcoxy inférieur)-alkyle inférieur, -alcényle inférieur ou -alcynyle inférieur, un radical carbamoyle dans lequel le fragment amino est éventuellement monosubstitué ou disubstitué, indépendamment l'un de l'autre, par un ou des groupes alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, un radical amino dans lequel le fragment amino est éventuellement monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, un groupe (alcanoyl inférieur)-, phényl-(alcanoyl inférieur)-, benzoyl-, (alcane inférieur)-sulfonyl-, benzènesulfonyl-amino, formyle, di-(alcoxy inférieur)-méthyle, oxy-(alkylène-oxy inférieur)-méthylène, hydroxy, alcoxy inférieur, alcényloxy inférieur, phényl-alcoxy inférieur, phénoxy, $S(O)_m$-R, m représentant 0, 1 ou 2 et R représentant un atome d'hydrogène ou un groupe alkyle inférieur, alcényle inférieur ou alcynyle inférieur, un radical alcanoyle inférieur, sulfamoyle, dans lequel le fragment amino est éventuellement monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux (alkylène inférieur)- ou (alkylène-oxy inférieur)-alkylène inférieur, ou représente $PO_nH_2$, n représentant 2 ou 3; $X_3$ représente -$CH_2$-; $R_3$ est le groupe carboxy, 5-tétrazolyle, $SO_3H$, $PO_2H_2$, $PO_3H_2$ ou un groupe halogéno-(alkyl inférieur)-sulfamoyle; les radicaux (hétéro)aromatiques, y compris les cycles A et B, étant éventuellement substitués, indépendamment les uns des autres, par un ou plusieurs substituants choisis parmi des atomes d'halogène et des groupes hydroxy, alcoxy inférieur, alcényloxy inférieur, des radicaux alkyle inférieur, alcényle inférieur, alcynyle inférieur, (alcoxy inférieur)-alkyle inférieur, -alcényle inférieur, -alcynyle inférieur, (alcényloxy inférieur)-alkyle inférieur, -alcényle inférieur et -alcynyle inférieur, éventuellement substitués chacun par un atome d'halogène ou par le groupe hydroxy, sous forme libre ou sous forme de sel.

5. Composé selon la revendication 1, de formule I, dans lequel $X_2$ représente un radical alkylène en $C_1$-$C_{10}$ ou alkylidène en $C_2$-$C_{10}$ qui sont éventuellement substitués par le groupe hydroxy ou par un groupe cycloalkyle en $C_3$-$C_7$,

cycloalcényle en $C_3$-$C_7$, phényle ou par un radical hétéroaromatique monocyclique à 5 ou 6 chaînons, comportant jusqu'à 4 hétéroatomes identiques ou différents, un atome de carbone d'un radical alkylène en $C_1$-$C_{10}$ ou alkylidène en $C_2$-$C_{10}$ pouvant être ponté par un groupe alkylène en $C_2$-$C_6$, et les radicaux cycliques étant pour leur part éventuellement substitués par un groupe carboxy éventuellement estérifié par un alcool dérivant d'un reste alkyle inférieur, phényl-alkyle inférieur, alcényle inférieur, alcynyle inférieur, (alcoxy inférieur)-alkyle inférieur, -alcényle inférieur ou -alcynyle inférieur, par un groupe carbamoyle dans lequel le fragment amino est éventuellement monosubstitué ou disubstitué, indépendamment l'un de l'autre, par un ou des groupes alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, par le groupe formyle, par un groupe di-(alcoxy inférieur)-méthyle, ou par un groupe oxy-(alkylène-oxy inférieur)-méthylène, ou $X_2$ représente un groupe cycloalkylène en $C_3$-$C_7$; $X_3$ représente un groupe alkylène inférieur ou alkylidène inférieur; les variables $X_1$, $R_1$, $R_2$, $R_3$ ont les significations indiquées dans la revendication 4; et les cycles (hétéro)aromatiques, y compris les cycles A et B, peuvent être substitués comme indiqué dans la revendication 4, sous forme libre ou sous forme de sel.

**6.** Composé selon la revendication 1, de formule I, dans lequel $R_1$ représente un radical alkyle inférieur, alcényle inférieur, halogéno-alkyle inférieur, -alcényle inférieur, hydroxy-alkyle inférieur, un radical cycloalkyle à 3-7 chaînons ou phényl-alkyle inférieur; $X_1$ représente CO, $SO_2$ ou -O-C(=O)-, l'atome de carbone du groupe carbonyle étant lié à l'atome d'azote indiqué dans la formule I; $X_2$ représente un radical alkylène en $C_1$-$C_{10}$ ou alkylidène en $C_1$-$C_7$, qui sont éventuellement substitués par le groupe hydroxy, carboxy, amino, guanidino, un radical cycloalkyle à 3-7 chaînons, cycloalcényle à 3- 7 chaînons, phényle, pyrrolyle, pyrazolyle, imidazolyle, triazolyle, tétrazolyle, furyle, thiényle ou pyridyle, qui pour leur part peuvent être éventuellement substitués en outre par un radical carboxy, (alcoxy inférieur)-carbonyle, phényl-(alcoxy inférieur)-carbonyle, par un radical carbamoyle dans lequel le fragment amino peut éventuellement être monosubstitué ou disubstitué, indépendamment l'un de l'autre, par un ou des groupes alkyle inférieur ou phényl-alkyle inférieur, ou un radical formyle, di-(alcoxy inférieur)-méthyle ou oxy-(alkylène-oxy inférieur)-méthylène; $R_2$ représente un groupe carboxy, (alcoxy inférieur)-, phényl-(alcoxy inférieur)-, (alcényloxy inférieur)-, (alcoxy inférieur)-(alcoxy inférieur)-carbonyle, un radical carbamoyle dans lequel le fragment amino est éventuellement monosubstitué ou disubstitué, indépendamment l'un de l'autre, par un ou des radicaux alkyle inférieur ou phényl-alkyle inférieur, ou est disubstitué par des radicaux alkylène inférieur, éventuellement condensés, au niveau de deux atomes de carbone contigus, avec un cycle benzénique, ou par des radicaux (alkylène-oxy inférieur)-alkylène inférieur, un radical amino dans lequel le fragment amino est éventuellement monosubstitué ou disubstitué, indépendamment l'un de l'autre, par un ou des radicaux alkyle inférieur ou phényl-alkyle inférieur, ou est disubstitué par des radicaux (alkylène inférieur)- ou (alkylène-oxy inférieur)-alkylène inférieur, un groupe (alcanoyl inférieur)-, phényl-(alcanoyl inférieur)-, benzoyl-, (alcane inférieur)-sulfonyl-, benzènesulfonylamino, formyle, di-(alcoxy inférieur)-méthyle, oxy-(alkylène-oxy inférieur)-méthylène, hydroxy, alcoxy inférieur, phényl-alcoxy inférieur, phénoxy, $S(O)_m$-R, m représentant 0, 1 ou 2 et R représentant un groupe alkyle inférieur, alcanoyle inférieur, sulfamoyle, dans lequel le fragment amino est éventuellement monosubstitué ou disubstitué, indépendamment l'un de l'autre, par un ou des groupes alkyle inférieur ou phényl-alkyle inférieur, ou représente $PO_nH_2$, n représentant 2 ou 3; $X_3$ est le groupe méthylène; $R_3$ représente le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, sont éventuellement substitués chacun en outre par un ou plusieurs substituants choisis parmi des atomes d'halogène et des groupes hydroxy, alcoxy inférieur, et des radicaux alkyle inférieur ou (alcoxy inférieur)-alkyle inférieur éventuellement substitués chacun par un atome d'halogène ou par le groupe hydroxy, sous forme libre ou sous forme de sel.

**7.** Composé selon la revendication 1, de formule I, dans lequel $R_1$ représente un radical alkyle inférieur, alcényle inférieur, halogéno-alkyle inférieur, -alcényle inférieur, hydroxy-alkyle inférieur, un radical cycloalkyle à 3-7 chaînons ou phényl-alkyle inférieur; $X_1$ représente CO ou $SO_2$; $X_2$ représente un radical alkylène en $C_1$-$C_{10}$ ou alkylidène en $C_1$-$C_7$, qui sont éventuellement substitués par un groupe hydroxy, un radical cycloalkyle à 3-7 chaînons, cycloalcényle à 3-7 chaînons, phényle, pyrrolyle, pyrazolyle, imidazolyle, triazolyle, tétrazolyle, furyle, thiényle ou pyridyle, qui peuvent pour leur part être éventuellement substitués en outre par un groupe carboxy, (alcoxy inférieur)-carbonyle, phényl-(alcoxy inférieur)-carbonyle, carbamoyle dans lequel le fragment amino est éventuellement monosubstitué ou disubstitué, indépendamment l'un de l'autre, par un ou des groupes alkyle inférieur ou phényl-alkyle inférieur, le radical formyle, un radical di-(alcoxy inférieur)-méthyle ou oxy-(alkylène-oxy inférieur)-méthylène; $R_2$ représente un radical carboxy, (alcoxy inférieur)-, phényl-(alcoxy inférieur)-, (alcényloxy inférieur)-, (alcoxy inférieur)-(alcoxy inférieur)-carbonyle, un radical carbamoyle dans lequel le fragment amino est éventuellement monosubstitué ou disubstitué, indépendamment l'un de l'autre, par un ou des groupes alkyle inférieur, phényl-alkyle inférieur, ou disubstitué par des radicaux (alkylène inférieur)-, ou (alkylène-oxy inférieur)- alkylène inférieur, un radical amino dans lequel le fragment amino est éventuellement monosubstitué ou disubstitué, indépendamment l'un de l'autre, par un ou des groupes alkyle inférieur, phényl-alkyle inférieur, ou disubstitué par des radicaux (alkylène inférieur)-

ou (alkylène-oxy inférieur)-alkylène inférieur, un groupe (alcanoyl inférieur)-, phényl-(alcanoyl inférieur)-, benzoyl-, (alcane inférieur)-sulfonyl-, benzènesulfonylamino, formyle, di-(alcoxy inférieur)-méthyle, oxy-(alkylène-oxy inférieur)-méthylène, hydroxy, alcoxy inférieur, phényl-alcoxy inférieur, phénoxy, $S(O)_m$-R, m représentant 0, 1 ou 2 et R représentant un groupe alkyle inférieur, un radical alcanoyle inférieur, sulfamoyle dans lequel le groupe amino est éventuellement monosubstitué ou disubstitué, indépendamment l'un de l'autre, par des groupes alkyle inférieur, phényl-alkyle inférieur, ou représente $PO_nH_2$, n représentant 2 ou 3; $X_3$ est le groupe méthylène; $R_3$ représente le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, sont éventuellement substitués chacun en outre par un ou plusieurs substituants choisis parmi des atomes d'halogène et des groupes hydroxy, alcoxy inférieur, des groupes alkyle inférieur ou (alcoxy inférieur)-alkyle inférieur éventuellement substitués chacun par un atome d'halogène ou par le groupe hydroxy, sous forme libre ou sous forme de sel.

**8.** Composé selon la revendication 1, de formule I, dans lequel $X_2$ représente un radical alkylène en $C_1$-$C_{10}$ ou alkylidène en $C_1$-$C_7$, qui sont éventuellement substitués par un groupe hydroxy, un radical cycloalkyle à 3-7 chaînons, cycloalcényle à 3-7 chaînons, phényle, pyrrolyle, pyrazolyle, imidazolyle, triazolyle, tétrazolyle, furyle, thiényle ou pyridyle, qui peuvent pour leur part être éventuellement substitués en outre par un groupe carboxy, (alcoxy inférieur)-carbonyle, phényl-(alcoxy inférieur)-carbonyle, carbamoyle dans lequel le fragment amino est éventuellement monosubstitué ou disubstitué, indépendamment l'un de l'autre, par un ou des groupes alkyle inférieur ou phényl-alkyle inférieur, ou peuvent être substitués par le groupe formyle, par un groupe di-(alcoxy inférieur)-méthyle, ou par un groupe oxy-(alkylène-oxy inférieur)-méthylène, un atome de carbone d'un radical alkylène en $C_1$-$C_{10}$ ou alkylidène en $C_1$-$C_7$ pouvant être ponté par un groupe alkylène en $C_2$-$C_6$, ou $X_2$ représente un groupe cycloalkylène en $C_3$-$C_7$; $X_3$ représente un groupe alkylène inférieur ou alkylidène inférieur; les variables $X_1$, $R_1$, $R_2$, $R_3$ ont les significations indiquées dans la revendication 7, et les cycles (hétéro)-aromatiques, y compris les cycles A et B, peuvent être substitués comme indiqué dans la revendication 7, sous forme libre ou sous forme de sel.

**9.** Composé selon la revendication 1, de formule I, dans lequel les variables $R_1$, $X_1$, $R_3$ ont les significations indiquées respectivement dans les revendications 1 à 7; $X_2$ représente un radical alkylène inférieur ou alkylidène inférieur éventuellement substitué par un groupe hydroxy, cycloalkyle à 3-7 chaînons, phényle ou imidazolyle; et $R_2$ représente un radical carboxy, (alcoxy inférieur)-, phényl-(alcoxy inférieur)-, (alcoxy inférieur)-(alcoxy inférieur)carbonyle, un radical carbamoyle qui est éventuellement monosubstitué ou disubstitué, indépendamment l'un de l'autre, par un ou des groupes alkyle inférieur ou phényl-alkyle inférieur, un radical amino, (alcanoyl inférieur)-, phényl-(alcanoyl inférieur)-, (alcane inférieur)-sulfonyl-amino, hydroxy, alcoxy inférieur, phényl-alcoxy inférieur ou phénoxy; $X_3$ représente $-CH_2-$; les radicaux (hétéro)aromatiques, y compris les cycles A et B, étant éventuellement substitués chacun par un ou plusieurs substituants choisis parmi des atomes d'halogène et des groupes trifluorométhyle, hydroxy, alcoxy inférieur, alkyle inférieur, hydroxyalkyle inférieur ou (alcoxy inférieur)-alkyle inférieur, sous forme libre ou sous forme de sel.

**10.** Composé selon la revendication 1, de formule I, dans lequel $X_2$ représente un radical alkylène inférieur ou alkylidène inférieur éventuellement substitué par un groupe hydroxy, cycloalkyle à 3-7 chaînons, cycloalcényle à 7 chaînons, phényle ou imidazolyle, un atome de carbone d'un radical alkylène inférieur ou alkylidène inférieur pouvant être ponté par un groupe alkylène en $C_2$-$C_6$, ou $X_2$ représente un groupe cycloalkylène en $C_3$-$C_7$; les variables $X_1$, $X_3$, $R_1$, $R_2$, $R_3$ ont les significations indiquées dans les revendications 7 à 9; et les cycles A et B, peuvent être substitués comme indiqué dans la revendication 9, sous forme libre ou sous forme de sel.

**11.** Composé selon la revendication 1, de formule

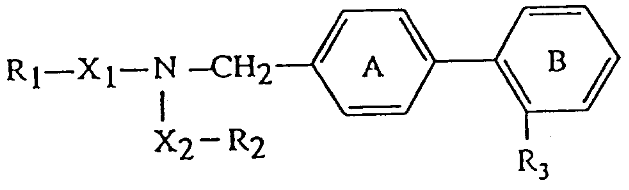

(Ia),

dans laquelle les variables $R_1$, $X_1$, $X_2$, $R_2$ et $R_3$ ont les significations données chacune dans l'une des revendications 1 à 10, et les cycles A et B, peuvent être substitués comme indiqué dans la revendication 10, sous forme libre ou sous forme de sel.

**12.** Composé selon la revendication 1, de formule Ia, dans lequel $X_2$ représente un radical alkylène inférieur ou alkylidène inférieur éventuellement substitué par un groupe hydroxy ou par un groupe cycloalkyle à 3-7 chaînons, un atome de carbone d'un radical alkylène inférieur ou alkylidène inférieur pouvant être ponté par un groupe alkylène en $C_2$-$C_6$, ou dans lequel $X_2$ représente un groupe cycloalkylène en $C_3$-$C_7$, et les variables $R_1$, $X_1$, $R_2$ et $R_3$ ont les significations indiquées chacune dans l'une des revendications 1 à 10, et les cycles A et B peuvent être substitués comme indiqué dans la revendication 10, sous forme libre ou sous forme de sel.

**13.** Composé selon la revendication 1, de formule Ia, dans lequel $X_2$ représente le groupe de formule

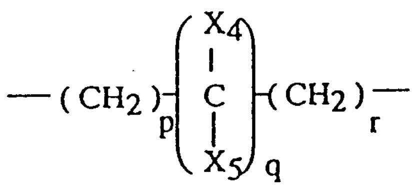

(Ib)

dans laquelle p représente 0 ou 1, q représente 1 et r représente 0 ou 1, ou dans laquelle p représente 1 à 8 et q ainsi que r représentent chacun 0; $X_4$ représente un radical phényle ou alkyle inférieur éventuellement substitué par un groupe hydroxy, cycloalkyle à 3-7 chaînons, phényle ou imidazolyle; et $X_5$ représente un atome d'hydrogène ou un groupe alkyle inférieur; $R_2$ représente un radical carboxy, (alcoxy inférieur)-carbonyle, phényl-(alcoxy inférieur)-carbonyle, (alcoxy inférieur)-(alcoxy inférieur)-carbonyle, hydroxy, alcoxy inférieur, phényl-alcoxy inférieur, phénoxy, amino, alcanoylamino inférieur, phénylalcanoylamino inférieur ou (alcane inférieur)-sulfonylamino; et les variables $R_1$, $X_1$ et $X_3$ ont les significations indiquées respectivement dans l'une des revendications 1 à 7; les radicaux (hétéro)-aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**14.** Composé selon la revendication 1, de formule Ia, dans lequel $X_2$ représente le groupe de formule Ib, dans lequel p représente 0 ou 1, q représente 1 et r représente 0 ou 1, ou dans lequel p représente 1 à 8 et q ainsi que r représentent chacun 0; $X_4$ représente un radical phényle ou alkyle inférieur éventuellement substitué par un groupe hydroxy, cycloalkyle à 3-7 chaînons, phényle ou imidazolyle; et $X_5$ représente un atome d'hydrogène ou un groupe alkyle inférieur; ou $X_4$ et $X_5$ forment ensemble un radical alkylène en $C_2$-$C_6$, tel qu'alkylène en $C_4$-$C_5$, ou $X_2$ représente un radical cycloalkylène en $C_3$-$C_7$, tel que cycloalkylène en $C_5$-$C_6$; $R_2$ représente un radical carboxy, (alcoxy inférieur)-carbonyle, phényl-(alcoxy inférieur)-carbonyle, (alcoxy inférieur)-(alcoxy inférieur)-carbonyle, hydroxy, alcoxy inférieur, phényl-alcoxy inférieur, phénoxy, amino, alcanoylamino inférieur, phényl-alcanoylamino inférieur ou (alcane inférieur)-sulfonylamino; et les variables $R_1$, $X_1$ et $R_3$ ont les significations indiquées respectivement dans l'une des revendications 1 à 7; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**15.** Composé selon la revendication 1, de formule Ia, dans lequel $R_1$ représente un radical alkyle inférieur, tel qu'un radical alkyle en $C_3$-$C_5$, ou un radical alcényle inférieur, tel qu'un radical alcényle en $C_3$-$C_5$; $X_1$ représente CO ou $SO_2$; $X_2$ représente le groupe de formule Ib, dans lequel p et r représentent 0 ou 1, et q représente 1; $X_4$ représente un radical alkyle inférieur, tel qu'un radical alkyle en $C_1$-$C_4$, ou phényle, éventuellement substitué par un groupe hydroxy, cycloalkyle à 3-7 chaînons tel que cyclohexyle, par un groupe phényle ou imidazolyle, tel que 4-imidazolyle, éventuellement substitué par un atome d'halogène ou par le groupe hydroxy; et $X_5$ représente un atome d'hydrogène ou un groupe alkyle inférieur, tel qu'un groupe alkyle en $C_1$-$C_4$; ou $X_4$ et $X_5$ représentent ensemble un groupe alkylène en $C_2$-$C_6$, tel qu'un groupe alkylène en $C_4$-$C_5$; ou $X_2$ représente un radical cycloalkylène en $C_3$-$C_7$, tel qu'un radical cycloalkylène en $C_5$-$C_6$,; $R_2$ représente un radical carboxy, (alcoxy inférieur)-carbonyle tel qu'un groupe alcoxy($C_2$-$C_5$)-carbonyle, phényl-(alcoxy inférieur)-carbonyle, tel que phényl-alcoxy-($C_1$-$C_4$)-carbonyle, un radical (alcoxy inférieur)-(alcoxy inférieur)-carbonyle, tel qu'un radical alcoxy($C_1$-$C_4$)-alcoxy($C_2$-$C_5$)-carbonyle, un groupe hydroxy ou alcoxy inférieur, tel qu'un groupe alcoxy en $C_1$-$C_4$; et $R_3$ représente le groupe carboxy ou 5-tétrazolyle; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**16.** Composé selon la revendication 1, de formule Ia, dans lequel $R_1$ représente un radical alkyle inférieur, tel qu'alkyle en $C_3$-$C_5$, ou alcényle inférieur, tel qu'alcényle en $C_3$-$C_5$; $X_1$ représente CO ou $SO_2$; $X_2$ représente le groupe de

formule Ib, dans lequel p et r représentent 0 ou 1 et q représente 1; $X_4$ représente un radical alkyle inférieur, tel qu'un radical alkyle en $C_1$-$C_4$, ou phényle, éventuellement substitué par un groupe hydroxy, cycloalkyle à 3-7 chaînons, phényle ou imidazolyle, tel que 4-imidazolyle, éventuellement substitué par un atome d'halogène ou par le groupe hydroxy; et $X_5$ représente un atome d'hydrogène ou un groupe alkyle inférieur, tel qu'un groupe alkyle en $C_1$-$C_4$; $R_2$ représente un groupe carboxy, (alcoxy inférieur)-carbonyle, tel qu'un groupe alcoxy($C_2$-$C_5$)-carbonyle, phényl-(alcoxy inférieur)-carbonyle, tel que phényl-alcoxy($C_1$-$C_4$)-carbonyle, (alcoxy inférieur)-(alcoxy inférieur)-carbonyle tel qu'un groupe alcoxy($C_1$-$C_4$)-alcoxy-($C_2$-$C_5$)-carbonyle, un groupe hydroxy ou alcoxy inférieur tel qu'un groupe alcoxy en $C_1$-$C_4$; et $R_3$ représente le groupe carboxy ou 5-tétrazolyle; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**17.** Composé selon la revendication 1, de formule Ia, dans lequel $R_1$ représente un radical alkyle inférieur, tel qu'un radical alkyle en $C_3$-$C_5$, ou bien un radical alcényle inférieur, tel qu'alcényle en $C_3$-$C_5$; $X_1$ représente CO ou $SO_2$; $X_2$ représente le groupe de formule Ib dans lequel p représente un nombre entier allant de 1 à 8 et q ainsi que r représentent 0; $R_2$ représente un groupe hydroxy, alcoxy inférieur tel qu'alcoxy en $C_1$-$C_4$, phényl-alcoxy inférieur tel que phényl-alcoxy($C_1$-$C_4$), phénoxy, un groupe alcanoyl-amino inférieur, tel qu'alcanoyl($C_1$-$C_4$)-amino, un groupe phényl-(alcanoyl inférieur)-amino, tel que phényl-alcanoyl($C_1$-$C_4$)-amino, un groupe (alcane inférieur)-sulfonylamino, tel qu'un groupe alcane($C_1$-$C_4$)-sulfonylamino; et $R_3$ représente le groupe carboxy ou, en premier lieu, 5-tétrazolyle; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**18.** Composé selon la revendication 1, de formule Ia, dans lequel $R_1$ représente un radical alkyle en $C_3$-$C_5$ ou, en second lieu, un radical alcényle en $C_3$-$C_5$; $X_1$ représente CO ou $SO_2$; $X_2$ représente un groupe de formule Ib dans lequel p et r représentent, indépendamment l'un de l'autre, 0 ou 1, et q représente 1; $X_4$ représente un groupe alkyle en $C_1$-$C_4$, hydroxyalkyle en $C_1$-$C_4$, cycloalkyl($C_3$-$C_7$)-alkyle($C_1$-$C_4$), phényl-alkyle($C_1$-$C_4$), ou imidazolyl-alkyle($C_1$-$C_4$); et $X_5$ représente un atome d'hydrogène ou un groupe alkyle en $C_1$-$C_4$; ou $X_4$ et $X_5$ représentent ensemble le groupe tétraméthylène ou pentaméthylène; $R_2$ représente un groupe carboxy ou alcoxy-($C_2$-$C_5$)-carbonyle, ainsi qu'un groupe phényl-alcoxy($C_1$-$C_4$)-carbonyle; et $R_3$ représente le groupe carboxy ou 5-tétrazolyle, sous forme libre ou sous forme de sel.

**19.** Composé selon la revendication 1, de formule Ia, dans lequel $R_1$ représente un groupe alkyle en $C_3$-$C_5$ ou, en second lieu, un groupe alcényle en $C_3$-$C_5$; $X_1$ représente CO ou $SO_2$; $X_2$ représence un groupe de formule Ib dans lequel p et r représentent chacun 0 ou 1 et q représente 1; $X_4$ représente un groupe alkyle en $C_1$-$C_4$, hydroxyalkyle en $C_1$-$C_4$, cycloalkyl($C_3$-$C_7$)-alkyle($C_1$-$C_4$), phényl-alkyle($C_1$-$C_4$), ou imidazolyl-alkyle($C_1$-$C_4$); et $X_5$ représente un atome d'hydrogène; $R_2$ représente un groupe carboxy ou alcoxy($C_2$-$C_5$)-carbonyle, ou un groupe phényl-alcoxy($C_1$-$C_4$)-carbonyle; et $R_3$ représente le groupe carboxy ou 5-tétrazolyle, sous forme libre ou sous forme de sel.

**20.** Composé selon la revendication 1, de formule Ia, dans lequel $R_1$ représente un groupe alkyle en $C_3$-$C_5$; $X_1$ représente CO; $X_2$ représente un groupe de formule Ib dans lequel q et r représentent 0 et p représente 1 à 3, ou dans lequel p et q représentent 1 et r représente 0; $X_4$ représente un groupe alkyle en $C_1$-$C_4$; $X_5$ représente un atome d'hydrogène ou un groupe alkyle en $C_1$-$C_4$; $R_2$ représente un groupe carboxy, alcoxy($C_2$-$C_5$)-carbonyle; et $R_3$ représente le groupe carboxy ou 5-tétrazolyle, sous forme libre ou sous forme de sel.

**21.** Composé selon l'une des revendications 1 à 20, dans lequel $R_3$ représente le groupe 5-tétrazolyle, sous forme libre ou sous forme de sel.

**22.** Composé selon la revendication 1, de formule Ia, dans lequel $R_1$ représente un groupe alkyle en $C_3$-$C_5$; $X_1$ représente CO; $X_2$ représente un groupe de formule Ib dans lequel p représente 0 ou 1, r représente 0 et q représente 1; $X_4$ représente un groupe alkyle en $C_1$-$C_4$; et $X_5$ représente un atome d'hydrogène ou un groupe alkyle en $C_1$-$C_4$; ou $X_4$ et $X_5$ représentent ensemble le groupe tétraméthylène ou pentaméthylène; $R_2$ représente un groupe carboxy ou alcoxy($C_2$-$C_5$)-carbonyle; et $R_3$ représente le groupe 5-tétrazolyle, sous forme libre ou sous forme de sel.

**23.** Composé selon la revendication 1, de formule Ia, dans lequel $R_1$ représente un groupe alkyle en $C_3$-$C_5$; $X_1$ représente CO; $X_2$ représente un groupe de formule Ib dans lequel p représente 0 ou 1, r représente 0 et q représente 1; $X_4$ et $X_5$ représentent ensemble le groupe tétraméthylène ou pentaméthylène; $R_2$ représente un groupe carboxy ou alcoxy($C_2$-$C_5$)-carbonyle; et $R_3$ représente le groupe tétrazolyle, sous forme libre ou sous forme de sel.

**24.** Composé selon la revendication 1, de formule Ia, dans lequel $R_1$ représente un groupe alkyle en $C_3$-$C_5$; $X_1$ représente CO; $X_2$ représente un groupe de formule Ib dans lequel p et r représentent 0 ou 1 et q représente 1: $X_4$ représente un groupe alkyle en $C_1$-$C_4$; et $X_5$ représente un atome d'hydrogène; $R_2$ représente un groupe carboxy ou alcoxy($C_2$-$C_5$)-carbonyle; et $R_3$ représente le groupe 5-tétrazolyle, sous forme libre ou sous forme de sel.

**25.** Composé de formule Ia selon l'une des revendications 13 à 24, dans lequel $X_2$ représente un groupe de formule Ib, q représente 1 et $X_4$ et $X_5$ ont des significations différentes, sous forme libre ou sous forme de sel, et dans lequel l'atome de carbone asymétrique concerné, portant les variables $X_4$ et $X_5$, a la configuration S.

**26.** (S)-N-(1-carboxy-2-méthylprop-1-yl)-N-pentanoyl-N-[2'-(1H-tétrazol-5-yl)biphényl-4-ylméthyl]amine, sous forme libre ou sous forme de sel, selon la revendication 1.

**27.** N-(2-carboxy-2,2-tétraméthylène-éthyl)-N-pentanoyl-N-[2'-(1H-tétrazol-5-yl)biphényl-4-ylméthyl]amine, sous forme libre ou sous forme de sel, selon la revendication 1.

**28.** N-[2-carboxy-2-éthylbut-1-yl)-N-pentanoyl-N-[2'-(1H-tétrazol-5-yl)biphényl-4-ylméthyl]amine, sous forme libre ou sous forme de sel, selon la revendication 1.

**29.** (S)-N-(1-carboxy-2-méthylprop-1-yl)-N-éthoxycarbonyl-N-[2'-(1H-tétrazol-5-yl)biphényl-4-ylméthyl]amine, sous forme libre ou sous forme de sel, selon la revendication 1.

**30.** N-(1-carboxycyclohexyl)-N-pentanoyl-N-[2'-(1H-tétrazol-5-yl)biphényl-4-ylméthyl]amine, sous forme libre ou sous forme de sel, selon la revendication 1.

**31.** Composé selon la revendication 1, choisi parmi les suivants:

(S)-N-(1-carboxyéthyl)-N-pentanoyl-N-[2'-(1H-tétrazol-5-yl)biphényl-4-ylméthyl]amine,
N-(2-hydroxyéthyl)-N-pentanoyl-N-[2'-(1H-tétrazol-5-yl)biphényl-4-ylméthyl]amine,
N-(2-éthoxycarbonyl-2,2-tétraméthylène-éthyl)-N-pentanoyl-N-[2'-(1H-tétrazol-5-yl)biphényl-4-ylméthyl]amine,
N-(2-éthoxycarbonyl-2-éthylbut-1-yl)-N-pentanoyl-N-[2'-(1H-tétrazol-5-yl)biphényl-4-ylméthyl]amine,
N-(2-éthoxycarbonyl-2-méthylprop-1-yl)-N-pentanoyl-N-[2'-(1H-tétrazol-5-yl)biphényl-4-ylméthyl]amine,
(S)-N-(1-hydroxyméthyl-2-méthylprop-1-yl)-N-pentanoyl-N-[2'-1H-tétrazol-5-yl)biphényl-4-ylméthyl]amine,
N-(2-éthoxycarbonyl-2,2-pentaméthylène-éthyl)-N-pentanoyl-N-[2'-(1H-tétrazol-5-yl)biphényl-4-ylméthyl]amine,
(S)-N-(1-carboxy-2-méthylprop-1-yl)-N-propyloxycarbonyl-N-[2'-(1H-tétrazol-5-yl)biphényl-4-ylméthyl]amine,
N-(2-carboxy-2-méthylpropyl)-N-pentanoyl-N-[2'-(1H-tétrazol-5-yl)biphényl-4-ylméthyl]amine,
N-(2-carboxy-2,2-pentaméthylène-éthyl)-N-pentanoyl-N-[2'-(1H-tétrazol-5-yl)biphényl-4-ylméthyl]amine,
(S)-N-(1-aminocarbonyl-2-méthylprop-1-yl)-N-pentanoyl-N-[2'-(1H-tétrazol-5-yl)biphényl-4-ylméthyl]amine et
(S)-N-(1-carboxy-2-méthylprop-1-yl)-N-5-oxopent-N-5-yl)-N-[2'-(1H-tétrazol-5-yl)biphényl-4-ylméthyl]amine,
chacun sous forme libre ou sous forme de sel.

**32.** Composé selon la revendication 1, choisi parmi les suivants:

N-carboxyméthyl-N-pentanoyl-N-[2'-(1H-tétrazol-5-yl)biphényl-4-ylméthyl]amine,
(S)-N-(1-méthoxycarbonyléthyl)-N-pentanoyl-N-[2'-(1H-tétrazol-5-yl)biphényl-4-ylméthyl]amine,
N-[1-carboxy-2-(4-fluorophényl)éthyl]-N-pentanoyl-N-[2'-(1H-tétrazol-5-yl)biphényl-4-ylméthyl]amine,
N-[2-(4-fluorophényl)-1-méthoxycarbonyléthyl]-N-pentanoyl-N-[2'-(1H-tétrazol-5-yl)biphényl-4-ylméthyl]amine,
N-[2-(4-fluorophényl)-1-hydroxyméthyléthyl]-N-pentanoyl-N-[2'-(1H-tétrazol-5-yl)biphényl-4-ylméthyl]amine,
N-(2'-carboxybiphényl-4-ylméthyl)-N-[1-carboxy-2-(4-fluorophényl)éthyl]-N-pentanoylamine,
N-(2'-carboxybiphényl-4-ylméthyl)-N-[2-(4-fluorophényl)-1-méthoxycarbonyléthyl]-N-pentanoylamine,
(S)-N-(2'-carboxybiphényl-4-ylméthyl)-N-(1-hydroxyméthyl-2-phényléthyl)-N-pentanoylamine,
(S)-N-(2'-carboxybiphényl-4-ylméthyl)-N-(1-hydroxyméthyl-2-imidazol-4-yléthyl)-N-pentanoylamine,
(R)-N-(1-carboxyéthyl)-N-pentanoyl-N-[2'-(1H-tétrazol-5-yl)biphényl-4-ylméthyl]amine,
(1S),(2S)-N-(1-carboxy-2-méthylbut-1-yl)-N-pentanoyl-N-[2'-(1H-tétrazol-5-yl)biphényl-4-ylméthyl]amine,
(1S),(2S)-N-(1-méthoxycarbonyl-2-méthylbut-1-yl)-N-pentanoyl-N-[2'-(1H-tétrazol-5-yl)biphényl-4-ylméthyl]-amine,
(S)-N-(1-carboxybut-1-yl)-N-pentanoyl-N-[2'-(1H-tétrazol-5-yl)biphényl-4-ylméthyl]amine,

(S)-N-(1-méthoxycarbonylbut-1-yl)-N-pentanoyl-N-[2'-(1H-tétrazol-5-yl)biphényl-4-ylméthyl]amine,
(S)-N-(1-carboxyéthyl)-N-hexanoyl-N-[2'-(1H-tétrazol-5-yl)-biphényl-4-ylméthyl]amine,
(S)-N-butanoyl-N-(1-carboxyéthyl-N-[2'-(1H-tétrazol-5-yl)biphényl-4-ylméthyl]amine,
(S)-N-(1-carboxyprop-1-yl)-N-pentanoyl-N-[2'-(1H-tétrazol-5-yl)-biphényl-4-ylméthyl]amine,
(S)-N-(1-carboxy-2-cyclohexyléthyl)-N-pentanoyl-N-[2'-(1H-tétrazol-5-yl)-biphényl-4-ylméthyl]amine,
(S)-N-(2-cyclohexyl-1-méthoxycarbonyléthyl)-N-pentanoyl-N-[2'-(1H-tétrazol-5-yl)-biphényl-4-ylméthyl]amine,
(R)-N-(1-carboxy-2-méthylprop-1-yl)-N-pentanoyl-N-[2'-(1H-tétrazol-5-yl)-biphényl-4-ylméthyl]amine,
N-(2-méthoxyéthyl)-N-pentanoyl-N-[2'-1H-tétrazol-5-yl)biphényl-4-ylméthyl]amine,
N-(2-benzyloxyéthyl)-N-pentanoyl-N-[2'-1H-tétrazol-5-yl)biphényl-4-ylméthyl]amine,
N-(3-méthoxyprop-1-yl)-N-pentanoyl-N-[2'-1H-tétrazol-5-yl)biphényl-4-ylméthyl]amine,
N-(3-benzyloxyprop-1-yl)-N-pentanoyl-N-[2'-1H-tétrazol-5-yl)-biphényl-4-ylméthyl]amine,
N-(3-hydroxyprop-1-yl)-N-pentanoyl-N-[2'-1H-tétrazol-5-yl)biphényl-4-ylméthyl]amine,
N-(1-méthoxycarbonyl-1-méthyléthyl)-N-pentanoyl-N-[2'-1H-tétrazol-5-yl)-biphényl-4-ylméthyl]amine,
N-(2-carboxyéthyl)-N-pentanoyl-N-[2'-(1H-tétrazol-5-yl)biphényl-4-ylméthyl]amine,
N-(2-carboxyprop-1-yl)-N-pentanoyl-N-[2'-(1H-tétrazol-5-yl)biphényl-4-ylméthyl]amine,
N-(1-carboxy-1-méthyléthyl)-N-pentanoyl-N-[2'-(1H-tétrazol-5-yl)biphényl-4-ylméthyl]amine,
N-(5-hydroxypent-1-yl)-N-pentanoyl-N-[2'-(1H-tétrazol-5-yl)biphényl-4-ylméthyl]amine,
N-(1-carboxyprop-2-yl)-N-pentanoyl-N-[2'-(1H-tétrazol-5-yl)biphényl-4-ylméthyl]amine,
N-(2-éthoxycarbonyl-3-méthylbut-1-yl)-N-pentanoyl-N-[2'-(1H-tétrazol-5-yl)biphényl-4-ylméthyl)amine,
N-(2-carboxy-3-méthylbut-1-yl)-N-pentanoyl-N-[2'-(1H-tétrazol-5-yl)biphényl-4-ylméthyl]amine,
N-(3-phénoxyprop-1-yl)-N-pentanoyl-N-[2'-(1H-tétrazol-5-yl)biphényl-4-ylméthyl]amine,
N-[2-(4-hydroxyphényl)éthyl]-N-pentanoyl-N-[2'-(1H-tétrazol-5-yl)biphényl-4-ylméthyl]amine,
N-[3-(4-hydroxyphényl)prop-1-yl]-N-pentanoyl-N-[2'-(1H-tétrazol-5-yl)biphényl-4-ylméthyl]amine,
N-[8-hydroxyoct-1-yl)-N-pentanoyl-N-[2'-(1H-tétrazol-5-yl)biphényl-4-ylméthyl]amine,
N-[2-méthanesulfonylaminoéthyl)-N-pentanoyl-N-[2'-(1H-tétrazol-5-yl)biphényl-4-ylméthyl]amine,
N-(3-acétylaminoprop-1-yl)-N-pentanoyl-N-[2'-(1H-tétrazol-5-yl)biphényl-4-ylméthyl]amine,
N-(2-méthoxy-2-oxo-1-phényléthyl)-N-pentanoyl-N-[2'-(1H-tétrazol-5-yl)biphényl-4-ylméthyl]amine,
N-(4-hydroxybut-2-yl)-N-pentanoyl-N-[2'-(1H-tétrazol-5-yl)biphényl-4-ylméthyl]amine,
N-(2-hydroxy-1-phényléthyl)-N-pentanoyl-N-[2'-(1H-tétrazol-5-yl)biphényl-4-ylméthyl]amine,
N-[3-(4-hydroxybenzylcarbonylamino)prop-1-yl]-N-pentanoyl-N-[2'-(1H-tétrazol-5-yl)biphényl-4-ylméthyl]amine,
N-(3-éthoxycarbonylcyclohexyl)-N-pentanoyl-N-[2'-(1H-tétrazol-5-yl)biphényl-4-ylméthyl]amine,
N-(3-carboxycyclohexyl)-N-pentanoyl-N-[2'-(1H-tétrazol-5-yl)biphényl-4-ylméthyl]amine,
*cis*-N-(4-carboxycyclohexyl)-N-pentanoyl-N-[2'-(1H-tétrazol-5-yl)biphényl-4-ylméthyl]amine,
*cis*-N-(2-éthoxycarbonylcyclohexyl)-N-pentanoyl-N-[2'-(1H-tétrazol-5-yl)biphényl-4-ylméthyl]amine,
*cis*-N-(2-carboxycyclohexyl)-N-pentanoyl-N-[2'-(1H-tétrazol-5-yl)biphényl-4-ylméthyl]amine,
N-[2-[2-(4-hydroxyphényl)éthylaminocarbonyl]-2,2-tétraméthylène-éthyl)-N-pentanoyl-N-[2'-(1H-tétrazol-5-yl)biphényl-4-ylméthyl]amine,
(S)-N-(1-[2-(4-hydroxyphényl)éthylaminocarbonyl]-2-méthylprop-1-yl)]-N-pentanoyl-N-[2'-(1H-tétrazol-5-yl)biphényl-4-ylméthyl]amine,
(S)-N-(1-carboxy-2,2-diméthylprop-1-yl)-N-pentanoyl-N-[2'-(1H-tétrazol-5-yl)biphényl-4-ylméthyl]amine,
(S)-N-(1-méthoxycarbonyl-2-méthylprop-1-yl)-N-pentanoyl-N-[2'-(1H-tétrazol-5-yl)biphényl-4-ylméthyl]amine,
N-(4-phénoxybut-1-yl)-N-pentanoyl-N-[2'-(1H-tétrazol-5-yl)biphényl-4-ylméthyl]amine,
N-(2-hydroxy-1-phényl-2-oxoéthyl)-N-pentanoyl-N-[2'-(1H-tétrazol-5-yl)biphényl-4-ylméthyl]amine,
(S)-N-(1-benzyloxycarbonyl-2-méthylprop-1-yl)-N-pentanoyl-N-[2'-(1H-tétrazol-5-yl)biphényl-4-ylméthyl]amine,
N-butanoyl-N-(1-carboxy-1-méthyléthyl)-N-[2'-(1H-tétrazol-5-yl)blphényl-4-ylméthyl]amine,
N-(4-hydroxybut-1-yl)-N-pentanoyl-N-[2'-(1H-tétrazol-5-yl)biphényl-4-ylméthyl]amine,
(S)-N-(1-benzyloxycarbonyl-2-méthylprop-1-yl)-N-[3-bromo-2'-(1H-tétrazol-5-yl)biphényl-4-ylméthyl]-N-pentanoylamine,
(S)-N-[3-bromo-2'-(1H-tétrazol-5-yl)biphényl-4-ylméthyl]-N-(1-carboxy-2-méthylprop-1-yl)-N-pentanoylamine,
N-(2-acétylaminoéthyl)-N-pentanoyl-N-[2'-(1H-tétrazol-5-yl)biphényl-4-ylméthyl]amine,
N-[2-(n-butoxycarbonyl)-2,2-tétraméthylène-éthyl]-N-pentanoyl-N-[2'-(1H-tétrazol-5-yl)biphényl-4-ylméthyl]amine,
N-(2-benzylaminocarbonyl-2,2-tétraméthylène-éthyl)-N-pentanoyl-N-[2'-(1H-tétrazol-5-yl)biphényl-4-ylméthyl]amine,

(S)-N-butyloxycarbonyl-N-(1-carboxy-2-méthylprop-1-yl)-N-[2'-(1H-tétrazol-5-yl)biphényl-4-ylméthyl]amine,
(S)-N-(1-carboxy-2-méthylprop-1-yl)-N-méthoxycarbonyl-N-[2'-(1H-tétrazol-5-yl)biphényl-4-ylméthyl]amine,
N-(2-diéthylaminocarbonyl-2,2-tétraméthylène-éthyl)-N-pentanoyl-N-[2'-(1H-tétrazol-5-yl)biphényl-4-ylméthyl]amine,
N-(2-méthyl-2-morpholine-4-ylcarbonylpropyl)-N-pentanoyl-N-[2'-(1H-tétrazol-5-yl)biphényl-4-ylméthyl]amine,
N-(1-carboxycyclopentyl)-N-pentanoyl-N-[2'-(1H-tétrazol-5-yl)biphényl-4-ylméthyl]amine,
N-(1-carboxy-1-éthylprop-1-yl)-N-pentanoyl-N-[2'-(1H-tétrazol-5-yl-)biphényl-4-ylméthyl]amine,
(S)-N-(5-amino-1-carboxypent-1-yl)-N-pentanoyl-N-[2'-(1H-tétrazol-5-yl)biphényl-4-ylméthyl]amine,
N-butanesulfonyl-N-(2-éthoxycarbonyl-2,2-pentaméthylène-éthyl)-N-[2'-(1H-tétrazol-5-yl)biphényl-4-ylméthyl]amine,
N-butanesulfonyl-N-(2-carboxy-2,2-pentaméthylène-éthyl)-N-[2'-(1H-tétrazol-5-yl)biphényl-4-ylméthyl]amine,
N-butanesulfonyl-N-(2-éthoxycarbonyl-2-méthylprop-1-yl)-N-[2'-(1H-tétrazol-5-yl)biphényl-4-ylméthyl]amine,
N-butanesulfonyl-N-(2-carboxy-2-méthylprop-1-yl)-N-[2'-(1H-tétrazol-5-yl)biphényl-4-ylméthyl]amine,
(S)-N-butanesulfonyl-N-(1-tert-butoxycarbonyléthyl)-N-[2'-(1H-tétrazol-5-yl)biphényl-4-ylméthyl]amine,
(S)-N-butanesulfonyl-N-(1-carboxyéthyl)-N-[2'-(1H-tétrazol-5-yl)biphényl-4-ylméthyl]amine,
(S)-N-butanesulfonyl-N-(1-carboxy-2-méthylprop-1-yl)-N-[2'-(1H-tétrazol-5-yl)biphényl-4-ylméthyl]amine,
(S)-N-(2-méthyl-1-méthylaminocarbonylprop-1-yl)-N-pentanoyl-N-[2'-(1H-tétrazol-5-yl)biphényl-4-ylméthyl]amine,
(S)-N-(1-diméthylaminocarbonyl-2-méthylprop-1-yl)-N-pentanoyl-N-[2'-(1H-tétrazol-5-yl)biphényl-4-ylméthyl]amine,
(2)-N-(2-méthyl-1-morpholine-4-ylcarbonylprop-1-yl)-N-pentanoyl-N-[2'-(1H-tétrazol-5-yl)biphényl-4-ylméthyl]amine,
(S)-N-(2'-carboxybiphényl-4-ylméthyl)-N-(1-carboxy-2-méthyl-prop-1-yl)-N-pentanoylamine,
(S)-N-(1,2-dicarboxyéthyl)-N-pentanoyl-N-[2'-(1H-tétrazol-5-yl)biphényl-4-ylméthyl]amine,
(S)-N-(1-carboxy-3-phénylprop-1-yl)-N-pentanoyl-N-[2'-(1H-tétrazol-5-yl)biphényl-4-ylméthyl]amine,
(S)-N-(2-cyclohexyl-1-hydroxyméthyl-éthyl)-N-pentanoyl-N-[2'-(1H-tétrazol-5-yl)biphényl-4-ylméthyl]amine,
(R)-N-(1-méthoxycarbonyl-2-méthylprop-1-yl)-N-pentanoyl-N-[2'-(1H-tétrazol-5-yl)biphényl-4-ylméthyl]amine,
(S)-N-(2-hydroxy-1-méthoxycarbonyl-éthyl)-N-pentanoyl-N-[2'-(1H-tétrazol-5-yl)biphényl-4-ylméthyl]amine,
N-pentanoyl-N-(1H-tétrazol-5-ylméthyl)-N-[2'-(1H-tétrazol-5-yl)biphényl-4-ylméthyl]amine,
N-pentanoyl-N-pyrid-3-ylméthyl)-N-[2'-(1H-tétrazol-5-yl)biphényl-4-ylméthyl]amine,
(S)-N-(1-carboxy-4-guanidinobut-1-yl)-N-pentanoyl-N-[2'-(1H-tétràzol-5-yl)biphényl-4-ylméthyl]amine,
N-(2-hydroxy-1-méthoxycarbonylprop-1-yl)-N-pentanoyl-N-[2'-(1H-tétrazol-5-yl)biphényl-4-ylméthyl]amine,
N-(1-benzyloxycarbonyl-1-méthyléthyl)-N-butanoyl-N-[2'-(1H-tétrazol-5-yl)biphényl-4-ylméthyl]amine,
(S)-N-(1-carboxy-3-méthylbut-1-yl)-N-pentanoyl-N-[2'-(1H-tétrazol-5-yl)biphényl-4-ylméthyl]amine,
N-(1-carboxy-2-hydroxyéthyl)-N-pentanoyl-N-[2'-(1H-tétrazol-5-yl)biphényl-4-ylméthyl]amine,
(S)-N-(1-carboxy-2-hydroxyéthyl)-N-pentanoyl-N-[2'-(1H-tétrazol-5-yl)biphényl-4-ylméthyl]amine,
(S)-N-(2-méthyl-1-(2-phényléthylaminocarbonyl)prop-1-yl]-N-pentanoyl-N-[2'-(1H-tétrazol-5-yl)biphényl-4-ylméthyl]amine,
(S)-N-(2-benzyloxy-1-hydroxyméthyléthyl)-N-pentanoyl-N-[2'-(1H-tétrazol-5-yl)biphényl-4-ylméthyl]amine,
(S)-N-(1-carboxy-2-méthylprop-1-yl)-N-pentanoyl-N-[2'-(1H-tétrazol-5-yl)biphényl-3-ylméthyl]amine,
(S)-N-(1-carboxy-2-méthylprop-1-yl)-N-pentanoyl-N-[3'-(1H-tétrazol-5-yl)biphényl-4-ylméthyl]amine,
(S)-N-[2-méthyl-1-(1,2,3,4-tétrahydroquinol-1-ylcarbonyl)prop-1-yl]-N-pentanoyl-N-[2'-(1H-tétrazol-5-yl)biphényl-4-ylméthyl]amine,
(S)-N-(2-méthyl-1-pipéridine-1-ylcarbonylprop-1-yl)-N-pentanoyl-N-[2'-(1H-tétrazol-5-yl)biphényl-4-ylméthyl]amine,
(2)-N-[2-méthyl-1-(1,2,3,4-tétrahydroisoquinol-2-yl-carbonyl)prop-1-yl]-N-pentanoyl-N-[2'-(1H-tétrazol-5-yl)biphényl-4-ylméthyl]amine,
N-(2-hydroxyméthyl-2-méthylprop-1-yl)-N-pentanoyl-N-[2'-(1H-tétrazol-5-yl)biphényl-4-ylméthyl]amine,
N-éthoxycarbonyl-N-(2-éthoxycarbonyl-2-méthylprop-1-yl)-N-[2'-(1H-tétrazol-5-yl)biphényl-4-ylméthyl]amine et
N-(2-carboxy-2-méthylprop-1-yl)-N-éthoxycarbonyl-N-[2'-(1H-tétrazol-5-yl)biphényl-4-ylméthyl]amine,
chacun sous forme libre ou sous forme de sel.

**33.** Composé selon l'une des revendications 1 à 32, sous forme libre ou sous forme d'un sel pharmaceutiquement utilisable, pour utilisation dans un procédé destiné au traitement thérapeutique de l'organisme humain ou animal.

**34.** Composé selon l'une des revendications 1 à 33, sous forme libre ou sous forme d'un sel pharmaceutiquement utilisable, pour utilisation en tant qu'antihypertenseur.

**35.** Composition pharmaceutique, contenant en tant que substance active un composé selon l'une des revendications 1 à 34, sous forme libre ou sous forme d'un sel pharmaceutiquement utilisable, éventuellement en plus d'adjuvants pharmaceutiques usuels.

**36.** Composition pharmaceutique à effet antihypertenseur selon la revendication 35, caractérisée en ce que l'on choisit une substance active à effet antihypertenseur.

**37.** Procédé pour la préparation d'un composé de formule

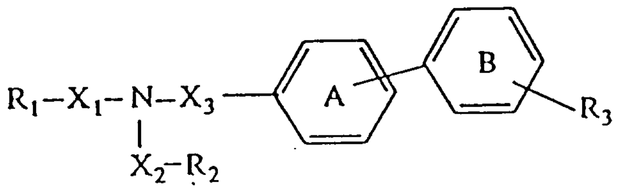

(I) · ou

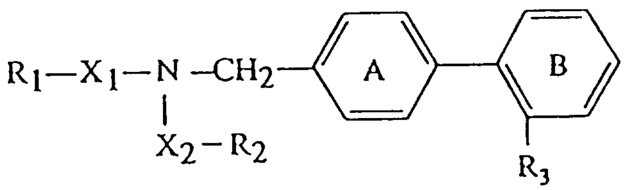

(Ia),

formules dans lesquelles $R_1$, $R_2$, $R_3$, $X_1$, $X_2$ et $X_3$ ainsi que les substituants des cyles A et B ont les significations indiquées chacune dans l'une des revendications 1 à 25, sous forme libre ou sous forme de sel, caractérisé en ce que

a) dans un composé de formule

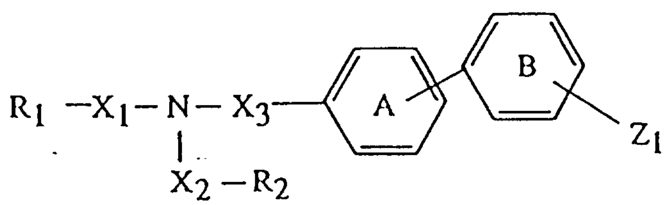

(II)

ou un sel de celui-ci, dans lequel $Z_1$ représente un radical pouvant être converti en $R_3$, on convertit $Z_1$ en $R_3$, ou
b) on fait réagir un composé de formule $R_1$-$X_1$OH (IIIa), un dérivé réactif de celui-ci ou un sel de celui-ci, avec un composé de formule

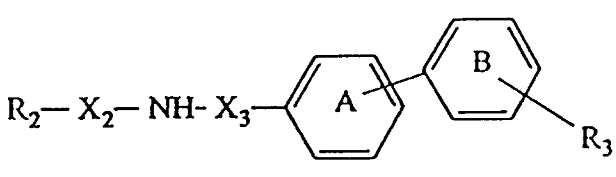

(IIIb)

ou un sel de celui-ci, et, si on le désire, on convertit dans chaque cas un composé I sous forme libre ou sous forme de sel, pouvant être obtenu conformément au procédé ou d'une autre façon, en un autre composé I, on scinde un mélange d'isomères, pouvant être obtenu conformément au procédé, et on isole les isomères recherchés, et/ou on convertit un composé I libre, pouvant être obtenu conformément au procédé, en un sel, ou on convertit un sel d'un composé I, pouvant être obtenu conformément au procédé, en le composé I libre ou en un autre sel.

**38.** Procédé pour la préparation d'une composition pharmaceutique selon la revendication 35 ou 36, caractérisé en ce que l'on met la substance active, éventuellement avec addition d'adjuvants pharmaceutiques usuels, sous forme d'une composition pharmaceutique.

**39.** Procédé selon la revendication 38, pour la préparation d'une composition pharmaceutique à effet antihypertenseur selon la revendication 34, caractérisé en ce que l'on choisit une substance active à effet antihypertenseur.

**40.** Utilisation d'un composé selon l'une des revendications 1 à 34, sous forme libre ou sous forme d'un sel pharmaceutiquement utilisable, pour la préparation d'une composition pharmaceutique.

**41.** Utilisation d'un composé selon l'une des revendications 1 à 34, sous forme libre ou sous forme d'un sel pharmaceutiquement utilisable, pour la préparation d'une composition pharmaceutique par voie non chimique.

**42.** Utilisation d'un composé selon l'une des revendications 1 à 34, sous forme libre ou sous forme d'un sel pharmaceutiquement utilisable, pour la préparation d'un agent antihypertenseur.

**43.** Utilisation d'un composé selon l'une des revendications 1 à 34, sous forme libre ou sous forme d'un sel pharmaceutiquement utilisable, pour la préparation d'une composition pharmaceutique destinée au traitement thérapeutique ou prophylactique de l'insuffisance cardiaque.

**44.** Utilisation d'un composé selon l'une des revendications 1 à 34, sous forme libre ou sous forme d'un sel pharmaceutiquement utilisable, pour la préparation d'une composition pharmaceutique destinée au traitement thérapeutique ou prophylactique de maladies qui sont provoquées par l'activité de l'angiotensine II.

**Revendications pour les Etats contractants suivants : ES, GR**

**1.** Procédé pour la préparation d'un composé de formule

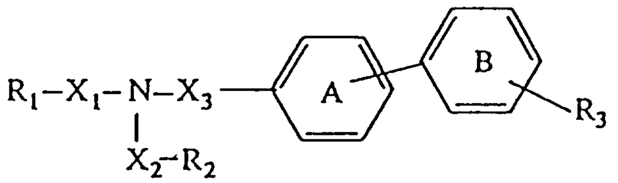

(I),

dans laquelle $R_1$ représente un radical alkyle inférieur, alcényle inférieur ou alcynyle inférieur, éventuellement substitué par un atome d'halogène ou par le groupe hydroxy, ou un radical cycloalkyle en $C_3$-$C_7$ ou cycloalcényle en $C_3$-$C_7$ ou phényl-alkyle inférieur, phényl-alcényle inférieur ou phényl-alcynyle inférieur; $X_1$ représente CO, $SO_2$ ou -O-C(=O)-, l'atome de carbone du groupe carbonyle étant lié à l'atome d'azote indiqué dans la formule I; $X_2$ représente un radical alkylène en $C_1$-$C_{10}$, alkylidène en $C_2$-$C_{10}$ ou cycloalkylène en $C_3$-$C_7$, éventuellement substitué par un groupe hydroxy, carboxy, amino, guanidino, cycloalkyle en $C_3$-$C_7$, cycloalcényle en $C_3$-$C_7$, phényle ou par un groupe aromatique monocyclique et à 5 ou 6 chaînons correspondant, qui comporte jusqu'à 4 hétéroatomes identiques ou différents, un atome de carbone du radical alkylène en $C_1$-$C_{10}$ ou alkylidène en $C_2$-$C_{10}$ pouvant en outre être ponté par un groupe alkylène en $C_2$-$C_6$, et un radical cycloalkyle en $C_3$-$C_7$ ou cycloalcényle en $C_3$-$C_7$ étant éventuellement une ou plusieurs fois substitué par un ou des groupes carboxy, carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, ou par un ou des groupes carbamoyle, carbamoyle dont le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, ou par un ou des groupes formyle, di-(alcoxy inférieur)-méthyle ou oxy-(alkylène-oxy inférieur)-méthylène; $R_2$ représente le groupe carboxy ou un groupe carboxy qui est estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, le groupe carbamoyle ou un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en

$C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, le groupe amino ou un groupe amino qui est monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, un groupe alcanoyle inférieur, phényl-(alcanoyl inférieur)-, benzoyl-, (alcane inférieur)-sulfonyl-, benzènesulfonyl-amino, formyle, di-(alcoxy inférieur)-méthyle, oxy-(alkylène-oxy inférieur)-méthylène, 1H-tétrazol-5-yle, pyridyle, hydroxy, alcoxy inférieur, alcényloxy inférieur, phényl-alcoxy inférieur, phénoxy, $S(O)_m$-R, m étant 0, 1 ou 2 et R représentant un atome d'hydrogène ou un radical alkyle inférieur, alcényle inférieur ou alcynyle inférieur, ou représente un groupe alcanoyle inférieur, sulfamoyle, sulfamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, ou représente $PO_nH_2$, n étant 2 ou 3; $X_3$ représente un groupe alkylène en $C_1$-$C_{10}$ ou alkylidène en $C_2$-$C_{10}$; $R_3$ est le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, étant éventuellement substitués, indépendamment les uns des autres, par des substituants choisis parmi des atomes d'halogène et des groupes hydroxy, alcoxy inférieur, alcényloxy inférieur, phényl-alcoxy inférieur, phénoxy, $S(O)_m$-R et des groupes alkyle inférieur, alcényle inférieur ou alcynyle inférieur éventuellement substitués par un atome d'halogène ou par le groupe hydroxy, les radicaux alkyle inférieur, alcényle inférieur ou alcynyle inférieur étant éventuellement interrompus par -O-, ainsi que, dans le cas de radicaux (hétéro)aromatiques, éventuellement substitués en outre par le groupe carboxy ou un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, par le groupe carbamoyle ou un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O- ou condensés, au niveau de deux atomes de carbone contigus, avec un cycle benzénique, par le groupe formyle ou par un groupe di-(alcoxy inférieur)-méthyle ou oxy(alkylène-oxy inférieur)-méthylène; les radicaux et groupes désignés par "inférieur" contenant jusqu'à 7 atomes de carbone; sous forme libre ou sous forme de sel;
caractérisé en ce que

a) dans un composé de formule

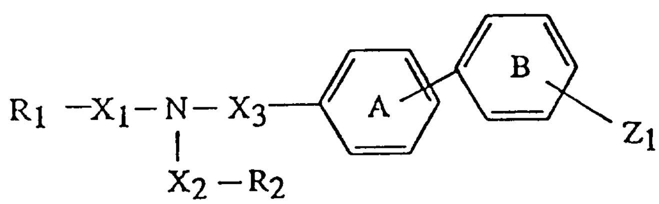
(II)

ou un sel de celui-ci, dans lequel $Z_1$ représente un radical pouvant être converti en $R_3$, on convertit $Z_1$ en $R_3$, ou
b) on fait réagir un composé de formule $R_1$-$X_1$OH (IIIa), un dérivé réactif de celui-ci ou un sel de celui-ci, avec un composé de formule

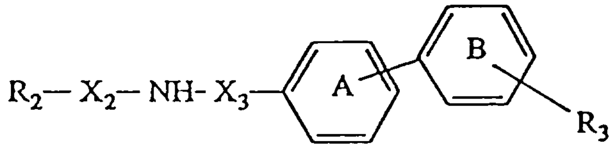
(IIIb)

ou un sel de celui-ci, et, si on le désire, on convertit dans chaque cas un composé I sous forme libre ou sous forme de sel, pouvant être obtenu conformément au procédé ou d'une autre façon, en un autre composé I, on scinde un mélange d'isomères, pouvant être obtenu conformément au procédé, et on isole les isomères recherchés, et/ou on convertit un composé I libre, pouvant être obtenu conformément au procédé, en un sel, ou on convertit un sel d'un composé I, pouvant être obtenu conformément au procédé, en le composé I libre ou en un autre sel.

2. Procédé selon la revendication 1, pour la préparation d'un composé de formule I dans lequel $R_2$ est différent d'un groupe carboxy et $R_3$ représente le groupe 5-tétrazolyle, caractérisé en ce que

(I) on part d'un composé de formule (II) dans lequel $Z_1$ représente le groupe cyano, et on le fait réagir avec $HN_3$ ou un sel alcalin de celui-ci, avec un azide de tri-(alkyl inférieur)-étain ou un azide de triphénylétain; ou
(II) on part d'un composé de formule (II) dans lequel $Z_1$ représente un groupe 5-tétrazolyle protégé par un groupe triphénylméthyle, benzyle éventuellement substitué par le radical nitro, un groupe (alcoxy inférieur)-méthyle, (alkyl inférieur)-thiométhyle, tri-(alkyl inférieur)-silyle, 2-cyanoéthyle, (alcoxy inférieur)-(alcoxy inférieur)-méthyle, benzyloxyméthyle ou phénacyle, et on élimine le groupe protecteur,

et, si on le désire, on convertit un composé de formule I, pouvant être obtenu selon le procédé, dans lequel $R_2$ est différent d'un groupe carboxy et $R_3$ représente le groupe 5-tétrazolyle, en un composé de formule I dans lequel $R_2$ est le groupe carboxy.

3. Procédé selon la revendication 1 ou 2 pour la préparation d'un composé de formule I dans lequel $R_1$ représente un radical alkyle inférieur, alcényle inférieur ou alcynyle inférieur éventuellement substitué par un atome d'halogène ou par le groupe hydroxy, ou un radical cycloalkyle en $C_3$-$C_7$ ou cycloalcényle en $C_3$-$C_7$ ou phényl-alkyle inférieur, phényl-alcényle inférieur ou phényl-alcynyle inférieur; $X_1$ représente CO ou $SO_2$; $X_2$ représente un radical alkylène en $C_1$-$C_{10}$ ou alkylidène en $C_2$-$C_{10}$ ou cycloalkylène en $C_3$-$C_7$, éventuellement substitué par un groupe hydroxy, cycloalkyle en $C_3$-$C_7$ ou cycloalcényle en $C_3$-$C_7$ ou phényle ou par un radical aromatique correspondant, monocyclique à 5 ou 6 chaînons, qui comporte jusqu'à 4 hétéroatomes identiques ou différents, un atome de carbone du radical alkylène en $C_1$-$C_{10}$ ou alkylidène en $C_2$-$C_{10}$ pouvant en outre être ponté par des groupes alkylène en $C_2$-$C_6$, et les groupes cycloalkylène en $C_3$-$C_7$ étant éventuellement substitués une ou plusieurs fois par un ou plusieurs groupes carboxy, carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, ou par le groupe carbamoyle ou un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, le groupe formyle, un groupe di(alcoxy inférieur)-méthyle ou oxy-(alkylène-oxy inférieur)-méthylène; $R_2$ représente le radical carboxy ou un radical carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, ou le radical carbamoyle ou un radical carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, le radical amino ou un radical amino qui est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente un radical (alcanoyl inférieur)-, phényl-(alcanoyl inférieur)-, benzoyl-, (alcane inférieur)-sulfonyl-, benzènesulfonyl-amino, formyle, di-(alcoxy inférieur)-méthyle, oxy-(alkylène-oxy inférieur)-méthylène, hydroxy, alcoxy inférieur, alcényloxy inférieur, phényl-alcoxy inférieur, phénoxy, $S(O)_m$-R, m représentant 0, 1 ou 2 et R représentant un atome d'hydrogène ou un groupe alkyle inférieur, alcényle inférieur ou alcynyle inférieur, un radical alcanoyle inférieur, le radical sulfamoyle ou un radical sulfamoyle dans lequel le fragment amino est monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $c_2$-$C_{10}$ étant éventuellement interrompus par -O-, ou représente $PO_nH_2$, n représentant 2 ou 3; $X_3$ représente un radical alkylène en $C_1$-$C_{10}$ ou alkylidène en $C_2$-$C_{10}$; $R_3$ est le groupe carboxy, 5-tétrazolyle, $SO_3H$, $PO_2H_2$, $PO_3H_2$ ou un groupe halogéno-(alkyl inférieur)-sulfamoyle; les radicaux (hétéro)aromatiques, y compris les cycles A et B, étant éventuellement substitués, indépendamment les uns des autres, par des substituants choisis parmi des atomes d'halogène et des groupes hydroxy, alcoxy inférieur, alcényloxy inférieur, phényl-alcoxy inférieur, phénoxy, $S(O)_m$-R et des radicaux alkyle inférieur, alcényle inférieur ou alcynyle inférieur éventuellement substitués par un atome d'halogène ou par le groupe hydroxy,

les radicaux alkyle inférieur, alcényle inférieur ou alcynyle inférieur étant éventuellement interrompus par -O-, ainsi que, dans le cas des radicaux (hétéro)aromatiques, éventuellement substitués en outre par le groupe carboxy, un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, par le groupe carbamoyle, par un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou par le groupe formyle, un groupe di-(alcoxy inférieur)-méthyle ou oxy(alkylène-oxy inférieur)-méthylène; sous forme libre ou sous forme de sel.

4. Procédé selon la revendication 1 ou 2 pour la préparation d'un composé de formule I dans lequel $R_1$ représente un radical alkyle inférieur, alcényle inférieur ou alcynyle inférieur éventuellement substitué par un atome d'halogène ou par le groupe hydroxy, ou un radical cycloalkyle en $C_3$-$C_7$ ou cycloalcényle en $C_3$-$C_7$ ou phényl-alkyle inférieur, phényl-alcényle inférieur ou phényl-alcynyle inférieur; $X_1$ représente CO ou $SO_2$; $X_2$ représente un radical alkylène en $C_1$-$C_{10}$ ou alkylidène en $C_2$-$C_{10}$ éventuellement substitué par un groupe hydroxy, cycloalkyle en $C_3$-$C_7$ ou cycloalcényle en $C_3$-$C_7$ ou phényle ou par un radical aromatique correspondant, monocyclique et à 5 ou 6 chaînons, qui comporte jusqu'à 4 hétéroatomes identiques ou différents; $R_2$ représente le groupe carboxy ou un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, le groupe carbamoyle ou un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente le groupe amino ou un groupe amino qui est monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou représente un groupe (alcanoyl inférieur)-, phényl-(alcanoyl inférieur)-, benzoyl-, (alcane inférieur)-sulfonyl-, benzènesulfonylamino, formyle, di-(alcoxy inférieur)-méthyle, oxy-(alkylène-oxy inférieur)-méthylène, hydroxy, alcoxy inférieur, alcényloxy inférieur, phényl-alcoxy inférieur ou phénoxy, $S(O)_m$-R, m représentant 0, 1 ou 2 et R représentant un atome d'hydrogène ou un groupe alkyle inférieur, alcényle inférieur ou alcynyle inférieur, un radical alcanoyle inférieur, ou représente le radical sulfamoyle ou un radical sulfamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des groupes alkylène en $C_1$-$C_{10}$ ou alkylidène en $C_2$-$C_{10}$, les groupes alkylène en $C_1$-$C_{10}$ ou alkylidène en $C_2$-$C_{10}$ étant éventuellement interrompus par -O-, ou représente $PO_nH_2$, n étant 2 ou 3; $X_3$ représente $-CH_2-$; $R_3$ est le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, étant éventuellement substitués, indépendamment les uns des autres, par des substituants choisis parmi des atomes d'halogène et des groupes hydroxy, alcoxy inférieur, alcényloxy inférieur, phényl-alcoxy inférieur, phénoxy, $S(O)_m$-R et des radicaux alkyle inférieur, alcényle inférieur ou alcynyle inférieur éventuellement substitués par un atome d'halogène ou par le groupe hydroxy, les radicaux alkyle inférieur, alcényle inférieur ou alcynyle inférieur étant éventuellement interrompus par -O-, et, dans le cas de radicaux (hétéro)aromatiques, étant éventuellement substitués en outre par le groupe carboxy ou par un groupe carboxy estérifié par un alcool dérivant d'un reste alkyle inférieur, phényl-alkyle inférieur, alcényle inférieur, alcynyle inférieur, (alcoxy inférieur)-alkyle inférieur, (alcoxy inférieur)-alcényle inférieur ou (alcoxy inférieur)-alcynyle inférieur, par le groupe carbamoyle ou par un groupe carbamoyle dans lequel le fragment amino est monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux alkylène en $C_1$-$C_{10}$ ou alkylidène en $C_2$-$C_{10}$, les radicaux alkylène en $C_1$-$C_{10}$ ou alkylidène en $C_2$-$C_{10}$ étant éventuellement interrompus par -O-, ou par le groupe formyle, un groupe di-(alcoxy inférieur)-méthyle ou oxy-(alkylène-oxy inférieur)-méthylène; sous forme libre ou sous forme de sel.

5. Procédé selon la revendication 1 ou 2 pour la préparation d'un composé de formule I dans lequel $R_1$ représente un radical alkyle inférieur, alcényle inférieur, alcynyle inférieur, halogéno-alkyle inférieur, -alcényle inférieur, -alcynyle inférieur, hydroxy-alkyle inférieur, -alcényle inférieur, -alcynyle inférieur, cycloalkyle en $C_3$-$C_7$, cycloalcényle en $C_3$-$C_7$, phényl-alkyle inférieur, phényl-alcényle inférieur ou phényl-alcynyle inférieur; $X_1$ représente CO ou $SO_2$; $X_2$ représente un radical alkylène en $C_1$-$C_{10}$ ou alkylidène en $C_2$-$C_{10}$ qui sont éventuellement substitués par le groupe hydroxy ou par un radical cycloalkyle en $C_3$-$C_7$, cycloalcényle en $C_3$-$C_7$, phényle ou par un radical hétéroaromatique

monocyclique à 5 ou 6 chaînons, comportant jusqu'à 4 hétéroatomes identiques ou différents, les radicaux cycliques étant pour leur part éventuellement substitués par un groupe carboxy éventuellement estérifié par un alcool dérivant d'un reste alkyle inférieur, phényl-alkyle inférieur, alcényle inférieur, alcynyle inférieur, (alcoxy inférieur)-alkyle inférieur, -alcényle inférieur ou -alcynyle inférieur, ou par un groupe carbamoyle dans lequel le fragment amino est éventuellement monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, le radical formyle, di-(alcoxy inférieur)-méthyle, oxy-(alkylène-oxy inférieur)-méthylène; $R_2$ représente un groupe carboxy éventuellement estérifié par un alcool dérivant d'un reste alkyle inférieur, phényl-alkyle inférieur, alcényle inférieur, alcynyle inférieur, (alcoxy inférieur)-alkyle inférieur, -alcényle inférieur ou -alcynyle inférieur, un radical carbamoyle dans lequel le fragment amino est éventuellement monosubstitué ou disubstitué, indépendamment l'un de l'autre, par un ou des groupes alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, un radical amino dans lequel le fragment amino est éventuellement monosubstitué ou disubstitué, indépendamment l'un de l'autre, par un ou des radicaux alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, un groupe (alcanoyl inférieur)-, phényl-(alcanoyl inférieur)-, benzoyl-, (alcane inférieur)-sulfonyl-, benzènesulfonyl-amino, formyle, di-(alcoxy inférieur)-méthyle, oxy-(alkylène-oxy inférieur)-méthylène, hydroxy, alcoxy inférieur, alcényloxy inférieur, phényl-alcoxy inférieur, phénoxy, $S(O)_m$-R, m représentant 0, 1 ou 2 et R représentant un atome d'hydrogène ou un groupe alkyle inférieur, alcényle inférieur ou alcynyle inférieur, un radical alcanoyle inférieur, sulfamoyle, dans lequel le fragment amino est éventuellement monosubstitué ou disubstitué, indépendamment l'un de l'autre, par un ou des groupes alkyle inférieur, alcényle inférieur, alcynyle inférieur, phényl-alkyle inférieur, phényl-alcényle inférieur ou phényl-alcynyle inférieur, ou disubstitué par des radicaux (alkylène inférieur)- ou (alkylène-oxy inférieur)-alkylène inférieur, ou représente $PO_nH_2$, n représentant 2 ou 3; $X_3$ représente $-CH_2-$; $R_3$ est le groupe carboxy, 5-tétrazolyle, $SO_3H$, $PO_2H_2$, $PO_3H_2$ ou un groupe halogéno-(alkyl inférieur)-sulfamoyle; les radicaux (hétéro)aromatiques, y compris les cycles A et B, étant éventuellement substitués, indépendamment les uns des autres, par un ou plusieurs substituants choisis parmi des atomes d'halogène et des groupes hydroxy, alcoxy inférieur, alcényloxy inférieur, des radicaux alkyle inférieur, alcényle inférieur, alcynyle inférieur, (alcoxy inférieur)-alkyle inférieur, -alcényle inférieur, -alcynyle inférieur, (alcényloxy inférieur)-alkyle inférieur, -alcényle inférieur et -alcynyle inférieur, éventuellement substitués chacun par un atome d'halogène ou par le groupe hydroxy, sous forme libre ou sous forme de sel.

6. Procédé selon la revendication 1 ou 2 pour la préparation d'un composé de formule I dans lequel $X_2$ représente un radical alkylène en $C_1$-$C_{10}$ ou alkylidène en $C_2$-$C_{10}$ qui sont éventuellement substitués par le groupe hydroxy ou par un groupe cycloalkyle en $C_3$-$C_7$, cycloalcényle en $C_3$-$C_7$, phényle ou par un radical hétéroaromatique monocyclique à 5 ou 6 chaînons, comportant jusqu'à 4 hétéroatomes identiques ou différents, un atome de carbone d'un radical alkylène en $C_1$-$C_{10}$ ou alkylidène en $C_2$-$C_{10}$ pouvant être ponté par un groupe alkylène en $C_2$-$C_6$, et les radicaux cycliques étant pour leur part éventuellement substitués par un groupe carboxy éventuellement estérifié par un alcool dérivant d'un reste alkyle inférieur, phényl-alkyle inférieur, alcényle inférieur, alcynyle inférieur, (alcoxy inférieur)-alkyle inférieur, -alcényle inférieur ou -alcynyle inférieur, par un groupe carbamoyle dans lequel le fragment amino est éventuellement monosubstitué ou disubstitué, indépendamment l'un de l'autre, par un ou des groupes alkyle inférieur, alcényle inférieur, alcynyle inférieur, phényl-alkyle inférieur, phényl-alcényle inférieur, phényl-alcynyle inférieur, ou disubstitué par des radicaux (alkylène inférieur)- ou (alkylène-oxy inférieur)-alkylène inférieur, par le groupe formyle, par un groupe di-(alcoxy inférieur)-méthyle, ou par un groupe oxy-(alkylène-oxy inférieur)-méthylène, ou $X_2$ représente un groupe cycloalkylène en $C_3$-$C_7$; $X_3$ représente un groupe alkylène inférieur ou alkylidène inférieur; les variables $X_1$, $R_1$, $R_2$, $R_3$ ont les significations indiquées dans la revendication 5; et les cycles (hétéro)aromatiques, y compris les cycles A et B, peuvent être substitués comme indiqué dans la revendication 5, sous forme libre ou sous forme de sel.

7. Procédé selon la revendication 1 ou 2 pour la préparation d'un composé de formule I dans lequel $R_1$ représente un radical alkyle inférieur, alcényle inférieur, halogéno-alkyle inférieur, -alcényle inférieur, hydroxy-alkyle inférieur, un radical cycloalkyle à 3-7 chaînons ou phényl-alkyle inférieur; $X_1$ représente CO, $SO_2$ ou -O-C(=O)-, l'atome de carbone du groupe carbonyle étant lié à l'atome d'azote indiqué dans la formule I; $X_2$ représente un radical alkylène en $C_1$-$C_{10}$ ou alkylidène en $C_1$-$C_7$, qui sont éventuellement substitués par le groupe hydroxy, carboxy, amino, guanidino, un radical cycloalkyle à 3-7 chaînons, cyclo-alcényle à 3-7 chaînons, phényle, pyrrolyle, pyrazolyle, imidazolyle, triazolyle, tétrazolyle, furyle, thiényle ou pyridyle, qui pour leur part peuvent être éventuellement substitués en outre par un radical carboxy, (alcoxy inférieur)-carbonyle, phényl-(alcoxy inférieur)-carbonyle, par un radical carbamoyle dans lequel le fragment amino peut éventuellement être monosubstitué ou disubstitué, indépendamment l'un de l'autre, par un ou des groupes alkyle inférieur ou phényl-alkyle inférieur, ou un radical formyle, di-(alcoxy

inférieur)-méthyle ou oxy-(alkylène-oxy inférieur)-méthylène; $R_2$ représente un groupe carboxy, (alcoxy inférieur)-, phényl-(alcoxy inférieur)-, (alcényloxy inférieur)-, (alcoxy inférieur)-(alcoxy inférieur)-carbonyle, un radical carbamoyle dans lequel le fragment amino est éventuellement monosubstitué ou disubstitué, indépendamment l'un de l'autre, par un ou des radicaux alkyle inférieur ou phényl-alkyle inférieur, ou est disubstitué par des radicaux alkylène inférieur, éventuellement condensés, au niveau de deux atomes de carbone contigus, avec un cycle benzénique, ou par des radicaux (alkylène-oxy inférieur)-alkylène inférieur, un radical amino dans lequel le fragment amino est éventuellement monosubstitué ou disubstitué, indépendamment l'un de l'autre, par un ou des radicaux alkyle inférieur ou phényl-alkyle inférieur, ou est disubstitué par des radicaux (alkylène inférieur)- ou (alkylène-oxy inférieur)-alkylène inférieur, un groupe (alcanoyl inférieur)-, phényl-(alcanoyl inférieur)-, benzoyl-, (alcane inférieur)-sulfonyl-, benzènesulfonylamino, formyle, di-(alcoxy inférieur)-méthyle, oxy-(alkylène-oxy inférieur)-méthylène, hydroxy, alcoxy inférieur, phényl-alcoxy inférieur, phénoxy, $S(O)_m$-R, m représentant 0, 1 ou 2 et R représentant un groupe alkyle inférieur, alcanoyle inférieur, sulfamoyle, dans lequel le fragment amino est éventuellement monosubstitué ou disubstitué, indépendamment l'un de l'autre, par un ou des groupes alkyle inférieur ou phényl-alkyle inférieur, ou représente $PO_nH_2$, n représentant 2 ou 3; $X_3$ est le groupe méthylène; $R_3$ représente le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, sont éventuellement substitués chacun en outre par un ou plusieurs substituants choisis parmi des atomes d'halogène et des groupes hydroxy, alcoxy inférieur, et des radicaux alkyle inférieur ou (alcoxy inférieur)-alkyle inférieur éventuellement substitués chacun par un atome d'halogène ou par le groupe hydroxy, sous forme libre ou sous forme de sel.

8. Procédé selon la revendication 1 ou 2 pour la préparation d'un composé de formule I dans lequel $R_1$ représente un radical alkyle inférieur, alcényle inférieur, halogéno-alkyle inférieur, -alcényle inférieur, hydroxy-alkyle inférieur, un radical cycloalkyle à 3-7 chaînons ou phényl-alkyle inférieur; $X_1$ représente CO ou $SO_2$; $X_2$ représente un radical alkylène en $C_1$-$C_{10}$ ou alkylidène en $C_1$-$C_7$, qui sont éventuellement substitués par un groupe hydroxy, un radical cycloalkyle à 3-7 chaînons, cycloalcényle à 3-7 chaînons, phényle, pyrrolyle, pyrazolyle, imidazolyle, triazolyle, tétrazolyle, furyle, thiényle ou pyridyle, qui peuvent pour leur part être éventuellement substitués en outre par un groupe carboxy, (alcoxy inférieur)-carbonyle, phényl-(alcoxy inférieur)-carbonyle, carbamoyle dans lequel le fragment amino est éventuellement monosubstitué ou disubstitué, indépendamment l'un de l'autre, par un ou des groupes alkyle inférieur ou phényl-alkyle inférieur, le radical formyle, un radical di-(alcoxy inférieur)-méthyle ou oxy-(alkylène-oxy inférieur)-méthylène; $R_2$ représente un radical carboxy, (alcoxy inférieur)-, phényl-(alcoxy inférieur)-, (alcényloxy inférieur)-, (alcoxy inférieur)-(alcoxy inférieur)-carbonyle, un radical carbamoyle dans lequel le fragment amino est éventuellement monosubstitué ou disubstitué, indépendamment l'un de l'autre, par un ou des groupes alkyle inférieur, phényl-alkyle inférieur, ou disubstitué par des radicaux (alkylène inférieur)-, ou (alkylène-oxy inférieur)-alkylène inférieur, un radical amino dans lequel le fragment amino est éventuellement monosubstitué ou disubstitué, indépendamment l'un de l'autre, par un ou des groupes alkyle inférieur, phényl-alkyle inférieur, ou disubstitué par des radicaux (alkylène inférieur)- ou (alkylène-oxy inférieur)-alkylène inférieur, un groupe (alcanoyl inférieur)-, phényl-(alcanoyl inférieur)-, benzoyl-, (alcane inférieur)-sulfonyl-, benzènesulfonylamino, formyle, di-(alcoxy inférieur)-méthyle, oxy-(alkylène-oxy inférieur)-méthylène, hydroxy, alcoxy inférieur, phényl-alcoxy inférieur, phénoxy, $S(O)_m$-R, m représentant 0, 1 ou 2 et R représentant un groupe alkyle inférieur, un radical alcanoyle inférieur, sulfamoyle dans lequel le groupe amino est éventuellement monosubstitué ou disubstitué, indépendamment l'un de l'autre, par des groupes alkyle inférieur, phényl-alkyle inférieur, ou représente $PO_nH_2$, n représentant 2 ou 3; $X_3$ est le groupe méthylène; $R_3$ représente le groupe carboxy, 5-tétrazolyle, $SO_3H$, $PO_2H_2$, $PO_3H_2$ ou un groupe halogéno-(alkyl inférieur)-sulfamoyle; et les radicaux (hétéro)aromatiques, y compris les cycles A et B, sont éventuellement substitués chacun en outre par un ou plusieurs substituants choisis parmi des atomes d'halogène et des groupes hydroxy, alcoxy inférieur, des groupes alkyle inférieur ou (alcoxy inférieur)-alkyle inférieur éventuellement substitués chacun par un atome d'halogène ou par le groupe hydroxy, sous forme libre ou sous forme de sel.

9. Procédé selon la revendication 1 ou 2 pour la préparation d'un composé de formule I dans lequel $X_2$ représente un radical alkylène en $C_1$-$C_{10}$ ou alkylidène en $C_1$-$C_7$, qui sont éventuellement substitués par un groupe hydroxy, un radical cycloalkyle à 3-7 chaînons, cycloalcényle à 3-7 chaînons, phényle, pyrrolyle, pyrazolyle, imidazolyle, triazolyle, tétrazolyle, furyle, thiényle ou pyridyle, qui peuvent pour leur part être éventuellement substitués en outre par un groupe carboxy, (alcoxy inférieur)-carbonyle, phényl-(alcoxy inférieur)-carbonyle, carbamoyle dans lequel le fragment amino est éventuellement monosubstitué ou disubstitué, indépendamment l'un de l'autre, par un ou des groupes alkyle inférieur ou phényl-alkyle inférieur, ou peuvent être substitués par le groupe formyle, par un groupe di-(alcoxy inférieur)-méthyle, ou par un groupe oxy-(alkylène-oxy inférieur)-méthylène, un atome de carbone d'un radical alkylène en $C_1$-$C_{10}$ ou alkylidène en $C_1$-$C_7$ pouvant être ponté par un groupe alkylène en $C_2$-$C_6$, ou $X_2$ représente un groupe cycloalkylène en $C_3$-$C_7$; $X_3$ représente un groupe alkylène inférieur ou alkylidène inférieur; les variables $X_1$, $R_1$, $R_2$, $R_3$ ont les significations indiquées dans la revendication 8, et les cycles (hétéro)aromatiques, y compris

les cycles A et B, peuvent être substitués comme indiqué dans la revendication 8, sous forme libre ou sous forme de sel.

**10.** Procédé selon la revendication 1 ou 2 pour la préparation d'un composé de formule I dans lequel les variables $R_1$, $X_1$, $R_3$ ont les significations indiquées respectivement dans les revendications 1 à 8; $X_2$ représente un radical alkylène inférieur ou alkylidène inférieur éventuellement substitué par un groupe hydroxy, cycloalkyle à 3-7 chaînons, phényle ou imidazolyle; et $R_2$ représente un radical carboxy, (alcoxy inférieur)-, phényl-(alcoxy inférieur)-, (alcoxy inférieur)-(alcoxy inférieur)-carbonyle, un radical carbamoyle qui est éventuellement monosubstitué ou disubstitué, indépendamment l'un de l'autre, par un ou des groupes alkyle inférieur ou phényl-alkyle inférieur, un radical amino, (alcanoyl inférieur)-, phényl-(alcanoyl inférieur)-, (alcane inférieur)-sulfonylamino, hydroxy, alcoxy inférieur, phényl-alcoxy inférieur ou phénoxy; $X_3$ représente $-CH_2-$; les radicaux (hétéro)aromatiques, y compris les cycles A et B, étant éventuellement substitués chacun par un ou plusieurs substituants choisis parmi des atomes d'halogène et des groupes trifluorométhyle, hydroxy, alcoxy inférieur, alkyle inférieur, hydroxyalkyle inférieur ou (alcoxy inférieur)-alkyle inférieur, sous forme libre ou sous forme de sel.

**11.** Procédé selon la revendication 1 ou 2 pour la préparation d'un composé de formule I dans lequel $X_2$ représente un radical alkylène inférieur ou alkylidène inférieur éventuellement substitué par un groupe hydroxy, cycloalkyle à 3-7 chaînons, cycloalcényle à 7 chaînons, phényle ou imidazolyle, un atome de carbone d'un radical alkylène inférieur ou alkylidène inférieur pouvant être ponté par un groupe alkylène en $C_2$-$C_6$, ou $X_2$ représente un groupe cycloalkylène en $C_3$-$C_7$; les variables $X_1$, $X_3$, $R_1$, $R_2$, $R_3$ ont les significations indiquées dans les revendications 8 à 10; et les cycles A et B, peuvent être substitués comme indiqué dans la revendication 10, sous forme libre ou sous forme de sel.

**12.** Procédé selon la revendication 1 ou 2 pour la préparation d'un composé de formule

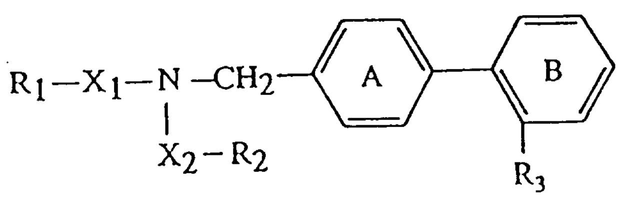

(Ia),

dans laquelle les variables $R_1$, $X_1$, $X_2$, $R_2$ et $R_3$ ont les significations données respectivement dans l'une des revendications 1 ou 3-11, et les cycles A et B, peuvent être substitués comme indiqué dans la revendication 11, sous forme libre ou sous forme de sel.

**13.** Procédé selon la revendication 1 ou 2 pour la préparation d'un composé de formule Ia dans lequel $X_2$ représente un radical alkylène inférieur ou alkylidène inférieur éventuellement substitué par un groupe hydroxy ou par un groupe cycloalkyle à 3-7 chaînons, un atome de carbone d'un radical alkylène inférieur ou alkylidène inférieur pouvant être ponté par un groupe alkylène en $C_2$-$C_6$, ou dans lequel $X_2$ représente un groupe cycloalkylène en $C_3$-$C_7$, et les variables $R_1$, $X_1$, $R_2$ et $R_3$ ont les significations indiquées respectivement dans l'une des revendications 1 ou 3-11; et les cycles A et B peuvent être substitués comme indiqué dans la revendication 11, sous forme libre ou sous forme de sel.

**14.** Procédé selon la revendication 1 ou 2 pour la préparation d'un composé de formule Ia, dans lequel $X_2$ représente le groupe de formule

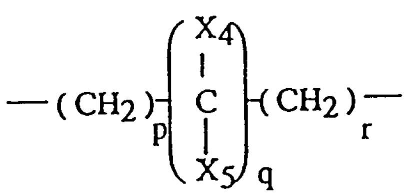

(Ib)

dans laquelle p représente 0 ou 1, q représente 1 et r représente 0 ou 1, ou dans laquelle p représente 1 à 8 et q ainsi que r représentent chacun 0; $X_4$ représente un radical phényle ou alkyle inférieur éventuellement substitué par un groupe hydroxy, cycloalkyle à 3-7 chaînons, phényle ou imidazolyle; et $X_5$ représente un atome d'hydrogène

ou un groupe alkyle inférieur; $R_2$ représente un radical carboxy, (alcoxy inférieur)-carbonyle, phényl-(alcoxy inférieur)-carbonyle, (alcoxy inférieur)-(alcoxy inférieur)-carbonyle, hydroxy, alcoxy inférieur, phényl-alcoxy inférieur, phénoxy, amino, alcanoylamino inférieur, phénylalcanoylamino inférieur ou (alcane inférieur)-sulfonylamino; et les variables $R_1$, $X_1$ et $X_3$ ont les significations indiquées respectivement dans l'une des revendications 1 ou 3-8; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**15.** Procédé selon la revendication 1 ou 2 pour la préparation d'un composé de formule Ia, dans lequel $X_2$ représente le groupe de formule Ib, dans lequel p représente 0 ou 1, q représente 1 et r représente 0 ou 1, ou dans lequel p représente 1 à 8 et q ainsi que r représentent chacun 0; $X_4$ représente un radical phényle ou alkyle inférieur éventuellement substitué par un groupe hydroxy, cycloalkyle à 3-7 chaînons, phényle ou imidazolyle; et $X_5$ représente un atome d'hydrogène ou un groupe alkyle inférieur; ou $X_4$ et $X_5$ forment ensemble un radical alkylène en $C_2$-$C_6$, tel qu'alkylène en $C_4$-$C_5$, ou $X_2$ représente un radical cycloalkylène en $C_3$-$C_7$, tel que cycloalkylène en $C_5$-$C_6$; $R_2$ représente un radical carboxy, (alcoxy inférieur)-carbonyle, phényl-(alcoxy inférieur)-carbonyle, (alcoxy inférieur)-(alcoxy inférieur)-carbonyle, hydroxy, alcoxy inférieur, phényl-alcoxy inférieur, phénoxy, amino, alcanoylamino inférieur, phényl-alcanoylamino inférieur ou (alcane inférieur)-sulfonylamino; et les variables $R_1$, $X_1$ et $R_3$ ont les significations indiquées respectivement dans l'une des revendications 1 ou 3-8; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**16.** Procédé selon la revendication 1 ou 2 pour la préparation d'un composé de formule Ia, dans lequel $R_1$ représente un radical alkyle inférieur, tel qu'un radical alkyle en $C_3$-$C_5$, ou un radical alcényle inférieur, tel qu'un radical alcényle en $C_3$-$C_5$; $X_1$ représente CO ou $SO_2$; $X_2$ représente le groupe de formule Ib, dans lequel p et r représentent 0 ou 1, et q représente 1; $X_4$ représente un radical alkyle inférieur, tel qu'un radical alkyle en $C_1$-$C_4$, ou phényle, éventuellement substitué par un groupe hydroxy, cycloalkyle à 3-7 chaînons tel que cyclohexyle, par un groupe phényle ou imidazolyle, tel que 4-imidazolyle, éventuellement substitué par un atome d'halogène ou par le groupe hydroxy; et $X_5$ représente un atome d'hydrogène ou un groupe alkyle inférieur, tel qu'un groupe alkyle en $C_1$-$C_4$; ou $X_4$ et $X_5$ représentent ensemble un groupe alkylène en $C_2$-$C_6$, tel qu'un groupe alkylène en $C_4$-$C_5$; ou $X_2$ représente un radical cycloalkylène en $C_3$-$C_7$, tel qu'un radical cycloalkylène en $C_5$-$C_6$,; $R_2$ représente un radical carboxy, (alcoxy inférieur)-carbonyle tel qu'un groupe alcoxy($C_2$-$C_5$)-carbonyle, phényl-(alcoxy inférieur)-carbonyle, tel que phényl-alcoxy-($C_1$-$C_4$)-carbonyle, un radical (alcoxy inférieur)-(alcoxy inférieur)-carbonyle, tel qu'un radical alcoxy($C_1$-$C_4$)-alcoxy$C_2$-$C_5$)-carbonyle, un groupe hydroxy ou alcoxy inférieur, tel qu'un groupe alcoxy en $C_1$-$C_4$; et $R_3$ représente le groupe carboxy ou 5-tétrazolyle; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**17.** Procédé selon la revendication 1 ou 2 pour la préparation d'un composé de formule Ia dans lequel $R_1$ représente un radical alkyle inférieur, tel qu'alkyle en $C_3$-$C_5$, ou alcényle inférieur, tel qu'alcényle en $C_3$-$C_5$; $X_1$ représente CO ou $SO_2$; $X_2$ représente le groupe de formule Ib, dans lequel p et r représentent 0 ou 1 et q représente 1; $X_4$ représente un radical alkyle inférieur, tel qu'un radical alkyle en $C_1$-$C_4$, ou phényle, éventuellement substitué par un groupe hydroxy, cycloalkyle à 3-7 chaînons, phényle ou imidazolyle, tel que 4-imidazolyle, éventuellement substitué par un atome d'halogène ou par le groupe hydroxy; et $X_5$ représente un atome d'hydrogène ou un groupe alkyle inférieur, tel qu'un groupe alkyle en $C_1$-$C_4$; $R_2$ représente un groupe carboxy, (alcoxy inférieur)-carbonyle, tel qu'un groupe alcoxy($C_2$-$C_5$)-carbonyle, phényl-(alcoxy inférieur)-carbonyle, tel que phényl-alcoxy($C_1$-$C_4$)-carbonyle, (alcoxy inférieur)-(alcoxy inférieur)-carbonyle tel qu'un groupe alcoxy($C_1$-$C_4$)-alcoxy-($C_2$-$C_5$)-carbonyle, un groupe hydroxy ou alcoxy inférieur tel qu'un groupe alcoxy en $C_1$-$C_4$; et $R_3$ représente le groupe carboxy ou 5-tétrazolyle; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxyalkyle inférieur, sous forme libre ou sous forme de sel.

**18.** Procédé selon la revendication 1 ou 2 pour la préparation d'un composé de formule Ia, dans lequel $R_1$ représente un radical alkyle inférieur, tel qu'un radical alkyle en $C_3$-$C_5$, ou bien un radical alcényle inférieur, tel qu'alcényle en $C_3$-$C_5$; $X_1$ représente CO ou $SO_2$; $X_2$ représente le groupe de formule Ib dans lequel p représente un nombre entier allant de 1 à 8 et q ainsi que r représentent 0; $R_2$ représente un groupe hydroxy, alcoxy inférieur tel qu'alcoxy en $C_1$-$C_4$, phényl-alcoxy inférieur tel que phényl-alcoxy-($C_1$-$C_4$), phénoxy, un groupe alcanoylamino inférieur, tel qu'alcanoyl($C_1$-$C_4$)-amino, un groupe phényl-(alcanoyl inférieur)-amino, tel que phényl-alcanoyl($C_1$-$C_4$)-amino, un groupe (alcane inférieur)-sulfonylamino, tel qu'un groupe alcane($C_1$-$C_4$)-sulfonylamino; et $R_3$ représente le groupe

carboxy ou, en premier lieu, 5-tétrazolyle; les radicaux (hétéro)aromatiques, y compris les cycles A et B, étant éventuellement substitués chacun par un atome d'halogène ou par un groupe trifluorométhyle, hydroxy, alcoxy inférieur, alkyle inférieur ou hydroxy-alkyle inférieur, sous forme libre ou sous forme de sel.

**19.** Procédé selon la revendication 1 ou 2 pour la préparation d'un composé de formule Ia, dans lequel $R_1$ représente un radical alkyle en $C_3$-$C_5$ ou, en second lieu, un radical alcényle en $C_3$-$C_5$; $X_1$ représente CO ou $SO_2$; $X_2$ représente un groupe de formule Ib dans lequel p et r représentent, indépendamment l'un de l'autre, 0 ou 1, et q représente 1; $X_4$ représente un groupe alkyle en $C_1$-$C_4$, hydroxyalkyle en $C_1$-$C_4$, cycloalkyl($C_3$-$C_7$)-alkyle($C_1$-$C_4$), phénylalkyle($C_1$-$C_4$), ou imidazolyl-alkyle($C_1$-$C_4$); et $X_5$ représente un atome d'hydrogène ou un groupe alkyle en $C_1$-$C_4$; ou $X_4$ et $X_5$ représentent ensemble le groupe tétraméthylène ou pentaméthylène; $R_2$ représente un groupe carboxy ou alcoxy($C_2$-$C_5$)-carbonyle, ainsi qu'un groupe phényl-alcoxy($C_1$-$C_4$)-carbonyle; et $R_3$ représente le groupe carboxy ou 5-tétrazolyle, sous forme libre ou sous forme de sel.

**20.** Procédé selon la revendication 1 ou 2 pour la préparation d'un composé de formule Ia, dans lequel $R_1$ représente un groupe alkyle en $C_3$-$C_5$ ou, en second lieu, un groupe alcényle en $C_3$-$C_5$; $X_1$ représente CO ou $SO_2$; $X_2$ représente un groupe de formule Ib dans lequel p et r représentent chacun 0 ou 1 et q représente 1; $X_4$ représente un groupe alkyle en $C_1$-$C_4$, hydroxyalkyle en $C_1$-$C_4$, cycloalkyl($C_3$-$C_7$)-alkyle($C_1$-$C_4$), phényl-alkyle($C_1$-$C_4$), ou imidazolyl-alkyle($C_1$-$C_4$); et $X_5$ représente un atome d'hydrogène; $R_2$ représente un groupe carboxy ou alcoxy($C_2$-$C_5$)-carbonyle, ou un groupe phényl-alcoxy($C_1$-$C_4$)-carbonyle; et $R_3$ représente le groupe carboxy ou 5-tétrazolyle, sous forme libre ou sous forme de sel.

**21.** Procédé selon la revendication 1 ou 2 pour la préparation d'un composé de formule Ia, dans lequel $R_1$ représente un groupe alkyle en $C_3$-$C_5$; $X_1$ représente CO; $X_2$ représente un groupe de formule Ib dans lequel q et r représentent 0 et p représente 1 à 3, ou dans lequel p et q représentent 1 et r représente 0; $X_4$ représente un groupe alkyle en $C_1$-$C_4$; $X_5$ représente un atome d'hydrogène ou un groupe alkyle en $C_1$-$C_4$; $R_2$ représente un groupe carboxy, alcoxy($C_2$-$C_5$)-carbonyle; et $R_3$ représente le groupe carboxy ou 5-tétrazolyle, sous forme libre ou sous forme de sel.

**22.** Procédé selon la revendication 1 ou 2 pour la préparation d'un composé de formule Ia dans lequel $R_3$ représente le groupe 5-tétrazolyle, sous forme libre ou sous forme de sel.

**23.** Procédé selon la revendication 1 ou 2 pour la préparation d'un composé de formule Ia dans lequel $R_1$ représente un groupe alkyle en $C_3$-$C_5$; $X_1$ représente CO; $X_2$ représente un groupe de formule Ib dans lequel p représente 0 ou 1, r représente 0 et q représente 1; $X_4$ représente un groupe alkyle en $C_1$-$C_4$; et $X_5$ représente un atome d'hydrogène ou un groupe alkyle en $C_1$-$C_4$; ou $X_4$ et $X_5$ représentent ensemble le groupe tétraméthylène ou pentaméthylène; $R_2$ représente un groupe carboxy ou alcoxy($C_2$-$C_5$)-carbonyle; et $R_3$ représente le groupe 5-tétrazolyle, sous forme libre ou sous forme de sel.

**24.** Procédé selon la revendication 1 ou 2 pour la préparation d'un composé de formule Ia, dans lequel $R_1$ représente un groupe alkyle en $C_3$-$C_5$; $X_1$ représente CO; $X_2$ représente un groupe de formule Ib dans lequel p représente 0 ou 1, r représente 0 et q représente 1; $X_4$ et $X_5$ représentent ensemble le groupe tétraméthylène ou pentaméthylène; $R_2$ représente un groupe carboxy ou alcoxy($C_2$-$C_5$)-carbonyle; et $R_3$ représente le groupe tétrazolyle, sous forme libre ou sous forme de sel.

**25.** Procédé selon la revendication 1 ou 2 pour la préparation d'un composé de formule Ia dans lequel $R_1$ représente un groupe alkyle en $C_3$-$C_5$; $X_1$ représente CO; $X_2$ représente un groupe de formule Ib dans lequel p et r représentent 0 ou 1 et q représente 1; $X_4$ représente un groupe alkyle en $C_1$-$C_4$; et $X_5$ représente un atome d'hydrogène; $R_2$ représente un groupe carboxy ou alcoxy($C_2$-$C_5$)-carbonyle; et $R_3$ représente le groupe 5-tétrazolyle, sous forme libre ou sous forme de sel.

**26.** Procédé selon la revendication 1 ou 2 pour la préparation d'un composé de formule Ia, selon l'une des revendications 14 à 25, dans lequel $X_2$ représente un groupe de formule Ib, q représente 1 et $X_4$ et $X_5$ ont des significations différentes, sous forme libre ou sous forme de sel, et dans lequel l'atome de carbone asymétrique concerné, portant les variables $X_4$ et $X_5$, a la configuration S.

**27.** Procédé selon la revendication 1 ou 2 pour la préparation de la (S)-N-(1-carboxy-2-méthylprop-1-yl)-N-pentanoyl-N-[2'-(1H-tétrazol-5-yl)biphényl-4-ylméthyl]amine, sous forme libre ou sous forme de sel, selon la revendication 1.

**28.** Procédé selon la revendication 1 ou 2 pour la préparation de la N-(2-carboxy-2,2-tétraméthylène-éthyl)-N-pentanoyl-N-[2'-(1H-tétrazol-5-yl)biphényl-4-ylméthyl]amine, sous forme libre ou sous forme de sel, selon la revendication 1.

**29.** Procédé selon la revendication 1 ou 2 pour la préparation de la N-(2-carboxy-2-éthylbut-1-yl)-N-pentanoyl-N-[2'-(1H-tétrazol-5-yl)biphényl-4-ylméthyl]amine, sous forme libre ou sous forme de sel, selon la revendication 1.

**30.** Procédé selon la revendication 1 ou 2 pour la préparation de la (S)-N-(1-carboxy-2-méthylprop-1-yl)-N-éthoxycarbonyl-N-[2'-(1H-tétrazol-5-yl)biphényl-4-ylméthyl]amine, sous forme libre ou sous forme de sel, selon la revendication 1.

**31.** Procédé selon la revendication 1 ou 2 pour la préparation de la N-(1-carboxycyclohexyl)-N-pentanoyl-N-[2'-(1H-tétrazol-5-yl)biphényl-4-ylméthyl]amine, sous forme libre ou sous forme de sel, selon la revendication 1.

**32.** Procédé selon la revendication 1 ou 2 pour la préparation d'un composé selon la revendication 1, choisi parmi les suivants:
(S)-N-(1-carboxyéthyl)-N-pentanoyl-N-[2'-(1H-tétrazol-5-yl)biphényl-4-ylméthyl]amine,
N-(2-hydroxyéthyl)-N-pentanoyl-N-[2'-(1H-tétrazol-5-yl)biphényl-4-ylméthyl]amine,
N-(2-éthoxycarbonyl-2,2-tétraméthylène-éthyl)-N-pentanoyl-N-[2'-(1H-tétrazol-5-yl)biphényl-4-ylméthyl]amine,
N-(2-éthoxycarbonyl-2-éthylbut-1-yl)-N-pentanoyl-N-[2'-(1H-tétrazol-5-yl)biphényl-4-ylméthyl]amine,
N-(2-éthoxycarbonyl-2-méthylprop-1-yl)-N-pentanoyl-N-[2'-(1H-tétrazol-5-yl)biphényl-4-ylméthyl]amine,
(S)-N-(1-hydroxyméthyl-2-méthylprop-1-yl)-N-pentanoyl-N-[2'-1H-tétrazol-5-yl)biphényl-4-ylméthyl]amine,
N-(2-éthoxycarbonyl-2,2-pentaméthylène-éthyl)-N-pentanoyl-N-[2'-(1H-tétrazol-5-yl)biphényl-4-ylméthyl]amine,
(S)-N-(1-carboxy-2-méthylprop-1-yl)-N-propyloxycarbonyl-N-[2'-(1H-tétrazol-5-yl)biphényl-4-ylméthyl]amine,
N-(2-carboxy-2-méthylpropyl)-N-pentanoyl-N-[2'-(1H-tétrazol-5-yl)biphényl-4-ylméthyl]amine,
N-(2-carboxy-2,2-pentaméthylène-éthyl)-N-pentanoyl-N-[2'-(1H-tétrazol-5-yl)biphényl-4-ylméthyl]amine,
(S)-N-(1-aminocarbonyl-2-méthylprop-1-yl)-N-pentanoyl-N-[2'-(1H-tétrazol-5-yl)biphényl-4-ylméthyl]amine et
(S)-N-(1-carboxy-2-méthylprop-1-yl)-N-5-oxopent-N-5-yl)-N-[2'-(1H-tétrazol-5-yl)biphényl-4-ylméthyl]amine,
chacun sous forme libre ou sous forme de sel.

**33.** Procédé selon la revendication 1 ou 2 pour la préparation d'un composé selon la revendication 1, choisi parmi les suivants:
N-carboxyméthyl-N-pentanoyl-N-[2'-(1H-tétrazol-5-yl)biphényl-4-ylméthyl]amine,
(S)-N-(1-méthoxycarbonyléthyl)-N-pentanoyl-N-[2'-(1H-tétrazol-5-yl)biphényl-4-ylméthyl]amine,
N-[1-carboxy-2-(4-fluorophényl)éthyl]-N-pentanoyl-N-[2'-(1H-tétrazol-5-yl)biphényl-4-ylméthyl]amine,
N-[2-(4-fluorophényl)-1-méthoxycarbonyléthyl]-N-pentanoyl-N-[2'-(1H-tétrazol-5-yl)biphényl-4-ylméthyl]amine,
N-[2-(4-fluorophényl)-1-hydroxyméthyléthyl]-N-pentanoyl-N-[2'-(1H-tétrazol-5-yl)biphényl-4-ylméthyl]amine,
N-(2'-carboxybiphényl-4-ylméthyl)-N-[1-carboxy-2-(4-fluorophényl)éthyl]-N-pentanoylamine,
N-(2'-carboxybiphényl-4-ylméthyl)-N-[2-(4-fluorophényl)-1-méthoxycarbonyléthyl]-N-pentanoylamine,
(S)-N-(2'-carboxybiphényl-4-ylméthyl)-N-(1-hydroxyméthyl-2-phényléthyl)-N-pentanoylamine,
(S)-N-(2'-carboxybiphényl-4-ylméthyl)-N-(1-hydroxyméthyl-2-imidazol-4-yléthyl)-N-pentanoylamine,
(R)-N-(1-carboxyéthyl)-N-pentanoyl-N-[2'-(1H-tétrazol-5-yl)biphényl-4-ylméthyl]amine,
(1S),(2S)-N-(1-carboxy-2-méthylbut-1-yl)-N-pentanoyl-N-[2'-(1H-tétrazol-5-yl)biphényl-4-ylméthyl]amine,
(1S),(2S)-N-(1-méthoxycarbonyl-2-méthylbut-1-yl)-N-pentanoyl-N-[2'-(1H-tétrazol-5-yl)biphényl-4-ylméthyl]amine,
(S)-N-(1-carboxybut-1-yl)-N-pentanoyl-N-[2'-(1H-tétrazol-5-yl)biphényl-4-ylméthyl]amine,
(S)-N-(1-méthoxycarbonylbut-1-yl)-N-pentanoyl-N-[2'-(1H-tétrazol-5-yl)biphényl-4-ylméthyl]amine,
(S)-N-(1-carboxyéthyl)-N-hexanoyl-N-[2'-(1H-tétrazol-5-yl)biphényl-4-ylméthyl]amine,
(S)-N-butanoyl-N-(1-carboxyéthyl-N-[2'-(1H-tétrazol-5-yl)biphényl-4-ylméthyl]amine,
(S)-N-(1-carboxyprop-1-yl)-N-pentanoyl-N-[2'-(1H-tétrazol-5-yl)-biphényl-4-ylméthyl]amine,
(S)-N-(1-carboxy-2-cyclohexyléthyl)-N-pentanoyl-N-[2'-(1H-tétrazol-5-yl)-biphényl-4-ylméthyl]amine,
(S)-N-(2-cyclohexyl-1-méthoxycarbonyléthyl)-N-pentanoyl-N-[2'-(1H-tétrazol-5-yl)-biphényl-4-ylméthyl]amine,
(R)-N-(1-carboxy-2-méthylprop-1-yl)-N-pentanoyl-N-[2'-(1H-tétrazol-5-yl)-biphényl-4-ylméthyl]amine,
N-(2-méthoxyéthyl)-N-pentanoyl-N-[2'-1H-tétrazol-5-yl)biphényl-4-ylméthyl]amine,
N-(2-benzyloxyéthyl)-N-pentanoyl-N-[2'-1H-tétrazol-5-yl)biphényl-4-ylméthyl]amine,
N-(3-méthoxyprop-1-yl)-N-pentanoyl-N-[2'-1H-tétrazol-5-yl)biphényl-4-ylméthyl]amine,
N-(3-benzyloxyprop-1-yl)-N-pentanoyl-N-[2'-1H-tétrazol-5-yl)-biphényl-4-ylméthyl]amine,

N-(3-hydroxyprop-1-yl)-N-pentanoyl-N-[2'-1H-tétrazol-5-yl)biphényl-4-ylméthyl]amine,
N-(1-méthoxycarbonyl-1-méthyléthyl)-N-pentanoyl-N-[2'-1H-tétrazol-5-yl)-biphényl-4-ylméthyl]amine,
N-(2-carboxyéthyl)-N-pentanoyl-N-[2'-(1H-tétrazol-5-yl)biphényl-4-ylméthyl]amine,
N-(2-carboxyprop-1-yl)-N-pentanoyl-N-[2'-(1H-tétrazol-5-yl)biphényl-4-ylméthyl]amine,
N-(1-carboxy-1-méthyléthyl)-N-pentanoyl-N-[2'-(1H-tétrazol-5-yl)biphényl-4-ylméthyl]amine,
N-(5-hydroxypent-1-yl)-N-pentanoyl-N-[2'-(1H-tétrazol-5-yl)biphényl-4-ylméthyl]amine,
N-(1-carboxyprop-2-yl)-N-pentanoyl-N-[2'-(1H-tétrazol-5-yl)biphényl-4-ylméthyl]amine,
N-(2-éthoxycarbonyl-3-méthylbut-1-yl)-N-pentanoyl-N-[2'-(1H-tétrazol-5-yl)biphényl-4-ylméthyl]amine,
N-(2-carboxy-3-méthylbut-1-yl)-N-pentanoyl-N-[2'-(1H-tétrazol-5-yl)biphényl-4-ylméthyl]amine,
N-(3-phénoxyprop-1-yl)-N-pentanoyl-N-[2'-(1H-tétrazol-5-yl)biphényl-4-ylméthyl]amine,
N-[2-(4-hydroxyphényl)éthyl]-N-pentanoyl-N-[2'-(1H-tétrazol-5-yl)biphényl-4-ylméthyl]amine,
N-[3-(4-hydroxyphényl)prop-1-yl]-N-pentanoyl-N-[2'-(1H-tétrazol-5-yl)biphényl-4-ylméthyl]amine,
N-[8-hydroxyoct-1-yl)-N-pentanoyl-N-[2'-(1H-tétrazol-5-yl)biphényl-4-ylméthyl]amine,
N-[2-méthanesulfonylaminoéthyl)-N-pentanoyl-N-[2'-(1H-tétrazol-5-yl)biphényl-4-ylméthyl]amine,
N-(3-acétylaminoprop-1-yl)-N-pentanoyl-N-[2'-(1H-tétrazol-5-yl)biphényl-4-ylméthyl]amine,
N-(2-méthoxy-2-oxo-1-phényléthyl)-N-pentanoyl-N-[2'-(1H-tétrazol-5-yl)biphényl-4-ylméthyl]amine,
N-(4-hydroxybut-2-yl)-N-pentanoyl-N-[2'-(1H-tétrazol-5-yl)biphényl-4-ylméthyl]amine,
N-(2-hydroxy-1-phényléthyl)-N-pentanoyl-N-[2'-(1H-tétrazol-5-yl)biphényl-4-ylméthyl]amine,
N-[3-(4-hydroxybenzylcarbonylamino)prop-1-yl]-N-pentanoyl-N-[2'-(1H-tétrazol-5-yl)biphényl-4-ylméthyl]amine,
N-(3-éthoxycarbonylcyclohexyl)-N-pentanoyl-N-[2'-(1H-tétrazol-5-yl)biphényl-4-ylméthyl]amine,
N-(3-carboxycyclohexyl)-N-pentanoyl-N-[2'-(1H-tétrazol-5-yl)biphényl-4-ylméthyl]amine,
*cis*-N-(4-carboxycyclohexyl)-N-pentanoyl-N-[2'-(1H-tétrazol-5-yl)biphényl-4-ylméthyl]amine,
*cis*-N-(2-éthoxycarbonylcyclohexyl)-N-pentanoyl-N-[2'-(1H-tétrazol-5-yl)biphényl-4-ylméthyl]amine,
*cis*-N-(2-carboxycyclohexyl)-N-pentanoyl-N-[2'-(1H-tétrazol-5-yl)biphényl-4-ylméthyl]amine,
N-[2-[2-(4-hydroxyphényl)éthylaminocarbonyl]-2,2-tétraméthylène-éthyl)-N-pentanoyl-N-[2'-(1H-tétrazol-5-yl)biphényl-4-ylméthyl]amine,
(S)-N-(1-[2-(4-hydroxyphényl)éthylaminocarbonyl]-2-méthylprop-1-yl)]-N-pentanoyl-N-[2'-(1H-tétrazol-5-yl)biphényl-4-ylméthyl]amine,
(S)-N-(1-carboxy-2,2-diméthylprop-1-yl)-N-pentanoyl-N-[2'-(1H-tétrazol-5-yl)biphényl-4-ylméthyl]amine,
(S)-N-(1-méthoxycarbonyl-2-méthylprop-1-yl)-N-pentanoyl-N-[2'-(1H-tétrazol-5-yl)biphényl-4-ylméthyl]amine,
N-(4-phénoxybut-1-yl)-N-pentanoyl-N-[2'-(1H-tétrazol-5-yl)biphényl-4-ylméthyl]amine,
N-(2-hydroxy-1-phényl-2-oxoéthyl)-N-pentanoyl-N-[2'-(1H-tétrazol-5-yl)biphényl-4-ylméthyl]amine,
(S)-N-(1-benzyloxycarbonyl-2-méthylprop-1-yl)-N-pentanoyl-N-[2'-(1H-tétrazol-5-yl)biphényl-4-ylméthyl]amine,
N-butanoyl-N-(1-carboxy-1-méthyléthyl)-N-[2'-(1H-tétrazol-5-yl)biphényl-4-ylméthyl]amine,
N-(4-hydroxybut-1-yl)-N-pentanoyl-N-[2'-(1H-tétrazol-5-yl)biphényl-4-ylméthyl]amine,
(S)-N-(1-benzyloxycarbonyl-2-méthylprop-1-yl)-N-[3-bromo-2'-(1H-tétrazol-5-yl)biphényl-4-ylméthyl]-N-pentanoylamine,
(S)-N-[3-bromo-2'-(1H-tétrazol-5-yl)biphényl-4-ylméthyl]-N-(1-carboxy-2-méthylprop-1-yl)-N-pentanoylamine,
N-(2-acétylaminoéthyl)-N-pentanoyl-N-[2'-(1H-tétrazol-5-yl)biphényl-4-ylméthyl]amine,
N-[2-(n-butoxycarbonyl)-2,2-tétraméthylène-éthyl]-N-pentanoyl-N-[2'-(1H-tétrazol-5-yl)biphényl-4-ylméthyl]amine,
N-(2-benzylaminocarbonyl-2,2-tétraméthylène-éthyl)-N-pentanoyl-N-[2'-(1H-tétrazol-5-yl)biphényl-4-ylméthyl]amine,
(S)-N-butyloxycarbonyl-N-(1-carboxy-2-méthylprop-1-yl)-N-[2'-(1H-tétrazol-5-yl)biphényl-4-ylméthyl]amine,
(S)-N-(1-carboxy-2-méthylprop-1-yl)-N-méthoxycarbonyl-N-[2'-(1H-tétrazol-5-yl)biphényl-4-ylméthyl]amine,
N-(2-diéthylaminocarbonyl-2,2-tétraméthylène-éthyl)-N-pentanoyl-N-[2'-(1H-tétrazol-5-yl)biphényl-4-ylméthyl]amine,
N-(2-méthyl-2-morpholine-4-ylcarbonylpropyl)-N-pentanoyl-N-[2'-(1H-tétrazol-5-yl)biphényl-4-ylméthyl]amine,
N-(1-carboxycyclopentyl)-N-pentanoyl-N-[2'-(1H-tétrazol-5-yl)biphényl-4-ylméthyl]amine,
N-(1-carboxy-1-éthylprop-1-yl)-N-pentanoyl-N-[2'-(1H-tétrazol-5-yl-)biphényl-4-ylméthyl]amine,
(S)-N-(5-amino-1-carboxypent-1-yl)-N-pentanoyl-N-[2'-(1H-tétrazol-5-yl)biphényl-4-ylméthyl]amine,
N-butanesulfonyl-N-(2-éthoxycarbonyl-2,2-pentaméthylène-éthyl)-N-[2'-(1H-tétrazol-5-yl)biphényl-4-ylméthyl]amine,
N-butanesulfonyl-N-(2-carboxy-2,2-pentaméthylène-éthyl)-N-[2'-(1H-tétrazol-5-yl)biphényl-4-ylméthyl]amine,
N-butanesulfonyl-N-(2-éthoxycarbonyl-2-méthylprop-1-yl)-N-[2'-(1H-tétrazol-5-yl)biphényl-4-ylméthyl]amine,
N-butanesulfonyl-N-(2-carboxy-2-méthylprop-1-yl)-N-[2'-(1H-tétrazol-5-yl)biphényl-4-ylméthyl]amine,
(S)-N-butanesulfonyl-N-(1-tert-butoxycarbonyléthyl)-N-[2'-(1H-tétrazol-5-yl)biphényl-4-ylméthyl]amine,
(S)-N-butanesulfonyl-N-(1-carboxyéthyl)-N-[2'-(1H-tétrazol-5-yl)biphényl-4-ylméthyl]amine,
(S)-N-butanesulfonyl-N-(1-carboxy-2-méthylprop-1-yl)-N-[2'-(1H-tétrazol-5-yl)biphényl-4-ylméthyl]amine,
(S)-N-(2-méthyl-1-méthylaminocarbonylprop-1-yl)-N-pentanoyl-N-[2'-(1H-tétrazol-5-yl)biphényl-4-ylméthyl]amine,
(S)-N-(1-diméthylaminocarbonyl-2-méthylprop-1-yl)-N-pentanoyl-N-[2'-(1H-tétrazol-5-yl)biphényl-4-ylméthyl]amine,
(S)-N-(2-méthyl-1-morpholine-4-ylcarbonylprop-1-yl)-N-pentanoyl-N-[2'-(1H-tétrazol-5-yl)biphényl-4-ylméthyl]amine,
(S)-N-(2'-carboxybiphényl-4-ylméthyl)-N-(1-carboxy-2-méthylprop-1-yl)-N-pentanoylamine,
(S)-N-(1,2-dicarboxyéthyl)-N-pentanoyl-N-[2'-(1H-tétrazol-5-yl)biphényl-4-ylméthyl]amine,
(S)-N-(1-carboxy-3-phénylprop-1-yl)-N-pentanoyl-N-[2'-(1H-tétrazol-5-yl)biphényl-4-ylméthyl]amine,
(S)-N-(2-cyclohexyl-1-hydroxyméthyl-éthyl)-N-pentanoyl-N-[2'-(1H-tétrazol-5-yl)biphényl-4-ylméthyl]amine,
(R)-N-(1-méthoxycarbonyl-2-méthylprop-1-yl)-N-pentanoyl-N-[2'-(1H-tetrazol-5-yl)biphényl-4-ylméthyl]amine,
(S)-N-(2-hydroxy-1-méthoxycarbonyl-éthyl)-N-pentanoyl-N-[2'-(1H-tétrazol-5-yl)biphényl-4-ylméthyl]amine,
N-pentanoyl-N-(1H-tétrazol-5-ylméthyl)-N-[2'-(1H-tétrazol-5-yl)biphényl-4-ylméthyl]amine,
N-pentanoyl-N-pyrid-3-ylméthyl)-N-[2'-(1H-tétrazol-5-yl)biphényl-4-ylméthyl]amine,
(S)-N-(1-carboxy-4-guanidinobut-1-yl)-N-pentanoyl-N-[2'-(1H-tétrazol-5-yl)biphényl-4-ylméthyl]amine,
N-(2-hydroxy-1-méthoxycarbonylprop-1-yl)-N-pentanoyl-N-[2'-(1H-tétrazol-5-yl)biphényl-4-ylméthyl]amine,
N-(1-benzyloxycarbonyl-1-méthyléthyl)-N-butanoyl-N-[2'-(1H-tétrazol-5-yl)biphényl-4-ylméthyl]amine,
(S)-N-(1-carboxy-3-méthylbut-1-yl)-N-pentanoyl-N-[2'-(1H-tétrazol-5-yl)biphényl-4-ylméthyl]amine,
N-(1-carboxy-2-hydroxyéthyl)-N-pentanoyl-N-[2'-(1H-tétrazol-5-yl)biphényl-4-ylméthyl]amine,
(S)-N-(1-carboxy-2-hydroxyéthyl)-N-pentanoyl-N-[2'-(1H-tétrazol-5-yl)biphényl-4-ylméthyl]amine,
(S)-N-(2-méthyl-1-(2-phényléthylaminocarbonyl)prop-1-yl]-N-pentanoyl-N-[2'-(1H-tétrasol-5-yl)biphényl-4-ylméthyl]amine,
(S)-N-(2-benzyloxy-1-hydroxyméthyléthyl)-N-pentanoyl-N-[2'-(1H-tétrazol-5-yl)biphényl-4-ylméthyl]amine,
(S)-N-(1-carboxy-2-méthylprop-1-yl)-N-pentanoyl-N-[2'-(1H-tétrazol-5-yl)biphényl-3-ylméthyl]amine,
(S)-N-(1-carboxy-2-méthylprop-1-yl)-N-pentanoyl-N-[3'-(1H-tétrazol-5-yl)biphényl-4-ylméthyl]amine,
(S)-N-[2-méthyl-1-(1,2,3,4-tétrahydroquinol-1-ylcarbonyl)prop-1-yl]-N-pentanoyl-N-[2'-(1H-tétrazol-5-yl)biphényl-4-ylméthyl]amine,
(S)-N-(2-méthyl-1-pipéridine-1-ylcarbonylprop-1-yl)-N-pentanoyl-N-[2'-(1H-tétrazol-5-yl)biphényl-4-ylméthyl]amine,
(S)-N-[2-méthyl-1-(1,2,3,4-tétrahydroisoquinol-2-ylcarbonyl)prop-1-yl]-N-pentanoyl-N-[2'-(1H-tétrazol-5-yl) biphényl-4-ylméthyl]amine,
N-(2-hydroxyméthyl-2-méthylprop-1-yl)-N-pentanoyl-N-[2'-(1H-tétrazol-5-yl)biphényl-4-ylméthyl]amine,
N-éthoxycarbonyl-N-(2-éthoxycarbonyl-2-méthylprop-1-yl)-N-[2'-(1H-tétrazol-5-yl)biphényl-4-ylméthyl]amine et
N-(2-carboxy-2-méthylprop-1-yl)-N-éthoxycarbonyl-N-[2'-(1H-tétrazol-5-yl)biphényl-4-ylméthyl]amine,
chacun sous forme libre ou sous forme de sel.

**34.** Procédé pour la préparation d'une composition pharmaceutique contenant, en tant que substance active, un composé selon l'une des revendications 1 ou 3 à 33, sous forme libre ou sous forme d'un sel pharmaceutiquement utilisable, éventuellement en plus d'adjuvants pharmaceutiques usuels, caractérisé en ce que l'on met la substance active, éventuellement avec addition d'adjuvants pharmaceutiques usuels, sous forme d'une composition pharmaceutique.

**35.** Utilisation d'un composé selon l'une des revendications 1 ou 3-33, sous forme libre ou sous forme d'un sel pharmaceutiquement utilisable, pour la préparation d'un agent antihypertenseur.

**36.** Utilisation d'un composé selon l'une des revendications 1 ou 3-33, sous forme libre ou sous forme d'un sel pharmaceutiquement utilisable, pour la préparation d'une composition pharmaceutique destinée au traitement thérapeutique ou prophylactique de l'insuffisance cardiaque.

**37.** Utilisation d'un composé selon l'une des revendications 1 ou 3-33, sous forme libre ou sous forme d'un sel pharmaceutiquement utilisable, pour la préparation d'une composition pharmaceutique destinée au traitement thérapeutique ou prophylactique de maladies qui sont provoquées par l'activité de l'angiotensine II.